# EUROPEAN PATENT SPECIFICATION

(11) **EP 2 049 502 B1**
(45) Date of publication and mention of the grant of the patent: **04.01.2012**
(21) Application number: 07819913.0
(22) Date of filing: 25.07.2007
(51) Int. Cl.: C07D 239/74, C07D 239/84, C07D 401/04, C07D 405/04, C07D 401/14, C07D 409/04, C07D 403/04, C07D 409/14, A61K 31/517, A61P 29/02, A61P 35/00, A61P 37/08

(54) **2,4-SUBSTITUTED QUINAZOLINES AS LIPID KINASE INHIBITORS**
2,4-SUBSTITUIERTE CHINAZOLINE ALS LIPIDKINASEHEMMER
QUINAZOLINES 2,4-SUBSTITUÉES UTILISÉES COMME INHIBITEURS DE LIPIDE KINASE

(30) Priority: 28.07.2006 EP 06118049
(43) Date of publication of application: 22.04.2009
(73) Proprietor: Novartis AG, 4056 Basel (CH)
(72) Inventor: STAUFFER, Frédéric, 68220 Hesingue (FR); FURET, Pascal, 68800 Thann (FR)
(74) Representative: Dyer, James
(86) International application number: PCT/EP2007/057669
(87) International publication number: WO 2008/012326

(56) References cited:
- WO-A-2004/072033
- WO-A-2004/099159
- WO-A-2005/012241
- WO-A-2005/016348
- US-A1- 2004 014 774

## Description

The invention relates to quinazolines substituted at least in the 4,6-position, the use of such a compound in the preparation of a pharmaceutical preparation or their use for the prophylactic and/or therapeutic treatment of one or more diseases selected from the group consisting of proliferative, inflammatory diseases, allergic diseases, obstructive airways diseases, and disorders commonly occurring in connection with transplantation, especially one or more diseases which respond to an inhibition of kinases of the P13-kinasia-related protein kinase family, especially lipid kinases and/or PI3 kinase (PI3K) and/or mTOR and/or DNA protein kinase and/or ATM and/or ATR and/or hSMG-1 activity, a compound of this type for use in the prophylactic and/or therapeutic treatment of one or more of the diseases just mentioned, a method for the preparation of a pharmaceutical formulation for use in one or more of the mentioned diseases, comprising mixing one of these compounds with at least one phamiaceutically acceptable carrier, and a method of treatment, comprising administering to a warm-blooded animal, including a human, especially in need of such treatment, a compound according to the invention, especially in an amount that is effective against a disease mentioned above; as well as to a process or method for the manufacture of a quinazoline substituted at least in the 4,6-position according to the invention; and to other aspects disclosed herein.

WO-A-2005 016348 describes quinazolin-4-one compounds.

WO04/072033 and WO06/044509 respectively describe TGFβ inhibitor quinazoline compounds having a substituted phenyl group at the 4-postion and a pyrazolyl group substituted by pyridine at the 6-position.

EP1382603 (WO02/088107) describes STAT6 inhibitor quinazoline compounds having a substituted phenyl or thiophene group at the 4-position and a substituted or unsubstituted pyrazolyl group at the 6-position.

WO97/42187 describes quinazoline compounds having an oxoindolyl group at the 4-position and a morpholine group at the 6-position.

In a first aspect, the invention provides a compound of the formula I, 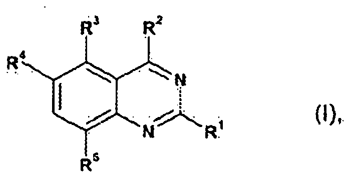 wherein
R¹ is hydrogen; or amino that is unsubstituted or monosubstituted with alkyl or cycloalkyl;
R² is an unsubstituted or substituted aryl or unsubstituted or substituted heteroaryl;
R⁴ is unsubstituted or substituted aryl or unsubstituted or substituted heteroaryl; and
R⁵ is hydrogen, methyl or methyl substituted with halogen;
with the proviso that if R⁴ is unsubstituted or substituted pyrazolyl then R¹ is amino that is unsubstituted or monosubstituted with alkyl (especially C₁-C₇-, more especially C₁-C₄-alkyl) or cycloalkyl (especially C₃-C₈-, more especially C₃-C₅-cycloalkyl) and R², R³ and R⁵ are as defined above;
and with the proviso that if R² is unsubstituted or substituted oxoindolyl, then R¹ is amino that is unsubstituted or monosubstituted with alkyl (especially C₁-C₇-, more especially C₁-C₄-alkyl) or cycloalkyl (especially C₃-C₈-, more especially C₃-C₅-cycloalkyl) and R³, R⁴ and R⁵ are as defined above;

or a tautomer thereof or a N-oxide thereof, or a pharmaceutically acceptable salt, or a hydrate or solvate thereof.

The general terms used hereinbefore and hereinafter preferably have within the context of this disclosure the following meanings, unless otherwise indicated, where more general terms where ever used may, independently of each other, be replaced by more specific definitions or remain, thus defining more preferred embodiments of the invention:

The prefix "lower" or "C₁-C₇-" denotes a radical having up to and including a maximum of 7, especially up to and including a maximum of 4 carbon atoms, the radicals in question being either linear or branched with single or multiple branching.

Alkyl (also in alkoxy or the like) preferably has up to 12 carbon atoms and is more preferably lower alkyl, especially C₁-C₄-alkyl.

Lower alkyl is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or preferably methyl.

Cycloalkyl is preferably cycloalkyl with from and including 3 up to and including 10 carbon atoms in the ring; cycloalkyl is more preferably C₃-C₈-cycloalkyl, still more preferably C₃-C₅-cycloalkyl, especially cyclopropyl, cyclobutyl or cyclopentyl.

Alkyl (e.g. methyl) which is substituted by halogen is preferably fluoroalkyl wherein 1 or more, preferably all (then the alkyl is a perfluoroalkyl) hydrogen atoms are substituted by fluoro, such as difluoromethyl or trifluoromethyl.

Halogen, halogeno (or halo) is especially fluoro, chloro, bromo, or iodo, especially fluoro, chloro or bromo.

Aryl preferably has 6 to 18 carbon atoms and is a mono-, di- or polycyclic (preferably up to tricyclic, more preferably up to bicyclic) unsaturated carbocyclic moiety with conjugated double bonds in the ring, especially phenyl, naphthyl, biphenylenyl, indacenyl, acenaphthylenyl, fluorenyl, phenalenyl, phenanthrenyl or anthracenyl. Naphthyl and preferably phenyl are especially preferred. Aryl is unsubstituted or substituted by one or more, e.g. one to three, substitutents preferably independently selected from the group consisting of C₁-C₇-alkyl, such as methyl, ethyl, n-propyl, isopropyl, n-butyl, isobutyl, sec-butyl or *tert-butyl*; C₂-C₇-alkenyl; C₂-C₇-alkinyl; C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino; by halo, by C₁-C₇-alkoxy, such as methoxy, and/or by halo-C₁-C₇-alkyl, such as trifluoromethyl, for example pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl, or N-mono- or N,N-di-(C₁-C₇-alkyl)-amino-substituted or unsubstituted pyrrolidino-C₁-C₇-alkyl; (pyrrolidinyl (especially pyrrolidino), piperidinyl (especially piperidino), piperazinyl (especially piperazino), pyridinyl, pyrimidinyl, pyrazinyl or pyridazinylyoxy-C₁-C₇-alkyl wherein pyrrolidinyl, piperidinyl, piperazinyl, pyridinyl,
pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), pyridinyl, pyrimidinyl, pyrazinyl or pyridazinyl)-carbonyl-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇-alkyl, such as trifluoromethyl; hydroxy-C₁-C₇-alkyl; C₁-C₇-alkoxy-C₁-C₇-alkyl, such as 3-methoxypropyl or 2-methoxyethyl; C₁-C₇-alkoxy-C₁-C₇-alkoxy-C₁-C₇-alkyl; phenyloxy- or naphthyloxy-C₁-C₇-alkyl; phenyl-C₁-C₇-alkoxy- or naphthyl-C₁-C₇-alkoxy-C₁-C₇-alkyl; amino-C₁-C₇-alkyl, such as aminomethyl; N-mono- or N,N-di-(C₁-C₇-alkyl and/or mono-C₁-C₇-alkoxy-C₁-C₇-alkyl and/or (mono- or di-C₁-C₇-alkyl)-amino)-C₁-C₇-alkyl)-amino-C₁-C₇-alkyl; C₁-C₇-alkoxy-C₁-C₇-alkylamino-C₁-C₇-alkyl; mono- or di-[C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-ralkyl, such as trifluoromethyl; (pyrrolidinyl (especially pyrrolidino), piperidinyl (especially piperidino), piperazinyl (especially piperazino), morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl, for example pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl, or N-mono- or N,N-di-(C₁-C₇-alkyl)-amino-substituted or unsubstituted pyrrolidino-C₁-C₇-alkyl; (pyrrolidinyl (especially pyrrolidino), piperidinyl (especially piperidino), piperazinyl (especially piperazino), pyridinyl, pyrimidinyl, pyrazinyl or pyridazinylyoxy-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-Cralkoxy, such as methoxy, and/or by halo-C₁-C₇-alkyl, such as trifluoromethyl; and/or (pyrrolidinyl (especially pyrrolidino), piperidinyl (especially piperidino), piperazinyl (especially piperazino), pyridinyl, pyrimidinyl, pyrazinyl or pyridazinyl)-carbonyl-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; especially naphthyl- or phenyl-C₁-C₇-alkyl]-amino-C₁-C₇-alkyl; C₁-C₇-alkanoylamino-C₁-C₇-alkyl; carboxy-C₁-C₇-alkyl; benzoyl- or naphthoylamino-C₁-C₇-alkyl; C₁-C₇-alkylsulfonylamino-C₁-C₇-alkyl; phenyl- or naphthylsulfonylamino-C₁-C₇-alkyl wherein phenyl or naphthyl is unsubstituted or substituted by one or more, especially one to three, C₁-C₇-alkyl moieties; phenyl- or naphthyl-C₁-C₇-alkylsulfonylaminO-C₁-C₇-alkyl;, halo, especially fluoro (preferred), chloro (preferred) or bromo; hydroxy; C₁-C₇-alkoxy; C₆-C₁₈-aryl-C₁-C₇-alkoxy in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by C₁-C₇-alkoxy, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; such as phenyl-C₁-C₇-alkoxy wherein phenyl is unsubstituted or substituted by C₁-C₇-alkoxy and/or halo; halo-C₁-C₇-alkoxy, such as trifluoromethoxy; hydroxy-C₁-C₇-alkoxy; C₁-C₇-alkoxy-C₁-C₇-alkoxy, such as 2-(methoxy)-ethoxy; amino-C₁-C₇-alkoxy, N-C₁-C₇-alkanoylamino-C₁-C₇-alkoxy; N-unsubstituted-, N-mono- or N,N-di-(C₁-C₇-alkyl)carbamoyl-C₁-C₇-alkoxy; phenyl- or naphthyloxy; phenyl- or naphthyl-C₁-C₇-alkyloxy; (pyrrolidinyl (especially pyrrolidino), piperidinyl (especially piperidino), piperazinyl (especially piperazino), pyridinyl, pyrimidinyl, pyrazinyl or pyridazinyl)-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), pyridinyl, pyrimidinyl, pyrazinyl or pyridazinylyoxy-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; C₁-C₇-alkanoyloxy; benzoyl- or naphthoyloxy; C₁-C₇-alkylthio; halo-C₁-C₇-alkthio, such as trifluoromethylthio; C₁-C₇-alkoxy-C₁-C₇-alkylthio; phenyl- or naphthylthio; phenyl- or naphthyl-C₁-C₇-alkylthio; C₁-C₇-alkanoylthio; benzoyl- or naphthaylthio; nitro; amino; mono- or di-(C₁-C₇-alkyl)-amino; mono- or di-(naphthyl- or phenyl-C₁-C₇-alkyl)-amino; C₁-C₇-alkanoylamino; benzoyl- or naphthoylamino; C₁-C₇-alkylsulfonylamino; phenyl- or naphthylsulfonylamino wherein phenyl or naphthyl is unsubstituted or substituted by one or more, especially one to three, C₁-C₇-alkyl moieties; phenyl- or naphthyl-C₁-C₇-alkylsulfonylamino; C₁-C₇-alkanoyl; C₁-C₇-alkoxy-C₁-C₇-alkanoyl; carboxyl (-COOH); C₁-C₇-alkoxy-carbonyl; phenoxy- or naphthoxycarbonyl; phenyl- or naphthyl-C₁-C₇-alkoxycarbonyl; C₁-C₁₀- especially C₁-C₄-alkylendioxy, such as methylendioxy or 1,2-ethylendioxy; carbamoyl; N-mono- or N,N-di-(C₁-C₇-alkyl, naphthyl-C₁-C₇-alkyl, phenyl-C₁-C₇-alkyl, pyrrolidinyl(especially pyrrolidino)-C₁-C₇-alkyl, piperidinyl (especially piperidinoyC₁-C₇-alkyl, piperazinyl- or N-C₁-C₇-alkyl)piperazinyl (especially piperazino or 4-C₁-C₇-alkylpiperazino)-C₁-C₇-alkyl, mono-C₁-C₇-alkoxy-C₁-C₇-alkyl and/or (N'-mono- or N,N'-di-(C₁-C₇-alkyl)-amino-C₁-C₇-alkylyamino-carbonyl, such as N-mono- or N,N-di-(C₁-C₇-alkyl)-aminocarbonyl; N-C₁-C₇-alkoxy-C₁-C₇-alkylcarbamoyl; pyrro-lidin-1-carbonyl; amino-N-pyrrolidin-1-carbonyl; N-mono- or N,N-di(C₁-C₇-alkyl)amino-pyrrolidin-1-carbonyl; piperidin-1-carbonylmorpholin-4-carbonyl; thiomorpholin-4-carbonyl; S-oxo-thiomorpholin-4-carbonyl; S,S-dioxothiomorpholin-4-carbonyl; piperazin-1-carbonyl; N-C₁-C₇-alkyl-piperazin-1-carbonyl; N-C₁-C₇-alkoxycarbonyl-piperazin-1-carbonyl; N-mono- or N,N-di-(C₁-C₇-alkyl)-amino-substituted or unsubstituted pyrrolidinyl-C₁-C₇-alkyl; cyano; C₁-C₇-alkenylene or -alkinylene; C₁-C₇-alkylsulfonyl; phenyl- or naphthylsulfonyl wherein phenyl or naphthyl is unsubstituted or substituted by one or more, especially one to three, C₁-C₇-alkyl moieties; phenyl- or naphthyl-C₁-C₇-alkylsulfonyl; sulfamoyl; N-mono or N,N-di-(C₁-C₇-alkyl, phenyl-, naphthyl-, phenyl-C₁-C₇-alkyl-, pyrrolidinyl(especially pyrrolidino)-C₁-C₇-alkyl, piperidinyl(especially piperidino)-C₁-C₇-alkyl, piperazinyl(especially piperazino)-C₁-C₇-alkyl, N-C₁-C₇-alkylpiperazinyl(especially 4-C₁-C₇-alkylpiperazino)-C₁-C₇-alkyl and/or naphthyl-C₁-C₇-alkyl)-aminosulfonyl, pyrazolyl, pyrazolidinyl, pyrrolyl, pyridyl that is unsubstituted or substituted by C₁-C₇-alkoxy, such as methoxy, and/or by halo-C₁-C₇-alkyl, such as trifluoromethyl, pyrrolidinyl, piperidinyl, morpholinyl, thiomorpholinyl, S-oxo-thiomorpholinyl, S,S-dioxothiomorpholinyl, piperazinyl, N-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxycarbonyl)-piperazinyl, 4-(phenyl-C₁-C₇-alkoxycarbonyl)-piperazinyl and 4-(naphthyl-C₁-C₇-alkoxycarbonyl)-piperazinyl. Especially preferably aryl is phenyl or naphthyl, each of which is unsubstituted or substituted by one or more, e.g. up to three, substituents independently selected from the group consisting of 2-amino-pyrimidin-5-yl-C₁-C₇-alkyl, halo, hydroxy, C₁-C₇-alkoxy, C₁-C₇-alkoxy-C₁-C₇-alkoxy, 4-C₁-C₇-alkyl-piperarzin-1-carbonyl-C₁-C₇-alkoxy, 4-pyrrolidino-piperidin-1-carbonyl-C₁-C₇-alkoxy, 4-pyrrolidino-piperidin-1-yl-C₁-C₇-alkoxy, 4-C₁-C₇-alkyl-piperazino-C₁-C₇-alkoxy, pyridin (e.g.-2)-yloxy-C₁-C₇-alkoxy, pyrimidin(e.g. -4)-yloxy-C₁-C₇-alkoxy, amino, N-mono- or N,N-di-(C₁-C₇-alkyl, phenyl-C₁-C₇-alkyl and/or naphthyl-C₁-C₇-alkyl)-amino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkyl)-carbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, N,N-di(C₁-C₇-alkyl)amino-pyrrolidin-1-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkyl)-sulfamoyl, pyrazolyl, especially pyrazolo, pyrazolidinyl, especially pyrazolidino, pyrrolyl, especially pyrrolin-1-yl, (unsubstituted or C₁-C₇-alkoxy- and/or halo-C₁-C₇-alkoxy-substituted pyridin(e.g. -3))-yl, pyrrolidinyl, especially pyrrolidino, piperidinyl, especially piperidino, piperazinyl, especially piperazino, 4-C₁-C₇-alkyl-piperazinyl, especially 4-C₁-C₇-alkyl-piperazino, 4-(phenyl-C₁-C₇-alkyl)-piperazinyl, especially 4-(phenyl-C₁-C₇-alkyl)-piperazino, 4-(naphthyl-C₁-C₇-alkyl)-piperazinyl, especially 4-(naphthyl-C₁-C₇-alkyl)-piperazino, 4-(C₁-C₇-alkoxycarbonyl)-piperazinyl, especially 4-(C₁-C₇-alkoxycarbonyl)-piperazino, 4-(phenyl-C₁-C₇-alkoxycarbonyl)-piperazinyl, especially 4-(phenyl-C₁-C₇-alkoxy-carbonylypiperazin, 4-(naphthyl-C₁-C₇-alkoxycarbonyl)-piperazinyl, especially 4-(naphthyl-C₁-C₇-alkoxycarbonyl)-piperazino, morpholinyl, especially morpholino, thiomorpholinyl, especially thiomorpholino, S-oxothiomorpholinyl, especially S-oxothiomorpholino, and S,S-dioxothiomorpholinyl, especially S,S-dioxothiomorpholino.

Heteroaryl is an unsaturated mono-, di- or polycyclic (preferably up to tricyclic, more preferably up to bicyclic) ring, preferably with 3 to 20, more preferably 5 to 16 ring atoms, including at least one, preferably up to 4, e.g. up to three ring heteroatoms selected from O, S, N and NH, which carries the maximum possible number of conjugated double bonds in the ring (that is, is unsaturated) and is unsubstituted or substituted by one or more, preferably up to three, substituents independently selected from the substituents mentioned above for aryl. Examples for preferred heteroaryl moieties are imidazolyl, thiophenyl, pyrrolyl, imidazolyl, pyrazolyl, pyridyl, pyrazinyl, pyrimidinyl, pyridazinyl, furyl, 2H- or 4H-pyranyl, oxazolyl, thiazolyl, 5H-indazolyl, indolyl, isoindolyl, quinolyl, isoquinolinyl, phthalazinyl, 1,8-naphthyridinyl, quinoxalinyl, quinazolinyl, cinnolinyl, indolizinyl, 4H-quinolizinyl, pteridinyl, purinyl, carbazolyl, beta-carbolinyl, acridinyl, phenanthridinyl, phenyzinyl, 1,7-phenanthrolinyl, perimidinyl, benzofuranyl, isobenzofuranyl, 2H-chromenyl, 4aH-isochromenyl, thianthrenyl, xanthenyl, phenoxathiinyl, phenoxazinyl or phenothiazinyl, each of which is unsubstituted or substituted as mentioned above; more preferably, pyrazolyl (especially as R⁴) and indolyl are excluded from the term "heteroaryl". Most preferably heteroaryl is pyrrolyl, thiophenyl, pyrazolyl (but only as R², not as R⁴), triazolyl, especially 1,2,4-triazolyl, pyridyl, quinolyl or quinoxalinyl, each of which is unsubstituted or substituted by one or more, especially up to three, substituents selected from the group consisting of halo, hydroxy, C₁-C₇-alkoxy, C₁-C₇-alkoxy-C₁-C₇-alkoxy, amino, N-mono- or N,N-di-(C₁-C₇-alkyl, phenyl-C₁-C₇-alkyl and/or naphthyl-C₁-C₇-alkyl)-amino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl and/or phenyl-C₁-C₇-alkyl)-carbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl and/or phenyl-C₁-C₇-alkyl)-sulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxy-carbonylypiperazinyl, 4-(phenyl-C₁-C₇-alkoxycarbonyl)-piperazinyl, 4-(naphthyl-C₁-C₇-alkoxycarbonyl)-piperazinyl, morpholinyl, thiomorpholinyl, S-oxothiomorpholinyl and S,S-dioxothiomorpholinyl.

An N-oxide derivative or pharmaceutically acceptable salt of each of the compounds of the formula 1 is also within the scope of this invention. For example, a nitrogen ring atom of the quinazole core or a nitrogen-containing heterocyclic (e.g. heteroaryl) substituent can form an N-oxide in the presence of a suitable oxidizing agent, e.g. a peroxide, such as m-chloro-perbenzoic acid or hydrogen peroxide.

Wherever a compound or compounds of the formula I are mentioned, this is further also intended to include N-oxides of such compounds, as well as tautomers of such compounds or N-oxides, also where not stated explicitly. Tautomerism may, for example, be present of the keto (or oxo)/enol type, the imine/amine (e.g. imine/enamine) type, the lactim/lactame type or the like.

The term "an N-oxide thereof, a tautomer thereof and/or a pharmaceutically acceptable salt thereof' especially means that a compound of the formula I may be present as such or in mixture with its N-oxide, as tautomer or in e.g. equilibrium reaction caused) mixture with its tautomer, or as a salt of the compound of the formula I and/or any of these embodiments. Compounds of the formula I can also be modified by appending appropriate functionalities to enhance selective biological properties. Modifications of this kind are known in the art and include those that increase penetration into a given biological system (e.g. blood, lymphatic system, central nervous system, testis), increase bioavailability, increase solubility to allow parenteral administration (e.g. injection, infusion), alter metabolism and/or alter the rate of secretion. Examples of this type of modifications include but are not limited to esterification, e.g. with polyethylene glycols, derivatisation with pivaloyloxy or fatty acid substituents, conversion to carbamates, hydroxylation of aromatic rings and heteroatom substitution in aromatic rings. Whereever compounds of the formula I, N-oxides and/or tautomers thereof are mentioned, this comprises such modified formulae, while preferably the molecules of the formula I, their N-oxides and/or their tautomers are meant.

In view of the close relationship between the novel compounds of the formula I in free form and those in the form of their salts, including those salts that can be used as intermediates, for example in the purification or identification of the novel compounds, any reference to the compounds or a compound of the formula I hereinbefore and hereinafter is to be understood as referring also to one or more salts, as appropriate and expedient, as well as to one or more solvates, e.g. hydrates.

Salts are formed, for example, as acid addition salts, preferably with organic or inorganic acids, from compounds of formula I with a basic nitrogen atom, especially the pharmaceutically acceptable salts. Suitable inorganic acids are, for example; halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, malonic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 4-toluenesulfonic acid, 2-naphthalenesulfonic acid, 1,5-naphthalene-disulfonic acid, 2- or 3-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In a preferred embodiment, the invention relates to a compound of the formula I wherein
R¹ is hydrogen; or amino that is unsubstituted or monosubstituted with C₁-C₇-alkyl or C₃-C₈ (preferably C₃-C₅)-cycloalkyl;
R² is unsubstituted or substituted aryl wherein aryl is selected from the group consisting of phenyl, naphthyl, biphenylenyl, indacenyl, acenaphthylenyl, fluorenyl, phenalenyl, phenanthrenyl and anthracenyl, each of which is unsubstituted or substituted by one or more, preferably up to three, substituents independently selected from the group consisting of C₁-C₇-alkyl; C₂-C₇-alkenyl; C₂-C₇-alkinyl; C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and is unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl, for example pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl, or N-mono- or N,N-di-(C₁-C₇-alkyl)-amino-substituted or unsubstituted pyrrolidino-C₁-C₇-alkyl; (pyrrolidinyl (especially pyrrolidino), piperidinyl (especially piperidino), piperazinyl (especially piperazino), pyridinyl, pyrimidinyl, pyrazinyl or pyridazinyl)-oxy-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as
methoxy, and/or by halo-C₁-C₇-alkyl, such as trifluoromethyl; (pyrrolidinyl (especially pyrrolidino), piperidinyl (especially piperidino), piperazinyl (especially piperazino), pyridinyl, pyrimidinyl, pyrazinyl or pyridazinyl)-carbonyl-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇-alkyl; hydroxy-C₁-C₇-alkyl; C₁-C₇-alkoxy-C₁-C₇-alkyl; C₁-C₇-alkoxy-C₁-C₇-alkoxy-C₁-C₇-alkyl; phenyloxy- or naphthyloxy-C₁-C₇-alkyl; phenyl-C₁-C₇-alkoxy- or naphthyl-C₁-C₇-alkoxy-C₁-C₇-alkyl; amino-C₁-C₇-alkyl; N-mono- or N,N-di-(C₁-C₇-alkyl and/or mono-C₁-C₇-alkoxy-C₁-C₇alkyl and/or (mono- or di-(C₁-C₇-alkyl)-amino-C₁-C₇-alkylyamino-C₁-C₇-alkyl; C₁-C₇-alkoxy-C₁-C₇-alkylamino-C₁-C₇-alkyl; mono- or di-[C₆-C₁₈-aryl-C₁-C₇-alkyl in which aryl is unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; (pyrrolidinyl, piperidinyl, piperazinyl, morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; (pyrrolidinyl, piperidinyl, piperazinyl, pyridinyl, pyrimidinyl, pyrazinyl or pyridazinyl)-oxy-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; and/or (pyrrolidinyl, piperidinyl, piperazinyl, pyridinyl, pyrimidinyl, pyrazinyl or pyridazinyl)-carbonyl-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; especially naphthyl- and/or phenyl-C₁-C₇-alkyl]-amino-C₁-C₇-alkyl; C₁-C₇-alkanoylamino-C₁-C₇-alkyl; carboxy-C₁-C₇-alkyl; benzoyl- or naphthoylamino-C₁-C₇-alkyl; C₁-C₇-alkylsulfonylamino-C₁-C₇-alkyl; phenyl- or naphthylsulfonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, halo; hydroxy; C₁-C₇-alkoxy; C₆-C₁₈-aryl-C₁-C₇-alkoxy in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by C₁-C₇-alkoxy, by
pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇-alkoxy; hydroxy-C₁-C₇-alkoxy; C₁-C₇-alkoxy-C₁-C₇-alkoxy; amino-C₁-C₇-alkoxy; N-C₁-C₇-alkanoylamino-C₁-C₇-alkoxy; N-unsubstituted-, N-mono- or N,N-di-(C₁-C₇-alkyl)carbamoyl-C₁-C₇-alkoxy; phenyl- or naphthyloxy; phenyl- or naphthyl-C₁-C-₇-alkyloxy; (pyrrolidinyl, piperidinyl, piperazinyl, pyridinyl, pyrimidinyl, pyrazinyl or pyridazinyl)-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; (pyrrolidinyl, piperidinyl, piperazinyl, pyridinyl, pyrimidinyl, pyrazinyl or pyridazinyl)-oxy-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyll; C₁-C₇-alkanoyloxy; benzoyl- or naphthoyloxy; C₁-C₇-alkylthio, halo-C₁-C₇-alkthio; C₁-C₇-alkoxy-C₁-C₇-alkylthio; phenyl- or naphthylthio; phenyl- or naphthyl-C₁-C₇-alkylthio; C₁-C₇-alkanoylthio; benzoyl- or naphthaylthio; nitro; amino; mono- or di-(C₁-C₇-alkyl)-amino; mono- or di-(naphthyl- or phenyl-C₁-C₇-alkylyamino; C₁-C₇-alkanoylamino; benzoyl- or naphthoylamino; C₁-C₇-alkylsulfonylamino; phenyl- or naphthylsulfonylamino wherein phenyl or naphthyl is unsubstituted or substituted by one or more, especially one to three, C₁-C₇-alkyl moieties; phenyl-or naphthyl-C₁-C₇-alkylsulfonylamino; C₁-C₇-alkanoyl; C₁-C₇-alkoxy-C₁-C₇-alkanoyl; carboxyl; C₁-C₇-alkoxy-carbonyl; phenoxy- or naphthoxycarbonyl; phenyl- or naphthyl-C₁-C₇-alkoxycarbonyl; C₁-C₁₀-, especially C₁-C₄-alkylendioxy; carbamoyl; N-mono- or N,N-di-(C₁-C₇-alkyl, naphthyl-C₁-C₇-alkyl, pyrrolidinyl-C₁-C₇-alkyl, piperidinyl -C₁-C₇-alkyl, piperazinyl- or N-C₁-C₇-alkylypiperazinyl-C₁-C₇-alkyl, phenyl-C₁-C₇-alkyl, mono-C₁-C₇-alkoxy-C₁-C₇-alkyl and/or (N'-mono- or N'N'-di-(C₁-C₇-alkyl)-amino-C₁-C₇-alkyl)-amino-carbonyl: N-C₁-C₇-alkoxy-C₁-C₇-alkylcarbamoyl; pyrrolidin-1-carbonyl; amino-N-pyrrolidin-1-carbonyl; N-mono- or N,N-di(C₁-C₇-alkyl)amino-pyrrolidin-1-carbonyl; piperidin-1-carbonyl; morpholin-4-carbonyl; thiomorpho-lin-4-carbonyl; S-oxo-thiomorpholin-4-carbonyl; S,S-dioxothiomorpholin-4-carbonyl; piperazin-1-carbonyl; N-C₁-C₇-alkyl-piperazin-1-carbonyl; N-C₁-C₇-alkoxycarbonyl-piperazin-1-carbonyl; N-mono- or N,N-di-(C₁-C₇-alkyl)-amino-substituted or unsubstituted pyrrolidinyl-C₁-C₇-alkyl; cyano; C₁-C₇-alkenylene or -alkinylene; C₁-C₇-alkylsulfonyl; phenyl- or naphthylsulfonyl wherein phenyl or naphthyl is unsubstituted or substituted by one or more, especially one to three, C₁-C₇-alkyl moieties; phenyl- or naphthyl-C₁-C₇-alkylsulfonyl; sulfamoyl; N-mono or N,N-di-(C₁-C₇-alkyl, phenyl-, naphthyl-, pyrrolidinyl(especially pyrrolidino)-C₁-C₇-alkyl, piperidinyl(especially piperidino)-C₁-C₇-alkyl, piperazinyl(especially piperazino)-C₁-C₇-alkyl, N-C₁-C₇-alkylpiperazinyl(especially 4-C₁-C₇-alkylpiperazino)-C₁-C₇-alkyl, phenyl-C₁-C₇-alkyl- and/or naphthyl-C₁-C₇-alkyl)-aminosulfonyl; pyrazolyl; pyrazolidinyl; pyrrolyl; pyridyl that is unsubstituted or substituted by C₁-C₇-alkoxy, and/or by halo-C₁-C₇-alkyl, pyrrolidinyl; piperidinyl; morpholinyl; thiomorpholinyl; S-oxo-thiomorpholinyl; S,S-dioxothiomorpholinyl; piperazinyl; N-C₁-C₇-alkyl-piperazinyl; 4-(phenyl-C₁-C₇-alkyl)-piperazinyl; 4-(naphthyl-C₁-C₇-alkyl)-piperazinyl; 4-(C₁-C₇-alkoxycarbonyl)-piperazinyl; 4-(phenyl-C₁-C₇-alkoxycarbonyl)-piperazinyl and 4-(naphthyl-C₁-C₇-alkoxycarbonyl)-piperazinyl;
or is unsubstituted or substituted heteroaryl where heteroaryl is selected from the group consisting of imidazolyl, thiophenyl, pyrrolyl, imidazolyl, pyridyl, pyrimidinyl, pyridazinyl, furyl, 2H- or 4H-pyranyl, oxazolyl, thiazolyl, 5H-indazolyl, isoindolyl, quinolyl, isoquinolinyl, phthalazinyl, 1,8-naphthyridinyl, quinoxalinyl, quinazolinyl, cinnolinyl, indolizinyl, 4H-quinolizinyl, pteridinyl, purinyl, carbazolyl, beta-carbolinyl, acridinyl, phenanthridinyl, phenyzinyl, 1,7-phenanthrolinyl, perimidinyl, benzofuranyl, isobenzofuranyl, 2H-chromenyl, 4aH-isochromenyl, thianthrenyl, xanthenyl, phenoxathiinyl, phenoxazinyl or phenothiazinyl or- preferably in an alternative embiodiment - if R¹ is amino or amino monosubstituted with C₁-C₇ (preferably C₁-C₄)alkyl or C₃-C₈(preferably C₃-C₅)-cycloakly, can also be pryrazolyl; each of which (= where each of the heteroaryls which are mentioned) is unsubstituted or substituted as mentioned above for aryl;
R³ is hydrogen, halogen, C₁-C₇-alkyl, C₁-C₇-alkoxy or cyano;
R⁴ is unsubstituted or substituted aryl or unsubstituted or substituted heteroaryl, each independently selected from unsubstituted or substituted aryl as defined for R² and unsubstituted or substituted heteroaryl where heteroaryl is selected from the group consisting of imidazolyl, thiophenyl, pyrazolyl, pyrrolyl, imidazolyl, pyridyl, pyrimidinyl, pyridazinyl, furyl, 2H- or 4H-pyranyl, oxazolyl, thiazolyl, 5H-indazolyl, indolyl, soindolyl, quinolyl, isoquinolinyl, phthalazinyl, 1,8-naphthyridinyl, quinoxalinyl, quinazolinyl, cinnolinyl, indolizinyl, 4H-quinolizinyl, pteridinyl, purinyl, carbazolyl, beta-carbolinyl, acridinyl, phenanthridinyl, phenyzinyl, 1,7-phenanthrolinyl, perimidinyl, benzofuranyl, isobenzofuranyl, 2H-chromenyl, 4aH-isochromenyl, thianthrenyl, xanthenyl, phenoxathiinyl, phenoxazinyl or phenothiazinyl, as defined for R²; and
R⁵ is hydrogen, methyl or methyl substituted with halogen;
or a tautomer thereof or a N-oxide thereof, or a pharmaceutically acceptable salt, or a hydrate or solvate thereof; as well as to its "use" as defined below.
In another preferred embodiment, the invention relates to a compound of the formula I wherein
R¹ is hydrogen, amino, N- alkylamino or C₃-C₅-cycloalkylamino,
R² is phenyl, naphthyl, pyrrolyl, thiophenyl, pyrazolyl, triazolyl, pyridyl, quinolyl or quinoxalinyl, or is pyrrolopyridinyl, especially1H-pyrrolo[2,3-b]pyridin-5-yl, each of which is unsubstituted or substituted by one or more substituents independently selected from the group consisting of halo, hydroxy, C₁-C₇-alkoxy, C₁-C₇-alkoxy-C₁-C₇-alkoxy, amino, N-mono-or N,N-di-(C₁-C₇-alkyl, phenyl-C₁-C₇-alkyl and/or naphthyl-C₁-C₇-alkyl)-amino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl, , N',N'-di-(C₁-C₇-alkyl)amino-C₁-C₇-alkyl, pyrrolidino-C₁-C₇-alkyl and/or phenyl-C₁-C₇-alkylycarbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl, N',N'-di-(C,-C₇-alkyl)amino-C₁-C₇-alkyl, pyrrolidino-C₁-C₇-alky and/or phenyl-C₁-C₇-alkylysulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxy-carbonylypiperazinyl, 4-(phenyl-C₁-C₇-alkoxycarbonyl)-piperazinyl, 4-(naphthyl-C₁-C₇-alkoxycarbonyl)-piperazinyl, morpholinyl, thiomorpholinyl, S-oxothiomorpholinyl and S,S-dioxothio-morpholinyl, and/or from 2-amino-pyrimidin-5-yl-C₁-C₇-alkyl, 4-C₁-C₇-alkyl-piperarzin-1-carbonyl-C₁-C₇-alkoxy, 4-pyrrolidino-piperidin-1-carbonyl-C₁-C₇-alkoxy, 4-pyrrolidino-piperidin-1-yl-C₁-C₇-alkoxy, 4-C₁-C₇-alkyl-piperazino-C₁-C₇-alkoxy, pyridin (e.g,-2)-yloxy-C₁-C₇-alkoxy, pyrimidin(e.g. -4)-yloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₇-alkoxy-substituted pyridin(e.g. -3))-yl; or alternatively or in addition selected from C₁-C₇-alkyl, halo-C₁-C₇-alkyl, such as trifluoromethyl, phenyl that is unsubstituted or substituted by one to three substituents independently selected from hydroxyl-C₁-C₇-alkyl, such as hydroxyl-methyl, C₁-C₇-alkoxy-C₁-C₇-alkyl, such as methoxymethyl, C₁-C₇-alkoxy, such as methoxy, amino and carbamoyl, C₁-C₇-alkanoylamino, such as acetylamino, cyano, 4-(C₁-C₇-alkanoyl)-piperazinyl, such as 4-acetyl-piperazin-1-yl, 4-(C₁-C₇-alkanesulfonyl)-piperazinyl, such as 4-methanesulfonyl-piperazin-1-yl, 2-oxo-pyrrolidin-1-yl, 2-oxo-azetidin-1-yl, 2-oxo-piperidin-1-yl and 3-C₁-C₇-alkyl-2-oxo-imidazolidin-1-yl, such as 3-methyl-2-oxo-imidazolidin-1-yl;
R³ is hydrogen, or it is halo, preferably hydrogen;
R⁴ is phenyl, naphthyl, pyrrolyl, thiophenyl, triazolyl, pyridyl, quinolinyl or quinoxalinyl, or is furanyl, such as furan-2-yl or 1H-pyrrolo[2,3-b]-pyridin-5-yl, (or, if R¹ is amino, N-C₁-C₄-alkylamino or C₃-C₅-cycloalkylamino, can (preferably in an alternative embodiment) also (= in addition to the other moieties just mentioned) be pyrazolyl), each of which is unsubstituted or substituted by one or more substituents independently selected from the group consisting of halo, hydroxy, C₁-C₇-alkoxy, C₁-C₇-alkoxy-C₁-C₇-alkoxy, amino, N-mono- or N,N-di-(C₁-C₇-alkyl, hydroxyl-C₁-C₇-alkyl, phenyl-C₁-C₇-alkyl and/or naphthyl-C₁-C₇-alkyl)-amino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl, , N',N'-di-(C₁-C₇-alkyl)amino-C₁-C₇-alkyl, pyrrolidino-C₁-C₇-alkyl and/or phenyl-C₁-C₇-alkyl)-carbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alky and/or phenyl-C₁-C₇-alkyl)sulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxycarbonyl)-piperazinyl, 4-(phenyl-C₁-C₇-alkoxycarbonyl)-piperazinyl, 4-(naphthyl-C₁-C₇-alkoxycarbonyl)-piperazinyl, morpholinyl, thiomorpholinyl, S-oxothiomorpholinyl and S,S-dioxothio-morpholinyl, and/or from 2-amino-pyrimidin-5-yl-C₁-C₇-alkyl, 4-C₁-C₇-alkyl-piperarzin-1-carbonyl-C₁-C₇-alkoxy, 4-pyrrolidino-piperidin-1-carbonyl-C₁-C₇-alkoxy, 4-pyrrolidino-piperidin-1-yl-C₁-C₇-alkoxy, 4-C₁-C₇-alkyl-piperazino-C₁-C₇-alkoxy, pyridin (e.g.-2)-yloxy-C₁-C₇-alkoxy, pyrimidin(e.g. -4)-yloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₇-alkoxy-substituted pyridin(e.g. -3))-yl; or alternatively or in addition selected from the group consisting of halo-C₁-C₇-alkyl, such as trifluoromethyl, amino-C₁-C₇alkyl, such as aminomethyl, amino-C₁-C₇-alkoxy, such as 3-aminopropoxy or 2-aminoethoxy, phenyl-C₁-C₇-alkoxy, such as benzyloxy, C₁-C₇-alkanoyl, such as formyl and cyano; and
R⁵ is hydrogen;
or a tautomer thereof or a N-oxide thereof, or a pharmaceutically acceptable salt, or a hydrate or solvate thereof; as well as to its "use" as defined below.

A more preferred embodiment of the invention relates to a compound of the formula I according to claim 1, wherein
R¹ is hydrogen, amino, methylamino, n-propylamino or cyclopropylamino;
R² is phenyl, 4-trifluoromethylphenyl, 3-fluorophenyl, 4-fluorophenyl, 4-chlorophenyl, 4-bromophenyl, 3-methoxyphenyl, 4-methoxyphenyl, 3-ethoxyphenyl, 3,4-dimethoxyphenyl, 4-ethoxy-3-methoxy-phenyl, 3,4-diethoxy-phenyl, 3-benzyloxy-4-methoxyphenyl, 4-(2-methoxyethoxy)-3-methoxy-phenyl, 4-trifluormethoxyphenyl, 4-methoxy-3-trifluoromethoxyphenyl, 4-(3-tert-butoxycarbonylamino-propoxy)-3-methoxy-phenyl, 4-(2-tert-butoxycarbonyl-aminoethoxy)-3-methoxy-phenyl, 3,4,5-trimethoxyphenyl, 3-chloro-4-n-propoxy-phenyl, 4-acetylaminophenyl, 4-carboxy-3-methoxyphenyl, 4-methoxycarbonyl-phenyl, 4-methoxycarbonyl-3-methoxyphenyl, 4-cyanophenyl, 4-biphenylyl, 4'-amino-biphenyl-4-yl, 4'-methoxy-biphenyl-4-yl, 4'-hydroxymethyl-biphenyl-4-yl, 4'-methoxymethyl-biphenyl-4-yl,3',4'-dimetho-xy-biphenyl-4-yl, 4'-carbamoyl-biphenyl-4-yl,4-carbamoylphenyl, 4-N-methylcarbamoyl-3-methoxy-phenyl, 4-(N,N-dimethylcarbamoyl)-phenyl, 4-(N-methylcarbamoyl)-phenyl, 4-(N,N-dimethyl-carbamoyl)-3-methoxy-phenyl, 4-(4-methylpiperazin-1-carbonyl)-3-methoxyphenyl, 4-(morpholin-4-carbonyl)-phenyl, 4-(4-morpholin-1-carbonyl)-3-methoxyphenyl, benzo[1,3]dioxol-5-yl, 2,3-dihydro-benzo[1,4]dioxin-6-yl, 4-(piperazin-1-yl)-phenyl, 4-(2-oxo-pyrrolidin-1-yl)-phenyl, 4-(2-oxo-azetidin-1-yl)-phenyl, 4-(2-oxo-piperidin-1-yl)-phenyl, 4-(3-methyl-2-oxo-imidazolidin-1-yl)-phenyl, 4-methanesulfonyl-phenyl, 4-sulfamoyl-phenyl, 4-N,N-dimethyl-sulfamoylphenyl, 4-pyrazolyl-phenyl, pyrrolyl, pyrazolyl, thiophenyl, especially thiophen-3-yl, 1,2,4-triazol-1-yl, 2-methoxy-pyridin-4-yl, 5-methoxy-pyridin-3-yl, 6-methoxy-pyridin-3-yl, 6-piperazino-pyridin-3-yl, 6-morpholin-4-yl-pyridin-3-yl, 1 H-pyrrolo[2,3-b]pyridin-5-yl, 4-[6-(4-methanesulfonyl)-piperazin-1-yl]-pyridin-3-yl, 5-(4-acetylpiperazin-1-yl)-pyridin-3-yl or 2-[4-(tert-butoxycarbonyl)piperazin-1-yl]-pyridin-4-yl;
R³ is hydrogen,
R⁴ is 3-hydroxyphenyl, 4-hydroxyphenyl, 4-hydroxy-3-methoxyphenyl, 3-hydroxy-4-n-propoxyphenyl, 3-ethoxyphenyl, 3,4-dimethoxyphenyl, 3,4-diethoxyphenyl, 3,4,5-trimethoxyphenyl, 3-ethoxy-4-methoxy-phenyl, 4-ethoxy-3-methoxyphenyl, 3-(2-methoxy-ethoxy)-4-methoxyphenyl, 3-methoxy-4-(2-methoxy-ethoxy)-phenyl, 3-benzyloxy-4-methoxyphenyl, 4-(3-aminopropoxy)-3-methoxy-phenyl, 5-(3-aminopropoxy)-3-methoxyphenyl, 4-(2-aminoethoxy)-3-methoxy-phenyl, 5-(2-aminoethoxy)-3-methoxy-phenyl, 3-fluoro-4-methoxyphenyl, 3-chloro-4-methoxy-phenyl, 3-chloro-4-n-propoxyphenyl, 4-(3-tert-butoxycarbonylaminopropoxy)-3-methoxy-phenyl, 4-(2-tert-butoxycarbonylamino-ethoxy)-3-methoxy-phenyl, 4-formyl-phenyl, 4-methoxycarbonyl-3-methoxyphenyl, 3-carbamoyl-phenyl, 4-carbamoylphenyl, 4-carbamoyl-3-methoxy-phenyl, 3-sulfamoyl-phenyl, N,N-dimethylaminosulfonylphenyl, benzo[1,3]dioxol-5-yl, 2,3-dihydro-benzo[1,4]dioxin-6-yl, 6-aminomethyl-pyridin-3-yl, pyridine-3-yl, 6-methoxy-pyridin-3-yl, 5-methoxy-pyridin-3-yl, 2-methoxy-pyridin-4-yl, 2-amino-pyridin-4-yl, 6-amino-pyridin-3-yl, 6-amino-5-trifluoromethylpyridin-3-yl, 6-dimethylamino-pyridin-3-yl, 6-methylamino-pyridin-3-yl, 6-isobutylamino-pyridin-3-yl, 6-(2-methoxyethylamino)-pyridin-3-yl, 6-(piperazin-1-yl)-pyridin-3-yl, 6-(4-tert-butoxycarbonyl-piperazin-1-yl)-pyridin-3-yl, 2-(piperazin-1-yl)-pyridin-4-yl, 6-carbamoyl-pyridin-3-yl, 2-cyano-pyridin-5-yl, 5-cyano-pyridin-3-yl, 6-(2-hydroxyethyl-amino)-pyridin-3-yl, 2-(4-tert-butoxycarbonyl-piperazin-1-yl)-pyridin-4-yl, 6-morpholin-4-yl-pyridin-3-yl, furan-2-yl, furan-3-yl, 1H-pyrrolo[2,3-b]pyridine-5-yl, or quinolin-3-yl and
R⁵ is hydrogen,
or a tautomer thereof or a N-oxide thereof, or a pharmaceutically acceptable salt, or a hydrate or solvate thereof.

A yet more preferred embodiment of the invention relates to a compound of the formula I wherein
R¹ is hydrogen, amino, methylamino, n-propylamino or cyclopropylamino;
R² is phenyl, 4-(2-amino-pyrimidin-5-ylmethyl)-phenyl, 3-(2-methoxy-6-trifluoromethyl)pyridin-3-yl-phenyl, 3-methoxyphenyl, 4-methoxyphenyl, 3,4-dimethoxyphenyl, 3,4,5-trimethoxyphenyl, 3-chloro-4-n-propoxy-phenyl, 4-carboxy-3-methoxyphenyl, 4-(2-pyridin-2-yloxyethoxy)-phenyl, 4-(2-pyrimidin-4-yloxyethoxy)-phenyl, 4-methoxycarbonyl-3-methoxyphenyl, 4-carbamoylphenyl, 4-N-methylcarbamoyl-3-methoxy-phenyl, 4-(N,N-dimethyl-carbamoyl)-3-methoxy-phenyl, 4-(4-methylpiperazin-1-carbonyl)-3-methoxyphenyl, 4-(4-morpholin-1-carbonyl)-3-methoxyphenyl, benzo[1,3]dioxol-5-yl, 2,3-dihydro-benzo[1,4]dioxin-6-yl, 4-[2-(4-pyrrolidino-piperidin-1-yl)-ethoxy]-phenyl, 4-(piperazin-1-yl)-phenyl, 4-[2-(4-methyl-piperazin-1-yl)-ethoxy]-phenyl, 4-[2-(4-ethyl-piperazin-1-yl)-ethoxy]-phenyl, 4-(4-methyl-piperazin-1-carbonylmethoxy)-phenyl, 4-(4-ethyl-piperazin-1-carbonylmethoxy)-phenyl, 4-(4-pyrrolidino-piperidin-1-carbonylmethoxy)-phenyl, 4-[N-(2-dimethylaminoethyl)-N-methylcarbamoyl]-phenyl, 4-[(R, S or R,S)-3-diethylamino-pyrrolidin-1-carbonyl)-phenyl, 4-sulfamoyl-phenyl, 4-N,N-dimethyl-sulfamoylphenyl, 4-[N-methyl-N-2-(pyrrolidinoethyl)-sulfamoyl]-phenyl, 4-pyrazolyl-phenyl, pyrrolyl, pyrazolyl, thiophenyl, 1,2,4-triazol-1-yl, 6-methoxy-pyridin-3-yl or 6-piperazino-pyridin-3-yl;
R³ is hydrogen,
R⁴ is 4-(2-amino-pyrimidin-5-ylmethyl)-phenyl, 3-(2-methoxy-6-trifluoromethyl)pyridin-3-yl-phenyl, 3-hydroxyphenyl, 4-hydroxyphenyl, 4-hydroxy-3-methoxyphenyl, 3-hydroxy-4-n-propoxyphenyl, 3,4-dimethoxyphenyl, 3,4,5-trimethoxyphenyl, 4-ethoxy-3-methoxyphenyl, 3-(2-methoxy-ethoxy)-4-methoxyphenyl, 3-methoxy-4-(2-methoxy-ethoxy)-phenyl, 3-fluoro-4-methoxyphenyl, 3-chloro-4-n-propoxyphenyl, 4-(2-pyridin-2-yloxyethoxy)-phenyl, 4-(2-pyrimidin-4-yloxyethoxy)phenyl, 4-[2-(4-pyrrolidino-piperidin-1-yl)-ethoxy]-phenyl, 4-[2-(4-methyl-piperazin-1-yl)-ethoxy]-phenyl, 4-[2-(4-ethyl-piperazin-1-yl)-ethoxy]-phenyl, 4-(4-methyl-piperazin-1-carbonylmethoxy)-phenyl, 4-(4-ethyl-piperazin-1-carbonylmethoxy)-phenyl, 4-(4-pyrrolidino-piperidin-1-carbonylmethoxy)-phenyl, 4-[N-(2-dimethylaminoethyl)-N-methylcarbamoyl]-phenyl, 4-[(R, S or R,S)-3-diethylamino-pyrrolidin-1-carbonyl)-phenyl, 4-methoxycarbonyl-3-methoxyphenyl, 4-carbamoylphenyl, N,N-dimethylaminosulfonylphenyl, 4-[N-methyl-N-2-(pyrrolidino-ethyl)-sulfamoyl]-phenyl, benzo[1,3]dioxol-5-yl, 2,3-dihydro-benzo[1,4]dioxin-6-yl, pyridine-3-yl, 6-methoxy-pyridin-3-yl, 5-methoxy-pyridin-3-yl, 2-amino-pyridin-4-yl, 6-amino-pyridin-3-yl, 6-(piperazin-1-yl)-pyridin-3-yl, 6-(4-tert-butoxycarbonyl-piperazin-1-yl)pyridin-3-yl, 2-(piperazin-1-yl)pyridin-4-yl or 2-(4-tert-butoxycarbonyl-piperazin-1-yl)-pyridin-4-yl, and
R⁵ is hydrogen,
or a tautomer thereof or a N-oxide thereof, or a pharmaceutically acceptable salt, or a hydrate or solvate thereof; as well as to its "use" as defined below.

Another more preferred embodiment of the invention relates to a compound of the formula I, wherein
R¹ is hydrogen, amino, methylamino, n-propylamino or cyclopropylamino;
R² is phenyl, 4-(2-amino-pyrimidin-5-ylmethyl)-phenyl, 3-(2-methoxy-6-trifluoromethyl)pyridin-3-yl, 3-methoxyphenyl, 4-methoxyphenyl, 3,4-dimethoxyphenyl, 3,4,5-trimethoxyphenyl, 3-chloro-4-n-propoxy-phenyl, 4-carboxy-3-methoxyphenyl, 4-(2-pyridin-2-yloxyethoxy)-phenyl, 4-(2-pyrimidin-4-yloxyethoxy)-phenyl, 4-methoxycarbonyl-3-methoxyphenyl, 4-carbamoylphenyl, 4-N-methylcarbamoyl-3-methoxy-phenyl, 4-(N,N-dimethyl-carbamoyl)-3-methoxyphenyl, 4-(4-methylpiperazin-1-carbonyl)-3-methoxyphenyl, 4-(4-morpholin-1-carbonyl)-3-methoxyphenyl, benzo[1,3]dioxol-5-yl, 2,3-dihydro-benzo[1,4]dioxin-6-yl, 4-[2-(4-pyrrolidino-piperidin-1-yl)-ethoxyl-phenyl, 4-(piperazin-1-yl)-phenyl, 4-[2-(4-methyl-piperazin-1-yl)-ethoxy]-phenyl, 4-[2-(4-ethyl-piperazin-1-yl)-ethoxy]-phenyl, 4-(4-methyl-piperazin-1-carbonylmethoxy)-phenyl, 4-(4-ethyl-piperazin-1-carbonylmethoxy)phenyl, 4-(4-pyrrolidino-piperidin-1-carbonylmethoxy)phenyl, 4-[N-(2-dimethylamino-ethyl)-N-methylcarbamoyl]-phenyl, 4-[(R, S or R,S)-3-diethylamino-pyrrolidin-1-carbonyl)-phenyl, 4-sulfamoyl-phenyl, 4-N, N-dimethyl-sulfamoylphenyl, 4-[N-methyl-N-2-(pyrrolidino-ethyl)-sulfamoyl]-phenyl, 4-pyrazolyl-phenyl, pyrrolyl, pyrazolyl, thiophenyl, 1,2,4-triazol-1-yl, 6-methoxy-pyridin-3-yl, or 6-piperazino-pyridin-3-yl;
R³ is hydrogen,
R⁴ is 4-(2-amino-pyrimidin-5-ylmethyl)-phenyl, 3-(2-methoxy-6-trifluoromethyl)pyridin-3-yl-phenyl, 3-hydroxyphenyl, 4-hydroxyphenyl, 4-hydroxy-3-methoxyphenyl, 3-hydroxy-4-n-propoxyphenyl, 3,4-dimethoxyphenyl, 3,4,5-trimethoxyphenyl, 4-ethoxy-3-methoxyphenyl, 3-(2-methoxy-ethoxy)-4-methoxyphenyl, 3-methoxy-4-(2-methoxy-ethoxy)-phenyl, 3-fluoro-4-methoxyphenyl, 3-chloro-4-n-propoxyphenyl, 4-(2-pyridin-2-yloxyethoxy)-phenyl, 4-(2-pyrimidin-4-yloxyethoxy)-phenyl, 4-[2-(4-pyrrolidino-piperidin-1-yl)-ethoxy]-phenyl, 4-[2-(4-methyl-piperazin-1-yl)-ethoxy]-phenyl, 4-[2-(4-ethyl-piperazin-1-yl)ethoxy]-phenyl, 4-(4-methyl-piperazin-1-carbonylmethoxy)-phenyl, 4-(4-ethyl-piperazin-1-carbonylmethoxy)-phenyl, 4-(4-pyrrolidino-piperidin-1-carbonylmethoxy)-phenyl, 4-[N-(2-dimethylaminoethyl)-N-methylcarbamoyl]-phenyl, 4-[(R, S or R,S)-3-diethylamino-pyrrolidin-1-carbonyl)-phenyl, 4-methoxycarbonyl-3-methoxyphenyl, 4-carbamoylphenyl, N,N-dimethyl-aminosulfonylphenyl, 4-[N-methyl-N-2-(pyrrolidino-ethyl)-sulfamoyl]-phenyl, benzo[1,3]dioxol-5-yl, 2,3-dihydro-benzo[1,4]dioxin-6-yl, pyridine-3-yl, 6-methoxy-pyridin-3-yl, 5-methoxy-pyridin-3-yl, 2-amino-pyridin-4-yl, 6-amino-pyridin-3-yl, 6-(piperazin-1-yl)-pyridin-3-yl, 6-(4-tert-butoxycarbonyl-piperazin-1-yl)-pyridin-3-yl, 2-(piperazin-1-yl)pyridin-4-yl or 2-(4-tert-butoxycarbonyl-piperazin-1-yl)-pyridin-4-yl, and
R⁵ is hydrogen,
or a tautomer thereof or a N-oxide thereof, or a pharmaceutically acceptable salt, or a hydrate or solvate thereof.

Especially preferred is a compound of the formula I as given in the Examples, as well as a way of its synthesis described therein, or a tautomer thereof or an N-oxide thereof, or a pharmaceutically acceptable salt, or a hydrate or solvate thereof; as well as its "use" as defined below.

Very preferred are also embodiment of the invention represented in the claims which are therefore incorporated by reference herein.

Surprisingly, it has now been found that the compounds of formula I have advantageous pharmacological properties and inhibit the activity of the lipid kinases, such as the PI3-kinase and/or members of the PI3-kinase-related protein kinase family (also called PIKK and include DNA-PK, ATM, ATR, hSMG-1 and mTOR), such as the DNA protein-kinase, and may be used to treat disease or disorders which depend on the activity of said kinases.

The phosphatidylinositol-3'-OH kinase (PI3K) pathway is one of the central signaling pathways that exerts its effect on numerous cellular functions including cell cycle progression, proliferation, motility, metabolism and survival. An activation of receptor tyrosine kinases causes PI3K to phosphorylate phosphatidylinositol-(4,5)-diphosphate, resulting in membrane-bound phosphatidylinositol-(3,4,5)-triphosphate. The latter promotes the transfer of a variety of protein kinases from the cytoplasm to the plasma membrane by binding of phosphatidylinositol-(3,4,5)-triphosphate to the pleckstrin-homology (PH) domain of the kinase. Kinases that are key downstream targets of PI3K include phosphoinositide-dependent kinase 1 (PDK1) and AKT (also known as Protein Kinase B). Phosphorylation of such kinases then allows for the activation or deactivation of numerous other pathways, involving mediators such as GSK3, mTOR, PRAS40, FKHD, NF-κB, BAD, Caspase-9, and the like. An important negative feedback mechanism for the PI3K pathway is PTEN, a phosphatase that catalyses the dephosphorylation of phosphatidylinositol-(3,4,5)-triphosphate to phosphorylate phosphatidylinositol-(4,5)-diphosphate. In more than 60 % of all solid tumors, PTEN is mutated into an inactive form, permitting a constitutive activation of the PI3K pathway. As most cancers are solid tumors, such an observation provides evidence that a targeting of PI3K itself or individual downstream kinases in the PI3K pathway provide a promising approach to mitigate or even abolish the dysregulation in many cancers and thus restore normal cell function and behaviour. This, however, does not exclude that other mechanisms may be responsible for the beneficial effects of PI3K activity modifying agents such as those in the present invention.

Having regard to their inhibitory effect on phosphatidylinositol 3-kinase enzymes, compounds of formula (I) in free or pharmaceutically acceptable salt form, are useful in the treatment of conditions which are mediated by the activation (including normal activity or especially overactivity) of one or more of the members of the PI3 kinase family, especially PI3 kinase enzyme, such as proliferative, inflammatory or allergic conditions, obstructive airways diseases and/or disorders commonly occurring in connection with transplantation.

"Treatment" in accordance with the invention may be therapeutic, e.g. symptomatic, or prophylactic. Preferred is the treatment of warm-blooded animals, especially humans.

Preferred is a compound of formula I for use or the use thereof in the treatment of a proliferative disease selected from a benign or malignant tumor, carcinoma of the brain, kidney, liver, adrenal gland, bladder, breast, stomach, gastric tumors, ovaries, colon, rectum, prostate, pancreas, lung, vagina or thyroid, sarcoma, glioblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, lymphomas, a mammary carcinoma or a leukemia. Other diseases include Cowden syndrome, Lhermitte-Dudos disease and Bannayan-Zonana syndrome, or diseases in which the PI3K/PKB pathway is aberrantly activated.

Compounds according to the invention are also of use in the treatment of inflammatory or obstructive airways (respiratory tract) diseases, resulting, for example, in reduction of tissue damage, airways inflammation, bronchial hyperreactivity, remodeling or disease progresssion. Inflammatory or obstructive airways diseases to which the present invention is applicable include asthma of whatever type or genesis including both intrinsic (non-allergic) asthma and extrinsic (allergic) asthma, e.g. mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma can be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Compounds of the formula I can be of use for other inflammatory or obstructive airways diseases and conditions to which the present invention is applicable and include acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy.

The invention also to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their anti-inflammatory activity, in particular in relation to inhibition of eosinophil activation, compounds of the invention are also of use in the treatment of eosinophil related disorders, e.g. eosinophilia, in particular eosinophil related disorders of the airways (e.g. involving morbid eosinophilic infiltration of pulmonary tissues) including hypereosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

Compounds of the invention are also of use in the treatment of inflammatory or allergic conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphigus, epidermolysis bullosa acquisita, and other inflammatory or allergic conditions of the skin.

Compounds of the invention may also be used for the treatment of other diseases or conditions, such as diseases or conditions having an inflammatory component, for example, treatment of diseases and conditions of the eye such as conjunctivitis, keratoconjunctivitis sicca, and vernal conjunctivitis, diseases affecting the nose including allergic rhinitis, and inflammatory disease in which autoimmune reactions are implicated or having an autoimmune component or aetiology, including autoimmune haematological disorders (e.g. haemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary billiary cirrhosis, uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Furthermore, the invention provides the use of a compound according to the definitions herein, or a pharmaceutically acceptable salt, or a hydrate or solvate thereof for the preparation of a medicament for the treatment of a proliferative disease, an inflammatory disease, an obstructive respiratory disease, or a disorder commonly occurring in connection with transplantation.

The invention expecially relates to the use of a compound of the formula I (or a pharmaceutical formulation comprising a compound of the formula I) in the treatment of one or more of the diseases mentioned above and below where the disease(s) respond or responds (in a beneficial way, e.g. by partial or complete removal of one or more of its symptoms up to complete cure or remission) to an inhibition of one or more kinases of the P13-kinase-related protein kinase family, most especially PI3 kinase (PI3K), especially where the kinase shows (in the context of other regulatory mechanisms) inadequately high or more preferably higher than normal (e.g. constitutive) activity.

Whereever the term "use" or "used" is mentioned, this is intended to include a compound of the formula I for use in the prophylactic and/or therapeutic treatment of a disease of a warm-blooded animal, especially a human, preferably of one or more diseases mentioned above or below, a method of use or a method of treatment comprising administering a compound of the formula I to a person in need of such treatment in an effective amount for the prophylactic and/or therapeutic treatment of a disease as mentioned above and below, the preparation or a method or preparation of a pharmaceutical formulation/preparation for use in the prophylactic and therapeutic treatment of a disease mentioned above and below, especially involving mixing a compound of the formula I (as therapeutically active ingredient) with at least one pharmaceutically acceptable carrier material, including making it ready for use in such treatment (e.g. adding an instruction insert (e.g. package leaflet or the like), formulation, appropriate preparation, adaptation for specific uses, customizing and the like), and the use of a compound of the formula I for such preparation, and/or all other prophylactic or therapeutic uses mentioned hereinbefore or below. All these aspects are embodiments of the present invention.

The efficacy of the compounds of formula I and salts thereof as PI3 kinase inhibitors can be demonstrated as follows:
The kinase reaction is performed in a final volume of 50 µL per well of a half area COSTAR, 96 well plate. The final concentrations of ATP and phosphatidyl inositol in the assay are 5 µM and 6 µg/mL respectively. The reaction is started by the addition of PI3 kinase p110β. The components of the assay are added per well as follows:
   - 10 µL test compound in 5% DMSO per well in columns 2-1.
   - Total activity is determined by addition 10 µL of 5% vol/vol DMSO in the first 4 wells of column 1 and the last 4 wells of column 12.
   - The background is determined by addition of 10 µM control compound to the last 4 wells of column 1 and the first 4 wells of column 12.
   - 2 mL 'Assay mix' are prepared per plate:
      1.912 mL of HEPES assay buffer
      8.33 µL of 3 mM stock of ATP giving a final concentration of 5 µM per well
      1 µL of [³³P]ATP on the activity date giving 0.05 µCi per well
      30 µL of 1 mg/mL PI stock giving a final concentration of 6 µg/mL per well
      5 µL of 1 M stock MgCl₂ giving a final concentration of 1 mM per well
   - 20 µL of the assay mix are added per well.
   - 2 mL 'Enzyme mix' are prepared per plate (x µL PI3 kinase p110β in 2 mL of kinase buffer). The Enzyme mix' is kept on ice during addition to the assay plates.
   - 20 µl 'Enzyme mix' are added/well to start the reaction.
   - The plate is then incubated at room temperature for 90 minutes.
   - The reaction is terminated by the addition of 50 µL WGA-SPA bead (wheat germ agglutinin-coated Scintillation Proximity Assay beads) suspension per well.
   - The assay plate was sealed using TopSeal-S) heat seal for polystyrene microplates, PerkinElmer LAS (Deutschland) GmbH, Rodgau, Germany) and incubated at room temperature for at least 60 minutes.
   - The assay plate was then centrifuged at 1500 rpm for 2 minutes using the Jouan bench top centrifuge (Jouan Inc., Nantes, France).
   - The assay plate was counted using a Packard TopCount, each well being counted for 20 seconds.
   - The volume of enzyme is dependent on the enzymatic activity of the batch in use.

Some of the compounds show a certain level of selectivity against the different paralogs PI3K alpha, beta, gamma and delta.

### Description of biochemical assay for DNA-PK:

The assay is conducted using the kit V7870 from Promega (SignaTECT® DNA-Dependent Protein Kinase Syste, comprises DNA-PK, biotinylated peptide substrate end further ingredients, Promega, Madison, Wisconsin, USA), that quantitates DNA-dependent protein kinase activity, both in purified enzyme preparations and in cell nuclear extracts. DNA-PK is a nuclear serine/threonine protein kinase that requires double-stranded DNA (dsDNA) for activity. The binding of dsDNA to the enzyme results in the formation of the active enzyme and also brings the substrate closer to the enzyme, allowing the phosphorylation reaction to proceed.

DNA-PK X5 reaction buffer (250 mM HEPES, 500 mM KCI, 50 mM MgCl₂, 1 mM EGTA, 0.5 mM EDTA, 5 mM DTT, pH to 7.5 with KOH) is diluted 1/5 in deionised water and BSA (stock = 10 mg/ml) is added to a final concentration of 0.1 mg/ml.

The activation buffer is made from 100 µg/ml of calf thymus DNA in control buffer (10 mM Tris-HCl (pH 7.4), 1 mM EDTA (pH 8.0)). Per tube, the reaction mix is composed of: 2.5 µl of activation or control buffers, 5 µl of X5 reaction buffer, 2.5 µl of p53-derived biotinylated peptide substrate (stock= 4mM), 0.2 µl of BSA (stock at 10 mg/ml) and 5 µl of [γ-³²P] ATP (5 µl of 0.5 mM cold ATP + 0.05 µl of Redivue [γ-³²P] ATP = Amersham AA0068-250 µCi, 3000Ci/mmol, 10 µCi/µl (now GE Gealthcare Biosciences AB, Uppsala, Sweden).

The DNA-PK enzyme (Promega V5811, concentration=100 U/µL) is diluted 1/10 in X1 reaction buffer and kept on ice until imminent use. 10.8 µl of the diluted enzyme is incubated with 1.2 µl of 100 µM compounds (diluted 1/100 in water from 10 mM stock in neat DMSO) for 10 minutes, at room temperature. During that time, 15.2 µl of the reaction mix is added to screw-capped tubes, behind Perspex glass. 9.8 µl of the enzyme is then transferred to the tubes containing the reaction mix and after 5 minutes incubation, at 30°C, the reaction is stopped by adding 12.5 µl of termination buffer (7.5 M guanidine hydrochloride).

After mixing well, a 10 µl aliquot of each tube is spotted onto a SAM2^{®} biotin capture membrane (Promega, Madison, Wisconsin, USA), which is left to dry for a few minutes. The membrane is then washed extensively to remove the excess free [γ-³²P] ATP and nonbiotinylated proteins: once for 30 seconds in 200 ml of 2M NaCl, 3 times for 2 minutes each in 200 ml of 2M NaCl, 4 times for 2 minutes each in 2M NaCl in 1% H₃P0₄ and twice for 30 seconds each in 100 ml of deionised water. The membrane is subsequently left to air-dry at room temperature for 30-60 minutes.

Each membrane square is separated using forceps and scissors and placed into a scintillation vial, after which 8 ml of scintillation liquid (Flo-Scint 6013547 from Perkin-Elmer) is added. The amount of ³²P incorporated into the DNA-PK biotinylated peptide substrate is then determined by liquid scintillation counting. In this test system, compounds of the formula I can be shown to have IC₅₀values in the range from 1 nM to 50 µM, e.g. from 1 nM to 10 µM.

The efficacy of the compounds of the invention in blocking the activation of the PI3K/PKB pathway can be demonstrated in cellular settings as follows:

### Protocol for the detection of phospho-PKB in U87MG cells by Elisa:

U87MG cells (human glioblastoma, ATCC No. HTB-14) are trypsinized, counted in a CASY cell counter (Schärffe systems, Göttingen, Germany), diluted in fresh complete DMEM high glucose medium to load ,per well,150µL cell suspension containing 4x10⁴ cells, and test plates incubated for 18 hours. In parallel, 50 µL of coating antibody, at the desired concentration in PBS/O is loaded in each well of the ELISA plates, and plates are kept for 2 h at room temperature. This ELISA assays is performed in black flat-bottom 96-well plates (Microtest^{™}, Falcon Becton-Dickinson, Ref: 353941) sealed with Plate Sealers (Costar-Corning, Ref: 3095). Medium in plates is discarded and replaced by complete DMEM high glucose medium containing either 0.1% DMSO or 0.1% inhibitor at titers (7) between 10 mM and 0.156 mM in DMSO. After 30 minutes of contact, the medium is quickly removed by aspiration, plates are then placed on ice and immediately cells lyzed with 70 µL of Lysis buffer. In parallel, the 96 wells plates prepared with the coating antibody (1/250 diluted (in PBS/O) Anti-Akt1 C-20, goat, Santa-Cruz-1618, Santa Cruz Biotechnology, Inc., Santa Cruz, California, USA) are washed 3 times 1 min with PBS/O containing 0.05% Tween 20 and 0.1% Top-Block^{®} (derivative of gelatine that blocks unspecific binding sites on surfaces; Sigma-Aldrich, Fluka, Buchs, Switzerland, Ref.: 37766), and remaining protein binding sites blocked to prevent non-specific interactions with 200 µL of PBS containing 3% Top Block®, for 2 h at room temperature. Well content is replaced with 50 µL of samples from treated cells, and plates are incubated for 3 h at 4°C. The ELISA assays are always done in parallel with the following controls, in 6 replicates: U87MG (untreated control) or Lysis buffer alone (LB). After 3 x 15 minutes washes, all wells received 50 µL of the secondary antibody (1/250 diluted (in 3% top block) Anti-S473P-PKB, rabbit, Cell Signaling-9271, Cell Signaling Technologies, Inc., Danvers, Massachusetts, USA)), and are incubated for 16 h at 4°C. After three washes, plates are incubated with the third and conjugated antibody (1/1000 diluted (in 3% top block) anti rabbit (HRP) Jackson Immuno Research 111-035-144) for 2 hours at room temperature. Finally, the immune-complexes are washed 2 times 15 seconds with PBS/O/ tween20 /top block ,1 time with 200µl of water and finally 200µl of water are left in each test well before a for 45 min incubation in darkness. The plates are then assayed with (SuperSignal^{®} ELISA pico Chemiluminescent substrate, Pierce, Ref: 27070, Pierce Biotechnology, Inc., Rockford, Illinois, USA). 100 µL of substrate are added, and plates shacked for 1 min. The luminescence is read immediately on a Top-Count NXT (Packard Bioscience) luminometer. Using this test system, IC₅₀ values in the range from 5 µM to 1 nM, more preferablely from 1.5 µM to 5 nM, can be found for compounds of the formula

There are also experiments to demonstrate the antitumor activity of compounds of the formula (I) *in vivo.*

For example, female Harlan (Indianapolis, Indiana, USA) athymic nu/nu mice with s.c. transplanted human glioblastoms U87MG tumors can be used to determine the anti-tumor activity of PI3 kinase inhibitors. On day 0, with the animals under peroral Forene® (1-chloro-2,2,2-trifluoroethyldifluormethylether, Abbot, Wiesbaden, Germany) narcosis, a tumor fragment of approximately 25 mg is placed under the skin on the animals' left flank and the small incised wound is closed by means of suture clips. When tumors reach a volume of 100 mm³, the mice are divided at random into groups of 6-8 animals and treatment commences. The treatment is carried out for a 2-3 weeks period with peroral, intravenous or intra-peritoneal administration once daily (or less frequently) of a compound of formula (I) in a suitable vehicle at defined doses. The tumors are measured twice a week with a slide gauge and the volume of the tumors is calculated.

As an alternative to cell line U87MG, other cell lines may also be used in the same manner, for example,
- the MDA-MB 468 breast adenocarcinoma cell line (ATCC No. HTB 132; see also In Vitro 14, 911-15 [1978]);
- the MDA-MB 231 breast carcinoma cell line (ATCC No. HTB-26; see also In Vitro 12, 331 [1976]);
- the MDA-MB 453 breast carcinoma cell line (ATCC No.HTB-131);
- the Colo 205 colon carcinoma cell line (ATCC No. CCL 222; see also Cancer Res. 38, 1345-55 [1978]);
- the DU145 prostate carcinoma cell line DU 145 (ATCC No. HTB 81; see also Cancer Res. 37, 4049-58 [1978]),
- the PC-3 prostate carcinoma cell line PC-3 (especially preferred; ATCC No. CRL 1435; see also Cancer Res. 40, 524-34 [1980]) and the PC-3M prostate carcinoma cell line;
- the A549 human lung adenocarcinoma (ATCC No. CCL 185; see also Int. J. Cancer 17, 62-70 [1976]),
- the NCI-H596 cell line (ATCC No. HTB 178; see also Science 246, 491-4 [1989]):
- the pancreatic cancer cell line SUIT-2 (see Tomioka et al., Cancer Res. 61, 7518-24 [2001]).

Compounds of the invention exhibit T cell inhibiting activity. More particular the compounds of the invention prevent T cell activation and/or proliferation in e.g. aqueous solution, e.g. as demonstrated in accordance with the following test method. The two-way MLR is performed according to standard procedures (J. Immunol. Methods, 1973, 2, 279 and Meo T. et al., Immunological Methods, New York, Academic Press, 1979, 227-39). Briefly, spleen cells from CBA and BALB/c mice (1.6 x 105 cells from each strain per well in flat bottom tissue culture microtiter plates, 3.2 x 105 in total) are incubated in RPMI medium containing 10% FCS, 100 U/ml penicillin, 100 µg/ml streptomycin (Gibco BRL, Basel, Switzerland), 50 µM 2-mercaptoethanol (Fluka, Buchs, Switzerland) and serially diluted compounds. Seven threefold dilution steps in duplicates per test compound are performed. After four days of incubation 1 µCi 3H-thymidine is added. Cells are harvested after an additional five-hour incubation period, and incorporated 3H-thymidine is determined according to standard procedures. Background values (low control) of the MLR are the proliferation of BALB/c cells alone. Low controls are subtracted from all values. High controls without any sample are taken as 100% proliferation. Percent inhibition by the samples is calculated, and the concentrations required for 50% inhibition (IC₅₀ values) are determined. In this assay, the compounds of the invention have IC₅₀ values in the range of 1 nM to 5 µM, preferably from 5 nM to 500 nM.

A compound of the formula (I) may also be used to advantage in combination with other antiproliferative compounds. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibittors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (TEMODAL®); kinesin spindle protein inhibitors, such as SB715992 or SB743921 from GlaxoSmithKline, or pentamidine/chlorpromazine from CombinatoRx; MEK inhibitors such as ARRY142886 from Array PioPharma, AZD6244 from AstraZeneca, PD181461 from Pfizer, leucovorin, EDG binders, antileukemia compounds, ribonucleotide reductase inhibittors, S-adenosylmethionine decarboxylase inhibitors, antiproliferative antibodies or other chemotherapeutic compounds. Further, alternatively or in addition they may be used in combination with other tumor treatment approaches, including surgery, ionizing radiation, photodynamic therapy, implants, e.g. with corticosteroids, hormones, or they may be used as radiosensitizers. Also, in anti-inflammatory and/or antiproliferative treatment, combination with anti-inflammatory drugs is included. Combination is also possible with antihistamine drug substances, bronchodilatatory drugs, NSAID or antagonists of chemokine receptors.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN. Formestane can be administered, e.g., in the form as it is marketed, e.g. under the trademark LENTARON. Fadrozole can be administered, e.g., in the form as it is marketed, e.g. under the trademark AFEMA. Anastrozole can be administered, e.g., in the form as it is marketed, e.g. under the trademark ARIMIDEX. Letrozole can be administered, e.g., in the form as it is marketed, e.g. under the trademark FEMARA or FEMAR. Aminoglutethimide can be administered, e.g., in the form as it is marketed, e.g. under the trademark ORIMETEN. A combination of the invention comprising a chemotherapeutic agent which is an aromatase inhibitor is particularly useful for the treatment of hormone receptor positive tumors, e.g. breast tumors.

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX. Raloxifene hydrochloride can be administered, e.g., in the form as it is marketed, e.g. under the trademark EVISTA. Fulvestrant can be formulated as disclosed in US 4,659,516 or it can be administered, e.g., in the form as it is marketed, e.g. under the trademark FASLODEX. A combination of the invention comprising a chemotherapeutic agent which is an antiestrogen is particularly useful for the treatment of estrogen receptor positive tumors, e.g. breast tumors.

The term "anti-androgen" as used herein relates to any substance which is capable of inhibiting the biological effects of androgenic hormones and includes, but is not limited to, bicalutamide (CASODEX), which can be formulated, e.g. as disclosed in US 4,636,505.

The term "gonadorelin agonisf" as used herein includes, but is not limited to abarelix, goserelin and goserelin acetate. Goserelin is disclosed in US 4,100,274 and can be administered, e.g., in the form as it is marketed, e.g. under the trademark ZOLADEX. Abarelix can be formulated, e.g. as disclosed in US 5,843,901.

The term "topoisomerase I inhibitor" as used herein includes, but is not limited to topotecan, gimatecan, irinotecan, camptothecian and its analogues, 9-nitrocamptothecin and the macromolecular camptothecin conjugate PNU-166148 (compound A1 in WO99/ 17804). Irinotecan can be administered, e.g. in the form as it is marketed, e.g. under the trademark CAMPTOSAR. Topotecan can be administered, e.g., in the form as it is marketed, e.g. under the trademark HYCAMTIN.

The term "topoisomerase II inhibitor" as used herein includes, but is not limited to the anthracyclines such as doxorubicin (including liposomal formulation, e.g. CAELYX), daunorubicin, epirubicin, idarubicin and nemorubicin, the anthraquinones mitoxantrone and losoxantrone, and the podophillotoxines etoposide and teniposide. Etoposide can be administered, e.g. in the form as it is marketed, e.g. under the trademark ETOPOPHOS. Teniposide can be administered, e.g. in the form as it is marketed, e.g. under the trademark VM 26-BRISTOL. Doxorubicin can be administered, e.g. in the form as it is marketed, e.g. under the trademark ADRIBLASTIN or ADRIAMYCIN. Epirubicin can be administered, e.g. in the form as it is marketed, e.g. under the trademark FARMORUBICIN. Idarubicin can be administered, e.g. in the form as it is marketed, e.g. under the trademark ZAVEDOS. Mitoxantrone can be administered, e.g. in the form as it is marketed, e.g. under the trademark NOVANTRON.

The term "microtubule active compound" relates to microtubule stabilizing, microtubule destabilizing compounds and microtublin polymerization inhibitors including, but not limited to taxanes, e.g. paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g. epothilone B or D or derivatives thereof. Paclitaxel may be administered e.g. in the form as it is marketed, e.g. TAXOL. Docetaxel can be administered, e.g., in the form as it is marketed, e.g. under the trademark TAXOTERE. Vinblastine sulfate can be administered, e.g., in the form as it is marketed, e.g. under the trademark VINBLASTIN R.P.. Vincristine sulfate can be administered, e.g., in the form as it is marketed, e.g. under the trademark FARMISTIN. Discodermolide can be obtained, e.g., as disclosed in US 5,010,099. Also included are Epothilone derivatives which are disclosed in WO 98/10121, US 6,194,181, WO 98/25929, WO 98/08849, WO 99/43653, WO 98/22461 and WO 00/31247. Especially preferred are Epothilone A and/or B.

The term "alkylating compound" as used herein includes, but is not limited to, cyclophosphamide, ifosfamide, melphalan or nitrosourea (BCNU or Gliadel). Cyclophosphamide can be administered, e.g., in the form as it is marketed, e.g. under the trademark CYCLOSTIN. Ifosfamide can be administered, e.g., in the form as it is marketed, e.g. under the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(2-methyl-1*H*-indol-3-yl)-ethyl]-amino]methyl]phenyl]-2*E-*2-propenamide and pharmaceutically acceptable salts thereof. It further especially includes Suberoylanilide hydroxamic acid (SAHA).

The term "antineoplastic antimetabolite" includes, but is not limited to, 5-Fluorouracil or 5-FU, capecitabine, gemcitabine, DNA demethylating compounds, such as 5-azacytidine and decitabine, methotrexate and edatrexate, and folic acid antagonists such as pemetrexed. Capecitabine can be administered, e.g., in the form as it is marketed, e.g. under the trademark XELODA. Gemcitabine can be administered, e.g., in the form as it is marketed, e.g. under the trademark GEMZAR..

The term "platin compound" as used herein includes, but is not limited to, carboplatin, cis-platin, cisplatinum and oxaliplatin. Carboplatin can be administered, e.g., in the form as it is marketed, e.g. under the trademark CARBOPLAT. Oxaliplatin can be administered, e.g., in the form as it is marketed, e.g. under the trademark ELOXATIN.

The term "compounds targeting/decreasing a protein or lipid kinase activity"; or a "protein or lipid phosphatase activity"; or "further anti-angiogenic compounds" as used herein includes, but is not limited to, protein tyrosine kinase and/or serine and/or threonine kinase inhibitors or lipid kinase inhibitors, e.g.,
a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g. a N-phenyl-2-pyrimidine-amine derivative, e.g. imatinib, SU101, SU6668 and GFB-111;
b) compounds targeting, decreasing or inhibiting the activity of the fibroblast growth factor-receptors (FGFR);
c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, such as those compounds disclosed in WO 02/092599 or such as OSI906, or antibodies that target the extracellular domain of IGF-I receptor such as CP-751871, R1507, AVE1642, IMC-A12, AMG479, MK-0646, SCH717454 or its growth factors;
d) compounds targeting, decreasing or inhibiting the activity of the Trk receptor tyrosine kinase family, or ephrin B4 inhibitors;
e) compounds targeting, decreasing or inhibiting the activity of the AxI receptor tyrosine kinase family;
f) compounds targeting, decreasing or inhibiting the activity of the Ret receptor tyrosine kinase;
g) compounds targeting, decreasing or inhibiting the activity of the Kit/SCFR receptor tyrosine kinase, e.g. imatinib;
h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g. imatinib;
i) compounds targeting, decreasing or inhibiting the activity of members of the c-Abl family, their gene-fusion products (e.g. BCR-Abl kinase) and mutants, such as compounds which target decrease or inhibit the activity of c-Abl family members and their gene fusion products, e.g. a N-phenyl-2-pyrimidine-amine derivative, e.g. imatinib or nilotinib (AMN107); PD180970; AG957; NSC 680410; PD173955 from ParkeDavis; or dasatinib (BMS-354825)
j) compounds targeting, decreasing or inhibiting the activity of members of the protein kinase C (PKC) and Raf family of serine/threonine kinases, members of the MEK, SRC, JAK, FAK, PDK1, PKB/Akt, and Ras/MAPK family members, and/or members of the cyclin-dependent kinase family (CDK) and are especially those staurosporine derivatives disclosed in US 5,093,330, e.g. midostaurin; examples of further compounds include e.g. UCN-01, safingol, BAY 43-9006, Bryostatin 1, Perifosine; limofosine; RO 318220 and RO 320432; GO 6976; Isis 3521; LY333531/LY379196; isochinoline compounds such as those disclosed in WO 00/09495; FTIs; PD184352 or QAN697 (a P13K inhibitor) or AT7519 (CDK inhibitor);
k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}-benzoic acid adamantyl ester; NSC 680410, adaphostin);
l) compounds targeting, decreasing or inhibiting the activity of the epidermal growth factor family of receptor tyrosine kinases (EGFR, ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180); e.g. trastuzumab (Herceptin™), cetuximab (Erbitux™), Iressa, Tarceva, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 03/013541; and
m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF.

Further anti-angiogenic compounds include compounds having another mechanism for their activity, e.g. unrelated to protein or lipid kinase inhibition e.g. thalidomide (THALOMID) and TNP-470.

Compounds which target, decrease or inhibit the activity of a protein or lipid phosphatase are e.g. inhibitors of phosphatase 1, phosphatase 2A, or CDC25, e.g. okadaic acid or a derivative thereof.

Compounds which induce cell differentiation processes are e.g. retinoic acid, α- γ- or δ-tocopherol or α- γ- or δ-tocotrienol.

The term cyclooxygenase inhibitor as used herein includes, but is not limited to, e.g. Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g. 5-methyl-2-(2'-chloro-6'-fluoroanilino)phenyl acetic acid, lumiracoxib.

The term "bisphosphonates" as used herein includes, but is not limited to, etridonic, clodronic, tiludronic, pamidronic, alendronic, ibandronic, risedronic and zoledronic acid. "Etridonic acid" can be administered, e.g., in the form as it is marketed, e.g. under the trademark DIDRONEL. "Clodronic acid" can be administered, e.g., in the form as it is marketed, e.g. under the trademark BONEFOS. "Tiludronic acid" can be administered, e.g., in the form as it is marketed, e.g. under the trademark SKELID. "Pamidronic acid" can be administered, e.g. in the form as it is marketed, e.g. under the trademark AREDIA™. "Alendronic acid" can be administered, e.g., in the form as it is marketed, e.g. under the trademark FOSAMAX. "Ibandronic acid" can be administered, e.g., in the form as it is marketed, e.g. under the trademark BONDRANAT. "Risedronic acid" can be administered, e.g., in the form as it is marketed, e.g. under the trademark ACTONEL. "Zoledronic acid" can be administered, e.g. in the form as it is marketed, e.g. under the trademark ZOMETA.

The term "mTOR inhibitors" relates to compounds which inhibit the mammalian target of rapamycin (mTOR) and which possess antiproliferative activity such as sirolimus (Rapamune®), everolimus (CerticanT™), CCI-779 and ABT578.

The term "heparanase inhibitor" as used herein refers to compounds which target, decrease or inhibit heparin sulfate degradation. The term includes, but is not limited to, PI-88.

The term " biological response modifier" as used herein refers to a lymphokine or interferons, e.g. interferon γ.

The term "inhibitor of Ras oncogenic isoforms", e.g. H-Ras, K-Ras, or N-Ras, as used herein refers to compounds which target, decrease or inhibit the oncogenic activity of Ras e.g. a "farnesyl transferase inhibitor" e.g. L-744832, DK8G557 or R115777 (Zarnestra).

The term "telomerase inhibitor" as used herein refers to compounds which target, decrease or inhibit the activity of telomerase. Compounds which target, decrease or inhibit the activity of telomerase are especially compounds which inhibit the telomerase receptor, e.g. telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase are e.g. bengamide or a derivative thereof.

The term "proteasome inhibitor" as used herein refers to compounds which target, decrease or inhibit the activity of the proteasome. Compounds which target, decrease or inhibit the activity of the proteasome include e.g. Bortezomid (Velcade™) and MLN 341.

The term "matrix metalloproteinase inhibitor" or ("MMP" inhibitor) as used herein includes, but is not limited to, collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat. (AG3340), metastat (NSC 683551) BMS-279251, BAY 12-9566, TAA211, MM1270B or AAJ996.

The term "compounds used in the treatment of hematologic malignancies" as used herein includes. but is not limited to, FMS-like tyrosine kinase inhibitors e.g. compounds targeting, decreasing or inhibiting the activity of FMS-like tyrosine kinase receptors (Flt-3R); interferon, 1-b-D-arabinofuransylcytosine (ara-c) and bisulfan; and ALK inhibitors e.g. compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, e.g. PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90 e.g., 17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to, trastuzumab (Herceptin™), Trastuzumab-DM1,erbitux, bevacizumab (Avastin™), rituximab (Rituxan^{®}), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), compounds of formula (I) can be used in combination with standard leukemia therapies, especially in combination with therapies used for the treatment of AML. In particular, compounds of formula (I) can be administered in combination with, e.g., farnesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

The term "antileukemic compounds" includes, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065, in particular, *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof and *N*-hydroxy-3-[4-[(2-hydroxyethyl){2-(1*H*-indol-3-yl)ethyl]-amino]methyl]phenyl]-2*E*-2-propenamide, or a pharmaceutically acceptable salt thereof, especially the lactate salt. Somatostatin receptor antagonists as used herein refers to compounds which target, treat or inhibit the somatostatin receptor such as octreotide, and SOM230 (pasireotide).

Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1, pp. 248-275 (1993).

The term "EDG binders" as used herein refers a class of immunosuppressants that modulates lymphocyte recirculation, such as FTY720.

The term "ribonucleotide reductase inhibitors" refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentostatin. Ribonucleotide reductase inhibitors are especially hydroxyurea or 2-hydroxy-1*H*-isoindole-1,3-dione derivatives, such as PL-1, PL-2, PL-3, PL-4, PL-5, PL-6, PL-7 or PL-8 mentioned in Nandy et al., Acta Oncologica, Vol. 33, No. 8, pp. 953-961 (1994).

The term "S-adenosylmethionine decarboxylase inhibitors" as used herein includes, but is not limited to the compounds disclosed in US 5,461,076.

Also included are in particular those compounds, proteins or monoclonal antibodies of VEGF disclosed in WO 98/35958, e.g. 1-(4-chloroanilino)-4-(4-pyridylmethyl)phthalazine or a pharmaceutically acceptable salt thereof, e.g. the succinate, or in WO 00/09495, WO 00/27820, WO 00/59509, WO 98111223, WO 00/27819 and EP 0 769 947; those as described by Prewett et al, Cancer Res, Vol. 59, pp. 5209-5218 (1999); Yuan et al., Proc Natl Acad Sci U S A, Vol. 93, pp. 14765-14770 (1996); Zhu et al., Cancer Res, Vol. 58, pp. 3209-3214 (1998); and Mordenti et al., Toxicol Pathol, Vol. 27, No. 1, pp. 14-21 (1999); in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g. rhuMAb and RHUFab, VEGF aptamer e.g. Macugon; FLT-4 inhibitors, FLT-3 inhibitors, VEGFR-2 IgG1 antibody, Angiozyme (RPI 4610) and Bevacizumab (Avastin™).

Photodynamic therapy as used herein refers to therapy which uses certain chemicals known as photosensitizing compounds to treat or prevent cancers. Examples of photodynamic therapy includes treatment with compounds, such as e.g. VISUDYNE and porfimer sodium. Angiostatic steroids as used herein refers to compounds which block or inhibit angiogenesis, such as, e.g., anecortave, triamcinolone. hydrocortisone, 11-α-epihydrocotisol, cortexolone, 17α-hydroxyprogesterone, corticosterone, desoxycorticosterone, testosterone, estrone and dexamethasone.

Implants containing corticosteroids refers to compounds, such as e.g. fluocinolone, dexamethasone.

"Other chemotherapeutic compounds" include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The compounds of the invention are also useful as co-therapeutic compounds for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/035668, WO 03/048181, WO 03/062259, WO 03/064445, WO 03/072592, non-steroidal glucocorticoid receptor agonists such as those described in WO 00/00531, WO 02/10143, WO 03/082280, WO 03/082787, WO 03/104195, WO 04/005229; LTB4 antagonists such LY293111, CGS025019C, CP-195543, SC-53228, BIIL 284, ONO 4057, SB 209247 and those described in US 5451700; LTD4 antagonists such as montelukast and zafirlukast; PDE4 inhibitors such cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelClD(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/ 018431, WO 04/018449, WO 04/018450, WO 04/018451, WO 04/018457, WO 04/018465, WO 04/019944, WO 04/019945, WO 04/045607 and WO 04/037805; A2a agonists such as those disclosed in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/ 039762, WO 04/039766, WO 04/045618 and WO 04/046083; A2b antagonists such as those described in WO 02/42298; and beta-2 adrenoceptor agonists such as albuterol (salbutamol), metaproterenol, terbutaline, salmeterol fenoterol, procaterol, and especially, formoterol and pharmaceutically acceptable salts thereof, and compounds (in free or salt or solvate form) of formula I of WO 0075114, which document is incorporated herein by reference, preferably compounds of the Examples thereof, especially a compound of formula 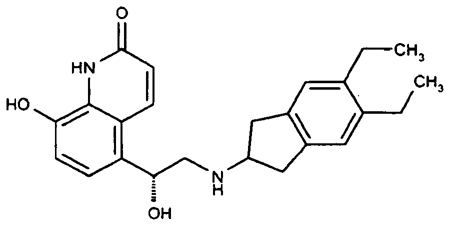 and pharmaceutically acceptable salts thereof, as well as compounds (in free or salt or solvate form) of formula I of WO 04/16601, and also compounds of WO 04/033412.

Suitable bronchodilatory drugs include anticholinergic or antimuscarinic compounds, in particular ipratropium bromide, oxitropium bromide, tiotropium salts and CHF 4226 (Chiesi), and glycopyrrolate, but also those described in WO 01/04118, WO 02/51841, WO 02/53564, WO 03/00840, WO 03/87094, WO 04/05285, WO 02/00652, WO 03/53966, EP 424021, US 5171744, US 3714357, WO 03/33495 and WO 04/018422.

Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in WO 03/099807, WO 04/026841 and JP 2004107299.

Other useful combinations of compounds of the invention with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzo-cyclohepten-8-yl]carbonyl]aminojphenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.

The structure of the active compounds identified by code nos., generic or trade names may be taken from the actual edition of the standard compendium "The Merck Index" or from databases, e.g. Patents International (e.g. IMS World Publications).

The above-mentioned compounds, which can be used in combination with a compound of the formula (I), can be prepared and administered as described in the art, such as in the documents cited above.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

The invention also provides a pharmaceutical preparation, comprising a compound of formula I as defined herein, or an N-oxide or a tautomer thereof, or a pharmaceutically acceptable salt of such a compound, or a hydrate or solvate thereof, and at least one pharmaceutically acceptable carrier.

A compound of formula I can be administered alone or in combination with one or more other therapeutic compounds, possible combination therapy taking the form of fixed combinations or the administration of a compound of the invention and one or more other therapeutic (including prophylactic) compounds being staggered or given independently of one another, or the combined administration of fixed combinations and one or more other therapeutic compounds. A compound of formula I can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The dose of a compound of the formula I or a pharmaceutically acceptable salt thereof to be administered to warm-blooded animals, for example humans of approximately 70 kg body weight, is preferably from approximately 3 mg to approximately 5 g, more preferably from approximately 10 mg to approximately 1.5 g per person per day, divided preferably into 1 to 3 single doses which may, for example, be of the same size. Usually, children receive half of the adult dose.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the invention in association with at least one pharmaceutical acceptable carrier or diluent may be manufactured in conventional manner by mixing with a pharmaceutically acceptable carrier or diluent.

The invention relates also to pharmaceutical compositions comprising an effective amount, especially an amount effective in the treatment of one of the above-mentioned disorders, of a compound of formula I or an N-oxide or a tautomer thereof together with one or more pharmaceutically acceptable carriers that are suitable for topical, enteral, for example oral or rectal, or parenteral administration and that may be inorganic or organic, solid or liquid. There can be used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilisers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectionning, dissolving or lyophilising processes, and comprises approximately from 1% to 99%, especially from approxtmatety 1% to approximately 20%, active ingredient(s).

Additionally, the present invention provides a compound of formula or an N-oxide or a tautomer thereof, or a pharmaceutically acceptable salt of such a compound, for use in a method for the treatment of the human, or animal body, especially for the treatment of a disease mentioned herein, most especially in a patient requiring such treatment.

The present invention also relates to the use of a compound of formula I or a tautomer thereof, or a pharmaceutically acceptable salt of such a compound, for the preparation of a medicament for the treatment of a proliferative disease, an inflammatory disease, or an obstructive airway disease, or disorders commonly occurring in connection with transplantation.

The compounds are provided to be useful in a method for the treatment of a proliferative, disease which responds to an inhibition of lipid kinases and/or P13-kinase-related protein kinases, in particular the PI3 kinase, and/or mTOR, and/or DNA protein kinase activity, which comprises administering a compound of formula I or a pharmaceutically acceptable salt thereof, wherein the radicals and symbols have the meanings as defined above, especially in a quantity effective against said disease, to a warm-blooded animal requiring such treatment.

Furthermore, the invention relates to a pharmaceutical composition for treatment of solid or liquid tumours in warm-btoodedanimats, including humans, comprising an antitumor effective dose of a compound of the formulae I as described above or a pharmaceutically acceptable salt of such a compound together with a pharmaceutical carrier.

### Manufacturing Process:

The invention relates also to a process for the manufacture of a compound of the formula I, an N-oxide thereof, a tautomer thereof and/or a salt thereof.

Compounds of the formula I. can be prepared according to or in analogy to methods that,
especially and according to the invention by a process comprising
a) for the manufacture of a compound of the formula I wherein R⁴ is bound to the central quinazoline moiety in formula I via a carbon atom, reacting a compound of the formula IIA, 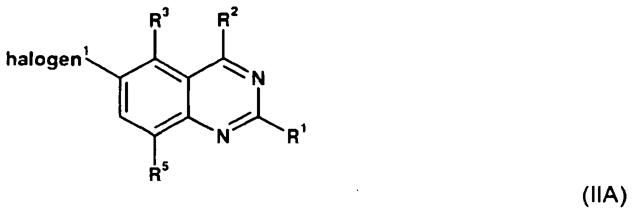 wherein R¹, R², R³ and R⁵ are as defined for a compound of the formula I and wherein halogen¹ is halo, preferably chloro, bromo or iodo, or is trifluoromethansulfonyloxy, under cross-coupling conditions with a boronic acid or boronic acid ester of the formula III,

   R⁴-D (III)

   wherein R⁴ is as defined for a compound of the formula I and is bound via a carbon atom to D and D is -B(OH₂) or a group of the formula A, 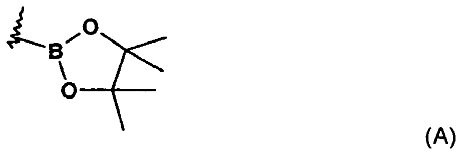 or
b) for the manufacture of a compound of the formula I wherein R² is bound to the central quinazoline moiety in formula I via a carbon atom, reacting a compound of the formula IIB, 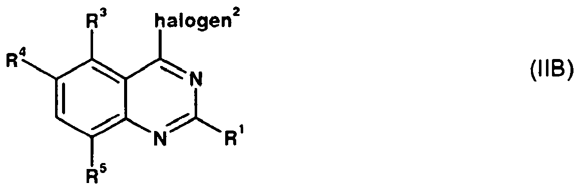 wherein R¹, R³, R⁴ and R⁵ are as defined for a compound of the formula I and halogen² is halo, preferably chloro, bromo or iodo, or is trifluoromethansulfonyloxy, under cross-coupling conditions with a boronic acid or boronic acid ester of the formula IV,

   R²-D (IV)

   wherein R² is as defined for a compound of the formula I and is bound via a carbon atom to D and D is -B(OH₂) or a group of the formula A given above;
   or
c) for the manufacture of a compound of the formula I wherein R² and R⁴ are identical and are bound to the central quinazoline moiety in formula I via a carbon atom, reacting a compound of the formula IIC, 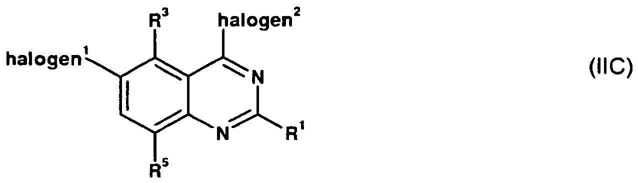 wherein R¹, R³ and R⁵ are as defined for a compound of the formula I and halogen¹ and halogen² are, independently of each other, halo, preferably chloro, bromo or iodo, or is trifluoromethansulfonyloxy, with a boronic acid or boronic acid ester of the formula V,

   R^{2,4}-D (V)

   wherein R^{2,4} is a moiety R² or R⁴ bound via a carbon atom to D and is otherwise as defined for a compound of the formula I and D is -B(OH₂) or a group of the formula A given above;
   or
d) for the manufacture of a compound of the formula I wherein R¹ is amino, N-mono-C₁-C₁₀ (preferably C₁-C₄)-alkyl-amino or N-mono-C₃-C₁₀(preferably C₃-C₅)-cycloalkylamino, reacting a compound of the formula IID, 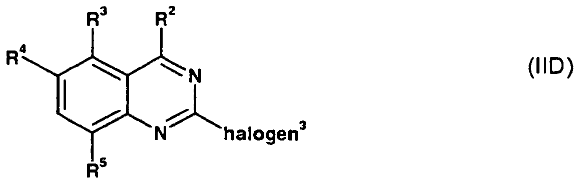 wherein R², R³, R⁴ and R⁵ are as defined for a compound of the formula I and wherein halogen³ is halo, preferably chloro, bromo or iodo, or is trifluoromethansulfonyloxy, with an amine of the formula VI

   R^{1*}-H (VI)

   wherein R^{1*} is amino, N-mono-C₁-C₁₀ (preferably C₁-C₄)-alkyl-amino or N-monO-C₃-C₁₀ (preferably C₃-C₅)-cycloalkylamino;
   or
e) for the manufacture of a compound of the formula I wherein R⁴ is heteroaryl with at least one ring nitrogen and is bound to the central quinazoline moiety in formula I via a nitrogen atom, reacting a compound of the formula IIA given above under a) with a compound of the formula VII,

   R^{4*}-H (VII)

   wherein R^{4*} is a nitrogen containing heteroaryl with at least one ring nitrogen and is bound to the hydrogen in formula VII via a nitrogen atom, under substitution conditions;
   or
f) for the manufacture of a compound of the formula I wherein R² is heteroaryl with at least one ring nitrogen and is bound to the central quinazoline moiety in formula I via a nitrogen atom, reacting a compound of the formula IIB given above under b) with a compound of the formula VIII,

   R^{2*}-H (VIII)

   wherein R^{6*} is a nitrogen containing heteroaryl with at least one ring nitrogen and is bound to the hydrogen in formula VIII via a nitrogen atom, under substitution conditions;
   or
g) for the manufacture of a compound of the formula I wherein R² and R⁴ are identical and are heteroaryl with at least one ring nitrogen and each of them is bound to the central quinazoline moiety in formula I via a nitrogen atom, reacting a compound of the formula IX,

   R^{2,4*}-H (IX)

   wherein R^{2,4*} is heteroaryl with at least one nitrogen atom and wherein R^{2,4*} is a moiety R² or R⁴ bound via a nitrogen atom to the hydrogen shown in formula IX and is otherwise as defined for a compound of the formula I, under substitution conditions with a compound of the formula IIC mentioned above; or
h) for the manufacture of a compound of the formula I wherein R⁴ is bound to the central quinazoline moiety in formula I via a carbon atom, reacting a boronic acid or boronic acid ester compound of the formula IIA^{*}, 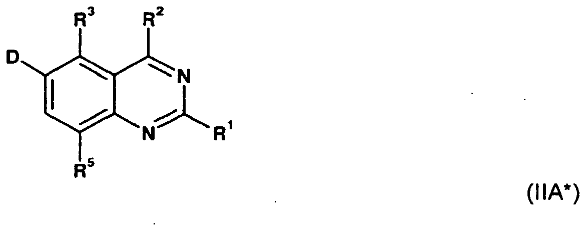 wherein R¹, R², R³ and R⁵ are as defined for a compound of the formula I and wherein D is -B(OH₂) or a group of the formula A, 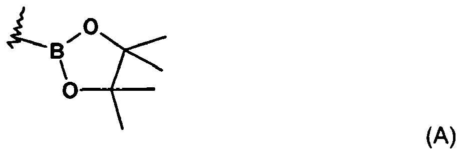 under cross coupling conditions with compound of the formula III*,

   R⁴-Hal (III*)

   wherein R⁴ is as defined for a compound of the formula I and is bound via a carbon atom to Hal and Hal is halo, preferably chloro, bromo or iodo, or is trifluoromethansulfonyloxy; where in any of the reactions represented under a) to h) functional groups in the starting materials can be present in protected form and in the obtainable compounds of the formula I carrying one or more protecting groups such protecting groups are removed;
   and, if desired, a compound of the formula I obtainable according to a process variant selected from a) to g) is converted into a different compound of the formula I, an obtainable salt of a compound of the formula I is converted into a different salt thereof, an obtainable free compound of the formula I is converted into a salt thereof, and/or an obtainable isomer of a compound of the formula I is separated from one or more different obtainable isomers of the formula I.

### Examples for preferred Reaction Conditions

In the following more detailed description of the processes, optional reactions and conversions, synthesis of starting materials and intermediates and the like, R¹, R², R³, R⁴ and R⁵ have the meanings given for a compound of the formula I or the compound mentioned specifically, while D is as defined for a compound of the formula (A), halogen¹ as for a compound of the formula IIA, halogen² as for a compound of the formula IIB, R^{2,4} as for a compound of the formula IV, R^{1*} as for a compound of the formula V, R^{4*} as for a compound of the formula VI, R^{2*} as for a compound of the formula VII, R^{2,4*} as for a compound of the formula VIII, Hal as for compound III*, in each case if not indicated otherwise, respectively.

Where useful or required, the reactions can take place under an inert gas, such as nitrogen or argon.

The reaction given under process variants a), b), c) and h), respectively, is preferably carried out under the conditions of a Suzuki-reaction, preferably in a mixture of a polar aprotic solvent, such as dimethylformamide (DMF) and water in the presence of a catalyst for the cross-coupling, especially a noble metal catalyst, preferably a palladium catalyst, such as palladium(II) complex, for example bis(triphenylphosphine)palladium (II) dichloride, in the presence of a base, such as potassium carbonate, sodium hydroxide or sodium carbonate, at a preferred temperature in the range from 80 °C to 130 °C; or according to a another preferred method in a cyclic ether solvent, e.g. tetrahydrofurane, in the presence of a catalyst for the cross coupling, especially a noble metal catalyst, preferably a palladium (0) complex, for example tris(dibenzylideneacetone)-dipalladium(0), in the presence of an appropriate ligand, such as 2-dicyclohexylphosphino-2',6'-dimethoxybiphenyl (SPhos), at a preferred temperature in the range from 80 to 150 °C; if required conducting the reaction in a sealed vessel (e.g. a seal reactor) if the boiling point of the reaction mixture is exceeded and especially if (as is a preferred embodiment) the heating is effected by microwave excitation.

The reaction conditions for process variants d), e), f) and g) (substitution) are preferably chosen from customary conditions of a nucleophilic aromatic substitution, e.g. carrying out the reaction, preferably in a sealed vessel (e.g. a seal reaction), in a polar solvent, such as an alcohol, e.g. ethanol, or an aprotic solvent, such as 1-methyl-2-pyrrolidone, preferably at a temperature in the range from 120 to 180 °C; preferably, the energy for heating is provided by microwave excitation.

### Protecting groups

If one or more other functional groups, for example carboxy, hydroxy, amino, or mercapto, are or need to be protected in a starting material, e.g. in any one or more starting materials of the formula IIA, IIA*, IIB, IIC, IID, III, III* IV, V, VI, VII, VIII or IX, because they should not take part in the reaction or disturb the reaction, these are such groups as are usually used in the synthesis of peptide compounds, and also of cephalosporins and penicillins, as well as nucleic acid derivatives and sugars. Protecting groups are such groups that are no longer present in the final compounds once they are removed, while groups that remain as substitutents are not protecting groups in the sense used here which is groups that are added at a certain intermediate stage and removed to obtain a final compound. For example, tert-butoxy if remaining in a compound of the formula I is a substituent, while if it is removed to obtain the final compound of the formula I it is a protecting group.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by acetolysis, protonolysis, solvolysis, reduction, photolysis or also by enzyme activity, for example under conditions analogous to physiological conditions, and that they are not present in the end-products. The specialist knows, or can easily establish, which protecting groups are suitable with the reactions mentioned above and below.

The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/I, Georg Thieme Verlag, Stuttgart 1974, in H.-D. Jakubke and H. Jescheit, "Aminosiuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag. Stuttgart 1974.

### Optional Reactions and Conversions

A compound of the formula I may be converted into a different compounds of the formula I.

For example, in a compound of the formula I wherein the substituent R¹, R² or R⁴ comprises an esterified carboxy group, such as C₁-C₇₋alkoxycarbonyl, this esterified carboxy group may be hydrolysed to give the corresponding free carboxy group, e.g. in the presence of a base, such as an alkali metal hydroxide, e.g. lithium hydroxide, in an appropriate solvent, e.g. a cyclic ether, such as dioxane, water or a mixture thereof, e.g. at temperatures in the range from 0 to 50 °C.

In a compound of the formula I wherein the substituent R¹, R or R⁴ comprises free carboxy group (e.g. obtainable by a preceding step as described in the last paragraph), this free carboxy group may be converted into a corresponding carbamoyl or N-mono or N,N-di-substituted carbamoyl group, e.g. by reaction with ammonia, N-mono- or N,N-di-(C₁-C₇-alkyl and/or phenyl-C₁-C₇-alkyl)-amine, piperidine, piperazine, 4-C₁-C₇-alkyl-piperazine, morpholine, thiomorpholine, S-oxo-thiomorpholine or S,S-dioxothiomorpholine; the reaction preferably takes place with the carboxy group in active form, more preferably under customary condensation conditions, where among the possible reactive derivatives of a carboxy group reactive esters (such as the hydroxybenzotriazole (HOBT), pentafluorophenyl, 4-nitrophenyl or N-hydroxysuccinimide ester), acid halogenides (such as the acid chloride or bromide) or reactive anhydrides (such as mixed anhydrides with lower alkanoic acids or symmetric anhydrides) are preferred. Reactive carbonic acid derivatives can preferably be formed *in situ.* The reaction is carried out by dissolving the corresponding compounds of the formula I carrying one or more carboxy substituents in a suitable solvent, for example a halogenated hydrocarbon, such as methylene chloride, *N,N*-dimethylformamide, *N,N*-dimethylacetamide, *N*-methyl-2-pyrrolidone, 4-(N,N-dimethylamino)-pyridine or acetonitrile, or a mixture of two or more such solvents, and by the addition of a suitable base, for example triethylamine, diisopropylethylamine (DIPEA) or *N*-methylmorpholine and, if the reactive derivative of the carboxyl substituent(s) is formed *in situ,* a suitable coupling agent that forms a preferred reactive derivative of the carboxy group *in situ,* for example dicydohexylcarbodiimide/1-hydroxybenzotriazole (DCC/ HOBT); bis(2-oxo-3-oxazolidinyl)phosphinic chloride (BOPCI); O-(1,2-dihydro-2-oxo-1-pyridyl)-*N,N,N',N'*-tetramethyluronium tetrafluoroborate (TPTU); O-benzotriazol-1-yl)-N,N,N',N'-tetramethyluronium tetrafluoroborate (TBTU); (benzotriazol-1-yloxy)-tripyrrolidinophosphonium-hexafluorophosphate (PyBOP), O-(1H-6-chlorobenzotriazoie-1-yl)-1,1,3.3-tetramethyluronium hexafluorophosphate, 1-(3-dimethylaminopropyl)-3-ethylcarbodiimide hydrochloride/hydroxybenzotriazole or/1-hydroxy-7-azabenzotriazole (EDC/HOBT or EDC/HOAt) or HOAt alone, or with (1-chloro-2-methyl-propenyl)-dimethylamine. For review of some other possible coupling agents, see e.g. Klauser, Bodansky, Synthesis (1972), 453-463. The reaction mixture is preferably stirred at a temperature of between approximately -20 and 50 °C, especially between 0 °C and 30 °C, e.g. at room temperature.

A nitrogen ring atom of the quinazole core or a nitrogen-containing heterocyclic (e.g. heteroaryl) substituent can form an N-oxide in the presence of a suitable oxidizing agent, e.g. a peroxide, such as m-chloro-perbenzoic acid or hydrogen peroxide.

Other reactions can be carried out as described, or in analogy to those mentioned, in the Examples.

Also in the optional process steps, carried out "if desired", functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

Salts of a compound of formula I with a salt-forming group may be prepared in a manner known *per se*. Acid addition salts of compounds of formula I may thus be obtained by treatment with an acid or with a suitable anion exchange reagent. A salt with two acid molecules (for example a dihalogenide of a compound of formula I) may also be converted into a salt with one acid molecule per compound (for example a monohalogenide); this may be done by heating to a melt, or for example by heating as a solid under a high vacuum at elevated temperature, for example from 130 to 170°C, one molecule of the acid being expelled per molecule of a compound of formula I.

Salts can usually be converted to free compounds, e.g. by treating with suitable basic compounds, for example with alkali metal carbonates, alkali metal hydrogencarbonates, or alkali metal hydroxides, typically potassium carbonate or sodium hydroxide.

Stereoisomeric mixtures, e.g. mixtures of diastereomers, can be separated into their corresponding isomers in a manner known *per se* by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

It should be emphasized that reactions analogous to the conversions mentioned in this chapter may also take place at the level of appropriate intermediates (and are thus useful in the preparation of corresponding starting materials).

### Starting materials:

The starting materials of the formulae IIA, IIA*, IIB, IIC, IID, III, III*, IV, V, VI, VII, VIII or IX, as well as other starting materials mentioned herein, e.g. below, can be prepared according to or in analogy to methods that are known in the art, are known in the art and/or are commercially available. Novel starting materials, as well as processes for the preparation thereof, are likewise an embodiment of the present invention. In the preferred embodiments, such starting materials are used and the reaction chosen are selected so as to enable the preferred compounds to be obtained.

For example, a compound of the formula IIA, IIB or IIC (where in the latter R¹ is amino or mono- or disubstituted amino as described for R¹ above) can be prepared from a compound of the formula X, 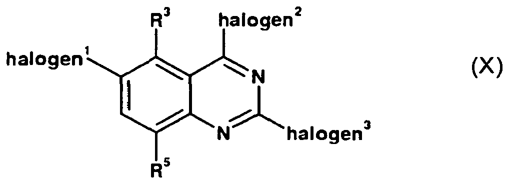 wherein R³ and R⁵ are as defined under formula I and halogen¹, halogen² and halogen³ are independently selected from halo, especially chloro, bromo or iodo, and from trifluoromethansulfonyloxy, by reacting it, in order to introduce C-bonded aryl or heteroaryl moieties, with a compound of formula III or IV in a cross-coupling (e.g. Suzuki) reaction, respectively, under preferred conditions as described above for the reaction variants a), b) or c) involving halogen¹ or halogen², respectively, or for the introduction of N-bound aryl or heteroaryl with a compound of the formula VII, VIII or IX or in the case of a compound of the formula IIC with a compound of the formula VI, in order to introduce the corresponding moiety R¹ other than hydrogen, in a nucleophilic aromatic substitution involving halogen¹ halogen² or halogen³, respectively, in each case preferably under the reaction described as preferred for reaction variants e), f), g) or d) mentioned above, respectively; which can take place in a sequential manner with the regio-selectivity being controlled by the reactivity of the respective halogen according to the used reaction conditions. The nature of halogen¹, halogen² and halogen³ are chosen such as to allow a certain level of selectivity for the given reaction to be performed with the chosen conditions, preferentially as described for the synthesis of a compound of the formula IIA, IIB or IID. Two sequential Suzuki-reactions (as well as nucleophilic amination reactions) can be performed independently or in one-pot without isolation of the first reaction product.

For example, a compound of formula IIA or IIB, wherein R¹ is hydrogen and R³ and R⁵ have the meanings as given under formula I, can be prepared from compound of the formula XI, 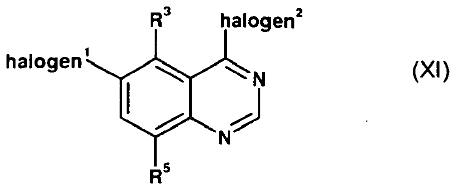 (which is also a compound of the formula IIC wherein R¹ is hydrogen which thus can be obtained as illustrated below for the compound of the formula XI)
wherein R⁵ is as defined for a compound of the formula I and halogen¹ and halogen² are as defined for a compound of the formula X, by reacting with compound of formula III or IV, respectively, in a cross-coupling (preferably Suzuki) reaction involving halogen¹ and halogen², as described above under process variants a) or b), respectively, or with a compound of the formula VII or VIII under substitution conditions, preferably conditions as described under process variants e) and f) mentioned above.

A compound of the formula X or XI, wherein R³ and R⁵ have the meanings as given under formula I, is prepared by hydroxyl to halogeno exchange with suitable halogenation reagent, such as phosphoroxychloride, in the absence or presence of an appropriate tertiary nitrogen base, e.g. diethylaniline, at preferred temperatures between 100°C and 140°C from the tautomeric carbonyl precursor of formula XII or XIII, respectively:

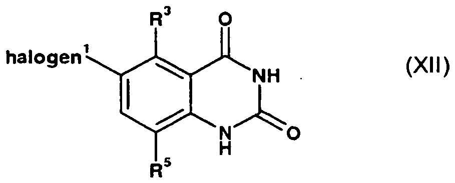

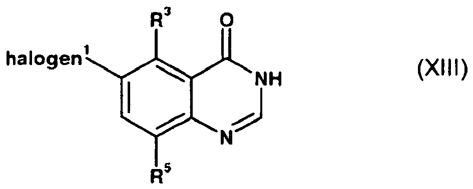

Alternatively, introduction of R⁴ substituent by cross-coupling (preferably Suzuki-) reaction with a compound of the formula III mentioned above (preferably under reaction conditions as described under process variants a) above) or nucleophilic substitution with a compound of the formula VII mentioned above (preferably under reaction conditions as described for process variant e) mentioned above) is carried out on an intermediate of the formula XII or XIII, followed by activation of the carbonyl intermediate to the halo intermediate, respectively, of formula XIV, 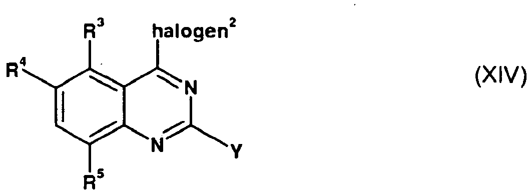 wherein R³, R⁴ and R⁵ have the meanings as given under formula I and Y is halogen or H.

This, if Y is hydrogen, is also an intermediate of the formula IIB wherein R¹ is hydrogen.

From the compound of the formula XIV, if Y is halogen, a starting material of the formula IID wherein halogen³ is halo is obtainable by cross-coupling (preferably under Suzuki conditions as described above for process variant b) it with a compound of the formula IV mentioned above or by nucleophilic substitution with a compound of the formula VIII (preferably under process conditions as described for process variant f) above) is accessible. The corresponding trifluoromethansulfonyl halogen³ can be obtained from this compound by nucleophilic substitution or by other methods.

The bicyclic intermediates of the formulae XII and XIII can be obtained from the anthranilic type derivative of formula XV, 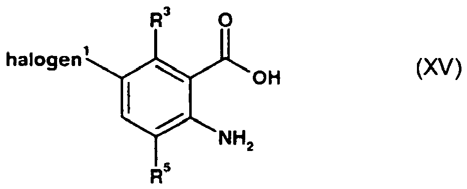 wherein R³ and R⁵ have the meanings as given under formula I, using neat urea (that is, a melt in urea) at a temperature between 130°C and 160°C or neat formamide at a preferred temperature between 130°C and 180°C.

An anthranilic intermediate of the formula XVI, 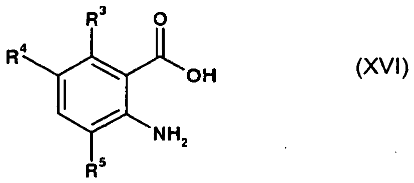 can be converted in the same manner to a compound of the formula XIV, and substituent R⁴ is introduced prior to formation of the bicycle using a cross-coupling reaction with a compound of the formula III given above (specially Suzuki-reaction under conditions as for process variant a) described above) or nucleophilic substitution with a compound of the formula VII given above, especially under reaction conditions as described above for process variant e).

A compound of the formula IIC wherein R¹ is amino, N-mono-C₁-C₁₀ (preferably C₁-C₄)-alkylamino or N-mono-C₃-C₁₀(preferably C₃-C₅)-cycloalkylamino as defined for a compound of the formula I can be obtained from a compound of the formula X given above by nucleophilic replacement with a compound of the formula VI wherein R¹* is as defined under process variant d) and preferably the reaction conditions described for it.

Compounds of the formula IIA* can be prepared from corresponding compounds of the formula IIA by replacing halogen¹¹ with the boronic or boronic ester group under conditions known in the art.

All remaining starting materials such as starting materials of the formula XII and III* are known, capable of being prepared according to known processes, or commercially obtainable; in particular, they can be prepared using processes as described or in analogy to those described in the Examples.

The following Examples serve to illustrate the invention without limiting its scope.

Temperatures are measured in degrees Celsius (°C). Unless otherwise indicated, the reactions take place at room temperature (rt).

Ratios of solvents (e.g. in eluents or solvent mixtures) are given in volume by volume (v/v). HPLC linear gradient between A = H₂O/TFA 1000:1 and B = acetonitrile/TFA 1000:1 Grad 1: 2-100 % Bin 4.5 min and 1 min at 100 % B; column: Chromolith Performance 100 mm x 4.5 mm (Merck, Darmstadt, Germany); flow rate 2 ml/min. Detection at 215 nM.

The following further abbreviations are used:
- Ac: acetyl
- brine: (at rt) saturated sodium chloride solution
- Celite: Celite®, filtering aid based on diatomaceous earth (Celite Corp., Lompoc, USA)
- DMA: N,N-dimethylacetamide
- DMAP: 4-dimethylaminopyridine
- ES-MS: Electrospray Mass Spectrometry
- Et: ethyl
- HPLC: High Performance Liquid Chromatography
- Isolute: Isolute® (Biotage AB, Uppsala, Sweden)
- JACS: Journal of the American Chemical Society
- LC-MS: Liquid Chromatography-Mass Spectrometry
- Me: methyl
- min: minute(s)
- NMP: 1-methyl-2-pyrrolidone
- NMR: Nuclear Magnetic Resonance
- Phe: phenyl
- PrOH: n-propanol
- RP-MPLC: Reversed-Phase Medium-Pressure Liquid Chromatography
- TFA: trifluoroacetic acid
- SPhos: 2-dicyclohexylphosphino-2',6'-dimethoxybiphenyl
- THF: tetrahydrofurane
- TPTU: O-(1,2-dihydro-2-oxo-1-pyridyl)-N,N,N',N'-tetramethyluroniumtetrafluoroborate
- tᵣₑₜ: retention time

### Example 1

### 4-(3,4-Dimethoxy-phenyl)-6-(6-piperazin-1-yl-pyridin-3-yl)-quinazoline

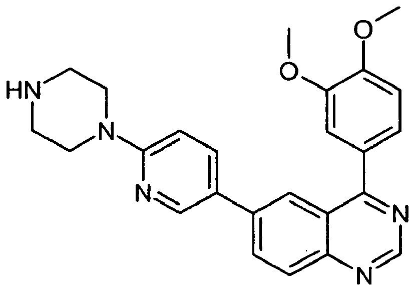

113 mg (0.214 mmol) of 4-{5-[4-(3,4-dimethoxy-phenyl)-quinazolin-6-yl]-pyridin-2-yl}-piperazine-1-carboxylic acid tert-butyl ester (Example **1a**) and 2 ml of TFA-H₂O (19:1) are stirred for 20 min. After this time, the reaction mixture is purified by preparative HPLC (H₂O/CH₃CN and 0.1% TFA). The pure fractions are basified with NaHCO₃, concentrated and extracted with EtOAc (2x). The organic layers are washed with brine, dried over Na₂SO₄, filtered and evaporated to provide the title compound as a yellow solid. ES-MS: 428 (M+H)⁺; analytical HPLC: tᵣₑₜ.= 52 min (Grad 1),

The starting materials are prepared as follows:

### Example 1a

### 4-{5-[4-(3,4-Dimethoxy-phenyl)-quinazolin-6-yl]-pyrldin-2-yl}-piperazine-1-carboxylic acid tert-butyl ester (which is also a compound of the formulae I according to the invention)

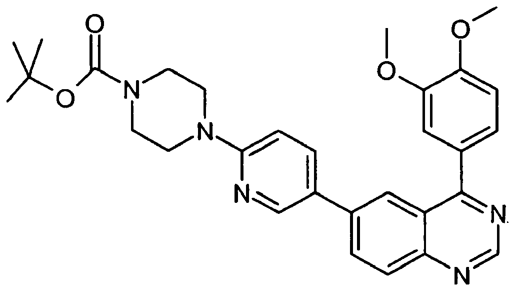

To 105 mg (0.41 mmol) of 6-bromo-4-chloro-quinazoline (Example **1c**), 18 mg (0.025 mmol) of bis(triphenylphosphine)palladium (II) dichloride (Fluka, Buchs, Switzerland) and 75 mg (0.41mmol) of 3,4-dimethoxyphenylboronic acid (Frontier Scientific, Logan, USA; **B1**) in 4 ml DMF under argon, 1 ml of a 1 M aqueous solution of K₂CO₃ is added. The mixture is stirred for 20 min at 105°C (oil bath). LC-MS confirms the formation of desired intermediate 6-bromo-4-(3,4-dimethoxy-phenyl)-quinazoline (Example **1b**). Then 192 mg (0.492) of 4-[5-(4,4,5,5-tetramethyl-[1,3,2]dioxaborolan-2-yl)-pyridin-2-yl]-piperazine-1-carboxylic acid tert-butyl ester (CB Research & Development, New Castle, USA; **B2**), 18 mg (0.025 mmol) of bis(triphenylphosphine)palladium (II) dichloride and 1 ml of a 1 M aqueous solution of K₂CO₃ are added. The reaction mixture is stirred for 1.5 h at 105'C under argon. After this time, the mixture is quenched with sat. aqueous NaHCO₃ and extracted with EtOAc (2x). The organic layer is washed with brine, dried over Na₂SO₄, filtered and evaporated *in vacuo.* The residue is purified by flash chromatography (CH₂Cl₂-MeOH 1:0 to 24:1) to give the title compound as a yellow solid. ES-MS: 528 (M + H)⁺; analytical HPLC: tᵣₑₜ. = 3.25 min (Grad 1).

### Example 1 b

### 6-Bromo-4-(3,4-dimethoxy-phenyl)-quinazoline

The intermediate compound in Example **1a** can also be synthesized in a separate batch and then be subjected to the second (the Suzuki) reaction in the one-pot synthesis in Example **1a**).

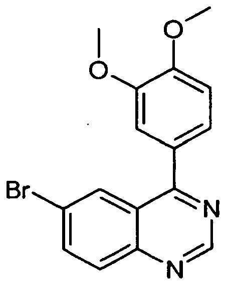

To 251 mg (1.03 mmol) of 6-bromo-4-chloro-quinazoline (Example **1c**), 44 mg (0.062 mmol) of bis(triphenylphosphine)palladium (II) dichloride (Fluka, Buchs, Switzerland) and 187 mg (1.03 mmol) of 3,4-dimethoxyphenylboronic acid **(B1)** in 10 ml DMF under argon, 2.6 ml of a 1 M aqueous solution of K₂CO₃ is added. The mixture is stirred for 20 min at 105°C (oil bath). After this time, the reaction mixture is quenched with sat. aqueous NaHCO₃ and extracted with EtOAc (2x). The organic layers are washed with water and brine, are dried over Na₂SO₄, filtered and evaporated *in vacuo.* The residue is purified by flash chromatography (CH₂Cl₂-Me0H 1:0 to 49:1) to give the title compound as a yellow solid. ES-MS: 345, 347 (M+H)+, Br pattern; analytical HPLC: tᵣₑₜ= 3.63 min (Grad 1).

### Example 1c

### 6-Bromo-4-chloro-quinazoline

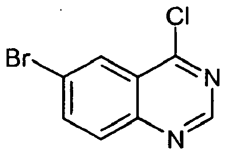

A mixture of 0.5 g (2.2 mmol) of 6-bromo-3H-quinazolin-4-one (Example **1d**), 0.7 ml (4.4 mmol) diethylaniline and 4 ml POCl₃ is stirred for 3 h at 125°C. After this time, the reaction mixture is cooled to rt and dropped into icy water. The precipitate is filtered and dried *in vacuo* overnight to give the title compound as a violet solid. Analytical HPLC: tᵣₑₜ= 3.51 min (Grad 1, partial hydrolysis in HPLC conditions); ¹H-NMR (CDCl₃): δ 9.08/s (1 H), 8.46/d (1 H), 8.06/dd (1H), 7.97/d (1H).

### Example 1d

**(following** F.R. Alexandre et al., Tetrahedron Lett., 2002, 43, p.3911**) 6-Bromo-3H-quinazolin-4-one**

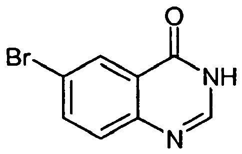

5 g (23 mmol) of 2-amino-5-bromobenzoic acid (Aldrich, Buchs, Switzerland) in 12 ml of formamide in a seal reactor are heated with microwave excitation for 1 h at 170°C. The reaction mixture is triturated with hot methanol and cooled at 4°C. The solid is filtered to give the title compound as an off-white solid. ES-MS: 225, 227 (M + H)⁺, Br pattern; analytical HPLC: tᵣₑₜ= 2.53 min (Grad 1).

### Example 2

### [4,6-Bis-(3,4-dimethoxy-phenyl)-quinazolin-2-yl]-n-propyl-amine

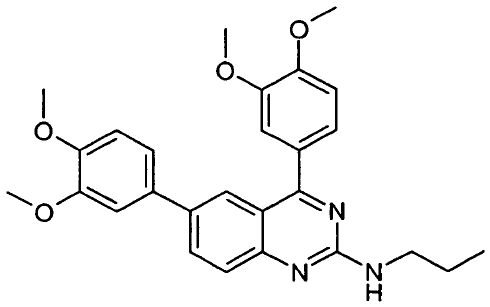

70 mg (0.16 mmol) of 2-chloro-4,6-bis-(3,4-dimethoxy-phenyl)-quinazoline (Example **2a**) and 47 mg (0.80 mmol) of n-propylamine (Aldrich, Buchs, Switzerland; **A1**) in 0.4 ml NMP are heated in a seal reactor with microwave excitation for 10 min at 150°C. After this time, the reaction mixture is diluted with 4 ml water and the precipitate is filtered over Celite. The solid is washed with water, and the solid is then dissolved in CH₂Cl₂, washed with brine, dried over Na₂SO₄, filtered and evaporated. The crude product is purified by preparative HPLC (H₂O/CH₂CN and 3% n-propanol). The pure fractions are concentrated and extracted with CH₂Cl₂ (2x) to provide the title compound as a yellow solid. ES-MS: 460 (M + H)⁺; analytical HPLC: tᵣₑₜ= 3.49 min (Grad 1).

The starting materials are prepared as follows:

### Example 2a

### 2-Chloro-4,6-bis(3,4-dimethoxy-phenyl)-quinazoline

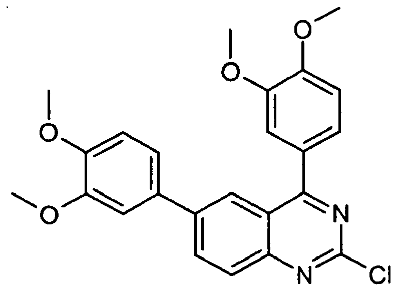

The title compound is obtained in a similar manner as in Example **1b** starting from 2,4-dichloro-6-(3,4-dimethoxy-phenyl)-quinazoline (Example **2b**); ES-MS: 437 (M + H)⁺, Cl pattern; analytical HPLC: tᵣₑₜ= 3.99 min (Grad 1).

### Example 2b

### 2,4-Dichloro-6-(3,4-dimethoxy-phenyl)-quinazoline

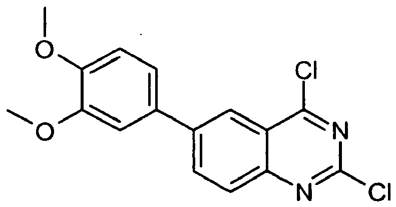

1.81 g (6.1 mmol) of 6-(3,4-dimethoxy-phenyl)-1H-quinazoline-2,4-dione (Example **2c**) in 20 ml POCl₃ is stirred for 6.5 h at 125°C. The reaction mixture is evaporated to dryness and then treated with chilly sat. aqueous NaHCO₃. The precipitate is filtered. The solid is dissolved in CH₂Cl₂, washed with chilly water, dried over MgSO₄, filtered and evaporated. The solid is triturated in CH₂Cl₂ and filtered off (2x). The combined filtrates are evaporated to dryness to yield the title compound as a yellow solid. ES-MS: 335 (M + H)⁺, 2Cl pattern; analytical H PLC: tᵣₑₜ= 4.03 min (Grad 1).

### Example 2c

### 6-(3,4-Dimethoxy-phenyl)-(1H, 3H)-quinazoline-2,4-dione

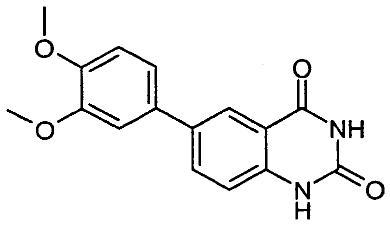

The title compound is obtained in a similar manner as in Example **1b** starting from 6-bromo-(*1H*,*3H*)-quinazoline-2,4-dione (Example **2d**); ES-MS: 299 (M + H)⁺; analytical HPLC: tᵣₑₜ= 2.73 min (Grad 1).

### Example 2d

**(following** H. Liu et al., JACS 2004, 126, p.1108**) 6-Bromo-*(1H,3H)*-quinazoline-2,4-dione**

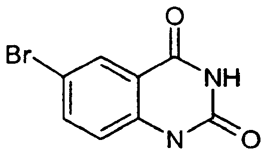

5 g (22.4 mmol) of 2-amino-5-bromobenzoic acid (Aldrich, Buchs, Switzerland) and 13.5 g (224 mmol) urea (Fluka, Buchs, Switzerland) are heated for 16 h at 150°C. The temperature is decreased to 100°C and one equivalent volume of water is added. The mixture is stirred 5 min and the resulting precipitate is filtered. The solid is triturated in glacial acetic acid, filtered and dried *in vacuo* to provide the title compound as an off-white solid. ES-MS: 241 (M + H)⁺, Br pattern; analytical HPLC: tᵣₑₜ= 2.48 min (Grad 1).

### Example 3

### 6-(6-Methoxy-pyridin-3-yl)-4-phenyl-quinazoline

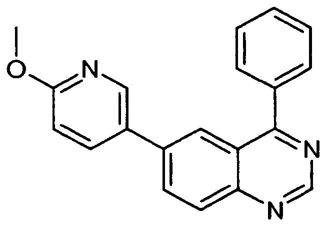

A mixture of 54 mg (0.20 mmol) of 4-chloro-6-(6-methoxy-pyridin-3-yl)-quinazoline (Example **3a**), 36 mg (0.30 mmol) of phenylboronic acid (Fluka, Buchs, Switzerland, **B3**), 4.6 mg (0.008 mmol) of tris(dibenzylideneacetone)-dipalladium(0) (Across, Basel, Switzerland), 6.5 mg (0.016 mmol) SPhos (synthesized following T.E. Barder et al., JACS 2005, 127, p.4685) and 126 mg (0.595 mmol) K₃PO₄ in 2 ml THF under argon in a seal reactor is heated with microwave excitation at 110°C for 1 h. The reaction mixture is quenched with sat. aqueous NaHCO₃ and extracted with EtOAc (2x). The organic layers are washed with brine, dried over Na₂SO₄, filtered and evaporated *in vacuo.* The residue is purified by flash chromatography (hexane-EtOAc 7:3 to 2:3) to give the title compound as a yellow solid. ES-MS: 314 (M + H)⁺; analytical HPLC: tᵣₑₜ= 3.74 min (Grad 1).

The starting materials are prepared as follows:

### Example 3a

### 4-Chloro-6-(6-methoxy-pyridin-3-yl)-quinazoline

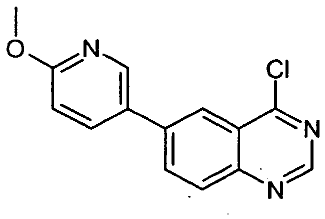

The title compound is obtained in a similar manner as in Example **2b** starting from 6-(6-methoxy-pyridin-3-yl)-3H-quinazolin-4-one (Example **3b**); ES-MS: 272 (M + H)⁺, Cl pattern; analytical HPLC: tᵣₑₜ= 3.51 min (Grad 1).

### Example 3b

### 6-(6-Methoxy-pyridin-3-yl)-3H-quinazolin-4-one

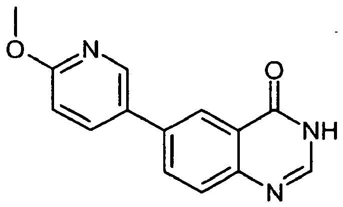

The title compound is obtained in a similar manner as in Example **1b** starting from 6-bromo-3H-quinazolin-4-one (Example **1d**) and 2-methoxy-5-pyridylboronic acid (Frontier Scientific, Logan, USA; **B4**); ES-MS: 254 (M + H)⁺; analytical HPLC: tᵣₑₜ= 2.50 min (Grad 1).

### Example 4

### 3-[2-Amino-4-(3,4-dimethoxy-phenyl)-quinazolin-6-yl]phenol

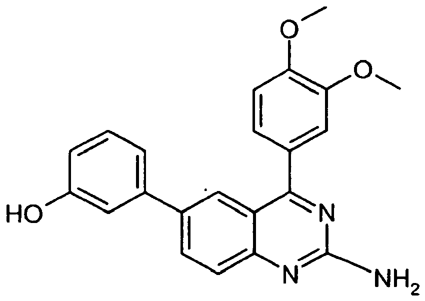

The title compound is obtained in a similar manner as in Example **1b** starting from 6-bromo4-(3,4-dimethoxy-phenyl)-quinazolin-2-ylamine (Example **4a**) and 3-hydroxyphenylboronic acid (Aldrich, Buchs, Switzerland; **B5**); ES-MS: 374 (M + H)⁺; analytical HPLC: tᵣₑₜ= 2.88 min (Grad 1).

The starting materials are prepared as follows:

### Example 4a

### 6-Bromo-4-(3,4-dimethoxy-phenyl)-quinazolin-2-ylamine

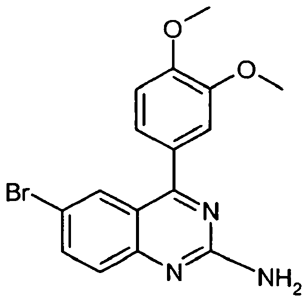

Two batches of 1.8 g (4.74 mmol) of 6-bromo-2-chloro-4-(3,4-dimethoxy-phenyl)-quinazoline (Example **4b**) and 20 ml (40 mmol) of 2 M ammonia in EtOH (Aldrich, Buchs, Switzerland; **A2**) are heated with microwave excitation in a seal reactor for 1 h at 170°C. The two batches are combined and evaporated to dryness. The residue is purified by flash chromatography (CH₂Cl₂-MeOH 1:0 to 97:3) to provide the title compound as a yellow solid. ES-MS: 360, 362 (M + H)⁺, Br pattern; analytical HPLC: tᵣₑₜ=2.92 min (Grad 1).

### Example 4b

### 6-Bromo-2-chloro-4-(3,4-dimethoxy-phenyl)-quinazoline

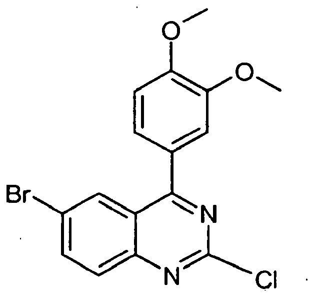

The title compound is obtained in a similar manner as in Example **1b** starting from 6-bromo-2,4-dichloro-quinazoline (Example **4c**); ES-MS: 374 (M + H)+; analytical HPLC: tᵣₑₜ= 2.88 min (Grad 1).

### Example 4c

### 6-Bromo-2,4-dichloro-quinazoline

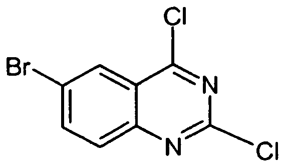

The title compound is obtained in a similar manner as in Example **1c** starting from 6-bromo-1H-quinazoline-2,4-dione (Example **2d**): analytical HPLC: tᵣₑₜ= 3.87 min (Grad 1).

Further commercialy available boronic acids;
**B6** 4-hydroxyphenylboronic acid (Lancaster, Morecambe, UK);
**B7** 3-methoxyphenylboronic acid (Aldrich, Buchs, Switzerland);
**B8** 2-chlorophenylboronic acid (Aldrich, Buchs, Switzerland);
**B9** 4-methoxyphenylboronic acid (Aldrich, Buchs, Switzerland);
**B10** 2-thienylboronic acid (Aldrich, Buchs, Switzerland);
**B11** 4-((1H-pyrazol-1-yl)phenyl)boronic acid (Anichem LLC, Monmouth Junction, USA);
**B12** 3-fluoro-4-methoxyphenylboronic acid (Aldrich, Buchs, Switzerland);
**B13** 3,4,5-trimethoxyphenylboronic acid (Aldrich, Buchs, Switzerland);
**B14** 3-methoxv-4-methoxycarbonylphenylboronic acid (Cuschem, Yonkers, USA);
**B15** 3,4-methylenedioxyphenylboronic acid (Aldrich, Buchs, Switzerland);
**B16** 2,3-dihydro-1,4-benzodioxin-6-ylboronic acid (Maybridge, Tintagel, UK);
**B17** 3-chloro-4-propoxyphenylboronic acid (Aldrich, Buchs, Switzerland);
**B18** 2-methoxy-4-(4,4,5,5-tetramethyl-1,3,2-dioxaborolan-2-yl)phenol (Aldrich, Buchs, Switzerland);
**B19** (4-aminocarbonylphenyl)boronic acid (Frontier Scientific, Logan, USA).
**B20** 5-(4,4,5,5-tetramethyl-[1,3,2]dioxaborolan-2-yl)-pyridin-2-ylamine (Aldrich, Buchs, Switzerland)
**B21** N,N-Dimethyl-4-(4,4,5,5-tetramethyl-[1,3,2]dioxaborolan-2-yl)-benzenesulfonamide (Frontier Scientific, Logan, USA)
**B22** 2-methoxy-4-pyridylboronic acid (Combi-blocks, San Diego, USA)
**B23** 3-ethoxyphenylboronic acid (Aldrich, Buchs, Switzerland)
**B24** 3-chlorophenylboronic acid (Aldrich, Buchs, Switzerland)
**B25** 2-benzyloxy-1-methoxy-4-(4,4,5,5-tetramethyl-1,3,2-dioxaborolan-2-yl)benzene (ABCR, Karlsruhe, Germany)
**B26** 4-methoxycarbonyphenylboronic acid (Aldrich, Buchs, Switzerland)
**B27** 3-quinoline boronic acid (Aros, Basel, Switzerland)
**B28** 5-methoxy-3-(4,4,5,5-tetramethyl-[1,3,2]dioxaborolan-2-yl)-pyridine (Aldrich, Buchs, Switzerland)
**B29** 3-(4,4,5,5-tetramethyl-[1,3,2]dioxaborolan-2-yl)-benzenesulfonamide (Frontier Scientific, Logan, USA)
**B30** 2-benzyloxy-1-methoxy-4-(4,4,5,5-tetramethyl-[1,3,2]dioxaborolan-2-yl)-benzene (ABCR, Karlsruhe, Germany)
**B31** 3-aminocarbonylphenylboronic acid (ABCR, Karlsruhe, Germany)
**B32** 4-chlorophenylboronic acid (Aldrich, Buchs, Switzerland)
**B33** 4-trifluoromethylphenylboronic acid (Aldrich, Buchs, Switzerland)
**B34** 3-chloro-4-methoxyphenylboronic acid (Aldrich, Buchs, Switzerland)
**B35** 3-thiopheneboronic acid (Aldrich, Buchs, Switzerland)
**B36** 5-(-4,4,5,5-tetramethyl-[1,3,2]dioxaborolan-2-yl)-1H-pyrrolo[2,3-b]pyridine (Alfa, Karlsruhe, Germany)
**B37** furane-3-boronic acid (Aldrich, Buchs, Switzerland)
**B38** 4-cyanophenylboronic acid (Aldrich, Buchs, Switzerland)
**B39** 3-formyphenylboronic acid (Fluka, Buchs, Switzerland)
**B40** 4-biphenylboronic acid (Aldrich, Buchs, Switzerland)
**B41** 2-cyano-5-(4,4,5,5-tetramethyl-[1,3,2]dioxaborolan-2-yl)-pyridine (Frontier Scientific, Logan, USA)
**B42** 4-bromophenylboronic acid (Aldrich, Buchs, Switzerland)
**B43** 4-aminomethylphenylboronic acid, hydrochloride (Frontier Scientific, Logan, USA)
**B44** 4-hydroxymethylphenylboronic acid (Aldrich, Buchs, Switzerland)
**B45** 4-trifluoromethoxyphenylboronic acid (Aldrich, Buchs, Switzerland)
**B46** 4-fluorophenylboronic acid (Aldrich, Buchs, Switzerland)
**B47** 3-fluorophenylboronic acid (Aldrich, Buchs, Switzerland)
**B48** 4-methylsulfonylphenylboronic acid (Aldrich, Buchs, Switzerland)
**B49** 4-acetaminophenylboronic acid (Aldrich, Buchs, Switzerland)
**B50** 3-cyano-5-(4,4,5,5-tetramethyl-[1,3,2]dioxaborolan-2-yl)pyridine (Frontier Scientific, Logan, USA)
**B51** 1-[5-(4,4,5,5-tetramethyl-1,3,2-dioxaborola n-2-yl)pyridin-2-yl)pyridine-2-yl]morpholine (Aldrich, Buchs, Switzerland)
**B52** 2-dimethylamino-pyridin-5-yl-boronic acid (Anichem, Monmouth Junction, USA)

### Synthesized boronic acids:

The following boronic acids are synthesized according to standard etherification procedures using commercially available halo reagents:
**B53** 1-ethoxy-2-methoxy-4-(4,4,5,5-tetramethyl-1,3,2-dioxaborolan-2-yl]benzene using iodoethane (Fluka, Buchs, Switzerland)
**B54** 2-[3-methoxy-4-(2-methoxy-ethoxy)-phenyl]-4,4,5,5-tetramethyl-[1,3,2]dioxaborolane using 2-bromoethyl methyl ether (Fluka, Buchs, Switzerland)
**B55** [4-(3-tert-butoxycarbonylamino-propoxy)-3-methoxy-phenyl]-boronic acid using tert-butyl N-(3-bromopropyl)carbamate (Fluka, Buchs, Switzerland)
**B56** [4-(2-tert-butoxycarbonylamino-ethoxy)-3-methoxy-phenyl]-boronic acid using tert-butyl N-(2-bromoethyl)carbamate (Fluka, Buchs, Switzerland)

The folowing boronic acid are prepared as follows:

**B57** 5-(4,4,5,5-Tetramethyl-[1,3,2]dioxaborolan-2-yl)-3-trifluoromethyl-pyridin-2-ylamine 8.04 g (31.7 mmol) of 5-Bromo-3-trifluoromethyl-pyridin-2-ylamine (B57a), 10.5 g (41.2 mmol) of 4,4,5,5,4',4',5',5'-Octamethyl-[2,2']bi[[1,3,2]dioxaborolanyl] (Aldrich, Buchs, Switzerland), 9.62 g (95.1 mmol) of KOAc in 100 ml dioxane are degassed with argon for 15 min. 776 mg (0.951 mmol) of bis(diphenylphosphino)ferrocene dichloropalladium(II)dichloromethane are added and the mixture is degassed for 15 more minutes. The reaction mixture is heated at 115°C for 8 h. After that time, the reaction mixture is filtered and the solvent evaporated. The residue is purified by simple filtration on silicagel (solvent system: t-butyl-methyl ether-EtOAc-NEt₃ = 50:50:0.1) to yield the title compound as almost colorless solid. ES-MS 289 (M+H)⁺; analytical HPLC: tᵣₑₜ= 1.68 min (Grad 1).

The starting material 5-Bromo-3-trifluoromethyl-pyridin-2-ylamine (**B57a**) is prepared as follows:

To a solution of 5.37 g (32.8 mmol) of 3-trifluoromethyl-pyridin-2-ylamine (Fluorochem, Derbyshire, UK) in 100 ml of dry CH3CN, 6.45 g of N-bromosuccinimide are added in 4 equal portions over a period of 1 h at 0-5°C under argon. The cooling bath is removed and stirring is continued for 3 h. The solvent is evaporated under vacuum, the residue is dissolved in EtOAc and washed with water and brine. The organic phase is dried over Na₂SO₄ and evaporated. The title compound is a reddish-yellow oil which is used after drying in the dark for 5 h at RT and under high vacuum in the next step without further purification. ES-MS: 241 (M-H)⁻.

### B58 2-(3,4-diethoxy-phenyl)-4,4,5,5-tetramethyl-[1,3,2]dioxaborolane

The title compound is synthesized in a similar manner as described in **B57** starting with 3,4-diethoxybromobenzene **B58a:** ES-MS: 293 (M + H)⁺; analytical HPLC: tᵣₑₜ= 3.94 min (Grad 1). The starting material 3,4-diethoxybromobenzene (**B58a)** is prepared as follows:

The title compound is obtained according to standard etherification procedures using commercially available iodoethane (Fluka, Buchs, Switzerland) and 4-bromocatechol (ABCR,
Karlsruhe, Germany): analytical HPLC: tᵣₑₜ= 3.79 min (Grad 1).

### B59 2-(3-trifluoromethoxy-4-methoxy-phenyl)-4,4,5,5-tetramethyl-[1,3,2]dioxaborolane

The title compound is synthesized in a similar manner as **B58** starting from 4-bromo-2(trifluroromethoxy)phenol (Manchester, Runcorn, UK) and iodomethane (Fluka, Buchs, Switzerland): analytical HPLC: tᵣₑₜ= 4.25 min (Grad 1).

### B60 2-isobutylamino-pyridin-5-ylboronic acid

The title compound is synthesized in a similar manner as **B57** starting from 5-bromo-2-isobutylamino-pyridine (**B60a**), the boronic pinacol ester being hydrolyzed during purifiction: ES-MS: 195 (M + H)⁺; analytical HPLC: tᵣₑₜ= 2.08 min (Grad 1).

The starting material 5-bromo-2-isobutylamino-pyridine (**B60a**) is prepared as follows: 600 mg (3.12 mmol) of 5-bromo-2-chloropyridine (Aldrich, Buchs, Switzerland) in 3.13 ml (31.2 mmol) isobutylamine (Fluka, Buchs, Switzerland) is heated in a microwave oven for 2 h at 170°C. The reaction mixture is quenched with 50 ml water and extracted with EtOAc (2x). The combined organic layers are washed with water (5x), dried over Na₂SO₄, filtered and evaporated. The crude product is purified by flash chromatography (CH₂Cl₂) to give the title compound as a white solid. ES-MS: 229, 231 (M + H)⁺, Br pattern; analytical HPLC: tᵣₑₜ= 2.31 min (Grad 1).

### B61 N-methyl-5-(4,4,5,5-tetramethyl-[1,3,2]dioxaborolan-2-yl)-pyridin-2-yl-amin

The title compound is synthesized in a similar manner as **B57** starting from 5-bromo-2-methylaminopyridine (**B61a**): ES-MS: 235 (M + H)⁺; analytical HPLC: tᵣₑₜ= 1.46 min (Grad 1). The starting material 5-bromo-2-methylaminopyridine (**B61a**) is prepared as follows:

The title compound is synthesized in a similar manner as **B60a** starting from **A4:** ES-MS: 187, 189 (M + H)⁺, Br pattern; analytical HPLC: tᵣₑₜ= 1.74 min (Grad 1).

**B62** N-(2-hydroxyethyl)-5-(4,4,5,5-tetramethyl-[1,3,2]dioxaborolan-2-yl)-pyridin-2-ylamine The title compound is synthesized in a similar manner as **B57** starting from 2-(5-bromopyridin-2-ylamino)-ethanol (**B62a**): ES-MS: 265 (M + H)⁺.

The starting material 2-(5-bromo-pyridin-2-ylamino)-ethanol (**B62a**) is prepared as follows: The title compound is synthesized in a similar manner as **B60a** starting from **A11:** ES-MS: 217, 219 (M + H)⁺, Br pattern; analytical HPLC: tᵣₑₜ= 1.66 min (Grad 1).

Commercially available amines:
**A3** cycropropylamine (Fluka, Buchs, Switzerland);
**A4** methylamine (8 M in EtOH (Fluka, Buchs, Switzerland));
**A5** morpholine (Fluka, Buchs, Switzerland);
**A6** N-methylpiperazine (Fluka, Buchs, Switzerland);
**A7** dimethylamine 2 M in THF (Aldrich, Buchs, Switzerland).
**A8** N-(2-methoxyethyl)methylamine (ABCR, Karlsruhe, Germany)
**A9** N,N-dimethylethylenediamine (Fluka, Buchs, Switzerland)
**A10** bis(2-methoxyethyl)amine (Fluka, Buchs, Switzerland)
**A11** 2-hydroxyethylamine (Fluka, Buchs, Switzerland)
**A12** 2-methoxyethylamine (Fluka, Buchs, Switzerland)

The following compounds (Table 1) are prepared in a similar manner as described in Example **1** by reacting 6-bromo-4-chloro-quinazoline (Example **1c**) with the appropriate boronic acid(s) (Process A), or are prepared in a similar manner as described in Example **3** starting from 6-bromo-3H-quinazolin-4-one (Example **1d**) and using the appropriate boronic acid(s) (Process B).

**Table 1**

| Example/ process | Boronic acids | Compound name | ES-MS (M+H)⁺ | tᵣₑₜ Grad 1 [min] |
|---|---|---|---|---|
| 5 / A | **B1 B17** | 6-(3-Chloro-4-n-propoxy-phenyl)-4-(3,4-dimethoxy-phenyl)-quinazoline | 435 | 4.54 |
| 6 / A | **B1 B18** | 4-[4-(3,4-Dimethoxy-phenyl)-quinazolin-6-yl]-2-methoxy-phenol | 389 | 3.24 |
| 7 / A | **B1 B16** | 6-(2,3-Dihydro-benzo[1,4]dioxin-6-yl)-4-(3,4-dimethoxy-phenyl)-quinazoline | 401 | 3.74 |
| 8 / A | **B1 B15** | 6-(Benzo[1,3]dioxol-5-yl)-4-(3,4-dimethoxy-phenyl)-quinazoline | 387 | 3.75 |
| 9 / A | **B1 B14** | 4-[4-(3,4-Dimethoxy-phenyl)-quinazolin-6-yl]-2-methoxy-benzoic acid methyl ester | 431 | 3.64 |
| 10 / A | **B1 B13** | 4-(3,4-Dimethoxy-phenyl)-6-(3,4,5-trimethoxy-phenyl)-quinazoline | 433 | 3.58 |
| 11 / B | **B1 B12** | 6-(3,4-Dimethoxy-phenyl)-4-(3-fluoro-4-methoxy-phenyl)-quinazoline | 391 | 3.80 |
| 12 / B | **B1 B14** | 4-[6-(3,4-Dimethoxy-phenyl)-quinazolin-4-yl]-2-methoxy-benzoic acid methyl ester | 431 | 3.68 |
| 13 / B | **B1 B17** | 4-(3-Chloro-4-n-propoxy-phenyl)-6-(3,4-dimethoxy-phenyl)-quinazoline | 435 | 4.46 |
| 14 / B | **B1 B13** | 6-(3,4-Dimethoxy-phenyl)-4-(3,4,5-trimethoxy-phenyl)-quinazoline | 433 | 3.63 |
| 15 / B | **B1 B16** | 4-(2,3-Dihydro-benzo[1,4]dioxin-6-yl)-6-(3,4-dimethoxy-phenyl)-quinazoline | 401 | 3.68 |
| 16 / B | **B1 B15** | 4-(Benzo[1,3]dioxol-5-yl)-6-(3,4-dimethoxy-phenyl)-quinazoline | 387 | 3.67 |
| 17 / A | **B2 B11** | 6-(6-Piperazin-1-yl-pyridin-3-yl)-4-(4-pyrazol-1-yl-phenyl)-quinazoline | 434 | 2.66 |
| 18 / A | **B1 B5** | 3-[4-(3,4-Dimethoxy-phenyl)-quinazolin-6-yl]-phenol | 359 | 3.27 |
| 19 / A | **B1 B6** | 4-[4-(3,4-Dimethoxy-phenyl)-quinazolin-6-yl]-phenol | 359 | 3,20 |
| 20 / A | **B1 B19** | 4-[4-(3,4-Dimethoxy-phenyl)-quinazolin-6-yl]-benzamide | 386 | 3.00 |
| 21 / A | **B1 B12** | 4-(3,4-Dimethoxy-phenyl)-6-(3-fluoro-4-methoxy-phenyl)-quinazoline | 391 | 3.83 |
| 22 / B | **B1 B3** | 6-(3,4-Dimethoxy-phenyl)-4-phenyl-quinazoline | 343 | 3,76 |
| 23 / B | **B1 B10** | 6-(3,4-Dimethoxy-phenyl)-4-thiophen-2-yl-quinazoline | 349 | 3.74 |
| 24 / B | **B1 B7** | 6-(3,4-Dimethoxy-phenyl)-4-(3-methoxy-phenyl)-quinazoline | 373 | 3.80 |
| 25 / B | **B1 B9** | 6-(3,4-Dimethoxy-phenyl)-4-(4-methoxy-phenyl)-quinazoline | 373 | 3.71 |
| 26 / B | **B1 B11** | 6-(3,4-Dimethoxy-phenyl)-4-(4-pyrazol-1-yl-phenyl)-quinazoline | 409 | 3.80 |
| 27 / B | **B1 B2** | 6-(3,4-Dimethoxy-phenyl)-4-(6-piperazin-1-yl-pyridin-3-yl)-quinazoline | 428 | 2.79 |
| 28 / A | **B2 B19** | 4-[6-(6-Piperazin-1-yl-pyridin-3-yl)-quinazolin-4-yl]-benzamide | 411 | 2.25 |
| 29 / A | **B4 B11** | 6-(6-Methoxy-pyridin-3-yl)-4-(4-pyrazol-1-yl-phenyl)-quinazoline | 380 | 3.70 |
| 30 / A | **B4 B19** | 4-[6-(6-Methoxy-pyridin-3-yl)-quinazolin-4-yl]-benzamide | 357 | 2.95 |
| 31 /A | **B2 B4** | 6-(6-Methoxy-pyridin-3-yl)-4-(6-piperazin-1-yl-pyridin-3-yl)-quinazoline | 399 | 2.67 |
| 32 / B | **B1 B1** | 4,6-Bis(3,4-dimethoxy-phenyl)-quinazoline | 403 | 3.55 |
| 33 / B | **B1 B19** | 4-(6-(3,4-Dimethoxy-phenyl)-quinazolin-4-yl]-benzamide | 386 | 3.12 |
| 34 / B | **B1 B4** | 6-(3,4-Dimethoxy-phenyl)-4-(6-methoxy-pyridin-3-yl)-quinazoline | 374 | 3.64 |
| 35 / A | B1 B4 | 4-(3,4-Dimethoxy-phenyl)-6-(6-methoxy-pyridin-3-yl)-quinazoline | 374 | 3.50 |
| 36 / A | **B2 B4** | 4-(6-Methoxy-pyridin-3-yl)-6-(6-piperazin-1-yl-pyridin-3-yl)-quinazoline | 399 | 2.47 |
| 37 / A | **B4 B10** | 6-(6-Methoxy-pyridin-3-yl)-4-thiophen-2-yl-quinazoline | 320 | 3.71 |
| 38 / A | **B4 B8** | 4-(2-Chloro-phenyl)-6-(6-methoxy-pyridin-3-yl)-quinazoline | 348 | 3.81 |
| 39 / B | **B4 B4** | 4,6-Bis(6-methoxy-pyridin-3-yl)-quinazoline | 345 | 3.56 |
| 40 / B | **B4 B9** | 4-(4-Methoxy-phenyl)-6-(6-methoxy-pyridin-3-yl)-quinazoline | 344 | 3.72 |
| 41 / A | **B1 B53** | 4-(3,4-Dimethoxy-phenyl)-6-(4-ethoxy-3-methoxy-phenyl)-quinazoline | 417 | 3.79 |
| 42 / A | **B1 B54** | 4-(3,4-Dimethoxy-phenyl)-6-[3-methoxy-4-(2-methoxy-ethoxy)-phenyl]-quinazoline | 447 | 3.58 |
| 43 / A | **B1 B20** | 5-[4-(3,4-Dimethoxy-phenyl)-quinazolin-6-yl]-pyridin-2-ylamine | 359 | 2.59 |
| 44 / B | **B1 B21** | 4-[6-(3,4-Dimethoxy-phenyl)-quinazolin-4-yl]-N, N-dimethyl-benzenesulfonamide | 450 | 3.70 |
| 45 / B | **B1 B22** | 6-(3,4-Dimethoxy-phenyl)-4-(4-ethoxy-3-methoxy-phenyl)-quinazoline | 417 | 3.74 |
| 46 / B | **B1, B23** | 6-(3,4-Dimethoxy-phenyl)-4-[3-methoxy-4-(2-methoxy-ethoxy)-phenyl]-quinazoline | 447 | 3.61 |
| 47 / A | **B1 B22** | 4-(3,4-Dimethoxy-phenyl)-6-(2-methoxy-pyridin-4-yl)-quinazoline | 374 | 3.37 |
| 48 / B | **B1 B22** | 6-(3,4-Dimethoxy-phenyl)-4-(2-methoxy-pyridin-4-yl)-quinazoline | 374 | 3.57 |
| 49 / A | **B1 B55** | (3-{4-[4-(3,4-Dimethoxy-phenyl)-quinazolin-6-yl]-2-methoxy-phenoxy}-propyl)-carbamic acid tert-butyl ester | 546 | 4.02 |
| 50 / B | **B1 B55** | (3-{4-[6-(3,4-Dimethoxy-phenyl)-quinazolin-4-yl]-2-methoxy-phenoxy}-propyl)-carbamic acid tert-butyl ester | 546 | 3.99 |
| 51 /A | **B1 B56** | (2-{4-[4-(3,4-Dimethoxy-phenyl)-quinazolin-6-yl]-2-methoxy-phenoxy}-ethyl)-carbamic acid tert-butyl ester | 532 | 3.87 |
| 52 / B | **B1 B56** | (2-{4-[6-(3,4-Dimethoxy-phenyl)-quinazolin-4-yl]-2-methoxy-phenoxy}-ethyl)-carbamic acid tert-butyl ester | 532 | 3.89 |
| 53 / B | **B1 B23** | 6-(3,4-Dimethoxy-phenyl)-4-(3-ethoxy-phenyl)-quinazoline | 387 | 3.97 |
| 54 / B | **B1 B24** | 4-(3-Chloro-phenyl)-6-(3,4-dimethoxy-phenyl)-quinazoline | 377 | 4.07 |
| 55 / B | **B1 B25** | 4-(3-Benzyloxy-4-methoxy-phenyl)-6-(3,4-dimethoxy-phenyl)-quinazoline | 479 | 4.12 |
| 56 / B | **B1 B26** | 4-[6-(3,4-Dimethoxy-phenyl)-quinazolin-4-yl]-benzoic acid methyl ester | 401 | 3.82 |
| 57 / A | **B1 B28** | 4-(3,4-Dimethoxy-phenyl)-6-(5-methoxy-pyridin-3-yl)-quinazoline | 374 | 2.73 |
| 58 / A | **B1 B14** | 4-[4-(3,4-Dimethoxy-phenyl)-quinazolin-6-yl]-2-methoxy-benzoic acid methyl ester | 431 | 3.63 |
| 59 / A | **B1 B27** | 4-(3,4-Dimethoxy-phenyl)-6-quinolin-3-yl-quinazoline | 394 | 3.00 |
| 60 / A | **B19 B28** | 4-[6-(5-Methoxy-pyridin-3-yl)-quinazolin-4-yl]-benzamide | 357 | 2.39 |
| 61 / A | **B19 B57** | 4-[6-(6-Amino-5-trifluoromethyl-pyridin-3-yl)-quinazolin-4-yl]-benzamide | 410 | 2.71 |
| 62 / A | **B1 B57** | 5-[4-(3,4-Dimethoxy-phenyl)-quinazolin-6-yl]-3-trifluoromethyl-pyridin-2-ylamine | 427 | 3.13 |
| 63 / A | **B1 B29** | 3-[4-(3,4-Dimethoxy-phenyl)-quinazolin-6-yl]-benzenesulfonamide | 422 | 3.04 |
| 64 / A | **B12 B15** | 4-Benzo[1,3]dioxol-5-yl-6-(3-fluoro-4-methoxy-phenyl)-quinazoline | 375 | 3.91 |
| 65 / B | **B1 B30** | 4-(3-Benzyloxy-4-methoxy-phenyl)-6-(3,4-dimethoxy-phenyl)-quinazoline | 479 | 4.10 |
| 66 / A | **B1 B31** | 3-[4-(3,4-Dimethoxy-phenyl)-quinazolin-6-yl]-benzamide | 386 | 3.01 |
| 67 / B | **B1 B32** | 4-(4-Chloro-phenyl)-6-(3,4-dimethoxy-phenyl)-quinazoline | 377 | 4.05 |
| 68 / B | **B1 B33** | 6-(3,4-Dimethoxy-phenyl)-4-(4-trifluoromethyl-phenyl)-quinazoline | 411 | 4.10 |
| 69 / A | **B1 B34** | 6-(3-Chloro-4-methoxy-phenyl)-4-(3,4-dimethoxy-phenyl)-quinazoline | 407 | 3.96 |
| 70 / B | **B1 B35** | 6-(3,4-Dimethoxy-phenyl)-4-thiophen-3-yl-quinazoline | 349 | 3.63 |
| 71 / A | **B1 B36** | 4-(3,4-Dimethoxy-phenyl)-6-(1H-pyrrolo[2,3-b]pyridin-5-yl)-quinazoline | 383 | 2.95 |
| 72 / A | **B19 B36** | 4-[6-(1H-Pyrrolo[2,3-b]pyridin-5-yl)-quinazolin-4-yl]-benzamide | 366 | 2.59 |
| 73 / A | **B19 B20** | 4-[6-(6-Amino-pyridin-3-yl)-quinazolin-4-yl]-benzamide | 342 | 2.30 |
| 74 / A | **B1 B30** | 6-(3-Benzyloxy-4-methoxy-phenyl)-4-(3,4-dimethoxy-phenyl)-quinazoline | 479 | 4.16 |
| 75 / B | **B12 B32** | 4-(4-Chloro-phenyl)-6-(3-fluoro-4-methoxy-phenyl)-quinazoline | 365 | 4.31 |
| 76 / A | **B19 B22** | 4-[6-(4-Ethoxy-3-methoxy-phenyl)-quinazolin-4-yl]-benzamide | 400 | 3.30 |
| 77 / A | **B19 B62** | 4-[6-(3,4-Diethoxy-phenyl)-quinazolin-4-yl]-benzamide | 414 | 3.47 |
| 78 / A | **B1 B37** | 4-(3,4-Dimethoxy-phenyl)-6-furan-3-yl-quinazoline | 333 | 3.47 |
| 79 / A | **B1 B38** | 4-[6-(3,4-Dimethoxy-phenyl)-quinazolin-4-yl]-benzonitrile | 368 | 3.65 |
| 80 / A | **B20 B38** | 4-[6-(6-Amino-pyridin-3-yl)-quinazolin-4-yl]-benzonitrile | 324 | 2.62 |
| 81 / A | **B1 B39** | 4-[4-(3,4-Dimethoxy-phenyl)-quinazolin-6-yl]-benzaldehyde | 371 | 3.56 |
| 82 / B | **B1 B40** | 4-Biphenyl-4-yl-6-(3,4-dimethoxy-phenyl)-quinazoline | 419 | 4.36 |
| 83 / B | **B1 B58** | 4-(3,4-Diethoxy-phenyl)-6-(3,4-dimethoxy-phenyl)-quinazoline | 431 | 3.84 |
| 84 / B | **B1 B59** | 6-(3,4-Dimethoxy-phenyl)-4-(4-methoxy-3-trifluoromethoxy-phenyl)-quinazoline | 457 | 4.08 |
| 85 / A | **B1 B59** | 4-(3,4-Dimethoxy-phenyl)-6-(4-methoxy-3-trifluoromethoxy-phenyl)-quinazoline | 457 | 4.08 |
| 86 / A | **B1 B41** | 5-[4-(3,4-Dimethoxy-phenyl)-quinazolin-6-yl]-pyridine-2-carbonitrile | 369 | 3.37 |
| 87 / B | **B1 B42** | 4-(4-Bromo-phenyl)-6-(3,4-dimethoxy-phenyl)-quinazoline | 421 423 | 4.10 |
| 88 / A | **B1 B58** | 6-(3,4-Diethoxy-phenyl)-4-(3,4-dimethoxy-phenyl)-quinazoline | 431 | 3.93 |
| 89 / B | **B1 B45** | 6-(3,4-Dimethoxy-phenyl)-4-(4-trifluoromethoxy-phenyl)-quinazoline | 427 | 4.15 |
| 90 / B | **B1 B46** | 6-(3,4-Dimethoxy-phenyl)-4-(4-fluoro-phenyl)-quinazoline | 361 | 3.79 |
| 91 / B | **B1 B47** | 6-(3,4-Dimethoxy-phenyl)-4-(3-fluoro-phenyl)-quinazoline | 361 | 3.80 |
| 92 / B | **B1 B48** | 6-(3,4-Dimethoxy-phenyl)-4-(4-methanesulfonyl-phenyl)-quinazoline | 421 | 3.37 |
| 93 / B | **B1 B49** | N-{4-[6-(3,4-Dimethoxy-phenyl)-quinazolin-4-yl]-phenyl}-acetamide | 400 | 3.25 |
| 94 / A | **B1 B50** | 5-[4-(3,4-Dimethoxy-phenyl)-quinazolin-6-yl]-nicotinonitrile | 369 | 3.25 |
| 95 / A | **B1 B60** | {5-[4-(3,4-Dimethoxy-phenyl)-quinazolin-6-yl]-pyridin-2-yl}-isobutyl-amine | 415 | 3.01 |
| 96 / B | **B1 B51** | 6-(3,4-Dimethoxy-phenyl)-4-(6-morpholin-4-yl-pyridin-3-yl)-quinazoline | 429 | 3.02 |
| 97 / A | **B1 B52** | {5-[4-(3,4-Dimethoxy-phenyl)-quinazolin-6-yl]-pyridin-2-yl}-dimethyl-amine | 387 | 2.72 |
| 98 / A | **B1 B61** | {5-[4-(3,4-Dimethoxy-phenyl)-quinazolin-6-yl]-pyridin-2-yl}-methyl-amine | 373 | 2.64 |
| 99 / A | **B1 B62** | 2-{5-[4-(3,4-Dimethoxy-phenyl)-quinazolin-6-yl]-pyridin-2-ylamino}-ethanol | 403 | 2.56 |
| 100 / B | **B1 B2** | 4-{5-[6-(3,4-Dimethoxy-phenyl)-quinazolin-4-yl]-pyridin-2-yl}-piperazine-1-carboxylic acid tert-butyl ester | 528 | 3.52 |

The following compounds (Table 2) are prepared in a similar manner as described in Example 2 by reacting 6-bromo-1H-quinazoline-2,4-dione (Example 2d) with the appropriate
boronic acid(s) and ammonia or a primary amine (Process C), or are prepared in a similar manner as described in Example **4** starting from 6-bromo-2,4-dichloro-quinazoline (Example **4c**) and using the appropriate boronic acid(s) and ammoniac or a primary amine (Process D):

**Table 2:**

| Example/ process | Boronic acids Amines | Compound name | ES-MS (M+H)⁺ | tᵣₑₜ Grad. 1 [min] |
|---|---|---|---|---|
| 101 / C | **B1, B1 A3** | [4,6-Bis-(3,4-dimethoxy-phenyl)-quinazolin-2-yl]-cyclopropyl-amine | 458 | 3.31 |
| 102 / C | **B1, B19 A2** | 4-[2-Amino-6-(3,4-dimethoxy-phenyl)-quinazol-in-4-yl]-benzamide | 401 | 2.66 |
| 103 / D | **B1, B12** | 4-(3,4-Dimethoxy-phenyl)-6-(3-fluoro-4-methoxy-phenyl)-quinazolin-2-ylamine | 406 | 3.25 |
| 104 / D | **B1, B14 A2** | 4-[2-Amino-4-(3,4-dimethoxy-phenyl)-quinaz-olin-6-yl]-2-methoxy-benzoic acid methyl ester | 446 | 3.11 |
| 105 / D | **B1, B13 A2** | 4-(3,4-Dimethoxyphenyl)-6-(3,4,5-trimethoxy-phenyl)-quinazolin-2-ylamine | 448 | 3.10 |
| 106 / D | **B1, B17 A2** | 6-(3-Chloro-4-n-propoxy-phenyl)-4-(3,4-dimethoxyphenyl)-quinazolin-2-ylamine | 450 | 3.80 |
| 107 / D | **B1, B6 A2** | 4-[2-Amino-4-(3,4-dimethoxy-phenyl)-quinazol-in-6-yl]-phenol | 374 | 2.81 |
| 108 / D | **B1, B16 A2** | 6-(2,3-Dihydro-benzo[1,4]dioxin-6-yl)-4-(3,4-dimethoxy-phenyl)-quinazolin-2-ylamine | 416 | 3.20 |
| 109 / C | **B1, B15 A2** | 4-(Benzo[1,3]dioxol-5-yl)-6-(3,4-dimethoxy-phenyl)-quinazolin-2-ylamine | 402 | 3.15 |
| 110 / C | **B1, B13 A2** | 6-(3,4-Dimethoxy-phenyl)-4-(3,4,5-trimethoxy-phenyl)-quinazolin-2-ylamine | 448 | 3.11 |
| 111 / D | **B1, B4 A2** | 6-(3,4-Dimethoxy-phenyl)-4-(6-methoxy-pyridin-3-yl)-quinazolin-2-ylamine | 389 | 3.03 |
| 112 / C | **B1, B1 A2** | 4,6-Bis(3,4-dimethoxy-phenyl)-quinazolin-2-yl-amine | 418 | 3.08 |
| 113 / C | **B4, B4 A2** | 4,6-Bis(6-methoxy-pyridin-3-yl)-quinazolin-2-yl-amine | 360 | 2.90 |
| 114 / D | **B1, B4 A2** | 4-(3,4-Dimethoxy-phenyl)-6-(6-methoxy-pyridin-3-yl)-quinazolin-2-ylamine | 389 | 2.97 |
| 115 / C | **B1, B1 A4** | N-[4,6-Bis-(3,4-dimethoxy-phenyl)-quinazolin-2-yl]-N-methyl-amine | 432 | 3.19 |
| 116 / D | **B1, B16 A2** | 4-(2,3-Dihydro-benzo[1,4]dioxin-6-yl)-6-(3,4-dimethoxy-phenyl)-quinazolin-2-ylamine | 416 | 3.19 |
| 117 / D | **B1, B20 A2** | 6-(6-Amino-pyridin-3-yl)-4-(3,4-dimethoxyphenyl)-quinazolin-2-ylamine | 374 | 2.27 |
| 118 / D | **B1, B27 A2** | 4-(3,4-Dimethoxy-phenyl)-6-quinolin-3-yl-quinazolin-2-ylamine | 409 | 2.63 |

### Example 119

### 4[6-(3,4-Dimethoxy-phenyl)-quinazolin-4-yl]-2-methoxy-benzoic acid

107 mg (0.248 mmol) of 4-[6-(3,4-dimethoxy-phenyl)-quinazolin-4-yl)-2-methoxy-benzoic acid methyl ester (Example **12**) in 2 ml dioxane is treated with 0.50 ml 1 M aqueous LiOH. The reaction mixture is stirred for 2.5 h at rt. After this time, 0.50 ml 1 M aqueous HCl are added and the precipitate is filtered. The solid is dissolved in CH₂Cl₂ and washed with water (2x), dried over Na₂SO₄, filtered and evaporated to give the title compound as a yellow solid. ES-MS: 417 (M + H)⁺; analytical HPLC: tᵣₑₜ = 3.30 min (Grad 1).

### Example 120

### 4-[6-(3,4-Dimethoxy-phenyl)-quinazolin-4-yl]-2-methoxy-benzoic acid-N-methylamide

60 mg (0.143 mmol) of 4-[6-(3,4-dimethoxy-phenyl)-quinazolin-4-yl]-2-methoxy-benzoic acid (Example **57**), 62 µl (0.357 mmol) diisopropylethylamine (Fluka, Buchs, Switzerland) and 42 mg (0.143 mmol) TPTU (Fluka, Buchs, Switzerland) in 1.5 ml DMA is stirred for 10 min at rt. The reaction mixture is added to a solution of 18 µl (0.143 mmol) **A4** and 4.5 mg (0.036 mmol) DMAP in 1.5 ml DMA. The reaction mixture is stirred for 10 min at rt. After this time, the reaction mixture is diluted with water and extracted with EtOAc (2x). The organic layers are washed with brine, dried over Na₂SO₄, filtered and evaporated to dryness. The residue is adsorbed on Isolute HM-N sorbent and purified by RP-MPLC (H₂O/CH₃CN and 3% PrOH), The pure fractions are concentrated and the product precipitates to provide the title compound as an off-white solid. ES-MS: 430 (M + H)⁺; analytical HPLC: tᵣₑₜ = 3.40 min (Grad 1).

The following compounds (Table 3) are prepared in a similar manner as described in Example **120** with the amine given:

**Table 3**

| Example | Amine | Compound Name | ES-MS (M+H)⁺ | tᵣₑₜ Grad. 1 [min] |
|---|---|---|---|---|
| 121 | **A2** | 4-[6-(3,4-Dimethoxy-phenyl)-quinazolin-4-yl]-2-methoxy-benzamide | 416 | 3.28 |
| 122 | **A7** | 4-[6-(3,4-Dimethoxy-phenyl)-quinazolin-4-yl]-2-methoxy-N, N-dimethyl-amide | 444 | 3.45 |
| 123 | **A6** | {4-[6-(3,4-Dimethoxy-phenyl)-quinazolin-4-yl]-2-methoxy-phenyl}-(4-methyl-piperazin-1-yl)-methanone | 499 | 2.98 |
| 124 | **A5** | {4-[6-(3,4-Dimethoxy-phenyl)-quinazolin-4-yl]-2-methoxy-phenyl}-morpholin-4-yl-methanone | 486 | 3.40 |

The following compounds (Table 4) are prepared in a similar manner as descibed in Example **120** starting with the amine given and 4-[6-(3,4-dimethoxy-phenyl)-quinazolin-4-yl]-benzoic acid, the hydrolysis side product of the synthesis of Example **56** (ES-MS: 387 (M + H )⁺; analytical HPLC: tᵣₑₜ = 3.33 min (Grad 1)):

**Table 4**

| Example | Amine | Compound Name | ES-MS (M+H)⁺ | tᵣₑₜ Grad. 1 [min] |
|---|---|---|---|---|
| 125 | **A7** | 4-[6-(3,4-Dimethoxy-phenyl)-quinazolin-4-yl]-N,N-dimethyl-benzamide | 414 | 3.40 |
| 126 | **A4** | 4-[6-(3,4-Dimethoxy-phenyl)-quinazolin-4-yl]-N-methyl-benzamide | 400 | 3.22 |
| 127 | **A6** | {4-[6-(3,4-Dimethoxy-phenyl)-quinazolin-4-yl]-phenyl}-(4-methyl-piperazin-1-yl)-methanone | 469 | 2.92 |
| 128 | **A5** | {4-[6-(3,4-Dimethoxy-phenyl)-quinazolin-4-yl]-phenyl}-morpholin-4-yl-methanone | 456 | 3.36 |

The following compounds (Table 5) are prepared by Susuki coupling in a similar manner as descibed in Example **3** or in Example **1b** starting with 4-(4-chloro-phenyl)-6-(3,4-dimethoxyphenyl)-quinazoline (Example **67**) or 4-(4-bromo-phenyl)-6-(3,4-dimethoxy-phenyl)-quinazoline (Example **87**) and the appropriate bornic acid:

**Table 5**

| Example | Boronic acid | Compound Name | ES-MS (M+H)⁺ | tret Grad. 1 [min] |
|---|---|---|---|---|
| 129 | **B43** | C-{4'-[6-(3,4-Dimethoxy-phenyl)-quinazolin-4-yl]-biphenyl-4-yl}-methylamine | 448 | 3.23 |
| 130 | **B9** | 6-(3,4-Dimethoxy-phenyl)-4-(4'-methoxy-biphenyl-4-yl)-quinazoline | 449 | 4.33 |
| 131 | **B44** | {4'-[6-(3,4-Dimethoxy-phenyl)-quinazolin-4-yl]-biphenyl-4-yl}-methanol | 449 | 3.76 |
| 132 | **B6** | 4'-[6-(3,4-Dimethoxy-phenyl)-quinazolin-4-yl]-biphenyl-4-ol | 435 | 3.76 |
| 133 | **B1** | 4-(3',4'-Dimethoxy-biphenyl-4-yl)-6-(3,4-dimethoxy-phenyl)-quinazoline | 479 | 4.11 |
| 134 | **B13** | 6-(3,4-Dimethoxy-phenyl)-4-(3',4',5'-trimethoxy-biphenyl-4-yl)-quinazoline | 509 | 4.11 |
| 135 | **B19** | 4'-[6-(3,4-Dimethoxy-phenyl)-quinazolin-4-yl]-biphenyl-4-carboxylic acid amide | 462 | 3.59 |

### Example 136

### 6-(3,4-Dimethoxy-phenyl)-4-(4'-methoxymethyl-biphenyl-4-yl)-quinazoline

The title compound is obtained by standard etherification of {4'-[6-(3,4-dimethoxy-phenyl)-quinazolin-4-yl]-biphenyl-4-yl}-methanol (Example 136) using iodomethane (Fluka, Buchs, Switzerland): ES-MS: 463 (M + H)⁺; analytical HPLC: tᵣₑₜ = 4.29 min (Grad 1).

### Example 137

### 3-{4-[4-(3,4-Dimethoxy-phenyl)-quinazolin-6-yl]-2-methoxy-phenoxy}-propylamine

The title compound is obtained in a similar manner as described in Example **1** starting with (3-{4-[4-(3,4-Dimethoxy-phenyl)-quinazolin-6-yl]-2-methoxy-phenoxy}-propyl)-carbamic acid tert-butyl ester (Example **49**): ES-MS: 446 (M + H)⁺; analytical HPLC: tᵣₑₜ = 2.93 min (Grad 1).

### Example 138

### 2-{4-[4-(3,4-Dimethoxy-phenyl)-quinazolin-6-yl]-2-methoxy-phenoxy}-ethylamine

The title compound is obtained in a similar manner as described in Example **1** starting with (2-{4-[4-(3,4-Dimethoxy-phenyl)-quinazolin-6-yl]-2-methoxy-phenoxy}-ethyl)-carbamic acid tert-butyl ester (Example **51**): ES-MS: 432 (M + H)⁺; analytical HPLC: tᵣₑₜ = 2.81 min (Grad 1).

### Example 139

### 3-{5-[4-(3,4-Dimethoxy-phenyl)-quinazolin-6-yl]-2-methoxy-phenoxy}-propylamine

The title compound is obtained in a similar manner as described in Example **1** starting with 6-(3-benzyloxy-4-methoxy-phenyl)-4-(3,4-dimethoxy-phenyl)-quinazoline (Example **74**): ES-MS: 446 (M + H)⁺; analytical HPLC: tᵣₑₜ = 2.89 min (Grad 1).

### Example 140

### 2-{5-[4-(3,4-Dimethoxy-phenyl)-quinazolin-6-yl]-2-methoxy-phenoxy}-ethylamine

The title compound is obtained in a similar manner as described in Example **1** starting with 6-(3-benzyloxy-4-methoxy-phenyl)-4-(3,4-dimethoxy-phenyl)-quinazoline (Example **74**) and using tert-butyl N-(2-bromoethyl)carbamate (Fluka, Buchs, Switzerland): ES-MS: 432 (M + H)⁺; analytical HPLC: tᵣₑₜ = 2.81 min (Grad 1).

### Example 141

### 4-(3,4-Dimethoxy-phenyl)-6-(3-ethoxy-4-methoxy-phenyl)-quinazoline

The title compound is obtained in a similar manner as described in Example **1** starting with 6-(3-benzyloxy-4-methoxy-phenyl)-4-(3,4-dimethoxy-phenyl)-quinazoline (Example **74**) and using iodoethane (Fluka, Buchs, Switzerland): ES-MS: 417 (M + H)⁺; analytical HPLC: tᵣₑₜ = 3.74 min (Grad 1).

### Example 142

### 4-[4-(3,4-Dimethoxy-phenyl)-quinazolin-6-yl]-2-methoxy-benzamide

The title compound is obtained in a similar manner as described in Example 120 using ammonia (A2) and starting with 4-[4-(3,4-dimethoxy-phenyl)-quinazolin-6-yl]-2-methoxy-benzoic acid (Example 151a): ES-MS: 415 (M + H)⁺; analytical HPLC: tᵣₑₜ = 3.15 min (Grad 1).

The starting material is prepared as follows:

### Example 142a

### 4-[4-(3,4-Dimethoxy-phenyl)-quinazolin-6-yl]-2-methoxy-benzoic acid

The title compound is obtained in a similar manner as described in Example 119 starting with 4-[4-(3,4-Dimethoxy-phenyl)-quinazolin-6-yl]-2-methoxy-benzoic acid methyl ester (Example 58): ES-MS: 417 (M + H)⁺; analytical HPLC: tᵣₑₜ = 3.22 min (Grad 1).

### Example 143

### 5-[4-(3,4-Dimethoxy-phenyl)-quinazolin-6-yl]-pyridine-2-carboxylic acid amide

A mixture of 80 mg (0.204 mmol) of 5-[4-(3,4-dimethoxy-phenyl)-quinazolin-6-yl]-pyridine-2-carbonitrile (Example **86**) in 2 ml dioxane and 0.51 ml (0.51 mmol) 1 M aqueous LiOH is stirred for 100 min at 100°C. The reaction mixture is quenched with 0.51 ml (0.51 mmol) 1 M aqueous HCl, diluted and extracted with EtOAc and CH₂Cl₂. The combined organic layer are dried over Na₂SO₄, filtered and evaporated *in vacuo.* The residue is purified by flash chromatography (CH₂Cl₂/MeOH 0% to 4%) to yield the title compound as a pale yellow solid. ES-MS: 387 (M + H)⁺; analytical HPLC: tᵣₑₜ = 2.99 min (Grad 1).

### Example 144

### C-{5-[4-(3,4-Dimethoxy-phenyl)-quinazolin-6-yl]-pyridin-2-yl}-methylamine

A mixture of 95 mg (0.204 mmol) of 5-[4-(3,4-dimethoxy-phenyl)-quinazolin-6-yl]-pyridine-2-carbonitrile (Example **86**) in 5 ml MeOH, 0.25 ml concentrated aqueous ammoniac and a spatula tip of Nickel Raney is shacked for 45 h at rt under 1 bar hydrogen. The catalyst is filtered off and washed with MeOH. The filtrate is evaporated *in vacuo.* The residue is purified by preparative RP-HPLC (H₂O/ CH₃CN and 0.1% TFA), The pure fractions are basified with NaHCO₃, concentrated and extracted with EtOAC (3x). The combined organic layers are dried over Na₂SO₄, filtered and evaporated to provide the title compound as an orange solid. ES-MS: 373 (M + H)⁺; analytical HPLC: tᵣₑₜ = 2.53 min (Grad 1).

### Example 145

### 6-(3,4-Dimethoxy-phenyl)-4-[6-(4-methanesulfonyl-piperazin-1-yl)-pyridin-3-yl]-quiriazoline

A mixture of 45 mg (0.1 mmol) of 6-(3,4-dimethoxy-phenyl)-4-(6-piperazin-1-yl-pyridin-3-yl)-quinazoline (Example **145a**) in 1.5 ml pyridine and 17.4 mg (0.15 mmol) methanesulfonyl chloride (Fluka, Buchs, Switzerland) is stirred for 70 min at rt. The reaction mixture is diluted with water and extracted with EtOAc (2x). The combined organic layer are washed with brine, dried over Na₂SO₄, filtered and evaporated *in vacuo*. The residue is adsorbed on silica gel and purified by flash chromatography (CH₂Cl₂/MeOH 0% to 3%) to yield the title compound as a yellow solid. ES-MS: 506 (M + H)⁺; analytical HPLC: tᵣₑₜ = 3.13 min (Grad 1).

The starting material is prepared as follows:

### Example 145a

### 6-(3,4-Dimethoxy-phenyl)-4-(6-piperazin-1-yl-pyridin-3-yl)-quinazoline

The title compound is synthesized in a similar manner as in Example 1 starting with 4-{5-[6-(3,4-dimethoxy-phenyl)-quinazolin-4-yl]-pyridin-2-yl}-piperazine-1-carboxylic acid tert-butyl ester (Example **100**): . ES-MS: 428 (M + H)⁺; analytical HPLC: tᵣₑₜ = 2.79 min (Grad 1).

### Example 146

### 1-(4-{5-[6-(3,4-Dimethoxy-phenyl)-quinazolin-4-yl]-pyridin-2-yl}-piperazin-1-yl)-ethanone

The title compound is synthesized in a similar manner as in Example **145** starting with 6-(3,4-dimethoxy-phenyl)-4-(6-piperazin-1-yl-pyridin-3-yl)-quinazoline (Example **145a**) and acetyl chloride (Fluka, Buchs, Switzerland): ES-MS: 470 (M + H)⁺; analytical HPLC: tᵣₑₜ = 2.98 min (Grad 1).

### Example 147

### 1-{4-[6-(3,4-Dimethoxy-phenyl)-quinazolin-4-yl]-phenyl}-pyrrolidin-2-one

Under Ar, a mixture of 75 mg (0.174 mmol) of 4-(4-bromo-phenyl)-6-(3,4-dimethoxy-phenyly quinazoline (Example **87**), 1 mg (0.0009 mmol) bis(dibenzylideneacetone) palladium (II) (Fluka, Buchs, Switzerland), 1.6 mg (0.0027 mmol) of Xantphos (9,9-dimethyl-9H-xanthene-4,5-diyl)bis[diphenylphosphine], Aldrich, Buchs, Switzerland) 81 mg (0.244 mmol) of cesium carbonate and 17.8 mg (0.209 mmol) 2-pyrrolidinone (Fluka, Buchs, Switzerland) in 0.18 ml dioxane is stirred for 22 h at 100°C. The reaction mixture diluted with EtOAc and washed with water and brine, dried over Na₂SO₄, filtered and evaporated *in vacuo*. The residue is adsorbed on silica gel and purified by flash chromatography (CH₂Cl₂/fMeOH 0% to 3%) to yield the title compound as a yellow solid. ES-MS: 426 (M + H)⁺; analytical HPLC: tᵣₑₜ = 3.55 min (Grad 1),

Commercially available cyclic aminocarbonyl starting materials:
- **L1**: 2-azetidinone (Fluka, Buchs, Switzerland)
- **L2**: 2-piperidinone (Fluka, Buchs, Switzerland)
- **L3**: 2-oxazolidinone (Fluka, Buchs, Switzerland)
- **L4**: 1-methyl-2-imidazolidinone (Acros, Basel, Switzerland)

The following compounds (Table 6) are prepared in a similar manner as described in Example **147** with the cyclic aminocarbonyl starting material given:

**Table 6**

| Example | Amine | Compound Name | ES-MS (M+H)⁺ | tᵣₑₜ Grad. 1 [min] |
|---|---|---|---|---|
| 148 | **L1** | 1-{4-[6-(3,4-Dimethoxy-phenyl)-quinazolin-4-yl]-phenyl}-azetidin-2-one | 412 | 3.52 |
| 149 | **L2** | 1-{4-[6-(3,4-Dimethoxy-phenyl)-quinazolin-4-yl]-phenyl}-piperidin-2-one | 440 | 3.58 |
| 150 | **L3** | 3-{4-[6-(3,4-Dimethoxy-phenyl)-quinazolin-4-yl]-phenyl}-oxazolidin-2-one | 428 | 3.44 |
| 151 | **L4** | 1-{4-[6-(3,4-Dimethoxy-phenyl)-quinazolin-4-yl]-phenyl}-3-methyl-imidazolidin-2-one | 441 | 3.49 |

### Example 152

### 6-(3,4-Dimethoxy-phenyl)-4-pyrazol-1-yl-quinazoline

A mixture of 64 mg (0.214 mmol) of 4-chloro-6-(3,4-dimethoxy-phenyl)-quinazoline (Example **152a**) and 73 mg pyrazole (Fluka, Buchs, Switzerland) in 2 ml DMF is stirred at rt overnight. The reaction mixture is quenched with water and extracted with EtOAc (2x). The organic layers are washed with brine, dried over Na₂SO₄, filtered and evaporated *in vacuo*. The residue is adsorbed on Isolute sorbent (Isolute HM-N) and purified by RP-MPLC (H₂O/ CH₃CN and 3% PrOH), The pure fractions are concentrated and the product precipitates. The solid is filtered off, dissolved in CH₂Cl₂, dried over Na₂SO₄, filtered and evaporated to provide the title compound as an off-white solid. ES-MS: 333 (M + H)⁺; analytical HPLC: tᵣₑₜ = 3.71 min (Grad 1),

The starting material is prepared as follows:

### Example 152a

### 4-Chloro-6-(3,4-dimethoxy-phenyl)-quinazoline

The title compound is synthesized in a similar manner as described in Example **3a** using **B1**; ES-MS: 301 (M + H)⁺; analytical HPLC: tᵣₑₜ = 3.63 min (Grad 1).

### Example 153

### 6-(3,4-Dimethoxy-phenyl)-4-[1,2,4]triazol-1-1-ly-auinazoline

The title compound is synthesized in a similar manner as described in Example **152** using 1,2,4-triazole (Fluka, Buchs, Switzerland); ES-MS: 334 (M + H)⁺; analytical HPLC: tᵣₑₜ = 3.41 min (Grad 1).

### Example 154

### 6-(3,4-Dimethoxy-phenyl)-4-pyrrol-1-quuinazoline

The title compound is synthesized in a similar manner as described in Example **152** using pyrrole (Fluka, Buchs, Switzerland) that is deprotonated beforehand with NaH; ES-MS: 332 (M + H)⁺; analytical HPLC: tᵣₑₜ = 3.75 min (Grad 1).

### Example 155

### 4,6-Bis-(3,4-dimethoxy-phenyl)-5-fluoro-quinazoline

The title compound is obtained in a similar manner as described in Example **3** starting from 5-fluoro-3H-quinazolin-4-one (Example **155a**) and using boronic acid **B1.** ES-MS: 421 (M + H)⁺; analytical HPLC: tret = 3.55 min (Grad 1).

### Example 155a

### 5-Fluoro-3H-quinazolin-4-one

2.7 g (11.4 mmol) of 2-amino-6-fluorobenzamide (ABCR, Karlsruhe, Germany) in 50 ml triethylorthoformate (Fluka, Buchs, Switzerland) is heated for 46 h at 130°C. The reaction mixture is evaporated to dryness *in vacuo*. The residue is triturated in hexane/EtOAc and the solid is filtered and dry to give the title compound as as an off-white solid. ES-MS: 243, 245 (M + H)+; analytical HPLC: tᵣₑₜ = 2.49min (Grad 1).

### Example 156

### 4-[6-(3,4-Dimethoxy-phenyl)-5-fluoro-quinazolin-4-yl]-benzamide

The title compound is obtained in a similar manner as described in Example **3** starting from 5-fluoro-3H-quinazolin-4-one (Example **155a**) and using boronic acid **B19.** ES-MS: 404 (M + H)+; analytical HPLC: tret = 3.09 min (Grad 1).

### Example 157

### {5-[4-(3,4-Dimethoxy-phenyl)-quinazolin-6-yl]-pyridin-2-yl}-(2-methoxy-ethyl)-amine

The title compound is synthesized by Suzuki coupling in a similar manner as described in Example **1b** starting from 4-(3,4-dimethoxy-phenyl)-6-(4,4,5,5-tetramethyl-[1,3,2]dioxaborolan-2-yl)-quinazoline (Example **157a**) and using (5-bromo-pyridin-2-yl)-(2-methoxy-ethyl)-amine (ES-MS: 231, 233 (M + H)+; analytical HPLC: tret = 1.96 min (Grad 1)) obtained in a similar manner as **B60a** using **A12**: ES-MS: 417 (M + H)+; analytical HPLC: tret = 2.73 min (Grad 1).

### Example 158

### 4-(3,4-Dimethoxy-phenyl)-6-(4,4,5,5-tetramethyl-[1,3,2]dioxaborolan-2-yl)-quinazoline

The title compound is synthesized in a similar manner as described in **B57** starting with 6-Bromo-4-(3,4-dimethoxy-pheny)-quinazoline (Example **1b**): ES-MS: 393 (M + H)+; analytical HPLC: tᵣₑₜ = 2.50 min (Grad 1).

### Example 159: Soft capsules

5000 soft gelatin capsule, each comprising as active ingredient 0.05 g of one of the compounds of formula I mentioned in the preceding Examples, are prepared as follows:

### Composition

| | |
|---|---|
| Active ingredient | 250 g |
| Lauroglycol | 2 litres |

Preparation process: The pulverized active ingredient is suspended in Lauroglykol® (propylene glycol laurate, Gattefosse S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. A compound of the formula I, 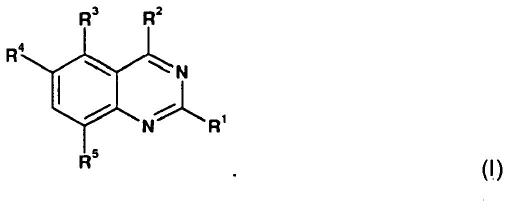 wherein
R¹ is hydrogen; or amino that is unsubstituted or monosubstituted with alkyl or cycloalkyl;
R² is an unsubstituted or substituted aryl or unsubstituted or substituted heteroaryl;
R³ is hydrogen, halogen, alkyl, alkoxy or cyano;
R⁴ is unsubstituted or substituted aryl or unsubstituted or substituted heteroaryl; and
R⁵ is hydrogen, methyl or methyl substituted with halogen;
with the proviso that if R⁴ is unsubstituted or substituted pyrazolyl then R¹ is amino that is unsubstituted or monosubstituted with alkyl or cycloalkyl and R², R³ and R⁵ are as defined above;
and with the proviso that if R² is unsubstituted or substituted oxoindolyl, then R¹ is amino that is unsubstituted or monosubstituted with alkyl or cycloalkyl and R³, R⁴ and R⁵ are as defined above;
or a tautomer thereof or a N-oxide thereof, or a salt, or a hydrate or solvate thereof.

2. A compound of the formula I according to claim 1, wherein
R¹ is hydrogen; or amino that is unsubstituted or monosubstituted with C₁-C₇ (preferably C₁-C₄)-alkyl or C₃-C₈ (preferably C₃-C₅)-cycloalkyl;
R² is unsubstituted or substituted aryl wherein aryl is selected from the group consisting of phenyl, naphthyl, biphenylenyl, indacenyl, acenaphthylenyl, fluorenyl, phenalenyl, phenanthrenyl and anthracenyl, each of which is unsubstituted or substituted by one or more, preferably up to three, substituents independently selected from the group consisting of C₁-C₇-alkyl; C₂-C₇-alkenyl; C₂-C₇-alkinyl; C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and is unsubstituted or substituted by C₁-C₁-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl, for example pyrrolidino-C₁-C₇-alkyl, piperidino-C₁-C₇-alkyl, morpholino-C₁-C₇-alkyl, thiomorpholinO-C₁-C₇-alkyl, N-C₁-C₇-alkyl-piperazino-C₁-C₇-alkyl, or N-mono- or N,N-di-(C₁-C₇-alkyl)-amino-substituted or unsubstituted pyrrolidino-C₁-C₇-alkyl; (pyrrolidinyl (especially pyrrolidino), piperidinyl (especially piperidino), piperazinyl (especially piperazino), pyridinyl, pyrimidinyl, pyrazinyl or pyridazinyl)-oxy-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), pyridinyl, pyrimidinyl, pyrazinyl or pyridazinylycarbonyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇ alkyl; hydroxy-C₁-C₇-alkyl; C₁-C₇-alkoxy-C₁-C₇-alkyl; C₁-C₇-alkoxy-C₁-C₇-alkoxy-C₁-C₇-alkyl; phenyloxy- or naphthyloxy-C₁-C₇-alkyl; phenyl-C₁-C₇alkoxy- or naphthyl-C₁-C₇-alkoxy-C₁-C₇. alkyl; amino-C₁-C₇-alkyl; N-mono- or N,N-di-(C₁-C₇-alkyl and/or mono-C₁-C₇-alkoxy-C₁-C₇alkyl and/or (mono- or di-(C₁-C₇-alkyl)-amino-C₁-C₇-alkyl)-amino-C₁-C₇-alkyl; C₁-C₇-alkoxy-C₁-C₇alkylamino-C₁-C₇alkyl; mono- or di-[C₆-C₁₈-aryl-C₁-C₇-alkyl in which aryl is unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; (pyrrolidinyl, piperidinyl, piperazinyl, morpholino, thiomorpholino, pyridinyl, pyrimidinyl, pyrazinyl or pyridazinyl)-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; (pyrrolidinyl, piperidinyl, piperazinyl, pyridinyl, pyrimidinyl, pyrazinyl or pyridazinyl)-oxy-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁C₇-alkyl; and/or (pyrrolidinyl, piperidinyl, piperazinyl, pyridinyl, pyrimidinyl, pyrazinyl or pyridazinyl)-carbonyl-C₁-C₇-alkyl wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyl; especially naphthyl- and/or phenyl-C₁-C₇-alkyl]-amino-C₁-C₇-alkyl; C₁-C₇-alkanoylamino-C₁-C₇-alkyl; carboxy-C₁-C₇alkyl; benzoyl- or naphthoylamino-C₁-C₇-alkyl; C₁-C₇-alkylsulfonylamino-C₁-C₇-alkyl; phenyl- or naphthylsulfonylamino-C₁-C₇-alkyl wherein phenyl or naphthyl is unsubstituted or substituted by one or more, especially one to three, C₁-C₇-alkyl moieties, phenyl- or naphthyl-C₁-C₇-alkylsulfonylamino-C₁-C₇-alkyl, halo; hydroxy; C₁-C₇-alkoxy; C₆-C₁₈-aryl-C₁-C₇-alkoxy in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by C₁-C₇-alkoxy, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇-alkoxy; hydroxy-C₁-C₇-alkoxy; C₁-C₇-alkoxy-C₁-C₇-alkoxy; amino-C₁-C₇-alkoxy; N-C₁-C₇-alkanoylamino-C₁-C₇-alkoxy; N-unsubstituted-, N-mono- or N,N-di-(C₁-C₇-ralkyl)carbamoyl-C₁-C₁-alkoxy; phenyl- or naphthyloxy; phenyl- or naphthyl-C₁-C₇-alkyloxy; (pyrrolidinyl, piperidinyl, piperazinyl, pyridinyl, pyrimidinyl, pyrazinyl or pyridazinyl)-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkytamino, by halo, by C₁-C₇-alkoxy and/or by halo-C₁-C₇-alkyl; (pyrrolidinyl, piperidinyl, piperazinyl, pyridinyl, pyrimidinyl, pyrazinyl or pyridazinyl)-oxy-C₁-C₇-alkoxy wherein pyrrolidinyl, piperidinyl, piperazinyl, pyridinyl, pyrimidinyl, pyrazinyl or pyridazinyl are unsubstituted or substituted by C₁-C₇-alkyl, by pyrrolidinyl, by piperazinyl, by amino, by N-mono- and/or N,N-di-C₁-C₇-alkylamino, by halo, by C₁-C₇-alkoxy and/or by halo-C₁-C₇-alkyll; C₁-C₇-alkanoyloxy; benzoyl- or naphthoyloxy; C₁-C₇-alkylthio, halo-C₁-C₇-alkthio; C₁-C₇-alkoxy-C₁-C₇-alkylthio; phenyl- or naphthylthio; phenyl- or naphthyl-C₁-C₇-alkylthio; C₁-C₇-alkanoylthio; benzoyl- or naphthaylthio; nitro; amino; mono- or di-(C₁-C₇-alkyl)-amino; mono- or di-(naphthyl- or phenyl-C₁-C₇-alkylyamino; C₁-C₇-alkanoylamino; benzoyl- or naphthoylamino; C₁-C₇-alkylsulfonylamino; phenyl- or naphthylsulfonylamino wherein phenyl or naphthyl is unsubstituted or substituted by one or more, especially one to three, C₁-C₇-alkyl moieties; phenyl-or naphthyl-C₁-C₇-alkylsulfonylamino; C₁-C₇-alkanoyl; C₁-C₇-alkoxy-C₁-C₇-alkanoyl; carboxyl; C₁-C₇-alkoxy-carbonyl; phenoxy- or naphthoxycarbonyl; phenyl- or naphthyl-C₁-C₇-alkoxycarbonyl, C₁-C₁₀-, especially C₁-C₄-alkylendioxy; carbamoyl; N-mono- or N,N-di-(C₁-C₇-alkyl, naphthyl-C₁-C₇-alkyl, pyrrolidinyl-C₁-C₇-alkyl, piperidinyl -C₁-C₇-alkyl, piperazinyl- or N-C₁-C₇-alkylypiperazinyl-C₁-C₇-alkyl, phenyl-C₁-C₇-alkyl, mono-C₁-C₇-alkoxy-C₁-C₇-alkyl and/or (N'-mono- or N'N'-di-(C₁-C₇-alkyl)-amino-C₁-C₇-alkylyamino-carbonyl; N-C₁-C₇-alkoxy-C₁-C₇-alkylcarbamoyl; pyrrolidin-1-carbonyl; amino-N-pyrrolidin-1-carbonyl; N-mono- or N,N-di(C₁-C₇-alkyl)amino-pyrrolidin-1-carbonyl; piperidin-1-carbonyl; morpholin-4-carbonyl; thiomorpho-lin-4-carbonyl; S-oxo-thiomorpholin-4-carbonyl; S,S-dioxothiomorpholin-4-carbonyl; piperazin-1-carbonyl; N-C₁-C₇-alkyl-piperazin-1-carbonyl; N-C₁-C₇-alkoxycarbonyl-piperazin-1-carbonyl; N-mono- or N,N-di-(C₁-C₇-alkyl)-amino-substituted or unsubstituted pyrrolidinyl-C₁-C₇-alkyl; cyano; C₁-C₇-alkenylene or -alkinylene; C₁-C₇-alkylsulfonyl; phenyl- or naphthylsulfonyl wherein phenyl or naphthyl is unsubstituted or substituted by one or more, especially one to three, C₁-C₇-alkyl moieties; phenyl- or naphthyl-C₁-C₇-alkylsulfonyl; sulfamoyl; N-mono or N,N-di-(C₁-C₇-alkyl, phenyl-, naphthyl-, pyrrolidinyl(especially pyrrolidino)-C₁-C₇-alkyl, piperidinyl(especially piperidino)-C₁-C₇-alkyl, piperazinyl(especially piperazino)-C₁-C₇-alkyl, N-C₁-C₇-alkylpiperazinyl(especially 4-C₁-C₇-alkylpiperazino)-C₁-C₇-alkyl, phenyl-C₁-C₇-alkyl- and/or naphthyl-C₁-C₇-alkyl)-aminosulfonyl; pyrazolyl; pyrazolidinyl; pyrrolyl; pyridyl that is unsubstituted or substituted by C₁-C₇-alkoxy, and/or by halo-C₁-C₇-alkyl, pyrrolidinyl; piperidinyl; morpholinyl; thiomorpholinyl; S-oxo-thiomorpholinyl; S,S-dioxothiomorpholinyl; piperazinyl; N-C₁-C₇-alkyl-piperazinyl; 4-(phenyl-C₁-C₇-alkyl)-piperazinyl; 4-(naphthyl-C₁-C₇-alkyl)-piperazinyl; 4-(C₁-C₇-alkoxycarbonyl)-piperazinyl; 4-(phenyl-C₁-C₇-alkoxycarbonyl)-piperazinyl and 4-(naphthyl-C₁-C₇-alkoxycarbonyl)-piperazinyl;
or is unsubstituted or substituted heteroaryl where heteroaryl is selected from the group consisting of imidazolyl, thiophenyl, pyrazolyl, pyrrolyl, imidazolyl, pyridyl, pyrimidinyl,
pyridazinyl, furyl, 2H- or 4H-pyranyl, oxazolyl, thiazolyl, 5H-indazolyl, isoindolyl, quinolyl, isoquinolinyl, phthalazinyl, 1,8-naphthyridinyl, quinoxalinyl, quinazolinyl, cinnolinyl, indolizinyl, 4H-quinolizinyl, pteridinyl, purinyl, carbazolyl, beta-carbolinyl, acridinyl, phenanthridinyl, phenyzinyl, 1,7-phenanthrolinyl, perimidinyl, benzofuranyl, isobenzofuranyl, 2H-chromenyl, 4aH-isochromenyl, thianthrenyl, xanthenyl, phenoxathiinyl, phenoxazinyl or phenothiazinyl, each of which is unsubstituted or substituted as mentioned above for aryl;
R³ is hydrogen, halogen, C₁-C₇-alkyl, C₁-C₇-alkoxy or cyano;
R⁴ is unsubstituted or substituted aryl or unsubstituted or substituted heteroaryl, independently selected from unsubstituted or substituted aryl as defined for R² and unsubstituted or substituted heteroaryl where heteroaryl is selected from the group consisting of imidazolyl, thiophenyl, pyrrolyl, imidazolyl, pyridyl, pyrimidinyl, pyridazinyl, furyl, 2H- or 4H-pyranyl, oxazolyl, thiazolyl, 5H-indazolyl, indolyl, isoindolyl, quinolyl, isoquinolinyl, phthalazinyl, 1,8-naphthyridinyl, quinoxalinyl, quinazolinyl, cinnolinyl, indolizinyl, 4H-quinolizinyl, pteridinyl, purinyl, carbazolyl, beta-carbolinyl, acridinyl, phenanthridinyl, phenyzinyl, 1,7-phenanthrolinyl, perimidinyl, benzofuranyl, isobenzofuranyl, 2H-chromenyl, 4aH-isochromenyl, thianthrenyl, xanthenyl, phenoxathiinyl, phenoxazinyl or phenothiazinyl, as defined for R²; or, if R¹ is amino or amino monosubstituted with C₁-C₇ (preferably C₁-C₄)-alkyl or C₃-C₈(preferably C₃-C₅)-cycloakly, can also be pryrazolyl, where each heteroaryl is unsubstituted or substituted as described above for aryl R²; and
R⁵ is hydrogen, methyl or methyl substituted with halogen;
or a tautomer thereof or a N-oxide thereof, or a pharmaceutically acceptable salt, or a hydrate or solvate thereof.

3. A compound of the formula I according to claim 1 wherein
R¹ is hydrogen, amino, N-mono-C₁-C₁₀ (preferably C₁-C₄)-alkylamino or C₃-C₈ (preferably C₃-C₅)-cycloalkylamino,
R² is phenyl, naphthyl, pyrrolyl, thiophenyl, pyrazolyl, triazolyl, pyridyl, quinolyl or quinoxalinyl, or is pyrrolopyridinyl, each of which is unsubstituted or substituted by one or more substituents independently selected from the group consisting of C₁-C₇-alkyl, halo-C₁-C₇-alkyl, phenyl that is unsubstituted or substituted by one to three substituents independently selected from hydroxyl-C₁-C₇-alkyl, C₁-C₇-alkoxy-C₁-C₇-alkyl, C₁-C₇-alkoxy, amino and carbamoyl, halo, hydroxy, C₁-C₇-alkoxy, C₁-C₇alkoxy-C₁-C₇-alkoxy, halo-C₁-C₇-alkoxy, such as trifluoromethoxy, amino, N-mono- or N,N-di-(C₁-C₇-alkyl, phenyl-C₁-C₇-alkyl and/or naphthyl-C₁-C₇-alkyl)-amino, C₁-C₇-alkanoylamino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, cyano, carbamoyl, N-mono- or N,N-di-(C₁-C₇-alkyl, N',N'-di-(C₁-C₁-alkyl)amino-C₁-C₇-alkyl, pyrrolidino-C₁-C₇-alkyl and/or phenyl-C₁-C₇-alkyly carbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkyl)-sulfamoyl, pyrazolyl, pyrazolidinyl, pyrrolyl, pyrrolidinyl, piperidinyl, piperazinyl, 4-C₁-C₇-alkyl-piperazinyl, 4-(pheny)-C₁-C₇-alky)-piperazinyl, 4-(naphthyl-C₁-C₇-alkyl)-piperazinyl, 4-(C₁-C₇-alkanoyly piperazinyl, 4-(C₁-C₇-alkoxycarbonyl)-piperazinyl, 4-(phenyl-C₁-C₇-alkoxycarbonyl)-piperazinyl, 4-(naphthyl-C₁-C₇-alkoxycarbonyl)-piperazinyl, 4-(C₁-C₇-alkanesulfonyl)-piperazinyl, 2-oxo-pyrrolidin-1-yl, 2-oxo-azetidin-1-yl, 2-oxo-piperidin-1-yl, 3-C₁-C₇-alkyl-2-oxo-imidazolidin-1-yl, morpholinyl, thiomorpholinyl, S-oxothiomorpholinyl and S,S-dioxothiomorpholinyl, and/or from 2-amino-pyrimidin-5-yl-C₁-C₇-alkyl, 4-C₁-C₇-alkyl-piperazin-1-carbonyl-C₁-C₇-alkoxy, 4-pyrrolidino-piperidin-1-carbonyl-C₁-C₇-alkoxy, 4-pyrrolidinopiperidin-1-yl-C₁-C₇-alkoxy, 4-C₁-C₇-alkyl-piperazino-C₁-C₇-alkoxy, pyridin (e.g.-2)-yloxy-C₁-C₇-alkoxy, pyrimidin(e.g. -4)-yloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₇-alkoxy-substituted) pyridin(e.g. -3))-yl;
R³ is hydrogen, or it is halo, preferably hydrogen,
R⁴ is phenyl, naphthyl, pyrrolyl, thiophenyl, triazolyl, pyridyl, quinolinyl, quinoxalinyl, furanyl or 1H-pyrrolo[2,3-b]-pyridin-5-yl, each of which is unsubstituted or substituted by one or more substituents independently selected from the group consisting of halo-C₁-C₇-alkyl, such as trifluoromethyl, amino-C₁-C₇alkyl, such as aminomethyl, halo, hydroxy, C₁-C₇-alkoxy, C₁-C₇-alkoxy-C₁-C₇-alkoxy, amino-C₁-C₇-alkoxy, phenyl-C₁-C₇-alkoxy, amino, N-mono- or N,N-di-(C₁-C₇-alkyl, hydroxyl-C₁-C₇-alkyl, phenyl-C₁-C₇-alkyl and/or naphthyl-C₁-C₇-alkyl)-amino, C₁-C₇-alkanoyl, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, cyano, carbamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkylycarbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxothiomorpholin-4-carbonyl, S,S-dioxothiomorpholin-4-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alky and/or phenyl-C₁-C₇-alky)-sulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxycarbonyl)-piperazinyl, 4-(phenyl-C₁-C₇-alkoxycarbonyl)-piperazinyl, 4-(naphthyl-C₁-C₇-alkoxycarbonyl)-piperazinyl, morpholinyl, thiomorpholinyl, S-oxothiomorpholinyl and S,S-dioxothiomorpholinyl, and/or from 2-amino-pyrimidin-5-yl-C₁-C₇ alkyl, 4-C₁-C₇-alkyl-piperarzin-1-carbonyl-C₁-C₇-alkoxy, 4-pyrrolidino-piperidin-1-carbonyl-C₁-C₇-alkoxy, 4-pyrrolidino-piperidin-1-yl-C₁-C₇-alkoxy, 4-C₁-C₇-alkyl-piperazino-C₁-C₇-alkoxy, pyridin (e.g.-2)-yloxy-C₁-C₇-alkoxy, pyrimidin(e.g. -4)-yloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₇-alkoxy-substituted) pyridin(e.g. -3))-yl; and
R⁵ is hydrogen;
or a tautomer thereof or a N-oxide thereof, or a pharmaceutically acceptable salt, or a hydrate or solvate thereof.

4. A compound of the formula I according to claim 1 wherein
R¹ is hydrogen, amino, N-mono-C₁-C₁₀ (preferably C₁-C₄)-alkylamino or C₃-C₈(preferably C₃-C₅)-cycloalkylamino,
R² is phenyl, naphthyl, pyrrolyl, thiophenyl, pyrazolyl, triazolyl, pyridyl, quinolyl or quinoxalinyl, each of which is unsubstituted or substituted by one or more substituents independently selected from the group consisting of halo, hydroxy, C₁-C₇-alkoxy, C₁-C₇-alkoxy-C₁-C₇-alkoxy, amino, N-mono- or N,N-di-(C₁-C₇-alkyl, phenyl-C₁-C₇-alkyl and/or naphthyl-C₁-C₇-alkylyamino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl, N',N'-di-(C₁-C₇-alkyl)amino-C₁-C₇alkyl, pyrrolidino-C₁-C₇-alkyl and/or phenyl-C₁-C₇-alkyl)-carbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkylysulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkylypiperazinyl, 4-(C₁-C₇-alkoxycarbonyl)-piperazinyl, 4-(phenyl-C₁-C₇-alkoxycarbonyl)-piperazinyl, 4-(naphthyl-C₁-C₇-alkoxycarbonyl)-piperazinyl, morpholinyl, thiomorpholinyl, S-oxothiomorpholinyl and S,S-dioxothiomorpholinyl, and/or from 2-amino-pyrimidin-5-yl-C₁-C₇-alkyl, 4-C₁-C₇-alkyl-piperazin-1-carbonyl-C₁-C₇-alkoxy, 4-pyrrolidino-piperidin-1-carbonyl-C₁-C₇-alkoxy, 4-pyrrolidino-piperidin-1-yl-C₁-C₇-alkoxy, 4-C₁-C₇-alkyl-piperazino-C₁-C₇alkoxy, pyridin (e.g.-2)-yloxy-C₁-C₇-alkoxy, pyrimidin(e.g. -4)-yloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₁-alkoxy-substituted) pyridin(e.g. -3))-yl;
R³ is hydrogen or halo, preferably hydrogen,
R⁴ is phenyl, naphthyl, pyrrolyl, thiophenyl, triazolyl, pyridyl, quinolinyl or quinoxalinyl each of which is unsubstituted or substituted by one or more substituents independently selected from the group consisting of halo, hydroxy, C₁-C₇-alkoxy, C₁-C₇-alkoxy-C₁-C₇-alkoxy, amino, N-mono- or N,N-di-(C₁-C₇-alkyl, phenyl-C₁-C₇-alkyl and/or naphthyl-C₁-C₇-alkyl)-amino, carboxy, C₁-C₇-alkoxycarbonyl, phenyl-C₁-C₇-alkoxycarbonyl, naphthyl-C₁-C₇-alkoxycarbonyl, phenoxycarbonyl, naphthoxycarbonyl, C₁-C₄-alkylendioxy, carbamoyl, N-mono- or N,N-di-(C₁-C₇-alkyl, N',N'-di-(C₁-C₇-alkyl)amino-C₁-C₇-alkyl, pyrrolidino-C₁-C₇-alkyl and/or phenyl-C₁-C₇-alkylycarbamoyl, piperidin-1-carbonyl, piperazin-1-carbonyl, 4-C₁-C₇-alkyl-piperazin-1-carbonyl, morpholin-4-carbonyl, thiomorpholin-4-carbonyl, S-oxo-thiomorpholin-4-carbonyl, S,S-dioxothiomorpholin-4-carbonyl, sulfamoyl, N-mono- or N,N-di-(C₁-C₇-alkyl, N',N'-di-(C₁-C₇-alkyl)amino-C₁-C₇-alkyl, pyrrolidino-C₁-C₇-alky and/or phenyl-C₁-C₇-alkyl)-sulfamoyl, pyrazolyl, pyrazolidinyl, pyrrolyl, pyrrolidinyl, piperidinyl, piperazinyl, 4-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxycarbonyl)-piperazinyl, 4-(pheny)-C₁-C₇-alkoxycarbonyl)-piperaziny), 4-(naphthyl-C₁-C₇-alkoxycarbonyl)-piperazinyl, morpholinyl, thiomorpholinyl, S-oxothiomorpholinyl and S,S-dioxothio-morpholinyl, and/or from 2-amino-pyrimidin-5-yl-C₁-C₇-alkyl, 4-C₁-C₇-alkyl-piperarzin-1-carbonyl-C₁-C₇-alkoxy, 4-pyrrolidino-piperidin-1-carbonyl-C₁-C₇-alkoxy, 4-pyrrolidino-piperidin-1-yl-Cₗ-C₇-alkoxy, 4-C₁-C₇-alkyl-piperazino-C₁-C₇-alkoxy, pyridin (e.g.-2)-yloxy-C₁-C₇-alkoxy, pyrimidin(e.g. -4yyloxy-C₁-C₇-alkoxy, N,N-di(C₁-C₇-alkyl)amino-pyrrolidin-1-carbonyl and (unsubstituted or C₁-C₇-alkoxy- and/or halo-C₁-C₇-alkoxy-substituted) pyridin(e.g. -3))-yl; and
R⁵ is hydrogen;
or a tautomer thereof or a N-oxide thereof, or a pharmaceutically acceptable salt, or a hydrate or solvate thereof.

5. A compound of the formula I according to claim 1, wherein
R¹ is hydrogen, amino, methylamino, n-propylamino or cyclopropylamino;
R² is phenyl, 3-methoxyphenyl, 4-methoxyphenyl, 3,4-dimethoxyphenyl, 3,4,5-trimethoxyphenyl, 3-chloro-4-n-propoxy-phenyl, 4-carboxy-3-methoxyphenyl, 4-methoxycarbonyl-3-methoxyphenyl, 4-carbamoylphenyl, 4-N-methylcarbamoyl-3-methoxy-phenyl, 4-(N,N-dimethyl-carbamoyl)-3-methoxy-phenyl, 4-(4-methylpiperazin-1-carbonyl)-3-methoxyphenyl, 4-(4-morpholin-1-carbonyl)-3-methoxyphenyl, benzo[1,3]dioxol-5-yl, 2,3-dihydro-benzo[1,4]dioxin-6-yl, 4-(piperazin-1-yl)-phenyl, 4-sulfamoyl-phenyl, 4-N,N-dimethyl-sulfamoylphenyl, 4-pyrazolyl-phenyl, pyrrolyl, pyrazolyl, thiophenyl, 1,2,4-triazol-1-yl, 6-methoxy-pyridin-3-yl, or 6-piperazino-pyridin-3-yl;
R³ is hydrogen,
R⁴ is 3-hydroxyphenyl, 4-hydroxyphenyl, 4-hydroxy-3-methoxyphenyl, 3-hydroxy-4-n-propoxyphenyl, 3,4-dimethoxyphenyl, 3,4,5-trimethoxyphenyl, 4-ethoxy-3-methoxyphenyl, 3-(2-methoxy-ethoxy)-4-methoxyphenyl, 3-methoxy-4-(2-methoxy-ethoxy)-phenyl, 3-fluoro-4-methoxyphenyl, 3-chloro-4-n-propoxyphenyl, 4-methoxycarbonyl-3-methoxyphenyl, 4-carbamoylphenyl, N,N-dimethyl-aminosulfonylphenyl, benzo[1,3]dioxol-5-yl, 2,3-dihydrobenzo[1,4]dioxin-6-yl, pyridine-3-yl, 6-methoxy-pyridin-3-yl, 5-methoxy-pyridin-3-yl, 2-amino-pyridin-4-yl, 6-amino-pyridin-3-yl, 6-(piperazin-1-y)-pyridin-3-yl, 6-(4-tert-butoxycarbonyl-piperazin-1-yl)-pyridin-3-yl, 2-(piperazin-1-yl)-pyridin-4-yl or 2-(4-tert-butoxycarbonyl-piperazin-1-yl)-pyridin-4-yl, and
R⁵ is hydrogen,
or a tautomer thereof or a N-oxide thereof, or a pharmaceutically acceptable salt, or a hydrate or solvate thereof.

6. A compound of the formula I according to claim 1, wherein
R¹ is hydrogen, amino, methylamino, n-propylamino or cyclopropylamino;
R² is phenyl, 4-trifluoromethylphenyl, 3-fluorophenyl, 4-fluorophenyl, 4-chlorophenyl, 4-bromophenyl, 3-methoxyphenyl, 4-methoxyphenyl, 3-ethoxyphenyl, 3,4-dimethoxyphenyl, 4-ethoxy-3-methoxy-phenyl, 3,4-diethoxy-phenyl, 3-benzyloxy-4-methoxyphenyl, 4-(2-methoxyethoxy)-3-methoxy-phenyl, 4-trifluormethoxyphenyl, 4-methoxy-3-trifluoromethoxyphenyl, 4-(3-tert-butoxycarbonylamino-propoxy)-3-methoxy-phenyl, 4-(2-tert-butoxycarbonyl-aminoethoxy)-3-methoxy-phenyl, 3,4,5-trimethoxyphenyl, 3-chloro-4-n-propoxy-phenyl, 4-acetylaminophenyl, 4-carboxy-3-methoxyphenyl, 4-methoxycarbonyl-phenyl, 4-methoxycarbonyl-3-methoxyphenyl, 4-cyanophenyl, 4-biphenylyl, 4'-amino-biphenyl-4-yl, 4'-methoxy-biphenyl-4-yl, 4'-hydroxymethyl-biphenyl-4-yl, 4'-methoxymethyl-biphenyl-4-y1,3',4'-dimetho-xy-biphenyl-4-yl, 4'-carbamoyl-biphenyl-4-y1,4-carbamoylphenyl, 4-N-methylcarbamoyl-3-methoxy-phenyl, 4-(N,N-dimethylcarbamoyl)-phenyl, 4-(N-methylcarbamoyl)-phenyl, 4-(N,N-dimethyl-carbamoyl)-3-methoxy-phenyl, 4-(4-methylpiperazin-1-carbonyl)-3-methoxyphenyl, 4-(morpholin-4-carbonyl)-phenyl, 4-(4-morpholin-1-carbonyl)-3-methoxyphenyl, benzo[1,3]di-oxol-5-yl, 2,3-dihydro-benzo[1,4]dioxin-6-yl, 4-(piperazin-1-yl)-phenyl, 4-(2-oxo-pyrrolidin-1-yl)-phenyl, 4-(2-oxo-azetidin-1-yl)-phenyl, 4-(2-oxo-piperidin-1-yl)-phenyl, 4-(3-methyl-2-oxo-imidazolidin-1-yl)-phenyl, 4-methanesulfonyl-phenyl, 4-sulfamoyl-phenyl, 4-N,N-dimethyl-sulfamoylphenyl, 4-pyrazolyl-phenyl, pyrrolyl, pyrazolyl, thiophenyl, especially thiophen-3-yl, 1,2,4-triazol-1-yl, 2-methoxy-pyridin-4-yl, 5-methoxy-pyridin-3-yl, 6-methoxy-pyridin-3-yl, 6-piperazino-pyridin-3-yl, 6-morpholin-4-yl-pyridin-3-yl, 1 H-pyrrolo[2,3-b]pyridin-5-yl, 4-[6-(4-methanesulfonyl)-piperazin-1-yl]-pyridin-3-yl, 5-(4-acetylpiperazin-1-yl)-pyridin-3-yl or 2-[4-(tert-butoxycarbonyl)-piperazin-1-yl]-pyridin-4-yl;
R³ is hydrogen,
R⁴ is 3-hydroxyphenyl, 4-hydroxyphenyl, 4-hydroxy-3-methoxyphenyl, 3-hydroxy-4-n-propoxyphenyl, 3-ethoxyphenyl, 3,4-dimethoxyphenyl, 3,4-diethoxyphenyl, 3,4,5-trimethoxyphenyl, 3-ethoxy-4-methoxy-phenyl, 4-ethoxy-3-methoxyphenyl, 3-(2-methoxy-ethoxy)-4-methoxyphenyl, 3-methoxy-4-(2-methoxy-ethoxy)-phenyl, 3-benzyloxy-4-methoxyphenyl, 4-(3-aminopropoxy)-3-methoxy-phenyl, 5-(3-aminopropoxy)-3-methoxyphenyl, 4-(2-aminoethoxy)-3-methoxy-phenyl, 5-(2-aminoethoxy)-3-methoxy-phenyl, 3-fluoro-4-methoxyphenyl, 3-chloro-4-methoxy-phenyl, 3-chloro-4-n-propoxyphenyl, 4-(3-tert-butoxycarbonylaminopropoxy)-3-methoxy-phenyl, 4-(2-tert-butoxycarbonylamino-ethoxy)-3-methoxy-phenyl, 4-formyl-phenyl, 4-methoxycarbonyl-3-methoxyphenyl, 3-carbamoyl-phenyl, 4-carbamoylphenyl, 4-carbamoyl-3-methoxy-phenyl, 3-sulfamoyl-phenyl, N,N-dimethyl-aminosulfonylphenyl, benzo[1,3]dioxol-5-yl, 2,3-dihydro-benzo[1,4]dioxin-6-yl, 6-aminomethyl-pyridin-3-yl, pyridine-3-yl, 6-methoxy-pyridin-3-yl, 5-methoxy-pyridin-3-yl, 2-methoxy-pyridin-4-yl, 2-amino-pyridin-4-yl, 6-amino-pyridin-3-yl, 6-amino-5-trifluoromethylpyridin-3-yl, 6-dimethylamino-pyridin-3-yl, 6-methylamino-pyridin-3-yl, 6-isobutylamino-pyridin-3-yl, 6-(2-methoxyethylamino)-pyridin-3-yl, 6-(piperazin-1-yl)-pyridin-3-yl, 6-(4-tert-butoxycarbonyl-piperazin-1-yl)-pyridin-3-yl, 2-(piperazin-1-yl)-pyridin-4-yl, 6-carbamoyl-pyridin-3-yl, 2-cyano-pyridin-5-yl, 5-cyano-pyridin-3-yl, 6-(2-hydroxyethyl-amino)-pyridin-3-yl, 2-(4-tert-butoxycarbonyl-piperazin-1-yl)-pyridin-4-yl, 6-morpholin-4-yl-pyridin-3-yl, furan-2-yl, furan-3-yl, 1H-pyrrolo[2,3-b]pyridine-5-yl, or quinolin-3-yl and
R⁵ is hydrogen,
or a tautomer thereof or a N-oxide thereof, or a pharmaceutically acceptable salt, or a hydrate or solvate thereof.

7. A compound of the formula I according to claim 1, selected from the group consisting of the following compounds:
4-(3,4-dimethoxy-phenyl)-6-(6-piperazin-1-yl-pyridin-3-yl)-quinazoline,
4-{5-[4-(3,4-dimethoxy-phenyl)-quinazolin-6-yl]-pyrldin-2-yl}-piperazine-1-carboxylic acid tert-butyl ester,
[4,6-bis-(3,4-dimethoxy-phenyl)-quinazolin-2-yl]-n-propyl-amine,
6-(6-methoxy-pyridin-3-yl)-4-phenyl-quinazoline,
3-[2-amino-4-(3,4-dimethoxy-phenyl)-quinazolin-6-yl]phenol,
6-(3-chloro-4-n-propoxy-phenyl)-4-(3,4-dimethoxy-phenyl)-quinazoline,
4-[4-(3,4-dimethoxy-phenyl)-quinazolin-6-yl]-2-methoxy-phenol,
6-(2,3-dihydro-benzo[1,4]dioxin-6-yl)-4-(3,4-dimethoxy-phenyl)-quinazoline,
6-(benzo[1,3]dioxol-5-yl)-4-(3,4-dimethoxy-phenyl)-quinazoline,
4-[4-(3,4-dimethoxy-phenyl)-quinazolin-6-yl]-2-methoxy-benzoic acid methyl ester,
4-(3,4-dimethoxy-phenyl)-6-(3,4,5-trimethoxy-phenyl)-quinazoline,
6-(3,4-dimethoxy-phenyl)-4-(3-fluoro-4-methoxy-phenyl)-quinazoline,
4-[6-(3,4-dimethoxy-phenyl)-quinazolin-4-yl]-2-methoxy-benzoic acid methyl ester,
4-(3-chloro-4-n-propoxy-phenyl)-6-(3,4-dimethoxy-phenyl)-quinazotine,
6-(3,4-dimethoxy-phenyl)-4-(3,4,5-trimethoxy-phenyl)-quinazoline,
4-(2,3-dihydro-benzo[1,4]dioxin-6-yl)-6-(3,4-dimethoxy-phenyl)-quinazoline,
4-(benzo[1,3]dioxol-5-yl)-6-(3,4-dimethoxy-phenyl)-quinazoline,
6-(6-piperazin-1-yl-pyridin-3-yl)-4-(4-pyrazol-1-yl-phenyl)-quinazoline, 3-[4-(3,4-dimethoxy-phenyl)-quinazolin-6-yl]-phenol,
4-[4-(3,4-dimethoxy-phenyl)-quinazolin-6-yl]-phenol,
4-[4-(3,4-dimethoxy-phenyl)-quinazolin-6-yl]-benzamide,
4-(3,4-dimethoxy-phenyly)-(3-fluoro-4-methoxy-phenyl)-quinazoline,
6-(3,4-dimethoxy-phenyl)-4-phenyl-quinazoline,
6-(3,4-dimethoxy-phenyl)-4-thiophen-2-yl-quinazoline,
6-(3,4-dimethoxy-phenyl)-4-(3-methoxy-phenyl)-quinazoline,
6-(3,4-dimethoxy-phenyl)-4-(4-methoxy-phenyl)-quinazoline,
6-(3,4-dimethoxy-phenyl)-4-(4-pyrazol-1-yl-phenyl)-quinazoline,
6-(3,4-dimethoxy-phenyl)-4-(6-piperazin-1-yl-pyridin-3-yl)-quinazoline,
4-[6-(6-piperazin-1-yl-pyridin-3-yl)-quinazolin-4-yl]-benzamide,
6-(6-methoxy-pyridin-3-yl)-4-(4-pyrazol-1-yl-phenyl)-quinazoline,
4-[6-(6-methoxy-pyridin-3-yl)-quinazolin-4-yl]-benzamide,
6-(6-methoxy-pyridin-3-yl)-4-(6-piperazin-1-yl-pyridin-3-yl)-quinazoline,
4,6-bis(3,4-dimethoxy-phenyl)-quinazoline,
4-(6-(3,4-dimethoxy-phenyl)-quinazolin-4-yl]-benzamide,
6-(3,4-dimethoxy-phenyl)-4-(6-methoxy-pyridin-3-yl)-quinazoline,
4-(3,4-dimethoxy-phenyl)-6-(6-methoxy-pyridin-3-yl)-quinazoline,
4-(6-methoxy-pyridin-3-yl)-6-(6-piperazin-1-yl-pyridin-3-ylyquinazoline,
6-(6-methoxy-pyridin-3-yl)-4-thiophen-2-yl-quinazoline,
4-(2-chloro-phenyl)-6-(6-methoxy-pyridin-3-yl)-quinazoline,
4,6-bis(6-methoxy-pyridin-3-yl)-quinazoline,
4-(4-methoxy-phenyl)-6-(6-methoxy-pyridin-3-yl)-quinazoline,
4-(3,4-dimethoxy-phenyl)-6-(4-ethoxy-3-methoxy-phenyl)-quinazoline,
[4,6-bis-(3,4-dimethoxy-phenyl)-quinazolin-2-yl]-cyclopropyl-amine,
4-[2-amino-6-(3,4-dimethoxy-phenyl)-quinazolin-4-yl]-benzamide,
4-(3,4-dimethoxy-phenyl)-6-(3-fluoro-4-methoxy-phenyl)-quinazolin-2-ylamine,
4-[2-amino-4-(3,4-dimethoxy-phenyl)-quinazolin-6-yl]-2-methoxy-benzoic acid methyl ester,
4-(3,4-dimethoxyphenylyl)-6-(3,4,5,trimethoxyphenyl)-quinazolin-2,ylamine,
6-(3-chloro-4-n-propoxy-phenyl)-4-(3,4-dimethoxyphenyl)-quinazolin-2-ylamine,
4-[2-amino-4-(3,4-dimethoxy-phenyl)-quinazolin-6-yl]-phenol,
6-(2,3-dihydro-benzo[1,4]dioxin-6-yl)-4-(3,4-dimethoxy-phenyl)-quinazolin-2-ylamine,
4-(benzo[1,3]dioxol-5-yl)-6-(3,4-dimethoxy-phenyl)-quinazolin-2-ylamine,
6-(3,4-dimethoxy-phenyl)-4-(3,4,5-trimethoxy-phenyl)-quinazolin-2-ylamine,
6-(3,4-dimethoxy-phenyl)-4-(6-methoxy-pyridin-3-yl)-quinazolin-2-ylamine,
4,6-bis(3,4-dimethoxy-phenyl)-quinazolin-2-ylamine,
4,6-bis(6-methoxy-pyridin-3-yl)-quinazolin-2-ylamine,
4-(3,4-dimethoxy-phenyl)-6-(6-methoxy-pyridin-3-yl)-quinazolin-2-ylamine,
N-[4,6-bis-(3,4-dimethoxy-phenyl)-quinazolin-2-yl]-N-methyl-amine,
4[6,(3,4-dimethoxy-phenyl)-quinazolin-4-yl]-2-methoxy-benzoic acid,
4-[6-(3,4-dimethoxy-phenyl)-quinazolin-4-yl]-2-methoxy-benzoic acid-N-methylamide,
4-[6-(3,4-dimethoxy-phenyl)-quinazolin-4-yl]-2-methoxy-benzamide,
4-[6-(3,4-dimethoxy-phenyl)-quinazolin-4-yl]-2-methoxy-N,N-dimethyl-amide,
{4-[6-(3,4-dimethoxy-phenyl)-quinazolin-4-yl]-2-methoxy-phenyl}-(4-methyl-pierazin-1-yl)-methanone,
{4-[6-(3,4-dimethoxy-phenyl)-quinazolin-4-yl]-2-methoxy-phenyl}-morpholin-4-yl-methanone;
4-(1,2,4-Triazol-1-yl)-6-(3,4-dimethoxy-phenyl)-quinazoline,
4-(3,4-dimethoxy-phenyl)-6-[3-methoxy-4-(2-methoxy-ethoxy)-phenyl]-quinazoline,
5-[4-(3,4-dimethoxy-phenyl)-quinazolin-6-yl]-pyridin-2-ylamine,
4-[6-(3,4-dimethoxy-phenyl)-quinazolin-4-yl]-N,N-dimethyl-benzenesulfonamide,
6-(3,4-dimethoxy-phenyl)-4-pyrazol-1-yl-quinazoline,
6-(3,4-dimethoxy-phenyl)-4-[1,2,4]triazol-1-yl-quinazoline, and
6-(3,4-dimethoxy-phenyl)-4-pyrrol-1-yl-quinazoline;
or in each case an N-oxide thereof, a tautomer thereof and/or a pharmaceutically acceptable salt thereof.

8. A compound of the formula I according to claim 1, wherein
R¹ is hydrogen, amino, methylamino, n-propylamino or cyclopropylamino;
R² is phenyl, 4-(2-amino-pyrimidin-5-ylmethyl)-phenyl, 3-(2-methoxy-6-trifluoromethyl)pyridin-3-yl-phenyl, 3-methoxyphenyl, 4-methoxyphenyl, 3,4-dimethoxyphenyl, 3,4,5-trimethoxyphenyl, 3-chloro-4-n-propoxy-phenyl, 4-carboxy-3-methoxyphenyl, 4-(2-pyridin-2-yloxyethoxy)-phenyl, 4-(2-pyrimidin-4-yloxyethoxy)-phenyl, 4-methoxycarbonyl-3-methoxyphenyl, 4-carbamoylphenyl, 4-N-methylcarbamoyl-3-methoxy-phenyl, 4-(N,N-dimethyl-carbamoyl)-3-methoxy-phenyl, 4-(4-methylpiperazin-1-carbonyl)-3-methoxyphenyl, 4-(4-morpholin-1-carbonyl)-3-methoxyphenyl, benzo[1,3]dioxol-5-yl, 2,3-dihydro-benzo[1,4]dioxin-6-yl, 4-[2-(4-pyrrolidino-piperidin-1-yl)-ethoxy]-phenyl, 4-(piperazin-1-yl)-phenyl, 4-[2-(4-methyl-piperazin-1-yl)-ethoxy]-phenyl, 4-[2-(4-ethyl-piperazin-1-yl)-ethoxy]-phenyl, 4-(4-methyl-piperazin-1-carbonylmethoxy)-phenyl, 4-(4-ethyl-piperazin-1-carbonylmethoxy)-phenyl, 4-(4-pyrrolidino-piperidin-1-carbonylmethoxy)-phenyl, 4-[N-(2-dimethylaminoethyl)-N-methylcarbamoyl]-phenyl, 4-[(R, S or R,S)-3-diethylamino-pyrrolidin-1-carbonyl)-phenyl, 4-sulfamoyl-phenyl, 4-N,N-dimethyl-sulfamoylphenyl, 4-[N-methyl-N-2-(pyrrolidinoethyl)-sulfamoyl]-phenyl, 4-pyrazolyl-phenyl, pyrrolyl, pyrazolyl, thiophenyl, 1,2,4-triazol-1-yl, 6-methoxy-pyridin-3-yl or 6-piperazino-pyridin-3-yl;
R³ is hydrogen,
R⁴ is 4-(2-amino-pyrimidin-5-ylmethyl)-phenyl, 3-(2-methoxy-6-trifluoromethyl)pyridin-3-yl-phenyl, 3-hydroxyphenyl, 4-hydroxyphenyl, 4-hydroxy-3-methoxyphenyl, 3-hydroxy-4-n-propoxyphenyl, 3,4-dimethoxyphenyl, 3,4,5-trimethoxyphenyl, 4-ethoxy-3-methoxyphenyl, 3-(2-methoxy-ethoxy)-4-methoxyphenyl, 3-methoxy-4-(2-methoxy-ethoxy)-phenyl, 3-fluoro-4-methoxyphenyl, 3-chloro-4-n-propoxyphenyl, 4-(2-pyridin-2-yloxyethoxy)-phenyl, 4-(2-pyrimidin-4-yloxyethoxy)-phenyl, 4-[2-(4-pyrrolidino-piperidin-1-yl)-ethoxy]-phenyl, 4-[2-(4-methyl-piperazin-1-yl)-ethoxy]-phenyl, 4-[2-(4-ethyl-piperazin-1-yl)-ethoxy]-phenyl, 4-(4-methyl-piperazin-1-carbonylmethoxy)-phenyl, 4-(4-ethyl-piperazin-1-carbonylmethoxy)-phenyl, 4-(4-pyrrolidino-piperidin-1-carbonylmethoxyl)-phenyl, 4-[N-(2-dimethylaminoethyl)-N-methylcarbamoyl]-phenyl, 4-[(R, S or R,S)-3-diethylamino-pyrrolidin-1-carbonyl)-phenyl, 4-methoxycarbonyl-3-methoxyphenyl, 4-carbamoylphenyl, N,N-dimethyl-aminosulfonylphenyl, 4-[N-methyl-N-2-(pyrrolidino-ethyl)-sulfamoyl]-phenyl, benzo[1,3]dioxol-5-yl, 2,3-dihydro-benzo[1,4]dioxin-6-yl, pyridine-3-yl, 6-methoxy-pyridin-3-yl, 5-methoxy-pyridin-3-yl, 2-amino-pyridin-4-yl, 6-amino-pyridin-3-yl, 6-(piperazin-1-yl)-pyridin-3-yl, 6-(4-tert-butoxycarbonyl-piperazin-1-yl)-pyridin-3-yl, 2-(piperazin-1-yl)-pyridin-4-yl or 2-(4-tert-butoxycarbony)-piperazin-1-yl)-pyridin-4-yl, and
R⁵ is hydrogen,
or a tautomer thereof or a N-oxide thereof, or a pharmaceutically acceptable salt, or a hydrate or solvate thereof.

9. A compound of the formula I according to claim 1, selected from the group consisting of the following compounds:
6-(3,4-dimethoxy-phenyl)-4-(4-ethoxy-3-methoxy-phenyl)-quinazoline;
6-(3,4-dimethoxy-phenyl)-4-[3-methoxy-4-(2-methoxy-ethoxy)-phenyl]-quinazoline;
4-(3,4-dimethoxy-phenyl)-6-(2-methoxy-pyridin-4-yl)-quinazoline;
6-(3,4-dimethoxy-phenyl)-4-(2-methoxy-pyridin-4-yl)-quinazoline;
(3-{4-[4-(3,4-dimethoxy-phenyl)-quinazolin-6-yl]-2-methoxy-phenoxy}-propyl)-carbamic acid tert-butyl ester;
(3-{4-[6-(3,4-dimethoxy-phenyl)-quinazolin-4-yl]-2-methoxy-phenoxy}-propyl)-carbamic acid tert-butyl ester;
(2-{4-[4-(3,4-dimethoxy-phenyl)-quinazolin-6-yl]-2-methoxy-phenoxy}-ethyl)-carbamic acid tert-butyl ester;
(2-{4-[6-(3,4-dimethoxy-phenyl)-quinazolin-4-yl]-2-methoxy-phenoxy}-ethyl)-carbamic acid tert-butyl ester;
6-(3,4-dimethoxy-phenyl)-4-(3-ethoxy-phenyl)-quinazoline;
4-(3-chloro-phenyl)-6-(3,4-dimethoxy-phenyl)-quinazoline;
4-(3-benzyloxy-4-methoxy-phenyl)-6-(3,4-dimethoxy-phenyl)-quinazoline;
4-[6-(3,4-dimethoxy-phenyl)-quinazolin-4-yl]-benzoic acid methyl ester;
4-(3,4-dimethoxy-phenyl)-6-(5-methoxy-pyridin-3-yl)-quinazoline
4-[4-(3,4-dimethoxy-phenyl)-quinazolin-6-yl]-2-methoxy-benzoic acid methyl ester;
4-(3,4-dimethoxy-phenyl)-6-quinolin-3-yl-quinazoline;
4-[6-(5-methoxy-pyridin-3-yl)-quinazolin-4-yl]-benzamide;
4-[6-(6-amino-5-trifluoromethy)-pyridin-3-yl)-quinazolin-4-yl]-benzamide;
5-[4-(3,4-dimethoxy-phenyl)-quinazolin-6-yl]-3-trifluoromethyl-pyridin-2-ylamine;
3-[4-(3,4-dimethoxy-phenyl)-quinazolin-6-yl]-benzenesutfonamide;
4-benzo[1,3]dioxol-5-yl-6-(3-fluoro-4-methoxy-phenyl)-quinazoline;
4-(3-benzyloxy-4-methoxy-phenyl)-6-(3,4-dimethoxy-phenyl)-quinazoline;
3-[4-(3,4-dimethoxy-phenyl)-quinazolin-6-yl]-benzamide;
4-(4-chloro-phenyl)-6-(3,4-dimethoxy-phenyl)-quinazoline;
6-(3,4-dimethoxy-phenyl)-4-(4-trifluoromethyl-phenyl)-quinazoline;
6-(3-chloro-4-methoxy-phenyl)-4-(3,4-dimethoxy-phenyl)-quinazoline;
6-(3,4-dimethoxy-phenyl)-4-thiophen-3-yl-quinazoline;
4-(3,4-dimethoxy-phenyl)-6-(1H-pyrrolo[2,3-b]pyridin-5-yl)-quinazoline;
4-[6-(1H-pyrrolo[2,3-b]pyridin-5-yl)-quinazolin-4-yl]-benzamide;
4-[6-(6-amino-pyridin-3-yl)-quinazolin-4-yl]-benzamide;
6-(3-benzyloxy-4-methoxy-phenyl)-4-(3,4-dimethoxy)-phenyl)-quinazoline;
4-(4-chloro-phenyl)-6-(3-fluoro-4-methoxy-phenyl)-quinazoline;
4-[6-(4-ethoxy-3-methoxy-phenyl)-quinazolin-4-yl]-benzamide;
4-[6-(3,4-diethoxy-phenyl)-quinazolin-4-yl]-benzamide;
4-(3,4-dimethoxy-phenyl)-6-furan-3-yl-quinazoline;
4-[6-(3,4-dimethoxy-phenyl)-quinazolin-4-yl]-benzonitrile;
4-[6-(6-amino-pyridin-3-yl)-quinazolin-4-yl]-benzonitrile;
4-[4-(3,4-dimethoxy-phenyl)-quinazolin-6-yl]-benzaldehyde;
4-biphenyl-4-yl-6-(3,4-dimethoxy-phenyl)-quinazoline;
4-(3,4-diethoxy-phenyl)-6-(3,4-dimethoxy-phenyl)-quinazoline;
6-(3,4-dimethoxy-phenyl)-4-(4-methoxy-3-trifluoromethoxy-phenyl)-quinazoline;
4-(3,4-dimethoxy-pheny)-6-(4-methoxy-3-trifluoromethoxy-phenyl)-quinazoline;
5-[4-(3,4-dimethoxy-phenyl)-quinazolin-6-yl]-pyridine-2-carbonitrile;
4-(4-bromo-phenyl)-6-(3,4-dimethoxy-phenyl)-quinazoline;
6-(3,4-diethoxy-phenyl)-4-(3,4-dimethoxy-phenyl)-quinazoline;
6-(3,4-dimethoxy-phenyl)-4-(4-trifluoromethoxy-phenyl)-quinazoline;
6-(3,4-dimethoxy-phenyl)-4-(4-fluoro-phenyl)-quinazoline;
6-(3,4-dimethoxy-phenyl)-4-(3-fluoro-phenyl)-quinazoline;
6-(3,4-dimethoxy-phenyl)-4-(4-methanesulfonyl-phenyl)-quinazoline;
N-{4-[6-(3,4-dimethoxy-phenyl)-quinazolin-4-yl]-phenyl}-acetamide;
5-[4-(3,4-dimethoxy-phenyl)-quinazolin-6-yl]-nicotinonitrile;
{5-[4-(3,4-dimethoxy-phenyl)-quinazolin-6-yl]-pyridin-2-yl}-isobutyl-amine;
6-(3,4-dimethoxy-phenyl)-4-(6-morpholin-4-yl-pyridin-3-yl)-quinazoline;
{5-[4-(3,4-dimethoxy-phenyl)-quinazolin-6-yl]-pyridin-2-yl}-dimethyl-amine;
{5-[4-(3,4-dimethoxy-phenyl)-quinazolin-6-yl]-pyridin-2-yl}-methyl-amine;
2-{5-[4-(3.4-dimethoxy-phenyl)-quinazolin-6-yl]-pyridin-2-ylamino}-ethanol;
4-{5-[6-(3,4-dimethoxy-phenyl)-quinazolin-4-yl]-pyridin-2-yl}-piperazine-1-carboxylic acid tert-butyl ester;
4-(2,3-dihydro-benzo[1,4]dioxin-6-yl)-6-(3,4-dimethoxy-phenyl)-quinazolin-2-ylamine;
6-(6-amino-pyridin-3-yl)-4-(3,4-dimethoxy-phenyl)-quinazolin-2-ylamine;
4-(3,4-dimethoxy-phenyl)-6-quinolin-3-yl-quinazolin-2-ylamine;
4-[6-(3,4-dimethoxy-phenyl)-quinazolin-4-yl]-N,N-dimethyl-benzamide;
4-[6-(3,4-dimethoxy-phenyl)-quinazolin-4-yl]-N-methyl-benzamide;
{4-[6-(3,4-dimethoxy-phenyl)-quinazolin-4-yl]-phenyl}-(4-methyl-piperazin-1-yl)-methanone;
{4-[6-(3,4-dimethoxy-phenyl)-quinazolin-4-yl]-phenyl}-morpholin-4-yl-methanone;
C-{4'-[6-(3,4-dimethoxy-phenyl)-quinazolin-4-yl]-biphenyl-4-yl}-methylamine;
6-(3,4-dimethoxy-phenyl)-4-(4'-methoxy-biphenyl-4-yl)-quinazoline;
{4'-[6-(3,4-dimethoxy-phenyl)-quinazolin-4-yl]-biphenyl-4-yl}-methanol;
4'-[6-(3,4-dimethoxy-phenyl)-quinazolin-4-yl]-biphenyl-4-ol;
4-(3',4'-dimethoxy-biphenyl-4-yl)-6-(3,4-dimethoxy-phenyl)-quinazoline;
6-(3,4-dimethoxy-phenyl)-4-(3',4',5'-trimethoxy-biphenyl-4-yl)-quinazoline;
4'-[6-(3,4-dimethoxy-phenyl)-quinazolin-4-yl]-biphenyl-4-carboxylic acid amide;
6-(3,4-dimethoxy-phenyl)-4-(4'-methoxymethyl-biphenyl-4-yl)-quinazoline;
3-{4-[4-(3,4-dimethoxy-phenyl)-quinazolin-6-yl]-2-methoxy-phenoxy}-propylamine;
2-{4-[4-(3,4-dimethoxy-phenyl)-quinazolin-6-yl]-2-methoxy-phenoxy}-ethylamine;
3-{5-[4-(3,4-dimethoxy-phenyl)-quinazolin-6-yl]-2-methoxy-phenoxy}-propylamine;
2-{5-[4-(3,4-dimethoxy-phenyl)-quinazolin-6-yl]-2-methoxy-phenoxy}-ethylamine;
4-(3,4-dimethoxy-phenyl)-6-(3-ethoxy-4-methoxy-phenyl)-quinazoline;
4-[4-(3,4-dimethoxy-phenyl)-quinazolin-6-yl]-2-methoxy-benzamide;
4-[4-(3,4-dimethoxy-phenyl)-quinazolin-6-yl]-2-methoxy-benzoic acid;
5-[4-(3,4-dimethoxy-phenyl)-quinazolin-6-yl]-pyridine-2-carboxylic acid amide;
C-{5-[4-(3,4-dimethoxy-phenyl)-quinazolin-6-yl]-pyridin-2-yl}-methylamine;
6-(3,4-dimethoxy-phenyl)-4-[6-(4-methanesulfonyl-piperazin-1-yl)-pyridin-3-yl]-quinazoline;
1-(4-{5-[6-(3,4-dimethoxy-phenyl)-quinazolin-4-yl]-pyridin-2-yl}-piperazin-1-yl)-ethanone;
1-{4-[6-(3,4-dimethoxy-phenyl)-quinazolin-4-yl]-phenyl}-pyrrolidin-2-one;
1-{4-[6-(3,4-dimethoxy-phenyl)-quinazolin-4-yl]-phenyl}-azetidin-2-one;
1-{4-[6-(3,4-dimethoxy-phenyl)-quinazolin-4-yl]-phenyl}-piperidin-2-one;
3-{4-[6-(3,4-dimethoxy-phenyl)-quinazolin-4-yl]-phenyl}-oxazolidin-2-one;
1-{4-[6-(3,4-dimethoxy-phenyl)-quinazolin-4-yl]-phenyl}-3-methyl-imidazolidin-2-one;
4,6-bis-(3,4-dimethoxy-phenyl)-5-fluoro-quinazoline;
4-[6-(3,4-dimethoxy-pheny)-5-fluoro-quinazolin-4-yl]-benzamide; {5-[4-(3,4-dimethoxy-phenyl)-quinazolin-6-yl]-pyridin-2-yl}-(2-methoxy-ethyl)-amine;
and
4-(3,4-dimethoxy-phenyl)-6-(4,4,5,5-tetramethyl-[1,3,2]dioxaborolan-2-yl)-quinazoline;
or a tautomer thereof or a N-oxide thereof, or a pharmaceutically acceptable salt, or a hydrate or solvate thereof.

10. A compound of the formula I, an N-oxide thereof, a tautomer thereof and/or a pharmaceutically acceptable salt thereof, according to any one of claims 1 to 9 for use in the treatment, including prophylactic treatment, of a warm-blooded animal, especially a human.

11. A compound of the formula 1, an N-oxide thereof, a tautomer thereof and/or a pharmaceutically acceptable salt thereof, according to claim 10 where the use is against one or more diseases selected from the group consisting of proliferative, inflammatory diseases, allergic diseases, obstructive airways diseases, and disorders commonly occurring in connection with transplantation, especially one or more diseases which respond to an inhibition of kinases of the PI3-kinase-related protein kinase family, especially lipid kinases and/or PI3 kinase (PI3K) and/or mTOR and/or DNA protein kinase and/or ATM and/or ATR and/or hSMG-1 activity.

12. A pharmaceutical preparation, comprising a compound of the formula I, an N-oxide thereof, a tautomer thereof and/or a pharmaceutically acceptable salt thereof, according to any one of claims 1 to 9 and at least one pharmaceutically acceptable carrier.

13. A method or process for the manufacture of a pharmaceutical preparation, comprising mixing a compound of the formula I, an N-oxide thereof, a tautomer thereof and/or a pharmaceutically acceptable salt thereof, according to any one of claims 1 to 9 with at least one pharmaceutically acceptable carrier material.

14. A process for the manufacture of a compound of the formula I according to any one of claims 1 to 9, comprising
a) for the manufacture of a compound of the formula I wherein R⁴ is bound to the central quinazoline moiety in formula I via a carbon atom, reacting a compound of the formula IIA, 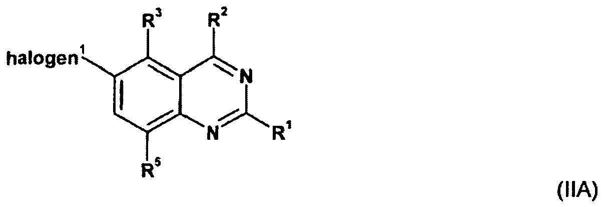 wherein R¹, R², R³ and R⁵ are as defined for a compound of the formula I and wherein halogen¹ is halo, preferably chloro, bromo or iodo, or is trifluoromethansulfonyloxy, under cross-coupling conditions with a boronic acid or boronic acid ester of the formula III,
R⁴-D (III)
wherein R⁴ is as defined for a compound of the formula I and is bound via a carbon atom to D and D is -B(OH₂) or a group of the formula A, 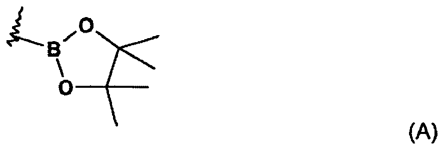 or
b) for the manufacture of a compound of the formula I wherein R² is bound to the central quinazoline moiety in formula I via a carbon atom, reacting a compound of the formula IIB, 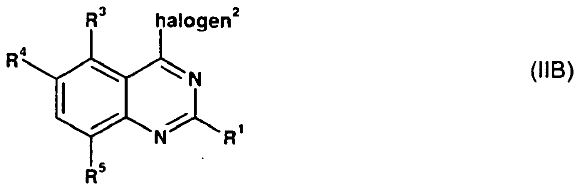 wherein R¹, R³, R⁴ and R⁵ are as defined for a compound of the formula I and halogen² is halo, preferably chloro, bromo or iodo, or is trifluoromethansulfonyloxy, under cross-coupling conditions with a boronic acid or boronic acid ester of the formula IV,
R²-D (IV)
wherein R² is as defined for a compound of the formula I and is bound via a carbon atom to D and D is -B(OH₂) or a group of the formula A given above;
or
c) for the manufacture of a compound of the formula I wherein R² and R⁴ are identical and are bound to the central quinazoline moiety in formula I via a carbon atom, reacting a compound of the formula IIC, 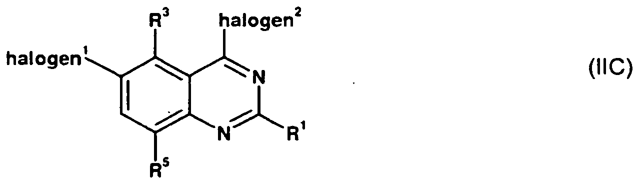 wherein R¹, R³ and R⁵ are as defined for a compound of the formula I and halogen¹ and halogen² are, independently of each other, halo, preferably chloro, bromo or iodo, or is trifluoromethansulfonyloxy, with a boronic acid or boronic acid ester of the formula V,
R^{2,4}-D (V)
wherein R^{2,4} is a moiety R² or R⁴ bound via a carbon atom to D and is otherwise as defined for a compound of the formula I and D is -B(OH₂) or a group of the formula A given above;
or
d) for the manufacture of a compound of the formula I wherein R¹ is amino, N-mono-C₁-C₁₀-alkyl-amino or N-mono-C₃-C₁₀-cycloalkylamino, reacting a compound of the formula IID, 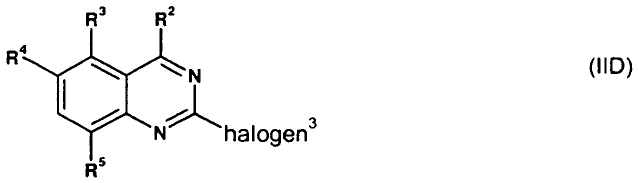 wherein R², R³, R⁴ and R⁵ are as defined for a compound of the formula I and wherein halogen³ is halo, preferably chloro, bromo or iodo, or is trifluoromethansulfonyloxy, with an amine of the formula VI
R^{1'}-H (VI)
wherein R^{1'} is amino, N-mono-C₁-C₁₀-alkyl-amino or N-mono-C₃-C₁₀-cycloalkylamino;
or
e) for the manufacture of a compound of the formula I wherein R⁴ is heteroaryl with at least one ring nitrogen and is bound to the central quinazoline moiety in formula I via a nitrogen atom, reacting a compound of the formula IIA given above under a) with a compound of the formula VII,
R^{4'}-H (VII)
wherein R⁴* is a nitrogen containing heteroaryl with at least one ring nitrogen and is bound to the hydrogen in formula VII via a nitrogen atom, under substitution conditions;
or
f) for the manufacture of a compound of the formula I wherein R² is heteroaryl with at least one ring nitrogen and is bound to the central quinazoline moiety in formula I via a nitrogen atom, reacting a compound of the formula IIB given above under b) with a compound of the formula VIII,
R^{2*} -H (VIII)
wherein R⁶* is a nitrogen containing heteroaryl with at least one ring nitrogen and is bound to the hydrogen in formula VIII via a nitrogen atom, under substitution conditions;
or
g) for the manufacture of a compound of the formula I wherein R² and R⁴ are identical and are heteroaryl with at least one ring nitrogen and each of them is bound to the central quinazoline moiety in formula I via a nitrogen atom, reacting a compound of the formula IX,
R^{2,4*}-H (IX)
wherein R^{2,4*} is heteroaryl with at least one nitrogen atom and wherein R^{2,4*} is a moiety R² or R⁴ bound via a nitrogen atom to the hydrogen shown in formula IX and is otherwise as defined for a compound of the formula I, under substitution conditions with a compound of the formula IIC mentioned above;or
h) for the manufacture of a compound of the formula I wherein R⁴ is bound to the central quinazoline moiety in formula I via a carbon atom, reacting a boronic acid or boronic acid ester compound of the formula IIA*, 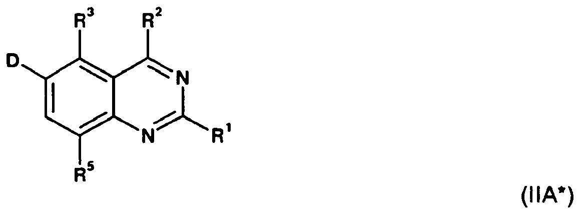 wherein R¹, R², R³ and R⁵ are as defined for a compound of the formula I and wherein D is -B(OH₂) or a group of the formula A, 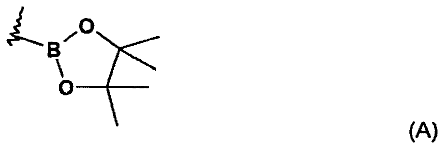 under cross coupling conditions with compound of the formula III*,
R⁴-Hal (III*)
wherein R⁴ is as defined for a compound of the formula I and is bound via a carbon atom to Hal and Hal is halo, preferably chloro, bromo or iodo, or is trifluoromethansulfonyloxy,
where in any of the reactions represented under a) to h) functional groups in the starting materials can be present in protected form and in the obtainable compounds of the formula I carrying one or more protecting groups such protecting groups are removed;
and, if desired, a compound of the formula I obtainable according to a process variant selected from a) to g) is converted into a different compound of the formula I, an obtainable salt of a compound of the formula I is converted into a different salt thereof,
an obtainable free compound of the formula I is converted into a salt thereof, and/or an obtainable isomer of a compound of the formula I is separated from one or more different obtainable isomers of the formula I.

15. The use of a compound of the formula I, an N-oxide thereof, a tautomer thereof and/or a pharmaceutically acceptable salt thereof, according to any one of claims 1 to 9 for the preparation of a pharmaceutical preparation for the treatment of a disease selected from the group consisting of proliferative, inflammatory diseases, allergic diseases, obstructive airways diseases, and disorders commonly occurring in connection with transplantation, especially one or more diseases which respond to an inhibition of kinases of the PI3-kinase-related protein kinase family, especially lipid kinases and/or PI3 kinase (PI3K) and/or mTOR and/or DNA protein kinase and/or ATM and/or ATR and/or hSMG-1 activity.

16. A combination of a compound of the formula I according to any one of claims 1 to 9 with one or more other therapeutic agents.

## Patentansprüche

1. Verbindung der Formel I, 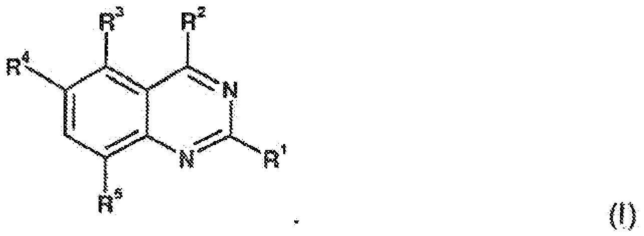 worin
R¹ für Wasserstoff oder Amino, das gegebenenfalls einfach durch Alkyl oder Cycloalkyl substituiert ist, steht;
R² für gegebenenfalls substituiertes Aryl oder gegebenenfalls substituiertes Heteroaryl steht;
R³ für Wasserstoff, Halogen, Alkyl, Alkoxy oder Cyano steht;
R⁴ für gegebenenfalls substituiertes Aryl oder gegebenenfalls substituiertes Heteroaryl steht und
R⁵ für Wasserstoff, Methyl oder durch Halogen substituiertes Methyl steht;
mit der Maßgabe, daß dann, wenn R⁴ für gegebenenfalls substituiertes Pyrazolyl steht, R¹ für Amino, das gegebenenfalls einfach durch Alkyl oder Cycloalkyl substituiert ist, steht und R³, R⁴ und R⁵ die oben angegebene Bedeutung besitzen;
und mit der Maßgabe, daß dann, wenn R² für gegebenenfalls substituiertes Oxoindolyl steht, R¹ für Amino, das gegebenenfalls einfach durch Alkyl oder Cycloalkyl substituiert ist, steht und R³, R⁴ und R⁵ die oben angegebene Bedeutung besitzen;
oder ein Tautomer davon oder ein N-Oxid davon oder ein Salz oder ein Hydrat oder Solvat davon.

2. Verbindung der Formel I nach Anspruch 1, worin
R¹ für Wasserstoff oder Amino, das gegebenenfalls durch C₁-C₇-Alkyl (vorzugsweise C₁-C₄-Alkyl) oder C₃-C₈-Cycloalkyl (vorzugsweise C₃-C₅-Cycloalkyl) substituiert ist, steht;
R² für gegebenenfalls substituiertes Aryl, wobei Aryl aus der Gruppe bestehend aus Phenyl, Naphthyl, Biphenylenyl, Indacenyl, Acenaphthylenyl, Fluorenyl, Phenalenyl, Phenanthrenyl und Anthracenyl ausgewählt ist, wobei jede dieser Gruppen gegebenenfalls durch einen oder mehrere, vorzugsweise bis zu drei, Substituenten substituiert ist, die unabhängig voneinander aus der Gruppe bestehend aus C₁-C₇-Alkyl; C₂-C₇-Alkenyl, C₂-C₇-Alkinyl; C₆-C₁₈-Aryl-C₁-C₇-alkyl, worin Aryl vorzugsweise für Phenyl, Naphthyl, Biphenylenyl, Indacenyl, Acenaphthylenyl, Fluorenyl, Phenalenyl, Phenanthrenyl oder Anthracenyl steht und gegebenenfalls durch C₁-C₇-Alkyl, wie Methyl oder Ethyl, durch Pyrrolidinyl, insbesondere Pyrrolidino, durch Piperazinyl, insbesondere Piperazino, durch Amino, durch N-Mono- und/oder
N,N-Di-C₁-C₇-alkylamino, durch Halogen, durch C₁-C₇-Alkoxy, wie Methoxy, und/oder durch Halogen-C₁-C₇-alkyl, wie Trifluormethyl, substituiert ist; (Pyrrolidinyl (insbesondere Pyrrolidino), Piperidinyl (insbesondere Piperidino), Piperazinyl (insbesondere Piperazino), Morpholino, Thiomorpholino, Pyridinyl, Pyrimidinyl, Pyrazinyl oder Pyridazinyl)-C₁-C₇-alkyl, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, wie Methyl oder Ethyl, durch Pyrrolidinyl, insbesondere Pyrrolidino, durch Piperazinyl, insbesondere Piperazino, durch Amino, durch N-Mono- und/oder N,N-Di-C₁-C₇-alkylamino, durch Halogen, durch C₁-C₇-Alkoxy, wie Methoxy, und/oder durch Halogen-C₁-C₇-alkyl, wie Trifluormethyl, substituiert ist, beispielsweise Pyrrolidino-C₁-C₇-alkyl, Piperidino-C₁-C₇-alkyl, Morpholino-C₁-C₇-alkyl, Thiomorpholino-C₁-C₇-alkyl, N-C₁-C₇-Alkylpiperazino-C₁-C₇-alkyl oder N-mono-oder N,N-di(C₁-C₇-alkyl) aminosubsitituiertes oder unsubstituiertes Pyrrolidino-C₁-C₇-alkyl; (Pyrrolidinyl (insbesondere Pyrrolidino), Piperidinyl (insbesondere Piperidino), Piperazinyl (insbesondere Piperazin), Pyridinyl, Pyrimidinyl, Pyrazinyl oder Pyridazinyl)oxy-C₁-C₇-alkyl, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, wie Methyl oder Ethyl, durch Pyrrolidinyl, insbesondere Pyrrolidino, durch Piperazinyl, insbesondere Piperazin, durch Amino, durch N-Mono- und/oder N,N-Di-C₁-C₇-alkylamino, durch Halogen, durch C₁-C₇-Alkoxy, wie Methoxy, und/oder durch Halogen-C₁-C₇-alkyl, wie Trifluormethyl, substituiert ist; (Pyrrolidinyl (insbesondere Pyrrolidino), Piperidinyl (insbesondere Piperidin), Piperazinyl (insbesondere Piperazino), Pyridinyl, Pyrimidinyl, Pyrazinyl oder Pyridazinyl) carbonyl-C₁-C₇-alkyl,
worin Pyrrolidinyl, Piperidinyl, Piperazinyl, Pyridinyl, Pyrimidinyl, Pyrazinyl oder Pyridazinyl gegebenenfalls durch C₁-C₇-Alkyl, wie Methyl oder Ethyl, durch Pyrrolidinyl, insbesondere Pyrrolidino, durch Piperazinyl, insbesondere Piperazino, durch Amino, durch N-Mono- und/oder N,N-Di-C₁-C₇-alkylamino, durch Halogen, durch C₁-C₇-Alkoxy, wie Methoxy, und/oder durch Halogen-C₁-C₇-alkyl, wie Trifluormethyl, substituiert ist; Halogen-C₁-C₇-alkyl; Hydroxy-C₁-C₇-alkyl; C₁-C₇-Alkoxy-C₁-C₇-alkyl; C₁-C₇-Alkoxy-C₁-C₇-alkoxy-C₁-C₇-alkyl; Phenyloxy- oder Naphthyloxy-C₁-C₇-alkyl; Phenyl-C₁-C₇-alkoxy- oder Naphthyl-C₁-C₇-alkoxy-C₁-C₇-alkyl; Amino-C₁-C₇-alkyl; N-Mono- oder N,N-Di(C₁-C₇-alkyl und/oder mono-C₁-C₇-alkoxy-C₁-C₇-alkyl und/oder (mono- oder di(C₁-C₇-alkyl)amino-C₁-C₇-alkyl)amino-C₁-C₇-alkyl; C₁-C₇-Alkoxy-C₁-C₇-alkylamino-C₁-C₇-alkyl; Mono- oder Di[C₆-C₁₈-aryl-C₁-C₇-alkyl, worin Aryl gegebenenfalls durch C₁-C₇-Alkyl, durch Pyrrolidinyl, durch Piperazinyl, durch Amino, durch N-Mono- und/oder N,N-Di-C₁-C₇-alkylamino, durch Halogen, durch C₁-C₇-Alkoxy und/oder durch Halogen-C₁-C₇-alkyl substituiert ist; (Pyrrolidinyl, Piperidinyl, Piperazinyl, Morpholino, Thiomorpholino, Pyridinyl, Pyrimidinyl, Pyraxinyl oder Pyridazinyl)-C₁-C₇-alkyl, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, durch Pyrrolidinyl, durch Piperazinyl, durch Amino, durch N-Mono- und/oder N,N-Di-C₁-C₇-alkylamino, durch Halogen, durch C₁-C₇-Alkoxy und/oder durch Halo-C₁-C₇-alkyl substituiert ist; (Pyrrolidinyl, Piperidinyl, Piperazinyl, Pyridinyl, Pyrimidinyl, Pyrazinyl oder Pyridazinyl)oxy-C₁-C₇-alkyl, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, durch Pyrrolidinyl, durch Piperazinyl,
durch Amino, durch N-Mono- und/oder N,N-Di-C₁-C₇-alkylamino, durch Halogen, durch C₁-C₇-Alkoxy und/oder durch Halogen-C₁-C₇-alkyl substituiert ist; und/oder (Pyrrolidinyl, Piperidinyl, Piperazinyl, Pyridinyl, Pyrimidinyl, Pyrazinyl oder Pyridazinyl)carbonyl-C₁-C₇-alkyl, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, durch Pyrrolidinyl, durch Piperazinyl, durch Amino, durch N-Mono- und/oder N,N-Di-C₁-C₇-alkylamino, durch Halogen, durch C₁-C₇-Alkoxy und/oder durch Halogen-C₁-C₇-alkyl substituiert ist; insbesondere [Naphthyl- und/oder Phenyl-C₁-C₇-alkyl]amino-C₁-C₇-alkyl; C₁-C₇-Alkanoylamino-C₁-C₇-alkyl; Carboxy-C₁-C₇-alkyl; Benzoyl- oder Naphthoylamino-C₁-C₇-alkyl; C₁-C₇-Alkylsulfonylamino-C₁-C₇-alkyl; Phenyl- oder Naphthylsulfonylamino-C₁-C₇-alkyl, worin Phenyl oder Naphthyl gegebenenfalls durch eine oder mehrere, insbesondere eine bis drei, C₁-C₇-Alkylgruppen substituiert ist, Phenyl- oder Naphthyl-C₁-C₇-alkylsulfonylamino-C₁-C₇-alkyl, Halogen; Hydroxy; C₁-C₇-alkoxy; C₆-C₁₈-Aryl-C₁-C₇-alkoxy, worin Aryl vorzugsweise für Phenyl, Naphthyl, Biphenylenyl, Indacenyl, Acenaphthylenyl, Fluorenyl, Phenalenyl, Phenanthrenyl oder Anthracenyl steht und gegebenenfalls durch C₁-C₇-Alkyl, wie Methyl oder Ethyl, durch C₁-C₇-Alkoxy, durch Pyrrolidinyl, insbesondere Pyrrolidino, durch Piperazinyl, insbesondere Piperazino, durch Amino, durch N-Mono- und/oder N,N-Di-C₁-C₇-alkylamino, durch Halogen, durch C₁-C₇-Alkoxy, wie Methoxy, und/oder durch Halogen-C₁-C₇-alkyl, wie Trifluorfnethyl, substituiert ist; Halogen-C₁-C₇-alkoxy; Hydroxy-C₁-C₇-alkoxy; C₁-C₇-Alkoxy-C₁-C₇-alkoxy; Amino-C₁-C₇-alkoxy; N-C₁-C₇-Alkanoylamino-C₁-C₇-alkoxy; N-unsubst.-, N-Mono- oder N,N-Di(C₁-C₇-alkyl)-carbamoyl-C₁-C₇-alkoxy; Phenyl- oder Naphthyloxy;
Phenyl- oder Naphthyl-C₁-C₇-alkyloxy; (Pyrrolidinyl, Piperidinyl, Piperazinyl, Pyridinyl, Pyrimidinyl, Pyrazinyl oder Pyridazinyl)-C₁-C₇-alkoxy, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, durch Pyrrolidinyl, durch Piperazinyl, durch Amino, durch N-Mono- und/oder N,N-Di-C₁-C₇-alkylamino, durch Halogen, durch C₁-C₇-Alkoxy und/oder durch Halogen-C₁-C₇-alkyl substituiert ist; (Pyrrolidinyl, Piperidinyl, Piperazinyl, Pyridinyl, Pyrimidinyl, Pyrazinyl oder Pyridazinyl) oxy-C₁-C₇-alkoxy, worin Pyrrolidinyl, Piperidinyl, Piperazinyl, Pyridinyl, Pyrimidinyl, Pyrazinyl oder Pyridazinyl gegebenenfalls durch C₁-C₇-Alkyl, durch Pyrrolidinyl, durch Piperazinyl, durch Amino, durch N-Mono- und/oder N,N-Di-C₁-C₇-alkylamino, durch Halogen, durch C₁-C₇-Alkoxy und/oder durch Halogen-C₁-C₇-alkyl substituiert ist; C₁-C₇-Alkanoyloxy; Benzoyl- oder Naphthoyloxy; C₁-C₇-Alkylthio, Halogen-C₁-C₇-alkylthio; C₁-C₇-Alkoxy-C₁-C₇-alkylthio; Phenyl oder Naphthylthio; Phenyl- oder Naphthyl-C₁-C₇-alkylthio; C₁-C₇-Alkanoylthio; Benzoyl- oder Naphthylthio; Nitro; Amino; Mono- oder Di(C₁-C₇-alkyl)amino ; Mono- oder Di(naphthyl- oder phenyl-C₁-C₇-alkyl)amino; C₁-C₇-Alkanoylamino; Benzoyl- oder Naphthoylamino; C₁-C₇-Alkylsulfonylamino; Phenyl- oder Naphthylsulfonylamino, worin Phenyl oder Naphthyl gegebenenfalls durch eine oder mehrere, insbesondere eine bis drei, C₁-C₇-Alkylgruppen substituiert ist; Phenyl-oder Naphthyl-C₁-C₇-alkylsulfonylamino; C₁-C₇-Alkanoyl; C₁-C₇-Alkoxy-C₁-C₇-alkanoyl; Carboxyl; C₁-C₇-Alkoxycarbonyl ; Phenoxy- oder Naphthoxycarbonyl; Phenyl- oder Naphthyl-C₁-C₇-alkoxycarbonyl; C₁-C₁₀-, insbesondere C₁-C₇-Alkylendioxy; Carbamoyl; N-Mono-oder N,N-Di(C₁-C₇-alkyl, naphthyl-C₁-C₇-alkyl, pyrrolidinyl-C₁-C₇-alkyl, piperidinyl-C₁-C₇-alkyl, piperazinyl- oder N-C₁-C₇-alkyl)piperaxinyl-C₁-C₇-alkyl, Phenyl-C₁-C₇-alkyl, Mono-C₁-C₇-alkoxy-C₁-C₇-alkyl und/oder (N'-Mono- oder N',N'-Di(C₁-C₇-alkyl)amino-C₁-C₇-alkyl)aminocarbonyl; N-C₁-C₇-Alkoxy-C₁-C₇-alkylcarbamoyl; Pyrrolidin-1-carbonyl; Amno-N-pyrrolidin-1-carbonyl; N-Mono- oder N,N-Di(C₁-C₇-alkyl)amino-pyrrolidin-1-carbonyl; Piperidin-1-carbonyl; Morpholin-4-carbonyl; Thiomorpholin-4-carbonyl; S-Oxothiomorpholin-4-carbonyl; S,S-Dioxothiomorpholin-4-carbonyl; Piperazin-1-carbonyl; N-C₁-C₇-Alkylpiperazin-1-carbonyl; N-C₁-C₇-Alkoxycarbonylpiperazin-1-carbonyl; N-mono- oder N,N-di(C₁-C₇-alkyl)aminosubstituiertes oder unsubstituiertes Pyrrolidinyl-C₁-C₇-alkyl; Cyano; C₁-C₇-Alkenylen oder -Alkinylen; C₁-C₇-Alkylsulfonyl; Phenyl- order Naphthylsulfonyl, worin Phenyl oder Naphthyl gegebenenfalls durch eine oder mehrere, insbesondere eine bis drei, C₁-C₇-Alkylgruppen substituiert ist; Phenyl- oder Naphthyl-C₁-C₇-alkylsulfonyl; Sulfamoyl; N-Mono-oder N,N-Di(C₁-C₇-alkyl, phenyl-, naphthyl-, pyrrolidinyl(insbesondere pyrrolidino)-C₁-C₇-alkyl, piperidinyl (insbesondere piperidino) -C₁-C₇-alkyl, piperazinyl (insbesondere piperazino)-C₁-C₇-alkyl, N-C₁-C₇-alkylpiperazinyl(insbesondere 4-C₁-C₇-alkyl-piperazino)-C₁-C₇-alkyl, phenyl-C₁-C₇-alkyl-und/oder naphthyl-C₁-C₇-alkyl) aminosulfonyl; Pyrazolyl; Pyrazolidinyl; Pyrrolyl; Pyridyl, das gegebenenfalls durch C₁-C₇-Alkoxy und/oder durch Halogen-C₁-C₇-alkyl substituiert ist; Pyrrolidinyl; Piperidinyl; Morpholinyl; Thiomorpholinyl; S-Oxothiomorpholinyl; S,S-Dioxothiomorpholinyl; Piperazinyl; N-C₁-C₇-Alkylpiperazinyl; 4-(Phenyl-C₁-C₇-alkyl)piperazinyl; 4-(Naphthyl-C₁-C₇-alkyl)piperazinyl; 4-(C₁-C₇-Alkoxy-carbonyl) piperazinyl; 4 - (Phenyl-C₁-C₇-alkoxycarbonyl)piperazinyl und 4-(Naphthyl-C₁-C₇-alkoxy-carbonyl)piperazinyl, ausgewählt sind,
oder für gegebenenfalls substituiertes Heteroaryl, wobei Heteroaryl aus der Gruppe bestehend aus Imidazolyl, Thiophenyl, Pyraxolyl, Pyrrolyl, Imidazolyl, Pyridyl, pyrimidinyl, Pyridazinyl, Furyl, 2H- oder 4H-Pyranyl, Oxazolyl, Thiazolyl, 5H-Indazolyl, Isoindolyl, Chinolyl, Isochinolinyl, Phthalazinyl, 1,8-Naphthyridinyl, Chinoxalinyl, Chinazolinyl, Cinnolinyl, Indolizinyl, 4H-Chinolizinyl, Pteridinyl, Purinyl, Carbazolyl, beta-Carbolinyl, Acridinyl, Phenanthridinyl, Phenyzinyl, 1, 7-Phenanthrolinyl, Perimidinyl, Benzofuranyl, Isobenzofuranyl, 2H-Chromenyl, 4aH-Isochromenyl, Thianthrenyl, Xanthenyl, Phenoxathiinyl, Phenoxazinyl oder Phenothiazinyl ausgewählt ist, wobei jede dieser Gruppen gegebenenfalls wie oben für Aryl definiert substituiert ist, steht;
R³ für Wasserstoff, Halogen, C₁-C₇-Alkyl, C₁-C₇-Alkoxy oder Cyano steht;
R⁴ für gegebenenfalls substituiertes Aryl oder gegebenenfalls substituiertes Heteroaryl steht, das unabhängig voneinander unter gegebenenfalls substituiertem Aryl gemäß der für R² angegebenen Definition und gegebenenfalls substitiertem Heteroaryl ausgewählt ist, wobei Heteroaryl aus der Gruppe bestehend aus Imidazolyl, Thiophenyl, Pyrrolyl, Imidazolyl, pyridyl, Pyrimidinyl, Pyridazinyl, Furyl, 2H- oder 4H-Pyranyl, oxazolyl, Thiazolyl, 5H-Indazolyl, Indoxyl, Isoindolyl, Chinolyl, Isochinolinyl, Phthalazinyl, 1,8-Naphthyridinyl, Chinoxalinyl, Chinazolinyl, Cinnolinyl, Indolizinyl, 4H-Chinolizinyl, Pteridinyl, Purinyl, Carbazolyl, beta-Carbolinyl, Acridinyl, Phenanthridinyl, Phenyzinyl, 1,7-Phenanthrolinyl, pyrimidinyl, Benzofuranyl, Isobenzofuranyl, 2H-Chromenyl, 4aH-Isochromenyl, Thianthrenyl, Xanthenyl, Phenoxathiinyl, Phenoxazinyl oder Phenothiazinyl gemäß der für R² angegebenen Definition ausgewählt ist; oder dann, wenn R³ für Amino oder einfach durch C₁-C₇-Alkyl (vorzugsweise C₁-C₄-Alkyl) oder C₃-C₈-Cycloalkyl (vorzugsweise C₃-C₅-cycloalkyl) substituiertes Amino steht, auch für Pyrazolyl stehen kann, wobei jedes Heteroaryl gegebenenfalls wie oben für Aryl-R² beschrieben substituiert ist; und
R⁵ für Wasserstoff, Methyl oder durch Halogen substituiertes Methyl steht;
oder ein Tautomer davon oder ein N-Oxid davon oder ein pharmazeutisch unbedenkliches Salz oder ein Hydrat oder Solvat davon.

3. Verbindung der Formel I nach Anspruch 1, worin
R¹ für Wässerstoff, Amino, N-Mono-C₁-C₁₀-alkylamino (vorzugsweise N-Mono-C₁-C₄-alkylamino) oder C₃-C₈-Cycloalkylamino (vorzugsweise C₃-C₅-Cycloalkyl-amino) steht;
R² für Phenyl, Naphthyl, Pyrrolyl, Thiophenyl, Pyrazolyl, Triazolyl, Pyridyl, Chinolyl oder Chinoxalinyl oder für Pyrrolopyridinyl steht, wobei jede dieser Gruppen gegebenenfalls durch einen oder mehrere Substituenten substituiert ist, die unabhängig aus der Gruppe bestehend aus C₁-C₇-Alkyl, Halogen-C₁-C₇-alkyl, Phenyl, das gegebenenfalls durch einen bis drei unabhängig voneinander unter Hydroxyl-C₁-C₇-alkyl, C₁-C₇-Alkoxy-C₁-C₇-alkyl, C₂-C₇-Alkoxy, Amino und Carbamoyl ausgewählte Substituenten substituiert ist, Halogen, Hydroxy, C₁-C₇-Alkoxy, C₁-C₇-Alkoxy-C₁-C₇-alkoxy, Halogen-C₁-C₇-alkoxy, wie Trifluormethoxy, Amino, N-Mono- oder N,N-Di(C₁-C₇-alkyl, phenyl-C₁-C₇-alkyl und/oder naphthyl-C₁-C₇-alkyl)amino, C₁-C₇-Alkanoylamino, Carboxy, C₁-C₇-Alkoxycarbonyl, Phenyl-C₁-C₇-alkoxycarbonyl, Naphthyl-C₁-C₇-alkoxycarbonyl, Phenoxycarbonyl, Naphthoxycarbonyl, C₁-C₄-Alkylendioxy, Cyano, Carbamoyl, N-Mono- oder N,N-Di(C₁-C₇-alkyl, N',N'-di(C₁-C₇-alkyl)amino-C₁-C₇-alkyl, pyrrolidino-C₁-C₇-alkyl und/oder phenyl-C₁-C₇-alkyl)carbamoyl, Piperidin-1-carbonyl, Piperazin-1-carbonyl, 4-C₁-C₇-Alkylpiperazin-1-carbonyl, Morpholin-4-carbonyl, Thiomorpholin-4-carbonyl, S-Oxothiomorpholin-4-carbonyl, S,S-Dioxothiomorpholin-4-carbonyl, Sulfamoyl, N-Mono-oder N,N-Di(C₁-C₇-alkyl, N',N'-di(C₁-C₇-alkyl)amino-C₁-C₇-alkyl, pyrrolidino-C₁-C₇-alkyl und/oder phenyl-C₁-C₇-alkyl)sulfamoyl, Pyrazolyl, Pyrazolidinyl, Pyrrolyl, Pyrrolidinyl, Piperidinyl, Piperazinyl, 4-C₁-C₇-Alkylpiperazinyl, 4-(Phenyl-C₁-C₇-alkyl)piperazinyl, 4-(Naphthyl-C₁-C₇-alkyl)piperazinyl, 4-(C₁-C₇-Alkanoyl)-piperazinyl, 4-(C₁-C₇-Alkoxycarbonyl)piperazinyl, 4-(Phenyl-C₁-C₇-alkoxycarbonyl) piperazinyl, 4-(Naphthyl-C₁-C₇-alkoxycarbonyl) piperazinyl, 4-(C₁-C₇-Alkansulfonyl)piperazinyl, 2-Oxopyrrolidin-1-yl, 2-Oxoazetidin-1-yl, 2-Oxopiperidin-1-yl, 3-C₁-C₇-Alkyl-2-oxoimidazolidin-1-yl, morpholinyl, Thiomorpholinyl, S-Oxothiomorpholinyl und S,S-Dioxo-thiomorpholinyl, und/oder unter 2-Aminopyrimidin-5-yl-C₁-C₇-alkyl, 4-C₁-C₇-Alkylpiperazin-1-carbonyl-C₁-C₇-alkoxy, 4-Pyrrolidinopiperidin-1-carbonyl-C₁-C₇-alkoxy, 4-Pyrrolidinopiperidin-1-yl-C₁-C₇-alkoxy, 4-C₁-C₇-Alkylpiperazino-C₁-C₇-alkoxy, Pyridinyloxy-C₁-C₇-alkoxy (z.B. Pyridin-2-yloxy-C₁-C₇-alkoxy), Pyrimidinyloxy-C₁-C₇-alkoxy (z.B. pyrimidin-4-yloxy-C₁-C₇-alkoxy), N,N-Di(C₁-C₇-alkyl)aminopyrrolidin-1-carbonyl und (unsubstituiertem oder C₁-C₇-alkoxy- und/oder halogen-C₁-C₇-alkoxy-substituiertem) Pyridinyl (z.B. Pyridin-3-yl), ausgewählt sind; R³ für Wasserstoff oder für Halogen, vorzugsweise Wasserstoff, steht;
R⁴ für Phenyl, Naphthyl, pyrrolyl, thiophenyl, Triazolyl Pyridyl, Chinolinyl, Chinoxalinyl, Furanyl oder 1H-Pyrrolo[2,3-b]pyridin-5-yl steht, wobei jede dieser Gruppen gegebenenfalls durch einen oder mehrere Substituenten substituiert ist, die unabhängig voneinander aus der Gruppe bestehend aus Halogen-C₁-C₇-alkyl, wie Trifluormethyl, Amino-C₁-C₇-alkyl, wie Aminomethyl, Halogen, Hydroxy, C₁-C₇-Alkoxy, C₁-C₇-Alkoxy-C₁-C₇-alkoxy, Amino-C₁-C₇-alkoxy, Phenyl-C₁-C₇-alkoxy, Amino, N-Mono- oder N,N-Di(C₁-C₇-alkyl, hydroxyl C₁-C₄-alkyl, phenyl-C₁-C₇-alkyl und/oder naphthyl-C₁-C₇-alkyl)amino, C₁-C₇-Alkanoyl, Carboxy, C₁-C₇-Alkoxycarbonyl, Phenyl-C₁-C₇-alkoxycarbonyl, Naphthyl-C₁-C₇-alkoxycarbonyl, Phenoxycarbonyl, Naphthoxycarbonyl, C₁-C₄-Alkylendioxy, Cyano, Carbamoyl, N-Mono- oder N,N-Di(C₁-C₇-alkyl, N',N'-di(C₁-C₇-alkyl)amino-C₁-C₇-alkyl, pyrrolidino-C₁-C₇-alkyl und/oder phenyl-C₁-C₇-alkyl)carbamoyl, Piperidin-1-carbonyl, Piperazin-1-carbonyl, 4-C₁-C₇-Alkylpiperazin-1-carbonyl, Morpholin-4-carbonyl, Thiomorpholin-4-carbonyl, S-Oxothiomorpholin-4-carbonyl, S,S-Dioxothiomorpholin-4-carbonyl, Sulfamoyl, N-Mono- oder N,N-Di(C₁-C₇-alkyl, N',N'-di (C₁-C₇-alkyl)amino-C₁-C₇-alkyl, pyrrolidino-C₁-C₇-alkyl und/ooer phenyl-C₁-C₇-alkyl) sulfamoyl, Pyrazolyl, Pyrazolidinyl, Pyrrolyl, Pyrrolidinyl, Piperidinyl, Piperazinyl, 4-C₁-C₇-Alkyl-piperazinyl, 4- (phenyl-C₁-C₇-alkyl)piperazinyl, 4-(Naphthyl-C₁-C₇-alkyl)piperazinyl, 4-(C₁-C₇-Alkoxycarbonyl) piperazinyl, 4-(Phenyl-C₁-C₇-alkoxycarbonyl) piperazinyl, 4-(Naphthyl-C₁-C₇-alkoxycarbonyl)piperazinyl, Morpholinyl, Thiomorpholinyl, S-Oxothiomorpholinyl und S,S-Dioxo-thiomorpholinyl und/oder unter 2-Aminopyrimidin-5-yl-C₁-C₇-alkyl, 4-C₁-C₇-Alkylpiperazin-1-carbonyl-C₁-C₇-alkoxy, 4-Pyrrolidinopiperidin-1-carbonyl-C₁-C₇-alkoxy, 4-Pyrrolidinopiperidin-1-yl-C₁-C₇-alkoxy, 4-C₂-C₇-Alkylpiperazino-C₁-C₇-alkoxy, Pyridinyloxy-C₁-C₇-alkoxy (z.B. Pyridin-2-yloxy-C₁-C₇-alkoxy), Pyrimidinyloxy-C₁-C₇-alkoxy (z.B. Pyrimidin-4-yloxy-C₁-C₇-alkoxy), N,N-Di(C₁-C₇-alkyl)aminopyrrolidin-1-carbonyl und (unsubstituiertem oder C₁-C₇-alkoxy- und/oder halogen-C₁-C₇-alkoxy-substituiertem) Pyridinyl (z.B. Pyridin-3-yl), ausgewählt sind; und
R⁵ für Wasserstoff steht;
oder ein Tautomer davon oder ein N-Oxid davon oder ein pharmazeutisch unbedenkliches Salz oder ein Hydrat oder Solvat davon.

4. Verbindung der Formel I nach Anspruch 1, worin
R¹ für Wasserstoff, amino, N-Mono-C₁-C₁₀-alkylamino (vorzugsweise N-Mano-C₁-C₄-alkylamino) oder C₃-C₈-Cycloalkylamino (vorzugsweises C₃-C₅-Cycloalkyl-amino) steht;
R² für Phenyl, Naphthyl, Pyrrolyl, thiophenyl, Pyrazolyl, Triazolyl, Pyridyl, Chinolyl oder Chinoxalinyl steht, wobei jede dieser Gruppen gegebenenfalls durch einen oder mehrere Substituenten substituiert ist, die unabhängig aus der Gruppe bestehend aus Halogen, Hydroxy, C₁-C₇-Alkoxy, C₁-C₇-Alkoxy-C₁-C₇-alkoxy, Amino, N-Mono-oder N,N-Di(C₁-C₇-alkyl, phenyl-C₁-C₇-alkyl und/oder naphthyl-C₁-C₇-alkyl) amino, Carboxy, C₁-C₇-Alkoxycarbonyl, Phenyl-C₁-C₇-alkoxycarbonyl, Naphthyl-C₁-C₇-alkoxycarbonyl, Phenoxycarbonyl, Naphthoxycarbonyl, C₁-C₄-Alkylendioxy, Carbamoyl, N-Mono-oder N,N-Di(C₁-C₇-alkyl, N',N'-di(C₁-C₇-alkyl) amino-C₁-C₇-alkyl, pyrrolidino-C₁-C₇-alkyl und/oder phenyl-C₁-C₇-alkyl) carbamoyl, Piperidin-1-carbonyl, Piperazin-1-carbonyl, 4-C₁-C₇-Alkylpiperazin-1-carbonyl, Morpholin-4-carbonyl, Thiomorpholin-4-carbonyl, S-Oxothiomorpholin-4-carbonyl, S,S-Dioxothiomorpholin-4-carbonyl, Sulfamoyl N-Mono-oder (C₁-C₇-alkyl, N',N'-di(C₁-C₇-alkyl) amino-C₁-C₇-alkyl, pyrrolidino-C₁-C₇-alkyl und/oder phenyl-C₁-C₇-alkyl) sulfamoyl, Pyrazolyl, Pyrazolidinyl, Pyrrolyl, Pyrrolidinyl, Piperidinyl, piperazinyl, 4-C₁-C₇-Alkylpiperazinyl, 4-(Phenyl-C₁-C₇-alkyl)piperazinyl, 4-(Naphthyl-C₁-C₇-alkyl) piperazinyl, 4- (C₁-C₇-Alkoxycarbonyl)-piperazinyl, 4-(Phenyl-C₁-C₇-alkoxycarbonyl)-piperazinyl, 4-(Naphthyl-C₁-C₇-alkoxycarbonyl)-piperazinyl, Morpholinyl, Thiomorpholinyl, S-Oxothiomorpholinyl und S,S-Dioxothiomorpholinyl, und/oder unter 2-Aminopyrimidin-5-yl-C₁-C₇-alkyl, 4-C₁-C₇-Alkylpiperazin-1-carbonyl-C₁-C₇-alkoxy, 4-Pyrrolidinopiperidin-1-carbonyl-C₁-C₇-alkoxy, 4-Pyrrolidinopiperidin-1-yl-C₁-C₇-alkoxy, 4-C₁-C₇-Alkylpiperazino-C₁-C₇-alkoxy, Pyridinyloxy-C₁-C₇-alkoxy (z.B. Pyridin-2-yloxy-C₁-C₇-alkoxy), Pyrimidinyloxy-C₁-C₇-alkoxy (z.B. Pyrimidin-4-yloxy-C₁-C₇-alkoxy), N,N-Di(C₁-C₇-alkyl) aminopyrrolidin-1-carbonyl und (unsubstituiertem oder C₁-C₇-alkoxy- und/oder halogen-C₁-C₇-alkoxy-substituiertem) Pyridinyl (z.B. Pyridin-3-yl), ausgewählt sind;
R³ für Wasserstoff oder für Halogen, vorzugsweise Wasserstoff, steht;
R⁴ für Phenyl, Naphthyl, Pyrrolyl, Thiophenyl, Triazolyl, Pyridyl, Chinolinyl oder Chinoxalinyl steht, wobei jede dieser Gruppen gegebenenfalls durch einen oder mehrere Substituenten substituiert ist, die unabhängig voneinander aus der Gruppe bestehend aus Halogen, Hydroxy, C₁-C₇-Alkoxy, C₁-C₇-Alkoxy-C₁-C₇-alkoxy, Amino, N-Mono-oder N,N-Di(C₁-C₇-alkyl, phenyl-C₁-C₇-alkyl und/oder naphthyl-C₁-C₇-alkyl amino, Carboxy, C₁-C₇-Alkoxycarbonyl, Phenyl-C₁-C₇-alkoxycarbonyl,
Naphthyl-C₁-C₇-alkoxycarbonyl, Phenoxycarbonyl, Naphthoxycarbonyl, C₁-C₄-Alkylendioxy, Carbamyl, N-Mono- oder N,N-Di(C₁-C₇-alkyl, N',N-di(C₁-C₇-alkyl) amino-C₁-C₇-alkyl, pyrrolidino-C₁-C₇-alkyl und/oder phenyl-C₁-C₇-alkyl)carbamoyl, Piperidin-1-carbonyl, Piperazin-1-carbonyl, 4-C₁-C₇-Alkylpiperazin-1-carbonyl, Morpholin-4-carbonyl, Thiomorpholin-4-carbonyl, S-Oxothiomorpholin-4-carbonyl, S,S-Dioxothiomorpholin-4-carbonyl, Sulfamoyl, N-Mono- oder N,N-Di(C₁-C₇-alkyl, N',N'-di(C₁-C₇-alkyl) amino-C₁-C₇-alkyl, pyrrolidino-C₁-C₇-alkyl und/oder phenyl-C₁-C₇-alkyl) sulfamoyl, pyrazolyl, Pyrazolidinyl, Pyrrolyl, Pyrrolidinyl, Piperidinyl, Piperazinyl, 4-C₁-C₇-Alkyl-piperazinyl, 4-(Phenyl-C₁-C₇-alkyl)piperazinyl, 4-(Naphthyl-C₁-C₇-alkyl)piperazinyl, 4- (C₁-C₇-Alkoxycarbonyl)piperazinyl, 4-(Phenyl-C₁-C₇-alkoxycarbonyl) piperazinyl, 4- (Naphthyl-C₁-C₇-alkoxycarbonyl)piperazinyl, Morpholinyl, Thiomorpholinyl, S-Oxothiomorpholinyl und S,S-Dioxothiomorpholinyl und/oder unter 2-Aminopyrimidin-5-yl-C₁-C₇-alkyl, 4-C₁-C₇-Alkylpiperazin-1-carbonyl-C₁-C₇-alkoxy, 4-Pyrrolidinopiperidin-1-carbonyl-C₁-C₇-alkoxy, 4-Pyrrolidinopiperidin-1-yl-C₁-C₇-alkoxy, 4-C₁-C₇-Alkylpiperazino-C₁-C₇-alkoxy, Pyridinyloxy-C₁-C₇-alkoxy (z.B. Pyridin-2-yloxy-C₁-C₇-alkoxy), Pyrimidinyloxy-C₁-C₇-alkoxy (z.B. Pyrimidin-4-yloxy-C₁-C₇-alkoxy), N,N-Di (C₁-C₇-alkyl)aminopyrrolidin-1-carbonyl und (unsubstituiertem oder C₁-C₇-alkoxy- und/oder halogen-C₁-C₇-alkoxy-substitulertem) Pyridinyl (z.B. Pyridin-3-yl), ausgewählt sind; und
R⁵ für Wasserstoff steht;
oder ein Tautomer davon oder ein N-Oxid davon oder ein pharmazeutisch unbedenkliches Salz oder ein Hydrat oder Solvat davon.

5. Verbindung der Formel I nach Anspruch 1, worin
R¹ für Wasserstoff, Amino, Methylamino, n-Propylamino oder Cyclopropylamino steht;
R² für Phenyl, 3-Methoxyphenyl, 4-Methoxyphenyl, 3,4-Dimethoxyphenyl, 3,4,5-Trimethoxyphenyl, 3-Chlor-4-n-propoxyphenyl, 4-Carboxy-3-methoxyphenyl, 4-Methoxycarbonyl-3-methoxyphenyl, 4-Carbamoylphenyl, 4-N-Methylcarbamoyl-3-methoxyphenyl, 4-(N,N-Dimethylcarbamoyl)-3-methoxyphenyl, 4-(4-Methylpiperazin-1-carbonyl)-3-methoxyphenyl, 4-(4-Morpholin-1-carbonyl)-3-methoxyphenyl, Benzo-[1,3]dioxol-5-yl, 2,3-Dihydrobenzo[1,4]dioxin-6-yl, 4-(Piperazin-1-yl)phenyl, 4-Sulfamoylphenyl, 4-N,N-Dimethylsulfamoylphenyl, 4-Pyrazolylphenyl, Pyrrolyl, Pyrazolyl, Thiophenyl, 1,2,4-Triazol-1-yl, 6-Methoxypyridin-3-yl oder 6-Piperazinopyridin-3-yl steht;
R³ für Wasserstoff steht;
R⁴ für 3-Hydroxyphenyl, 4-Hydroxyphenyl, 4-hydroxyl 3-methoxyphenyl, 3-Hydroxy-4-n-propoxyphenyl, 3,4-Dimethoxyphenyl, 3,4,5-Trimethoxyphenyl, 4-Ethoxy-3-methoxyphenyl, 3-(2-Methoxyethoxy)-4-methoxyphenyl, 3-Methoxy-4-(2-methoxyethoxy)phenyl, 3-Fluor-4-methoxyphenyl, 3-Chlor-4-n-propoxyphenyl, 4-Methoxycarbonyl-3-methoxyphenyl, 4-Carbamoylphenyl, N,N-Dimethylaminosulfonylphenyl, Benzo-[1,3]dioxol-5-yl, 2,3-Dihydrobenzo[1,4]dioxin-6-k1, Pyridin-3-yl, 6-Methoxypyridin-3-yl, 5-Methoxypyridin-3-yl, 2-Aminopyridin-4-yl, 6-Aminopyridin-3-yl, 6-(Piperazin-1-yl)pyridin-3-yl, 6-(4-tert.-Butoxycarbonylpiperazin-1-yl)pyridin-3-yl, 2-(Piperazin-1-yl)pyridin-4-yl oder 2-(4-tert. -Butoxycacbonylpiperazin-1-yl)pyridin-4-yl steht und
R⁵ für Wasserstoff steht;
oder ein Tautomer davon oder ein N-Oxid davon oder ein pharmazeutisch unbedenkliches Salz oder ein Hydrat oder Solvat davon.

6. Verbindung der Formel 1 nach Anspruch 1, worin
R¹ für Wasserstoff, Amino, Methylamine, n-Propylamino oder Cyclopropylamino steht;
R² für Phenyl, 4-Trifluormethylphenyl, 3-Fluorphenyl, 4-Fluorphenyl, 4-Chlorphenyl, 4-Bromphenyl, 3-Methoxyphenyl 4-Methoxyphenyl, 3-Ethoxyphenyl, 3,4-Dimethoxyphenyl, 4-Ethoxy-3-methoxyphenyl, 3,4-Diethoxyphenyl, 3-Benzyloxy-4-methoxyphenyl, 4-(2-Methoxyethoxy)-3-methoxyphenyl, 4-Trifluormethoxyphenyl, 4-Methoxy-3-trifluormethoxyphenyl, 4-(3-tert.-Butoxycarbonyl-aminopropoxy)-3-methoxyphenyl, 4-(2-tert.-Butoxy-carbonylaminoethoxy)-3-methoxyphenyl, 3,4,5-Trimethoxyphenyl, 3-Chlor-4-n-propoxyphenyl, 4-Acetylaminophenyl, 4-Carboxy-3-methoxyphenyl, 4-Methoxycarbonylphenyl, 4-Methoxycarbonyl-3-methoxyphenol, 4-Cyanophenyl, 4-Biphenylyl, 4'-Aminobiphenyl-4-yl, 4'-Methoxybiphenyl-4-yl, 4'-Hydroxymethylbiphenyl-4-yl, 4'-Methoxymethylbiphenyl-4-yl, 3', 4'-Dimethoxybiphenyl-4-yl, 4'-Carbamoylbiphenyl-4-yl, 4-Carbamoylphenyl, 4-N-Methylcarbamoyl-3-methoxyphenyl, 4-(N,N-dimethylcarbamoyl)phenyl, 4-(N-Methylcarbamoyl)-phenyl, 4-(N-N-Dimethylcarbamoyl)-3-methoxyphenyl, 4-(4-Methylpiperazin-1-carbonyl)-3-methoxyphenyl, 4-(Morpholin-4-carbonyl)phenyl, 4-(4-Morpholin-1-carbonyl)-3-methoxyphenyl, Benzo[1,3]dioxol-5-yl, 2,3-Dihydrobenzo[1,4]dioxin-6-yl, 4-(Piperazin-1-yl)phenyl, 4-(2-Oxopyrrolidin-1-yl)phenyl, 4-(2-Oxoazetidin-1-yl)phenyl, 4-(2-Oxopiperidin-1-yl)-phenyl, 4-(3-Methyl-2-oxoimidazolidin-1-yl)phenyl, 4-Methansulfonylphenyl, 4-Sulfamoylphenyl, 4-N,N-Dimethylsulfamoylphenyl, 4-Pyrazolylphenyl, Pyrrolyl, Pyrazolyl, Thiophenyl, insbesondere Thiophen-3-yl, 1,2,4-Triazol-1-yl, 2-Methoxypyridin-4-yl, 5-Methoxypyridin-3-yl, 6-Methoxypyridin-3-yl, 6-Piperazinopyridin-3-yl, 6-Morpholin-4-ylpyridin-3-yl, 1H-Pyrrolo[2,3-b]-pyridin-5-yl, 4-[6-(4-Methansulfonyl)piperazin-1-yl]pyridin-3-yl, 5-(4-Acetylpiperazin-1-yl)-pyridin-3-yl oder 2-[4-(tert.-Butoxycarbonyl)-piperazin-1-yl]pyridin-4-yl steht;
R³ für Wasserstoff steht;
R⁴ für 3-Hydroxyphenyl, 4-Hydroxyphenyl, 4-Hydroxy-3-methoxyphenyl, 3-Hydroxy-4-n-propoxyphenyl, 3-Ethoxyphenyl, 3,4-Dimethoxyphenyl, 3,4-Diethoxyphenyl, 3,4,5-Trimethoxyphenyl, 3-Ethoxy-4-methoxyphenyl, 4-Ethoxy-3-methoxyphenyl, 3-(2-Methoxyethoxy)-4-methoxyphenyl, 3-Methoxy-4-(2-methoxyethoxy)phenyl, 3-Benzyloxy-4-methoxyphenyl, 4-(3-Aminopropoxy)-3-methoxyphenyl, 5-(3-Aminopropoxy)-3-methoxyphenyl, 4-(2-Aminoethoxy)-3-methoxyphenyl, 5-(2-Aminoethoxy)-3-methoxyphenyl, 3-Fluor-4-methoxyphenyl, 3-Chlor-4-methoxyphenyl, 3-Chlor-4-n-proproxyphenyl, 4-(3-tert.-Butoxycarbonylaminopropoxy)-3-methoxyphenyl, 4-(2-tert.-Butoxycarbonylaminoethoxy)-3-methoxyphenyl, 4-Formylphenyl, 4-Methoxycarbonyl-3-methoxyphenyl, 3-Carbamoylphenyl (4-Carbamoylphenyl, 4-Carbamoyl-3-methoxyphenyl, 3-Sulfamoylphenyl, N,N-Dimethylaminosulfonylphenyl, Benzo[1,3]dioxol-5-yl, 2,3-Dihydrobenzo[1,4]dioxin-6-yl, 6-Aminomethylpyridin-3-yl, pyridin-3-yl, 6-Methoxypyridin-3-yl, 5-Methoxypyridin-3-yl, 2-Methoxypyridin-4-yl, 2-Aminopyridin-4-yl, 6-Aminopyridin-3-yl, 6-Amino-5-trifluormethylpyridin-3-yl, 6-Dimethylaminopyridin-3-yl, 6-Methylaminopyridin-3-yl, 6-Isobutylaminopyridin-3-yl, 6-(2-Methoxyethylamino)-pyridin-3-yl, 6-(Piperazin-1-yl)pyridin-3-yl, 6-(4-tert.-Butoxycarbonyl-piperazin-1-yl)pyridin-3-yl, 2-(Piperazin-1-yl)pyridin-4-yl, 6-Carbamoylpyridin-3-yl, (2-Cyanopyridin-5-yl, 5-Cyanopyridin-3-yl, 6-(2-Hydroxyethylamino)pyridin-3-yl, 2-(4-tert.-Butoxycarbonyl-piperazin-1-)pyridin-4-yl, 6-morpholin-4-ylpyridin-3-yl, Furan-2-yl, Furan-3-yl, 1H-Pyrrolo[2,3-b]pyridin-5-yl oder Chinolin-3-yl. steht und
R⁵ für Wasserstoff steht;
oder ein Tautomer davon oder ein N-Oxid davon oder ein pharmazeutisch unbedenkliches Salz oder ein Hydrat oder Solvat davon.

7. Verbindung der Formel I nach Anspruch 1, ausgewählt aus der Gruppe bestehend aus den folgenden Verbindungen:
4-(3,4-Dimethoxyphenyl)-6-(6-piperazin-1-yl-pyridin-3-yl)chinazolin.
4-{5-[4-(3,4-Dimethoxyphenyl)chinazolin-6-yl]-pyridin-2-yl}piperazin-1-carbonsäure-tert.-butylester,
[4,6-Bis(3,4-dimethoxyphenyl)chinazolin-2-yl]-n-propylamin,
6-(6-Methoxypyridin-3-yl)-4-phenylchinazolin, 3-[2-Amino-4-(3,4-dimethoxyphenyl)chinazolin-6-yl]phenol,
6-(3-Chlor-4-n-propoxyphenyl)-4-(3,4-dimethoxyphenyl)chinazolin,
4-[4-(3,4-Dimethoxyphenyl)chinazolin-6-yl]-2-methoxyphenol,
6-(2,3-Dihydrobenzo[1,4]dioxin-6-yl)-4-(3,4-dimethoxyphenyl)chinazolin,
6-(Benzo[1,3]dioxol-5-yl)-4-(3,4-dimethoxyphenyl)-chinazolin,
4-[4-(3,4-Dimethoxyphenyl)chinazolin-6-yl]-2 methoxybenzoesäuremethylester,
4-(3,4-Dimethoxyphenyl)-6-(3,4,5-trimethoxyphenyl)chinazolin,
6-(3,4-Dimethoxyphenyl)-4-(3-fluor-4-methoxyphenyl)chinazolin,
4-[6-(3,4-Dimethoxyphenyl)chinazolin-4-yl]-2-methoxybenzoesäuremethylester,
4-(3-Chlor-4-n-propoxyphenyl)-6-(3,4-dimethoxyphenyl)chinazolin.
6-(3,4-Dimethoxyphenyl)-4-(3,4,5-trimethoxyphenyl)chinazolin,
4-(2,3-Dibydrobenzo[1,4]dioxin-6-yl)-6-(3,4-dimethoxyphenyl)chinazolin,
4-(Benzo[1,3]dioxol-5-yl)-6-(3,4-dimethoxyphenyl)-chinazolin,
6-(6-Piperazin-1-ylpyridin-3-yl)-4-(4-pyrazol-1-ylphenyl)chinazolin,
3-[4-(3,4-Dimethoxyphenyl)chinazolin-6-yl] phenol,
4-[4-(3,4-Dimethoxyphenyl)chinazolin-6-yl]phenol,
4-[4-(3,4-Dimethoxyphenyl)chinazolin-6-yl]benzamid,
4-(3,4-Dimethoxyphenyl-6-(3-fluor-4-methoxyphenyl)chinazolin,
6-(3,4-Dimethoxyphenyl)-4-phenylchinazolin,
6-(3,4-Dimethoxyphenyl)-4-thiophen-2-ylchinazolin,
6-(3,4-Dimethoxyphenyl)-4-(3-methoxyphenyl)-chinazolin,
6-(3,4-Dimethoxyphenyl)-4-(4-methoxyphenyl)-chinazolin,
6-(3,4-Dimethoxyphenyl)-4-(4-pyrazol-1-yl-phenyl)-chinazolin,
6-(3,4-Dimethoxyphenyl)-4-(6-piperazin-1-yl-pyridin-3-yl)chinazolin,
4-[6-(6-Piperazin-1-ylpyridin-3-yl)chinazolin-4-yl]benzamid,
6-(6-Methoxypyridin-3-yl)-4-(4-pyrazol-1-yl-phenyl)chinazolin,
4-[6-(6-Methoxypyridin-3-yl)chinazolin-4-yl]benzamid,
6-(6-Methoxypyridin-3-yl)-4-(6-piperazin-1-yl-pyridin-3-yl)chinazolin,
4,6-Bis(3,4-Dimethoxyphenyl)chinazolin,
4-(6-(3,4-Dimethoxyphenyl)chinazolin-4-yl]benzamid,
6-(3,4-Dimethoxyphenyl)-4-(6-methoxypyridin-3-yl)-chinazolin,
4-(3,4-Dimethoxyphenyl)-6-(6-methoxypyridin-3-yl)-chinazolin,
4-(6-Methoxypyridin-3-yl)-6-(6-piperazin-1-yl-pyridin-3-yl)chinazolin,
6-(6-Methoxypyridin-3-yl)-4-thiophen-2-yl-chinazolin,
4-(2-Chlorphenyl)-6-(6-methoxypyridin-3-yl)-chinazolin,
4,6-Bis(6-methoxypyridin-3-yl)chinazolin,
4-(4-Methoxyphenyl)-6-(6-methoxypyridin-3-yl)-chinazolin,
4-(3,4-Dimethoxyphenyl)-6-(4-ethoxy-3-methoxyphenyl) chinazolin,
[4,6-Bis-(3,4-dimethoxyphenyl)chinazolin-2-yl]-cyclopropylamin,
4-[2-Amino-6-(3,4-dimethoxyphenyl)chinazolin-4-yl]benzamio,
4-(3,4-Dimethoxyphenyl)-6-(3-fluor-4-methoxyphenyl)chinazolin-2-ylamin,
4-[2-Amino-4-(3,4-dimethoxyphenyl)chinazolin-6-yl]-2-methoxybenzoesäuremethylester,
4-(3,4-Dimethoxyphenyl)-6-(3,4,5-trimethoxyphenyl)chinazolin-2-ylamin,
6-(3-Chlor-4-n-propoxyphenyl)-4-(3,4-dimethoxyphenyl)chinazolin-2-ylamin,
4-[2-Amino-4-(3,4-dimethoxyphenyl)chinazolin-6-yl]phenol,
6-(2,3-Dihydrobenzo[1,4]dioxin-6-yl)-4-(3,4-dimethoxyphenyl)chinazolin-2-ylamin,
4-(Benzo[1,3]dioxol-5-yl)-6-(3,4-dimethoxyphenyl)-chinazolin-2-ylamin,
6-(3,4-Dimethoxyphenyl)-4-(3,4,5-trimethoxyphenyl)chinazolin-2-ylamin,
6-(3,4-Dimethoxyphenyl)-4-(6-methoxypyridin-3-yl)-chinazolin-2-ylamin,
4,6-Bis(3,4-dimethoxyphenyl)chinazolin-2-ylamin,
4,6-Bis(6-methoxypyridin-3-yl)chinazolin-2-ylamin,
4-(3,4-Dimethoxyphenyl)-6-(6-methoxypyridin-3-yl)-chinazolin-2-ylamin,
N-[4,6-Bis-(3,4-dimethoxyphenyl)chinazolin-2-yl]-N-methylamin,
4[6-(3,4-Dimethoxyphenyl)chinazolin-4-yl]-2-methoxybenzoesäure,
4-[6-(3,4-Dimethoxyphenyl)chinazolin-4-yl]-2-methoxybenzoesäure-N-methylamid,
4-[6-(3,4-Dimethoxyphenyl)chinazolin-4-yl]-2-methoxybenzamid,
4-[6-(3,4-Dimethoxyphenyl)chinazolin-4-yl]-2-methoxy-N,N-dimethylamid,
{4-[6-(3,4-Dimethoxyphenyl)chinazolin-4-yl]-2-methoxyphenyl}-(4-methylpiperazin-1-yl)methanon,
{4-[6-(3,4-Dimethoxyphenyl)chinazolin-4-yl]-2-methoxyphenyl}morpholin-4-ylmethanon;
4-(1,2,4-Triazol-1-yl)-6-(3,4-dimethoxyphenyl)-chinazolin,
4-(3,4-Dimethoxyphenyl)-6-[3-methoxy-4-(2-methoxy-ethoxy)phenyl]chinazolin,
5-[4-(3,4-Dimethoxyphenyl)chinazolin-6-yl]pyridin-2-ylamin,
4-[6-(3,4-Dimethoxyphenyl)chinazolin-4-yl]-N,N-dimethylbenzolsulfonamid,
6-(3,4-Dimethoxyphenyl)-4-pyrazol-1-ylchinazolin,
6-(3,4-Dimethoxyphenyl)-4-[1,2,4]triazol-1-yl-chinazolin und
6-(3,4-Dimethoxyphenyl)-4-pyrrol-1-ylchinazolin;
oder jeweils ein N-Oxid davon, ein Tautomer davon und/oder ein pharmazeutisch unbedenkliches Salz davon.

8. Verbindung der Formel I nach Anspruch 1, worin
R¹ für Wasserstoff, Amino, Methylamino, n - Propylamino oder Cyclopropylamino steht;
R² für Phenol, 4-(2-Aminopyrimidin-5-ylmethyl)-phenyl, 3-(2-Methoxy-6-trifluormethyl)pyridin-3-ylphenyl, 3-Methoxyphenyl, 4-Methoxyphenyl, 3,4-Dimethoxyphenyl, 3,4,5-Trimethoxyphenyl, 3-Chlor-4-n-propoxyphenyl, 4-Carboxy-3-methoxyphenyl, 4-(2-Pyridin-2-yloxyethoxy)phenyl, 4-(2-Pyrimidin-4-yloxyethoxy)phenyl, 4-Methoxycarbonyl-3-methoxyphenyl, 4-Carbamoylphenyl, 4-N-Methylcarbamoyl-3-methoxyphenyl, 4-(N,N-Dimethylcarbamoyl)-3-methoxyphenyl, 4-(4-Methylpiperazin-1-carbonyl)-3-methoxyphenyl, 4-(4-Morpholin-1-carbonyl)-3-methoxyphenyl, Benzo[1,3]dioxol-5-yl, 2,3-Dihydrobenzo[1,4]dioxin-6-yl, 4-[2-(4-Pyrrolidino-piperidin-1-yl)ethoxy]phenyl, 4-(Piperazin-1-yl)-phenyl, 4-[2-(4-methylpiperazin-1-yl)ethoxy]-phenyl, 4-[2-(4-Ethylpiperazin-1-yl)ethoxy]phenyl, 4-(4-Methylpiperazin-1-carbonylmethoxy)phenyl, 4-(4-Ethylpiperazin-1-carbonylmethoxy)phenyl, 4-(4-Pyrrolidinopiperidin-1-carbonylmethoxy)phenyl, 4-[N-(2-Dimethylaminoethyl)-N-methylcarbamoyl]-phenyl, 4-[(R,S oder R,S)-3-Diethylamino-pyrrolidin-1-carbonyl)phenyl, 4-sulfamoylphenyl, 4-N,N-Dimethylsulfamoylphenyl, 4-[N-Methyl-N-2-(pyrrolidinoethyl)sulfamol]phenyl, 4-Pyrazolylphenyl, Pyrrolyl, Pyrazolyl, Thiophenyl, 1,2,4-Triazol-1-yl, 6-Methoxypyridin-3-yl oder 6-Piperazinopyridin-3-yl steht;
R³ für Wasserstoff steht;
R⁴ für 4-(2-Aminopyrimidin-5-ylmethyl)phenyl, 3-(2-Methoxy-6-trifluormethyl)pyridin-3-ylphenyl, 3-Hydroxyphenyl, 4-Hydroxyphenyl, 4-Hydroxy-3-methoxyphenyl, 3-Hydroxy-4-n-propoxyphenyl, 3,4-Dimethoxyphenyl, 3,4,5-Trimethoxyphenyl, 4-Ethoxy-3-methoxyphenyl, 3-(2-Methoxyethoxy)-4-methoxyphenyl, 3-Methoxy-4-(2-methoxyethoxy)phenyl, 3-Fluor-4-methoxyphenyl, 3-Chlor-4-n-propoxyphenyl, 4-(2-Pyridin-2-yloxyethoxy)phenyl, 4-(2-Pyrimidin-4-yloxyethoxy)phenyl, 4-[2-(4-Pyrrolidinopiperidin-1-yl)ethoxy]phenyl, 4-[2-(4-Methylpiperazin-1-yl)ethoxy]phenyl, 4-[2-(4-Ethylpiperazin-1-yl)ethoxy]phenyl, 4-(4-methylpiperazin-1-carbonylmethoxy)phenyl, 4-(4-Ethylpiperazin-1-carbonylmethoxy)phenyl, 4-(4-Pyrrolidinopiperidin-1-carbonylmethoxy)phenyl, 4-[N-(2-Dimethylaminoethyl)-N-methylcarbamoyl]-phenyl, 4-[(R,S oder R,S)-3-Diethylaminopyrrolidin-1-carbonyl)phenyl, 4-Methoxycarbonyl-3-methoxyphenyl, 4-Carbamoylphenyl, N,N-Dimethylaminosulfonylphenyl, 4-[N-Methyl-N-2-(pyrrolidinoethyl)sulfamoyl]phenyl, Benzo[1,3]dioxol-5-yl, 3,3-Dihydrobenzo[1,4]dioxin-6-yl, Pyridin-3-yl, 6-Methoxypyridin-3-yl, 5-Methoxypyridin-3-yl, 2-Aminopyridin-4-yl, 6-Aminopyridin-3-yl, 6-(Piperazin-1-yl)pyridin-3-yl, 6-(4-tert.-Butoxy-carbonylpiperazin-1-yl)pyridin-3-yl, 2-(Piperazin-1-yl)pyridin-4-yl oder 2-(4-tert.-Butoxycarbonyl-piperazin-1-yl)pyridin-4-yl steht und
R⁵ für Wasserstoff steht;
oder ein Tautomer davon oder ein N-Oxid davon oder ein pharmazeutisch unbedenkliches Salz oder ein Hydrat oder Solvat davon.

9. Verbindung der Formel I nach Anspruch 1, ausgewählt aus der Gruppe betstehend aus den folgenden Verbindungen:
6-(3,4-Dimethoxyphenyl)-4-(4-ethoxy-3-methoxyphenyl)chinazolin;
6-(3,4-Dimethoxyphenyl)-4-[3-methoxy-4-(2-methoxy-ethoxy)phenyl]chinazolin;
4-(3,4-Dimethoxyphenyl)-6-(2-methoxypyridin-4-yl)-chinazolin;
6-(3,4-Dimethoxyphenyl)-4-(2-methoxypyridin-4-yl)-chinazolin;
(3-{4-[4-(3,4-Dimethoxyphenyl)chinazolin-6-yl]-2-methoxyphenoxy}propyl)carbamidsäureure-tert.-butyl-ester;
(3-{4-[6-(3,4-Dimethoxyphenyl)chinazolin-4-yl]-2-methoxyphenoxy}propyl)carbamidsäure-tert.-butylester;
(2-{4-[4-(3,4-Dimethoxyphenyl)chinazolin-6-yl]-2-methoxyphenoxy}ethyl)carbamidsäure-tert.-butylester;
(2-{4-[6-(3,4-Dimethoxyphenyl)chinazolin-4-yl]-2-methoxyphenoxy}ethyl)carbamidsäure-tert.-butylester;
6-(3,4-Dimethoxyphenyl)-4-(3-ethoxyphenyl)-chinazolin;
4-(3-Chlorphenyl)-6-(3,4-dimethoxyphenyl)-chinazolin;
4-(3-Benzyloxy-4-methoxyphenyl)-6-(3,4-dimethoxy-phenyl)chinazolin;
4-[6-(3,4-Dimethoxyphenyl)chinazolin-4-yl]benzoesäuremethylester;
4-(3,4-Dimethoxyphenyl)-6-(5-methoxypyridin-3-yl)-chinazolin
4-[4-(3,4-Dimethoxyphenyl)chinazolin-6-yl]-2-methoxybenzoesäuremethylester;
4-(3,4-Dimethoxyphenyl)-6-chinolin-3-ylchinazolin;
4-[6-(5-Methoxypyridin-3-yl)chinazolin-4-yl]benz-amid;
4-[6-(6-Amino-5-trifluormethyl-pyridin-3-yl)-chinazolin-4-yl]benzamid;
5-[4-(3,4-Dimethoxyphenyl)chinazolin-6-yl]-3-trifluormethylpyridin-2-ylamin;
3-[4-(3,4-Dimethoxyphenyl)chinazolin-6-yl]benzolsulfonamid;
4-Benzo[1,3]dioxol-5-yl-6-(3-fluor-4-methoxyphenyl)chinazolin;
4-(3-Benzyloxy-4-methoxyphenyl)-6-(3,4-dimethoxy-phenyl)chinazolin;
3-[4-(3,4-Dimethoxyphenyl)chinazolin-6-yl]benzamid;
4-(4-Chlorphenyl)-6-(3,4-dimethoxyphenyl)-chinazolin;
6-(3,4-Dimethoxyphenyl)-4-(4-trifluormethylphenyl)chinazolin;
6-(3-Chlor-4-methoxyphenyl)-4-(3,4-dimethoxyphenyl)chinazolin;
6-(3,4-Dimethoxyphenyl)-4-thiophen-3-ylchinazolin;
4-(3,4-Dimethoxyphenyl)-6-(1H-pyrrolo[2,3-b]-pyridin-5-yl)chinazolin;
4-[6-(1H-Pyrrolo[2,3-b]pyridin-5-yl)chinazolin-4-yl]benzamid;
4-[6-(6-Aminopyridin-3-yl)chinazolin-4-yl]benz-amid;
6-(3-Benzyloxy-4-methoxyphenyl)-4-(3,4-dimethoxyphenyl)chinazolin:
4-(4-Chlorphenyl)-6-(3-fluor-4-methoxyphenyl)-chinazolin;
4-[6-(4-Ethoxy-3-methoxyphenyl)chinazolin-4-yl]-benzamid;
4-[6-(3,4-Diethoxyphenyl)chinazolin-4-yl]benzamid;
4-(3,4-Dimethoxyphenyl)-6-furan-3-ylchinazolin;
4-[6-(3,4-Dimethoxyphenyl)chinazolin-4-yl]benzo-nitril;
4-[6-(6-Aminopyridin-3-yl)chinazolin-4-yl]benzonitril;
4-[4-(3,4-Dimethoxyphenyl)chinazolin-6-yl]benz-aldehyd;
4-Biphenyl-4-yl-6-(3,4-dimethoxyphenyl)chinazolin;
4-(3,4-Diethoxyphenyl)-6-(3,4-dimethoxyphenyl)-chinazolin;
6-(3,4-Dimethoxyphenyl)-4-methoxy-3-trifluor-methoxyphenyl)chinazolin;
4-(3,4-Dimethoxyphenyl)-6-(4-methoxy-3-trifluor-methoxyphenyl)chinazolin;
5-[4-(3,4-Dimethoxyphenyl)chinazolin-6-]pyridin-2-carbonitril:
4-(4-Bromphenyl)-6-(3,4-dimethoxyphenyl)-chinazolin;
6-(3,4-Diethoxyphenyl)-4-(3,4-dimethoxyphenyl)-chinazolin;
6-(3,4-Dimethoxyphenyl)-4-(4-trifluormethoxyphenyl)chinazolin;
6-(3,4-Dimethoxyphenyl)-4-(4-fluorphenyl)-chinazolin;
6-(3,4-Dimethoxyphenyl)-4-(3-fluorphenyl)-chinazolin;
6-(3,4-Dimethoxyphenyl)-4-(4-methansulfonyl-phenyl)chinazolin;
N-{4-[6-(3,4-Dimethoxyphenyl)chinazolin-4-yl]-phenyl}acetamid;
5-[4-(3,4-Dimethoxyphenyl)chinazolin-6-yl]-nicotinonitrile;
{5-[4-(3,4-Dimethoxyphenyl)chinazolin-6-yl]-pyridin-2-yl}isobutylamin;
6-(3,4-Dimethoxyphenyl)-4-(6-morpholin-4-yl-pyridin-3-yl)chinazolin;
{5-[4-(3,4-Dimethoxyphenyl)chinazolin-6-yl]-pyridin-2-yl}dimethylamin;
{5-[4-(3,4-Dimethoxyphenyl)chinazolin-6-yl]-pyridin-2-yl}methylamin;
2-{5-[4-(3,4-Dimethoxyphenyl)chinazolin-6-yl]-pyridin-2-ylamino}ethanol;
4-{5-[6-(3,4-Dimethoxyphenyl)chinazolin-4-yl]-pyridin-2-yl}piperazin-1-carbonsäure-tert.-butylester;
4-(2,3-Dihydrobenzo[1,4]dioxin-6-yl)-6-(3,4-dimethoxyphenyl)chinazolin-2-ylamin;
6-(6-Aminopyridin-3-yl)-4-(3,4-dimethoxyphenyl)-chinazolin-2-ylamin;
4-(3,4-Dimethoxyphenyl)-6-chinolin-3-ylchinazolin-2-ylamin;
4-[6-(3,4-Dimethoxyphenyl)chinazolin-4-yl]-N,N-dimethylbenzamid;
4-[6-(3,4-Dimethoxyphenyl)chinazolin-4-yl]-N-methylbenzamid;
{4-[6-(3,4-Dimethoxyphenyl)chinazolin-4-yl]-phenyl}(4-methylpiperazin-1-yl)methanon;
{4-[6-(3,4-Dimethoxyphenyl)chinazolin-4-yl]-phenyl}morpholin-4-ylmethanon;
C-{4'-[6-(3,4-Dimethoxyphenyl)chinazolin-4-yl]-biphenyl-4-yl}methylamin;
6-(3,4-Dimethoxyphenyl)-4-(4'-methoxybiphenyl-4-yl)chinazolin;
{4'-[6-(3,4-Dimethoxyphenyl)chinazolin-4-yl]-biphenyl-4-yl}methanol;
4'-[6-(3,4-Dimethoxyphenyl)chinazolin-4-yl]-biphenyl-4-ol;
4-(3',4'-Dimethoxybiphenyl-4-yl)-6-(3,4-dimethoxyphenyl)chinazolin;
6-(3,4-Dimethoxyphenyl)-4-(3',4',5'-trimethoxy-biphenyl-4-yl)chinazolin;
4'-[6-(3,4-Dimethoxyphenyl)chinazolin-4-yl]-biphenyl-4-carbonsäureamid;
6-(3,4-Dimethoxyphenyl)-4-(4'-methoxymethyl-biphenyl-4-yl)chinazolin;
3-{4-[4-(3,4-Dimethoxyphenyl)chinazolin-6-yl]-2-methoxyphenoxy}propylamin;
2-{4-[4-(3,4-Dimethoxyphenyl)chinazolin-6-yl]-2-methoxyphenoxy}ethylamin;
3-{5-[4-(3,4-Dimethoxyphenyl)chinazolin-6-yl]-2-methoxy-phenoxy}propylamin;
2-{5-[4-(3,4-Dimethoxyphenyl)chimazolin-6-yl]-2-methoxyphenoxy}ethylamin;
4-(3,4-Dimethoxyphenyl)-6-(3-ethoxy-4-methoxy-phenyl)chinazolin,
4-[4-(3,4-Dimethoxyphenyl)chinazolin-6-yl]-2-methoxy-benzamid,
4-[4-(3,4-dimethoxyphenyl)chinazolin-6-yl]-2-methoxybenzoesäure;
5-[4-(3,4-Dimethoxyphenyl)chinazolin-6-yl]pyridin-2-carbonsäureamid;
c-{5-[4-(3,-4-Dimethoxyphenyl)chinazolin-6-yl]-pyridin-2-yl}methylamin:
6-(3,4-Dimethoxyphenyl)-4-[6-(4-methansulfonyl-piperazin-1-yl)pyridin-3-yl]chinazolin;
1-(4-{5-[6-(3,4-dimethoxyphenyl)chinazolin-4-yl]-pyridin-2-yl}-piperazin-1-yl)ethanon;
1-{4-[6-(3,4-Dimethoxyphenyl)chinazolin-4-yl]-phenyl}pyrrolidin-2-on;
1-{4-[6-(3,4'-Dimethoxyphenyl)chinazolin-4-yl]-phenyl}azetidin-2-on;
1-{4-[6-(3,4-Dimethoxyphenyl)chinazolin-4-yl] phenyl}piperidin-2-on;
3-{4-[6(3,4"Dimethoxyphenyl)chinazolin-4-yl]-phenyl}oxazolidin-2-one
1-{4-[6-(3,4-Dimethoxyphenyl)chinazolin-4-yl]-phenyl}-3-methylimidazolidin-2-on;
4,6-Bis-(3,4-dimethoxyphenyl)-5-fluorchinazolin;
4-[6-(3,4-Dimethoxyphenyl)-5-fluorchinszolin-4-yl]benzamid;
{5-[4-(3,4-Dimethoxyphenyl)chinazolin-6-yl]-pyridin-2-yl}-(2-methoxyethyl)amin und
4-(3,4-Dimethoxyphenyl)-6-(4,4,5,5-tetramethyl-[1,3,2]dioxaborolan-2-yl)chinazolin;
oder ein Tautomer davon oder ein N-Oxid davon oder ein pharmazeutisch unbedenkliches Salz oder ein Hydrat oder Solvat davon.

10. Verbindung der Formel I, ein N-Oxid davon, ein Tautomer davon und/oder ein pharmazeutisch unbedenkliches Salz davon nach einem der Ansprüche 1 bis 9 zur Verwendung bei der Behandlung, einschließlich der prophylaktischen Behandlung, eines warmblütern, insbesondere eines Menschen.

11. Verbindung der Formel I, ein N-Oxid davon, ein Tautonier davon und/oder ein pharmazeutisch unbedenkliches Salz davon nach Anspruch. 10, wobei es sich bei der Verwendung um die Verwendung gegen eine oder mehrere Erkrankungen aus der Gruppe bestehend aus proliferative, entzündlichen Erkrankungen, allergischen Erkrankungen, obstruktiven Ateinwecjserkrankungen und Störungen, die gemeinhin in Verbindung mit Transplantation auftreten, insbesondere eine oder mehrere Erkrankungen, die auf eine Inhibierung der Aktivität von Kinasen der PIKK-Familie (PIKK = PI3-kinase-related protein kinase), insbesondere Lipidkinasen und/oder PI3-Kinase (PI3K) und/oder mTOR und/oder DNA-Proteinkinase und/oder AM und/oder ATR und/oder hSY;.C,-1, ansprechen, handelt.

12. Pharmazeutische Zubereitung, umfassend eine Verbindung der Formel I, J ein N-Oxid davon, ein Tautomer davon und/oder ein pharmazeutisch unbedenkliches Salz davon nach einem der Ansprüche 1. bis 9 und mindestens einen pharmazeutisch unbedenklichen Träger.

13. Methode oder Verfahren zur Herstellung einer pharmazeutischen Zubereitung, bei derhzw, dem man eine Verbindung der Formel I, ein N-Oxid davon, ein Tautomer davon und/oder ein pharmazeutisch unbedenkliches Salz davon mach der Ansprüche 1 bis 9 mit mindestens einem pharmazeutisch unbedenklichen Träger mischt.

14. Verfahren zur Herstellung einer Verbindung der Formel 1 nach einem der Ansprüche 1 bis 9, bei dem man
a) zur Herstellung einer Verbindung der Formel I, worin R⁴ über ein Kohlenstoffatom an die zentrale Chinazolineinheit in Formel I gebunden ist, eine Verbindung der Formel IIA, 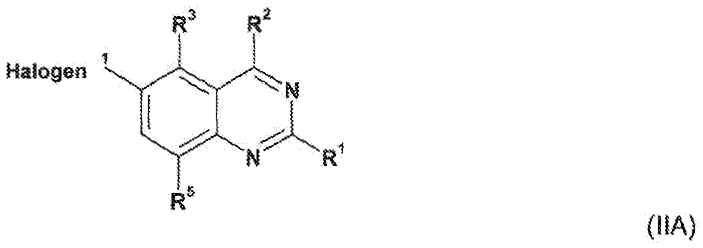 worin R¹ R², R³ und R⁵ die für eine Verbindung der Formel I angegebene Bedeutung besitzen und Halogen¹ für Halogen, vorzugsweise Chlor, Brom oder Iod, oder Trifluormethansulfonyloxy steht, unter Kreuskupplungsbedingun gen mit einer Boronsäure oder einem Boronsäureester der Formel III,
R⁴-D (III)
worin R⁴ die für eine Verbindung der Formel 1 angegebene Bedeutung besitzt und über ein Kohlenstoffatom an D gebunden ist und D für -B(OH)-, i oder eine Gruppe der Formel in, 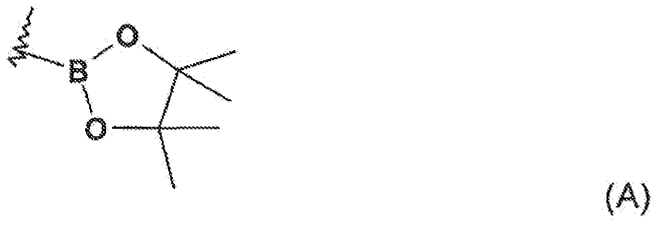 steht, umsetzt;
oder
b) zur Herstellung einer Verbindung- der Formel, worin R² über ein Kohlenstoffatom an die zentrale Chinazolineinheit in Formel 1 gebunden ist, eine Verbindung der Formel IIB, 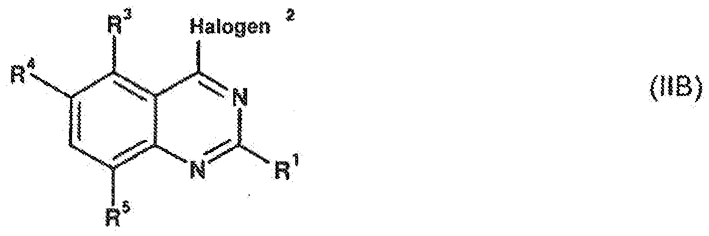 worin R¹ R³, R⁴ und R⁵ die für eine Verbindung der Formel I angegebene Bedeutung besitzen und Halogen² für Halogen, vorzugsweise Chlor, Brom oder lod, oder Trifluormethansulfonyloxy steht, unter Kreuzkupplungsbedingungen mit einer Boronsäure oder einem Boronsäureester der Formel IV,
R²-D (IV)
worin R² die für eine Verbindung der Formel I angegebene Bedeutung besitzt und über ein Kohlenstoffatom an D gebunden ist und D für 2 oder eine Gruppe der oben angegebenen Formel A steht, umsetzt;
oder
c) zur Herstellung einer Verbindung der Formel I, worin R² und. R⁴ identisch sind und über ein Kohlenstoffatom an die zentrale Chinazolineinheit in Formel I gebunden sind, eine Verbindung der Formel IIC, 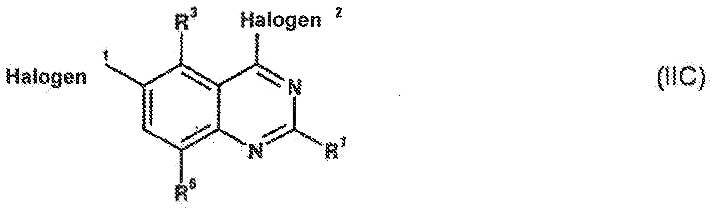 worin R¹ R³ und R⁵ die für eine Verbindung der Formel I angegebene Bedeutung besitzen und Halogen¹ und Halogen² unabhängig voneinander für Halogen, vorzugsweise Chlor, Brom oder Iod, oder Trifluormethansulfonyloxy stehen, mit einer Boronsäure oder einem Boronsäureester der Formel V,
R^{2,4}-D (V)
worin R^{2,1} für eine Einheit R² oder R⁴ steht, die über ein Kohlenstoffatom an D gebunden ist und ansonsten die für eine Verbindung der Formel I angegebene Bedeutung besitzt, und D für B(OH)₂ oder eine Gruppe der oben angegebenen Formel A steht, umsetzt;
oder
d) zur Herstellung einer Verbindung der Formel I, worin R¹ für Amino, N-Mono-C₁-C₃₀-alkylamino oder N-Mono-C₃-C₁₀-cycloalkylamino steht, eine Verbindung der Formel IID, 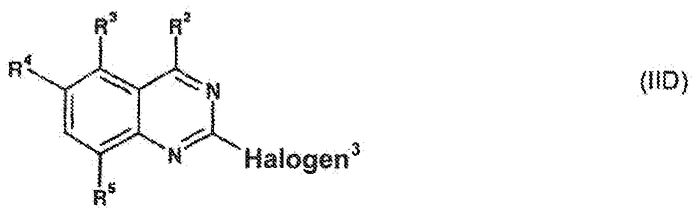 worin R², R³, R⁴ und R⁵ die für eine Verbindung der Formel I angegebene Bedeutung besitzen und Halogen³ für Halogen, vorzugsweise Chlor, Brom oder Iod, oder Trifluormethansulfonyloxy steht, mit einem Amin der Formel VI,
R¹⁺-H (VI)
worin R^{1*} für amino N-Mono-C₃-C₁₀-alkylamino oder N-Mono-C₃-C₁₀-cycloalkylamino steht, umsetzt;
oder
e) zur Herstellung einer Verbindung der Formel I, worin R⁴ für Heteroaryl mit mindestens einem Ringstickstoff steht und über ein Stickstoffatom an die zentrale Chinazolineinheit in Formel I gebunden ist, eine Verbindung der oben unter a) angegebenen Formel IIA unter Substitutionsbedingungen mit einer Verbindung der Formel VII,
R^{4*}-H (VII)
worin R^{4*} für ein stickstoffhaltiges Heteroaryl mit mindestens einem Ringstockstoff, das über ein Stickstoffatom, an den Wasserstoff in Formel VII gebunden ist, steht, umsetzt;
oder
f) zur Herstellung einer Verbindung der Formel I, worin R² für Heteroaryl mit mindestens einem Ringstickstoff steht und über ein Stickstoffatom an die zentrale Chinazolineinheit in Formel I gebunden ist, eine Verbindung der oben unter b) angegebenen Formel IIB unter Substitiationsbedingungen mit einer Verbindung der Formel VIII,
R^{2*}-H (VIII)
worin R^{2*} für ein stickstoffhaltiges Heteroaryl mit mindestens einem Ringstickstoff, das über ein Stickstoffatom an den Wasserstoff in Formel VIII gebunden ist, steht, umsetzt;
oder
g) zur Herstellung einer Verbindung der Formel I, worin R² und R⁴ identisch sind und für Heteroaryl mit mindestens einem Ringstickstoff stehen und jeweils über ein Stickstoffatom an die zentrale Chinazolineinheit in Formel I gebunden ist, eine Verbindung der Formel IX,
R^{2,4*} -H (IX)
worin R^{2,4*} für Heteroaryl mit mindestens einem Stickstoffatom steht und R^{2,4*} für eine Gruppierung R² oder R⁴ steht, die über ein Stickstoffatom an den in Formel IX gezeigten Wasserstoff gebunden ist und ansonsten die für eine Verbindung der Formel 1 angegebene Bedeutung beisitzt, unter Substitutionsbedingungen mit einer Verbindung der oben erwähnten Formel IIC umsetzt;
oder
h) zur Herstellung einer Verbindung der Formel I, worin R⁴ über ein Kohlenstoffatom an die zentrale Chinoazolineinheit in Formel I gebunden ist, eine BOronsaure- oder Boronsaureesterverbindung der Formel IIA*, 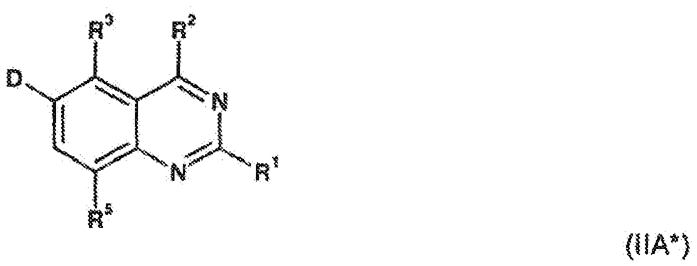 worin R¹, R², R³ und R⁵ die für eine Verbindung der Formel I angegebene Bedeutung besitzen und D für -B(OH)₂ oder eine Gruppe der Formel A_{,} 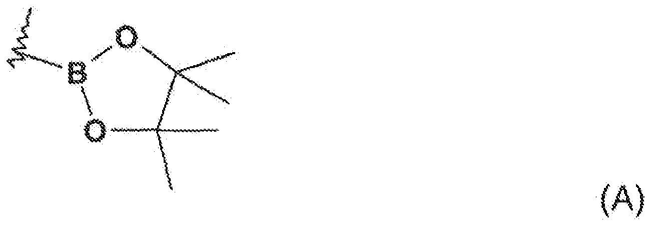
steht, unter Kreuzkupplungsbedingungen mit einer Verbindung der Formel III*,
R⁴-Hal (III*)
worin R⁴ die für eine Verbindung der Formel I angegebene Bedeutung besitzt und über ein Kohlenstoffatom au Hal gebunden ist und Hal für Halogen, vorzugsweise Chlor, Brom oder Tod, oder Trifluormethansulfonyloxy steht, umsetzt;
wobei bei allen unter a) bis h) vorgestellten Umsetzungen funktionelle Gruppen in den Ausgangsstoffen in geschützter Form vorliegen können und in den erhältlichen Verbindungen der Formel I, die eine oder mehrere Schutzgruppen tragen, derartige Schutzgruppen abgespalten werden;
und gegebenenfalls eine gemäß einer unter a) bis g) ausgewählten Verfahrensvariante erhältlich Verbindung der Formel I in eine andere Verbindung der Formel I umwandelt, ein erhältliches Salz einer Verbindung der Formel I in ein anderes Salz davon umwandelt, eine erhältliche freie Verbindung der Formel I in ein Salz davon umwandelt und/oder ein erhältliches isomer einer Verbindung der Formel I von einem oder mehreren anderen erhältlichen Isomeren der Formel I trennt.

15. verwendung einer Verbindung der Formel I, eines N-Oxids davon, eines Tautomers davon und/oder eines pharmazeutisch unhedenklichen Salzes davon nach einem der Ansprüche 1 bis 9 zur Herstellung einer pharmazeutischen zubereitung zur Behandlung einer Erkrankung aus der Gruppe bestehend aus proliferativen, entzündlichen Erkrankungen, allergischen Erkrankungen, obstruktiven Atemwegserkrankungen und Störungen, die gemeinhin in Verbindung mit Transplantation auftreten, insbesondere eine oder mehrere Erkrankungen, die auf eine Inhibierung der Aktivität von Kinasen der PIKK-Familie (PIKK = PI3-kinase-related protein kinase), insbesondere Lipidkinasen und/oder PI3-Kinase (PI3K) und/oder mTOR und/oder DNA-Proteinkinase und/oder ATM und/oder ATR und/oder hSMG-1, ansprechen.

16. Kombination einer Verbindung der Formel I nach einem der Ansprüche 1 bis 9 mit einem oder mehreren anderen Therapeutika.

## Revendications

1. Composé de formule I : 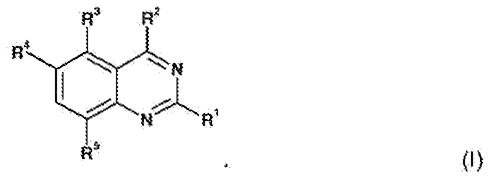 dans laquelle
R¹ est hydrogène ; ou amino qui est non substitué ou monosubstitué par alkyle ou cycloalkyle ;
R² est aryle non substitué ou substitué ou hétéroaryle non substitué ou substitué ;
R³ est hydrogène, halogène, alkyle, alcoxy ou cyano ;
R⁴ est acyle non substitué ou substitué ou hétéroaryle non substitué ou substitué ; et
R⁵ est hydrogène, méthyle ou méthyle substitué par halogène ;
à condition que si R⁴ est pyrazolyle non substitué ou substitué alors R¹ est amino qui est non substitué ou monosubstitué par alkyle ou cycloalkyl et R², R³ et R⁵ sont tels que définis ci-dessus ;
et à condition, que si R² est oxoindolyle non substitué ou substitué, alors R¹ est amino qui est non substitué ou monosubstitué par alkyle ou cycloalkyle et R³, R⁴ et sont tels que définis ci-dessus ;
ou un tautomère de celui-ci ou un N-oxyde de celui-ci, ou un sel, ou un hydrate ou solvat de celui-ci.

2. Composé de formule I selon la revendication 1, dans laquelle
R¹ est hydrogène ; ou amino qui est non substitué ou monosubstitué par C₃-C₇ (préférablement C₁-C₄) alkyle ou C₃-C₈ (préférablement C₃-C₅) Cycloalkyle ;
R² est aryle non substitué ou substitué, où aryle est choisi dans le groupe constitué par phényle, naphtyle, biphenylényle, indacényle, acénaphtylényle, fluorényle, phénalényle, phénanthrényle et anthracényle, chacun d'entre eux étant non substitue ou substitué par un ou plusieurs, préférablement jusqu'à trois, substituant indépendants choisis dans le groupe constitué par C₁-C₇-alkyle ; C₂-C₇-alcérlyle ; C₂-C₇-alcynyle ; C₆-C₁₈-aryl -C₁-C₇-alkyle où aryle est préférablement phényle, naphtyle, biphenylényle, indacényle, acénaphtylényle, fluorényle, phénalényle, phénanthrényle ou anthracényle et est non substitué ou substitué par C₃-C₇-alkyle, tel que méthyle ou éthyle, par pyrrolidinyle, notamment pyrrolidino, par pipérazinyle, notamment pipérazino, par amino, par N-mono- et/ou N,N-di-C₁-C₇-alkylamino, par halogèno, par C₁-C₇-alcoxy, tel que méthoxy, et/ou par halogéno-C₁-C₇-alkyle, tel que trifluorométhyle (pyrrolidinyle (notamment pyrrolidino), pipéridinyle (notamment pipéridino), pipérazinyle (notamment piperazino), morpholino, thiomorpholino, pyridinyle, pyrimidinyle, pyrazinyle ou pyridazinyl)-C₁-C₇-alkyle où pyrrolidinyle, pipéridinyle, pipérazinyle, pyridinyle, pyrimidinyle, pyrazinyle ou pyridazinyle sont non substitués ou substitués par C₃-C₇-alkyle, tel que méthyle ou éthyle, par pyrrolidinyle, notamment pyrrolidino, par pipérazinyle, notamment pipérazino, par amino, par N-mono- et/ou N,N-di-C₁-C₇-alkylamino, par halogéno, par C₁-C₇-alcoxy, tel que méthoxy, et/ou par halogéno-C₁-C₇-alkyle, tel que trifluorométhyle, par exemple pyrrolidino-C₁-C₇-alkyle, pipéridino-C₁-C₇-alkyle, morpholino-C₁-C₇-alkyle, thiomorpholino-C₁-C₇-alkyl, N-C₁-C₇-alkyl-pipérazino-C₁-C₇-alkyle, ou N-mono-ou N,N-di-(C₁-C₇-alkyl)-amino-substitué ou non substitué pyrrolidino-C₁-C₇-alkyle; (pyrrolidinyle (notamment
pyrrolidino), pipéridinyle (notamment pipéridino), pipérazinyle (notamment pipérazino), pyridinyle, pyrimidinyle, pyrazinyle ou pyridazinyl) -oxy-C₁-C₇-alkyl où pyrrolidinyle, pipéridinyle, pipérazinyle, pyridinyle, pyrimidinyle, pyrazinyle ou pyridazinyle sont non substitués ou substitués par C₁-C₇-alkyle, tel que méthyle ou éthyle, par pyrrolidinyle, notamment pyrrolidino, par pipérazinyle, notamment pipérazino, par amino, par N-mono- et/ou N,N-di-C₁-C₇-alkylamino, par halogéno, par C₁-C₇-alcoxy, tel que méthoxy, et/ou par halogéno-C₁-C₇-alkyle, tel que trifluorométhyle ; (pyrrolidinyle (notamment pyrrolidino), pipéridinyle (notamment pipéridino), pipérazinyle (notamment piperazino), pyridinyle, pyrimidinyle, pyrazinyle ou pyridazinyl) -carbonyl-C₁-C₇-alkyle où pyrrolidinyle, pipéridinyle, pipérazinyle, pyridinyle, pyrimidinyle, pyrazinyle ou pyridazinyl sont non substitués ou substitués par C₁-C₇-alkyle, tel que méthyle ou éthyle, par pyrrolidinyle, notamment pyrrolidino, par pipérazinyle, notamment pipérazino, par amino, par N-mono- et/ou N,N-di-C₁-C₇-alkylamino, par halogène, par C₁-C₇-alcoxy, tel que méthoxy, et/ou par hallogéno-C₁-C₇-alkyle, tel que trifluorométhyle ; halogéno-C₁-C₇-alkyle ; hydroxy-C₁-C₇-alkyle ; C₁-C₇-alcoxy-C₁-C₇-alkyle ; C₁-C₇-alcoxy-C₁-C₇-alcoxy-alkyle ; phényloxy- ou naphtyloxy-C₁-C₇-alkyle ; phényl-C₁-C₇-alcoxy- ou naphtyl-C₁-C₇-alcoxy-C₁-C₇-alkyle ; amino-C₁-C₇-alkyle ; N-mono- ou N,N-di-C₁-C₇-alkyle et/ou mono-C₁-C₇-alcoxy-C₁-C₇-alkyle et/ou (mono- ou di-(C₁-C₇-alkyl)-amino-C₁-C₇-ralkyl)-amino-C₁-C₇-alkyle ; C₁-C₇-alcoxy-C₁-C₇-alkylamino-C₁-C₇-alkyle ; mono- ou di-[C₆-C₁₈-aryl-C₁-C₇-alkyle où aryle est non substitué ou substitué par C₁-C₇-alkyle, par pyrrolidinyle, par pipérazinyle, par amino, par N-mono- et/ou N,N-di-C₁-C₇-alkylamino, par halogène, par C₁-C₇-alcoxy et/ou par halogéno-C₁-C₇-alkyle ; (pyrrolidinyle, pipéridinyle, pipérazinyle, morpholino, thiomorpholino, pyridinyle, pyrimidinyle, pyrazinyle ou pyridazinyl)-C₁-C₇-alkyle où pyrrolidinyl, pipéridinyle, pipérazinyle, pyridinyle,
pyrimidinyle, pyrazinyle ou pyridazinyle sont non substitués ou substitués par C₁-C₇-alkyle, par pyrrolidinyle, par pipérazinyle, par amino, par N-mono-et/ou N,N-di-C₁-C₇-alkylamino, par halogène, par C₁-C₇-alcoxy et/ou par halogéno-C₁-C₇-alkyle ; (pyrrolidinyle, pipéridinyle, pipérazinyle, pyridinyle, pyrimidinyle, pyrazinyle ou pyridazinyl)-oxy-C₁-C₇-alkyle où pyrrolidinyle, pipéridinyle, pipérazinyle, pyridinyle, pyrimidinyle, pyrazinyle ou pyridazinyle sont non substitués ou substitués par C₁-C₇-alkyle, par pyrrolidinyle, par pipérazinyle, par amino, par N-mono-et/ou N,N-di-C₁-C₇-alkylamino, par halogéno, par C₁-C₇-alcoxy et/ou par halogéno-C₁-C₇-alkyle ; et/ou (pyrrolidinyle, pipéridinyle, pipérazinyle, pyridinyle, pyrimidinyle, pyrazinyle ou pyridazinyl) -carbonyl-C₁-C₇-alkyle où pyrrolidinyle, pipéridinyle, pipérazinyle, pyridinyle, pyrimidinyle, pyrazinyle ou pyridazinyl sont non substitués ou substitués par C₁-C₇-alkyle, par pyrrolidinyle, par piperazinyl, par amino, par N-mono-et/ou N,N-di-C₁-C₇-alkylamino, par halogène, par C₁-C₇-alcoxy et/ou par halogéno-C₁-C₇-alkyle ; notamment naphtyl- et/ou phényl-C₁-C₇-alkyl]-amino-C₁-C₇-alkyle ; C₁-C₇-alcanoylamino-C₁-C₇-alkyle ; carboxy-C₁-C₇-alkyle ; benzoyl- ou naphtoylaminom-C₁-C₇-alkyle ; C₁-C₇-alkylsulfonylamino-C₁-C₇-alkyle ; phényl- ou naphtylsulfonylamino-C₁-C₇-alkyle où phényle ou naphtyle est non substitué ou substitué par un ou plusieurs, notamment de un à trois, motifs C₁-C₇-alkyle, phényl- ou naphtyl-C₁-C₇-alkylsulfonylamino-C₁-C₇-alkyle, halogéno ; hydroxy; C₁-C₇-alcoxy ; C₆-C₁₈-aryl-C₁-C₇-alkyle où aryle est préférablement phényle, naphtyle, biphenylenyle, indacényle, acénaphtylényle, fluorényle, phénalényle, phénanthrényle ou anthracényle et non substitué ou substitué par C₁-C₇-alkyle, tel que méthyle ou éthyle, par C₁-C₇-alcoxy par pyrrolidinyle, notamment pyrrolidino, par pipérazinyle, notamment pipérazino, par amino, par N-mono- et/ou N,N-di-C₁-C₇-alkylamino, par halogéno, par C₁-C₇-alcoxy, tel que méthoxy, et/ou par halogéno-C₁-C₇-alkyle, tel que trifluorométhyle ;
halogéno-C₁-C₇-alcoxy ; hydroxy-C₁-C₇-alcoxy ; C₁-C₇-alcoxy-C₁-C₇-alcoxy ; amino-C₁-C₇-alcoxy ; N-C₁-C₇-alcanoylamino-C₁-C₇-alcoxy ; N-non substitué-, N-mono-ou N,N-di-(C₁-C₇-alkyl)carbamoyl-C₁-C₇-alcoxy ; phényl-ou naphtyloxy ; phényl- ou naphtyl-C₁-C₇-alkyloxy ; (pyrrolidinyle, pipéridinyle, pipérazinyle, pyridinyle, pyrimidinyle, pyrazinyle ou pyridazinyl)-C₁-C₇-alcoxy où pyrrolidinyle, pipéridinyle, pipérazinyle, pyridinyle, pyrimidinyle, pyrazinyle ou pyridazinyle sont non substitués ou substitués par C₁-C₇-alkyle, par pyrrolidinyle, par pipérazinyle, par amino, par N-mono-et/ou N,N-di-C₁-C₇-alkylamino, par halogéno, par C₁-C₇-alcoxy et/ou par halogéno-C₁-C₇-alkyle ; (pyrrolidinyle, pipéridinyle, pipérazinyle, pyridinyle, pyrimidinyle, pyrazinyle ou pyridazinyl)-oxy-C₁-C₇-alcoxy où pyrrolidinyle, pipéridinyle, pipérazinyle, pyridinyle, pyrimidinyle, pyrazinyle ou pyridazinyl sont non substitués ou substitués par C₁-C₇-alkyle, par pyrrolidinyle, par pipérazinyle, par amino, par N-mono-et/ou N,N-di-C₁-C₇-alkylamino, par halogéno, par C₁-C₇-alcoxy et/ou par halogéno-C₁-C₇-alkyle ; C₁-C₇-alcanoyloxy ; benzoyl- ou naphtoyloxy ; C₁-C₇-alkylthio, halogéno-C₁-C₇-alkylthio ; C₁-C₇-alcoxy-C₁-C₇-alkylthio ; phényl- ou naphtylthio ; phényl- ou naphtyl-C₁-C₇-alkylthio ; C₁-C₇-alcanoylthio ; benzoyl- ou naphtaylthio ; nitro ; amino ; mono- ou di-(C₁-C₇-alkyl)-amino ; mono- ou di-(naphtyl- ou phényl-C₁-C₇-alkyl)-amino ; C₁-C₇-alcanoylamino ; benzoyl- , ou naphtoylamino ; C₁-C₇-alkylsulfonylamino ; phényl- ou naphtylsulfonylamino où phényle ou naphtyle est non substitué ou substitué par un ou plusieurs, notamment de un à trois, motifs C₁-C₇-alkyle ; phényl- ou naphtyl-C₁-C₇-alkylsulfonylamino ; C₁-C₇-alcanoyle ; C₁-C₇-alcoxy-C₁-C₇-alcanoyle ; carboxyle ; C₁-C₇-alcoxy-carbonyle ; phénoxy- ou naphtoxycarbonyle ; phényl- ou naphtyl-C₁-C₇-alcoxycarbonyle : C₁-C₁₀-, notamment C₁-C₄-alcylènedioxy ; carbamoyle ; N-mono- ou N,N-di-(C₁-C₇-alkyle, naphtyl-C₁-C₇-alkyle, pyrrolidinyl-C₁-C₇-alkyle, pipéridinyle-C₁-C₆-alkyle, pipérazinyl- ou N-C₁-C₇-alkyl -pipérazinyl-C₁-C₇-alkyle, phényl-C₁-C₇-alkyle, mono-C₁-C₇-alcoxy-C₁-C₇-alkyle et/ou (N'-mono- ou N'N'-di-(C₁-C₇-alkyle) amino-C₁-C₇-alkyle) -aminocarbonyle : N-C₁-C₇-alcoxy-C₁-C₇-alkylcarbamoyle ; pyrrolidin-1-carbonyle ; amino-N-pyrrolidin-1-carbonyle ; N-mono- ou N,N-di -C₁-C₇-alkyle) amino-pyrrolidin-1-carbonyle ; pipéridin-1-carbonyle ; morpholin-4-carbonyle ; thiomorpholin-4-carbonyle ; S-oxo-thiomorpholin-4-carbonyle ; S,S-dioxothiomorpholin-4-carbonyle ; pipérazin-1-carbonyle ; N-C₁-C₇-alkyl-pipérazin-1-carbonyle ; N-C₁-C₇-alcoxycarbonyl-pipérazin-1-carbonyle ; N-mono- ou N,N-di-(C₁-C₇-alkyl)-amino-substitué ou non substitué pyrrolidinyl-C₁-C₇-alkyle ; cyano ; C₁-C₇-alcénylène ou -alcynylène ; C₁-C₇-alkylsulfonyle ; phényl- ou naphtylsulfonyle ou phényle ou naphtyle est non substitué ou substitué par un ou plusieurs, notamment de un à trois, motifs C₁-C₇-alkyle ; phényl- ou naphtyl-C₁-C₇-alkylsulfonyle ; sulfamoyle ; N-mono- ou N,N-di-(C₁-C₇-alkyle, phényl-, naphtyl-, pyrrolidinyl (notamment pyrrolidino)-C₁-C₇)-alkyle, pipéridinyl (notamment pipéridino)-C₁-C₇-alkyle, pipérazinyl (notamment pipérazino)-C₁-C₇-alkyle, N-C₁-C₇-alkylpiperazinyl (notamment 4-C₁-C₇-alkylpipérazino)-C₁-C₇-alkyle, phényl-C₁-C₇)-alkyl- et/ou naphtyl-C₁-C₇-alkyl)-aminosulfonyle ; pyrazolyle ; pyrazolidinyle ; pyrrolyle ; pyridyle que est non substitué ou substitué par C₁-C₇-alcoxy, et/ou par halogéno-C₁-C₇-alkyle, pyrrolidinyle ; pipéridinyle ; morpholinyle ; thiomorpholinyle ; S-oxo-thiomorpholinyle ; S,S-dioxothiomorpholinyle ; pipérazinyle ; N-C₁-C₇-alkyl-piperazinyle ; 4-(phényl-C₁-C₇-alkyl)-pipérazinyle ; 4-(naphtyl-C₁-C₇-alkyl)-pipérazinyle ; 4-(C₁-C₇-alcoxy-carbony)-pipérazinyle ; 4-(phényl-C₁-C₇-alcoxy-carbonyl) -pipérazinyle et 4-(naphtyl-C₁-C₇-alcoxy-carbonyl)-pipérazinyle ;
ou est hétéroaryle non substitué ou substitué où hétéroaryle est choisi dans le groupe constitué par imidazolyle, thiophényle, pyrazolyl, pyrrolyle, imidazolyle, pyridyle, pyrimidinyle, pyridazinyl, furyle, 2H- ou 4H-pyranyle, oxazolyle, thiazolyle, 5H-indazolyle, isoindolyle, quinolyl, isoquinoléinyle, phtalazinyle, 1,8-naphtyridinyle, quinoxalinyle, quinazolinyle, cinnolinyle, indolizinyle, 4H-quinolizinyle, ptéridinyle, purinyle, carbazolyle, bêta-carbolinyle, acridinyle, phénanthridinyle, phényzinyle, 1,7-phenanthrolinyle, périmidinyle, benzofuranyle, isobenzofuranyle, 2H-chroményle, 4aH-isochroményle, thianthrényle, xanthényle, phénoxathiinyle, phénoxazinyle ou phénothiazinyle, chacun d'entre eux étant non substitué ou substitué tel que mentionné ci-dessus pour aryle ;
R³ est hydrogène, halogène, C₁-C₇-alkyle, C₁-C₇-alcoxy ou cyano ;
R⁴ est aryle non substitué ou substitué ou hétéroaryle non substitué ou substitué, choisi indépendamment parmi aryle non substitué ou substitué tel que défini pour R² et hétéroaryle non substitué ou substitué où hétéroaryle est choisi dans le groupe constitué par imidazolyle, thiophényle, pyrrolyle, imidazolyle, pyridyle, pyrimidinyle, pyridazinyle, furyle, 2H- ou 4H-pyranyle, oxazolyle, thiazolyle, 5H-indazolyle, indolyle, isoindolyl, quinolyle, isoquinoléinyle, phtalazinyle, 1,8-naphtyridinyle, quinoxalinyle, quinazolinyle, cinnolinyle, indolizinyle, 4H-quinolizinyle, ptéridinyle, purinyle, carbazolyle, bêta-carbolinyle, acridinyle, phénanthridinyle, phényzinyle, 1, 7-phénanthrolinyle, périmidinyle, benzofuranyle, isobenzofuranyle, 2H-chroményle, 4aH-isochroményle, thianthrényle, xanthényle, phénoxathiinyle, phénoxazinyle ou phénothiazinyle, tel que défini pour R² ; ou, si R¹ est amino ou amino monosubstitué par C₁-C₇(préférablement C₁-C₄)alkyle ou C₃-C₈(préférablement C₃-C₅)cycloalkyle, peut également être pyrazolyle, où chaque hétéroaryle est non substitué ou substitué tel que décrit ci-dessus pour aryle R² ; et
R⁵ est hydrogène, méthyle ou méthyle substitué par halogène ;
ou un tautomère de celui-ci ou un N-oxyde de celui-ci, ou un sel pharmaceutiquement acceptable, ou un hydrate ou solvate de celui-ci.

3. Composé de formule I selon la revendication 1, dans laquelle
R¹ est hydrogène, amino, N-mono-C₁-C₁₀-(préférablement C₁-C₄)alkylamino ou C₃-C₈(préférablement C₃-C₅)cycloalkylamino ;
R² est phényle, naphtyle, pyrrolyle, thiophényle, pyrazolyle, triazolyle, pyridyle, quinolyle ou quinoxalinyle, ou est pyrrolopyridinyle, chacun d'entre eux étant non substitué ou substitué par un ou plusieurs substituants choisis indépendamment dans le groupe constitué par C₁-C₇-alkyle, halogéno-C₁-C₇-alkyle, phényle qui est non substitué ou substitue par de un à trois substituants choisis indépendamment parmi hydroxy-C₁-C₇-alkyle, C₁-C₇-alcoxy-C₁-C₇-alkyle, C₁-C₇-alcoxy, amino et carbamoyle, halogèno, hydroxy, C₁-C₇-alcoxy, C₁-C₇-alcoxy-C₁-C₇-alcoxy, halogéno-C₁-C₇-alcoxy, tel que trifluorométhoxy, amino, N-mono- ou N,N-di-(C₁-C₇-alkyle, phényl-C₁-C₇-alkyle et/ou naphtyl-C₁-C₇-alkyl)-amino, C₁-C₇-alcanoylamino, carboxy, C₁-C₇-alcoxycarbonyle, phényl-C₁-C₇-alcoxycarbonyle, naphtyl-C₁-C₇-alcoxycarbonyle, phénoxycarbonyle, naphtoxycarbonyle, C₁-C₄-alcylènedioxy, cyano, carbamoyle, N-mono- ou N,N-di-(C₁-C₇-alkyle, N',N'-di-(C₁-C₇-alkyle)-amino-C₁-C₇-alkyle, pyrrolidino-C₁-C₇-alkyle et/ou phényl-C₁-C₇-alkyle) -carbamoyle, pipéridin-1-carbonyle, pipérazin- 1-carbonyle, 4-C₁-C₇-alkyl-piperazin-1-carbonyle, morpholin-4-carbonyle, thiomorpholin-4-carbonyle, S-oxo-thiomiorpholin-4-carbonyle, S,S-dioxothiomorpholin-4-carbonyle, sulfamoyle, N-mono- ou N,N-di-(C₁-C₇-alkyle, N',N'-di-(C₁-C₇-alkyl)amino-C₁-C₇-alkyle, pyrrolidino-C₁-C₇-alkyle et/ou phényl-C₁-C₇-alkyl)-sulfamoyle, pyrazolyl, pyrazolidinyle, pyrrolyle, pyrrolidinyle, pipéridinyle, pipérazinyle, 4-C₁-C₇-alkyl-pipérazinyle, 4- (phényl-C₁-C₇-alkyl)-pipérazinyle, 4- (naphtyl-C₁-C₇-alkyl) -pipérazinyle, 4-(C₁-C₇ alcanoyl)-pipérazinyle, 4-(C₁-C₇-alcoxycarbonyl)-pipérazinyle, 4- (phényl-C₁-C₇-alcoxycarbonyl)-pipérazinyle, 4- (naphtyl-C₁-C₇-alcoxycarbonyl) - pipérazinyle, 4-(C₁-C₇-alcanesulfonyl)-pipérazinyle, 2-oxo-pyrrolidin-1-yle, 2-oxo-azétidin-1-yle, 2-oxo-pipéridin-1-yle, 3-C₁-C₇-alkyl-2-oxo-imidazolidin-1-yle, morpholinyle, thiomorpholinyle, S-oxothiomorpholinyle et S,S-dioxothiomorpholinyle, et/ou parmi 2-amino-pyrimidin-5-yl-C₁-C₇-alkyle, 4-C₁-C₇-alkyl-pipérazin-1-carbonyl-C₁-C₇-alcoxy, 4-pyrrolidino-pipéridin-1-carbonyl-C₁-C₇-alcoxy, 4-pyrrolidino-pipéridin-1-yl-C₃-C₇-alcoxy, 4-C₁-C₇-alkyl-pipérazino-C₁-C₇-alcoxy, pyridine (par exemple -2)-yloxy-C₁-C₇-alcoxy, pyrimidine (par exemple -4)-yloxy-C₁-C₇-alcoxy, N,N-di(C₁-C₇-alkyl) amino-pyrrolidin-1-carbonyle et (non substituée ou C₁-C₇-alcoxy- et/ou halogéno-C₁-C₇-alcoxy-substituée) pyridine (par exemple -3))-yle ;
R³ est hydrogène, ou est halogéno, préférablement hydrogène,
R⁴ est phényle, naphtyle, pyrrolyle, thiophényle, triazolyle, pyridyl, quinoléinyle, quinoxalinyle, furanyle ou 1H-pyrrolo[2,3-b]-pyridin-5-yle, chacun d'entre eux étant non substitué ou substitué par un ou plusieurs substituants choisis indépendamment dans le groupe constitué par halogéno-C₁-C₇-alkyle, tel que trifluorométhyle, amino-C₁-C₇-alkyle, tel que aminométhyle, halogène, hydroxy, C₁-C₇-alcoxy, C₁-C₇-alcoxy-C₁-C₇-alcoxy, amino-C₁-C₇-alcoxy, phényl-C₁-C₇-alcoxy, amino, N-mono- ou N,N-di-(C₁-C₇-alkyle, hydroxy, C₁-C₇-alkyle, phényl-C₁-C₇-alkyle et/ou naphtyl-C₁-C₇-alkyl)-amino, C₁-C₇-alcanoyle, carboxy, C₁-C₇-alcoxycarbonyle, phényl-C₁-C₇-alcoxycarbonyle, naphthyl-C₁-C₇-alcoxycarbonyle, phénoxycarbonyle, naphtoxycarbonyle, C₁-C₄-alcylènedioxy, cyano, carbamoyle, N-mono- ou N,N-di-(C₁-C₇-alkyle, N',N'-di-(C₁-C₇-alkyl) amino-C₁-C₇-alkyle, pyrrolidino-C₁-C₇-alkyle et/ou phényl-C₁-C₇-alkyl)-carbamoyle, pipéridin-1-carbonyle, pipérazin-1-carbonyle, 4-C₁-C₇-alkyl-pipérazin-1-carbonyle, morpholin-4-carbonyle, thiomorpholin-4-carbonyle, S-oxo-thiomorpholin-4-carbonyle, S,S-dioxothiomorpholin-4-carbonyle, sulfamoyle, N-mono- ou N,N-di-(C₁-C₇-alkyle, N',N'-di-(C₁-C₇-alkyl)amino-C₁-C₇-alkyle, pyrrolidino-C₁-C₇-alkyle et/ou phényl-C₁-C₇-alkyl)-sulfamoyle, pyrazolyle, pyrazolidinyle, pyrrolyle, pyrrolidinyle, pipéridinyle, pipérazinyle, 4-C₁-C₇-alkyl-pipérazinyle, 4-(phényl-C₁-C₇-alkyl)-pipérazinyle, 4-(naphtyl-C₁-C₇-alkyl)-pipérazinyle, 4-(C₁-C₇-alcoxycarbonyl)-pipérazinyle, 4-(phényl-C₁-C₇-alcoxycarbonyl)-pipérazinyle, 4-(naphtyl-C₁-C₇-alcoxy-carbonyl)-pipérazinyle, morpholinyle, thiomorpholinyle, S-oxothiomorpholinyle et S,S-dioxothiomorpholinyle, et/ou parmi 2-amino-pyrimidin-5-yl-C₁-C₇-alkyle, 4-C₁-C₇-alkyl-pipérazin-1-carbonyl-C₁-C₇-alcoxy, 4-pyrrolidino-pipéridin-1-carbonyl-C₁-C₇-alcoxy, 4-pyrrolidino-pipéridin-1-yl-C₁-C₇-alcoxy, 4-C₁-C₇-alkyl-pipérazino-C₁-C₇-alcoxy, pyridine (par exemple -2)-yloxy-C₁-C₇-alcoxy, pyrimidine (par exemple -4)-yloxy-C₁-C₇-alcoxy, N,N-di (C₁₋C₇-alkyl) amino-pyrrolidin-1-carbonyle et (non substituée ou C₁-C₇-alcoxy- et/ou halogéno-C₁-C₇-alcoxy-substituée) pyridine (par exemple -3))-yle ; et
R⁵ est hydrogène,
ou un tautomère de celui-ci ou un N-oxyde de celui-ci, ou un sel pharmaceutiquement acceptable, ou un hydrate ou solvat de celui-ci.

4. Composé de formule I selon la revendication. 1, dans laquelle
R¹ est hydrogène, amino, N-mono-C₁-C₁₀(prérérablement C₁-C₄) alkylamlno ou C₃-C₈(préférablement C₃-C₅)-cycloalkylamino,
R² est phényle, naphtyle, pyrrolyle, thiophényle, pyrazolyle, triazolyle, pyridyle, quinolyle ou quinoxalinyle, chacun d'entre eux étant non substitué ou substitué par un ou plusieurs substituants choisis indépendamment dans le groupe constitué par halogéne, hydroxy, C₁-C₇-alcoxy, C₁-C₇-alcoxy-C₁-C₇-alcoxy, amino, N-mono- ou N,N-di-(C₁-C₇-alkyle, phényl-C₁-C₇-alkyle et/ou naphtyl-C₁-C₇-alkyl)-amino, carboxy, C₁-C₇-alcoxycarbonyle, phényl-C₁-C₇-alcoxycarbonyle, naphthyl-C₁-C₇-alcoxycarbonyle, phénoxycarbonyle, naphtoxycarbonyle, C₁-C₄-alcylènedioxy, carbamoyle, N-mono- ou N,N-di-(C₁-C₇-alkyle, N',N'-di-(C₁-C₇-alkyl)amino-C₁-C₇-alkyle, pyrrolidino-C₁-C₇-alkyle et/ou phényl-C₁-C₇-alkyl)-carbamoyle, pipéridin-1-carbonyle, pipérazin-1-carbonyle, 4-C₁-C₇-alkyl-pipérazin-1-carbonyle, morpholin-4-carbonyle, thiomorpholin-4-carbonyle, S-oxo-thiomorpholin-4-carbonyle, S,S-dioxothiomorpholin-4-carbonyle, sulfamoyle, N-mono- ou N,N-di-(C₁-C₇-alkyle, N',N'-di-(C₁-C₇-alkyl)amino-C₁-C₇-alkyle, pyrrolidino-C₁-C₇-alkyle et/ou phényl-C₁-C₇-alkyl)-sulfamoyle, pyrazolyl, pyrazolidinyle, pyrrolyle, pyrrolidinyle, pipéridinyle, pipérazinyle, 4-C₁-C₇-alkyl-pipérazinyle, 4-(phényl-C₁-C₇-alkyl)-pipérazinyle, 4-(naphtyl-C₁-C₇-alkyl)-pipérazinyle, 4-(C₁-C₇-alcoxy-carbonyl)-pipérazinyle, 4-(phényl-C₁-C₇-alcoxycarbonyl)-pipérazinyle, 4-(naphtyl-C₁-C₇-alcoxycarbonyl)-pipérazinyle, morpholinyle, thiomorpholinyle, S - oxothiomorpholinyle et S,S-dioxothiomorpholinyle, et/ou parmi 3-amino-pyrimidin-5-yl-C₁-C₇-alkyle, 4-C₁-C₇-alkyl-pipérazin-1-carbonyl-C₁-C₇-alcoxy, 4-pyrrolidino-pipéridin-1-carbonyl-C₁-C₇-alcoxy, 4-pyrrolidino-pipéridin-1-yl-C₁-C₇-alcoxy, 4-C₁-C₇-alkyl-pipérazino-C₁-C₇-alcoxy, pyridine (par exemple -2)-yloxy-C₁-C₇-alcoxy, pyrimidine (par exemple -4)-yloxy-C₁-C₇-alcoxy, N,N-di(C₁-C₇-alkyl)amino-pyrrolidin-1-carbonyle et (non substituée ou C₁-C₇-alcoxy- et/ou halogéno-C₁-C₇alcoxy-substituée) pyridine (par exemple -3)-yle ;
R³ est hydrogène ou halogène, préférablement hydrogène ; R⁴ est phényle, naphtyle, pyrrolyle, thiophényle, triazolyle, pyridyle, quinoléinyle ou quinoxalinyle, chacun d'entre eux étant non substitué ou substitué par un ou plusieurs substituants choisis indépendamment dans le groupe constitué par halogène, hydroxy, C₁-C₇-alcoxy, C₁-C₇-alcoxy-C₁-C₇-alcoxy, amino, N-mono- ou N,N-di-(C₁-C₇-alkyle, phényl-C₁-C₇-alkyle et/ou naphtyl-C₁-C₇-alkyl)-amino, carboxy, C₁-C₇-alcoxycarbonyle, phényl-C₁-C₇-alcoxycarbonyle, naphtyl-C₁-C₇-alcoxycarbonyle, phénoxycarbonyle, naphtoxycarbonyle, C₁-C₄-alcylène-dioxy, carbamoyle, N-mono- ou N,N-di-(C₁-C₇-alkyle, N',N'-di-(C₁-C₇-alkyl) amino-C₁-C₇-alkyle, pyrrolidino-C₁-C₇-alkyle et/ou phényl-C₁-C₇-alkyl)-carbamoyle, pipéridin-1-carbonyle, pipérazin-1-carbonyle, 4-C₁-C₇-alkyl-pipérazin-1-carbonyle, morpholin-4-carbonyle, thiomorpholin-4-carbonyl, S-oxo-thiomorpholin-4-carbonyle, S,S-dioxothiomorpholin-4-carbonyle, sulfamoyle, N-mono- ou N,N-di-(C₁-C₇-alkyle, N',N'-di-(C₁-C₇-alkyl) amino-C₁-C₇-alkyle, pyrrolidino-C₁-C₇-alkyle et/ou phényl-C₁-C₇-alkyl)-sulfamoyle, pyrazolyle, pyrazolidinyle, pyrrolyle, pyrrolidinyle, pipéridinyle, pipérazinyle, 4-C₁-C₇-alkyl-pipérazinyle, 4-(phényl-C₁-C₇-alkyl)-pipérazinyle, 4-(naphtyl-C₁-C₇-alkyl)-pipérazinyle, 4-(C₁-C₇-alcoxycarbonyl)pipérazinyle, 4-(phényl-C₁-C₇-alcoxycarbonyl) -pipérazinyle, 4 - (naphtyl-C₁-C₇-alcoxycarbonyl) -pipérazinyle, morpholinyle, thiomorpholinyle, S-oxothiomorpholinyle et S,S-dioxothiomorpholinyle, et/ou parmi 2-amino-pyrimidin-5-yl-C₁-C₇-alkyle, 4-C₁-C₇-alkyl-pipérazin-1-carbonyl-C₁-C₇-alcoxy, 4-pyrrolidino-pipéridin-1-carbonyl-C₁-C₇-alcoxy, 4-pyrrolidino-pipéridin-1-yl-C₁-C₇-alcoxy, 4-C₁-C₇-alkyl-pipérazino-C₁-C₇-alcoxy, pyridine (par exemple -2)-yloxy-C₁-C₇-alcoxy, pyrimidine (par exemple -4)-yloxy-C₁-C₇-alcoxy, N,N-di(C₁-C₇-alkyl)amino-pyrrolidini-1-carbonyle et (non substituée ou C₁-C₇-alcoxy- et/ou halogéno-C₁-C₇-alcoxy-substituée) pyridine (par exemple -3))-yle ; et
R⁵ est hydrogène ;
ou un tautomère de celui-ci ou un N-oxyde de celui-ci, ou un sel pharmaceutiquement acceptable, ou un hydrate on solvate de celui-ci.

5. Composé de formule I selon la revendication 1, dans laquelle
R¹ est hydrogène, amino, méthylamino, n-propylamino ou cyclopropylamino ;
R² est phényle, 3-méthoxyphényle, 4-méthoxyphényle, 3,4-diméthoxyphényle, 3,4,5-triméthoxyphényle, 3-chloro-4-n-propoxyphényle, 4-carboxy-3-méthoxyphényle, 4-méthoxycarbonyl-3-méthoxyphényle, 4-carbamoylphényle, 4-N-méthylcarbamoyl-3-méthoxyphényle, 4-(N,N-di-méthyl-carbamoyl)-3-méthoxyphényle, 4-(4-méthylpipérazin-1-carbonyl)-3-méthoxyphényle, 4-(4-morpholin-1-carbonyl)-3-méthoxyphényle, benzo[1,3]dioxol-5-yle, 2,3-dihydrobenzo[1,4]dioxin-6-yle, 4-(pipérazin-1-yl)-phényle, 4-sulfamoyl-phényle, 4-N,N-diméthyl-sulfamoylphényle, 4-pyrazolyl-phényle, pyrrolyle, pyrazolyle, thiophényle, 1,2,4-triazol-1-yle, 6-méthoxy-pyridin-3-yle, ou 6-pipérazino-pyridin-3-yle; R³ est hydrogène ;
R⁴ est 3-hydroxyphényle, 4-hydroxyphényle, 4-hydroxy-3-méthoxyphényle, 3-hydroxy-4-n-propoxyphényle, 3,4-diméthoxyphényle, 3,4,5-triméthoxyphényle, 4-éthoxy-3-méthoxyphényle, 3-(2-methoxyéthoxy)-4-méthoxyphényle, 3-méthoxy-4-(2-méthoxyéthoxy)-phényle, 3-fluoro-4-méthoxyphényle, 3-chloro-4-n-propoxyphényle, 4-méthoxycarbonyle-3-méthoxyphényle, 4-carbamoylphényle, N,N-diméthylaminosulfonylphényle, benso[1,3]dioxol-5-yle, 2,3-dihydrobenzo[1,4]dioxin-6-yle, pyridine-3-yle, 6-méthoxy-pyridin-3-yle, 5-méthoxy-pyridin-3-yle, 2-amino-pyridin-4-yle, 6-amino-pyridin-3-yle, 6-(pipérazin-1-yl)-pyridin-3-yle, 6-(4-tertio-butoxy-carbonyl-pipérazin-1-yl)-pyridin-3-yle, 2-(pipérazin-1-yl)-pyridin-4-yle ou 2-(4-tertio-butoxycarbonyl-pipérazin-1-yl)-pyridin-4-yle ; et
R⁵ est hydrogène ;
ou un tautomère de celui-ci ou un N-oxyde de celui-ci, ou un sel pharmaceutiquement acceptable, ou un hydrate ou solvat de celui-ci.

6. Composé de formule I selon la revendication 1, dans laquelle
R¹ est hydrogène, amino, méthylamino, n-propylamino ou cyclopropylamino ;
R² est phényle, 4-trifluorométhylphényle, 3-fluorophényle, 4-fluorophényle, 4-chlorophényle, 4-bromophényle, 3-méthoxyphényle, 4-méthoxyphényle 3-éthoxyphényle, 3,4-diméthoxyphényle, 4-éthoxy-3-méthoxyphényle, 3,4-diéthoxyphényle, 3-benzyloxy-4-méthoxyphényle, 4-(2-méthoxyéthoxy)-3-thoxyphényle, 4-trifluorométhoxyphényle, 4-méthoxy-3-trifluorométhoxyphényle, 4-(3-tertio-butoxycarbonylamino-propoxy)-3-méthoxyphényle, 4-(2-tertio-butoxycarbonyl-aminoéthoxy)-3-méthoxyphényle, 3,4,5-triméthoxyphényle, 3-chloro-4-n-propoxyphényle, 4-acétylaminophényle, 4-carboxy-3-méthoxyphényle, 4-méthoxycarbonyl-phényle , 4-méthoxycarbonyl-3-méthoxyphényle, 4-cyanophényle, 4-biphénylyle, 4'-amino-biphényl-4-yle, 4-méthoxybiphényl-4-yle, 4'-hydroxyméthyl-biphényl-4-yle, 4'-méthoxyméthyl-biphényl-4-yle, 3',4'-diméthoxy-biphényl-4-yle, 4'-carbamoyl-biphényl-4-yle, 4-carbamoylphényle, 4-N-méthylcarbamoyl-3-méthoxyphényle, 4-(N,N-diméthylcarbamoyl)-phényle, 4-(N-méthylcarbamoyl)-phényle, 4-(N,N-diméthylcarbamoyl)-3-méthoxyphenyle, 4-(4-méthylpipérazin-1-carbonyl) -3-méthoxyphényle, 4-(morpholin-4-carbonyl) -phényle, 4-(4-morpholin-1-carbonyl)-3-méthoxyphényle, benzo[1,3]-dioxol-5-yle, 2,3-dihydrobenzo[1,4]dioxin-6-yle, 4-(pipérazin-1-yl)-phényle, 4-(2-oxo-pyrrolidin-1-yl)-phényle, 4-(2-oxo-azétidin-1-yl)-phényle, 4-(2-oxo-pipéridin-1-yl)-phényle, 4-(3-méthyl-2-oxo-imidazolidin-1-yl)-phényle, 4-méthanesulfonyl-phényle, 4-sulfamoyl-phényle, 4-N,N-diméthylsulfamoylphényle, 4-pyrazolyl-phényle, pyrrolyle, pyrazolyle, thiophenyle, notamment thiophén-3-yle, 1,2,4-triazol-1-yle, 2-méthoxy-pyridin-4-yle, 5-méthoxy-pyridin-3-yle, 6-méthoxy-pyridin-3-yle, 6-pipérazino-pyridin-3-yle, 6-morpholin-4-yl-pyridin-3-yle, 1H-pyrrolo[2,3-b]pyridin-5-yle, 4-[6-(4-méthanesulfonyl)-pipérazin-1-yl]-pyridin-3-yle, 5-(4-acétylpipérazin-1-yl)-pyridin-3-yle ou 2-[4-(tertio-butoxycarbonyl)-pipérazin-1-yl]-pyridin-4-yle;
R³ est hydrogène ;
R⁴ est 3-hydroxyphényle, 4-hydroxyphényle, 4-hydroxy-3-méthoxyphényle, 3-hydroxy-4-n-propoxyphényle, 3-éthoxy-phényle, 3,4-diméthoxyphényle, 3,4-diéthoxyphényle, 3,4,5-triméthoxyphényle, 3-éthoxy-4-méthoxyphényle, 4-éthoxy-3-méthoxyphényle, 3-(2-méthoxyéthoxy)-4-méthoxyphényle, 3-méthoxy-4- (2-méthoxyéthoxy)phényle, 3 - benzyloxy-4-méthoxyphényle, 4-(3-aminopropoxy)-3-méthoxyphényle, 5-(3-aminopropoxy)-3-méthoxyphényle, 4-(2-aminoéthoxy)-3-méthoxyphényle, 5-(2-aminoethoxy)-3-méthoxyphényle, 3-fluoro-4-méthoxyphényle, 3-chloro,-4-méthoxyphényle, 3-chloro-4-n-propoxyphényle, 4-(3-tertio-butoxycarbonylaminopropoxy)-3-méthoxyphényle, 4-(2-tertio-butoxycarbonylaminoéthoxy)-3-methoxyphenyle 4-formyl-phényle, 4-méthoxycarbonyl-3-méthoxyphényle, 3-carbamoyl-phényle, 4-carbamoylphényle, 4-carbamoyl-3-méthoxyphényle, 3-sulfamoyl-phényle, N,N-diméthyl-aminosulfonylphényle, benzo [1,3] dioxol-5-yle, 2,3-dihydrobenzo[1,4]dioxin-6-yle, 6-aminométhyl-pyridin-3-yle, pyridine-3-yle, 6-méthoxy-pyridin-3-yle, 5-méthoxy-pyridin-3-yle, 2-methoxy-pyridin-4-yle, 2-amino-pyridin-4-yle, 6-amino-pyridin-3-yle, 6-amino-5-trifluorométhylpyridin-3-yle, 6-diméthylamino-pyridin-3-yle, 6-méthylamino-pyridin-3-yle, 6-isobutylamino-pyridin-3-yle, 6-(2-méthoxyéthylamino)-pyridin-3-yle, 6-(pipérazin-1-yl)-pyridin-3-yle, 6-(4-tertio-butoxy-carbonyl-pipérazin-1-yl)-pyridin-3-yle, 2-(pipérazin-1-yl)-pyridin-4-yle, 6-carbamoyl-pyridin-3-yle-, 2-cyano-pyridin-5-yle, 5-cyano-pyridin-3-yle, 6 (2-hydroxy-éthyl-amino)-pyridin-3-yle, 2-(4-tertio-butoxycarbonyl-pipérazin-1-yl)-pyridin-4-yle, 6-morpholin-4-yl-pyridin-3-yle, furan-2-yle, furan-3-yle, 1H-pyrrolo[2,3-b]pyridine-5-yle, ou quinoléin-3-yle ; et R⁵ est hydrogène;
ou un tautomère de celui-ci ou un N-oxyde de celui-ci, ou un sel pharmaceutiquement acceptable, ou un hydrate ou solvat de celui-ci.

7. Composé de formule I selon la revendication 1, choisi dans le groupe constitué par les composés suivants :
la 4-(3,4-diméthoxyphényl)-6-(6-pipérazin-1-yl-pyridin-3-yl)quinazoline,
l'ester tertio-butylique de l'acide 4-{5-[4-(3,4-diméthoxyphényl)quinazolin-6-yl]-pyridin-2-yl}pipérazine-1-carboxylique,
la [4,6-bis-(3,4-diméthoxyphényl)quinazolin-2-yl]-n-propyl-amine,
la 6-(6-méthoxy-pyridin-3-yl)-4-phényl-quinazoline,
le 3-[2-amino-4-(3,4-diméthoxyphényl)quinazolin-6-yl]phénol,
la 6-(3-chloro-4-n-propoxyphényl)-4-(3,4-diméthoxy-phényl)quinazoline,
le 4-[4-(3,4-diméthoxyphényl)quinazolin-6-yl]-2-méthoxy-phénol,
la 6-(2,3-dihydrobenzo[1,4]dioxin-6-yl)-4-(3,4-diméthoxyphényl) quinazoline,
la 6- (benzo[1,3]dioxol-5-yl)-4-(3,4-diméthoxyphényl) - quinazoline,
l'ester méthylique de l'acide 4-[4-(3,4-diméthoxy-phényl)quinazolin-6-yl]-2-méthoxybenzoïque,
la 4-(3,4-diméthoxyphényl)-6-(3,4,5-triméthoxy-phényl)quinazoline,
la 6-(3,4-diméthoxyphényl)-4-(3-fluoro-4-méthoxy-phényl)quinazoline,
l'ester méthylique de l'acide 4-[6-(3,4-diméthoxy-phényl)quinazolin-4-yl-2-méthoxybensoïque,
la 4-(3-chloro-4-n-propoxyphényl)-6-(3,4-diméthoxy-phényl)quinazoline,
la 6-(3,4-dimethoxyphényl)-4-(3,4,5-triméthoxyphényle)-quinazoline,
la 4-(2,3-dihydrobenzo[1,4]dioxin-6-yl)-6-(3,4-diméthoxyphényl)quinazoline,
la 4-(benzo[1,3]dioxol-5-yl)-6-(3,4-diméthoxyphényl)-quinazoline,
la 6-(6-pipérazin-1-yl-pyridin-3-yl)-4-(4-pyrazol-1-yl-phényl)quinazoline,
le 3-[4-(3,4-diméthoxyphényl)quinazolin-6-yl]phénol,
le 4-[4-(3,4-diméthoxyphényl)quinazolin-6-yl]phénol,
le 4-[4-(3,4-diméthoxyphényl)quinazolin-6-yl]-benzamide,
la 4-(3,4-diméthoxyphényl)-6-(3-fluoro-4-méthoxy-phényl)quinazoline,
la 6-(3,4-diméthoxyphényl)-4-phényl-quinazoline,
la 6-(3,4-diméthoxyphényl)-4-thiophén-2-yl-quinazoline,
la 6-(3,4-diméthoxyphényl)-4-(3-méthoxyphényl)-quinazoline,
la 6-(3,4-diméthoxyphényl)-4-(4-méthoxyphényl)-quinazoline,
la 6-(3,4-diméthoxyphényl)-4-(4-pyrazol-1-yl-phényl)-quinazoline,
la 6-(3,4-diméthoxyphényl)-4-(6-pipérazin-1-yl-pyridin-3-yl)quinazoline,
le 4-[6-(6-pipérazin-1-yl-pyridin-3-yl)-quinazolin-4-yl]-benzamide,
la 6-(6-methoxy-pyridin-3-yl)-4-(4-pyrazol-1-yl-phényl)quinazoline,
le 4-[6-(6-méthoxy-pyridin-3-yl)-quinazolin-4-yl]-benzamide,
la 4-(6-(6-méthoxy-pyridin-3-yl)-4-(6-quinazolin-1-yl-pyridin-3-yl)quinazoline,
la 4,6-bis(3,4-diméthoxyphényl) quinazoline,
le 4-(6-(3,4-diméthoxyphényl)-quinazolin-4-yl]-benzamide,
la 6-(3,4-diméthoxyphényl)-4-(6-méthoxy-pyridin-3-yl)-quinazoline,
la 4-(3,4-diméthoxyphényl)-6-(6-méthoxy-pyridin-3-yl)quinazoline,
la 4-(6-méthoxy-pyridin-3yl)-6-(6-pipérazin-1-yl-pyridin-3-yl)quinzoline,
la 6-(6-méthoxy-pyridin-3-yl)-4-thiophen-2-yl-quinazoline,
la 4-(2-chlorophényl)-6-(6-méthoxy-pyridin-3-yl)quinazoline,
la 4,6-bis(6-méthoxy-pyridin-3-yl)quinazoline,
la 4-(4-méthoxyphényl)-6-(6-méthoxy-pyridin-3-yl)quinazoline,
la 4-(3,4-diméthoxyphényl)-6-(4-éthoxy-3-méthoxy-phenyl)quinazoline,
la [4,6-bis-(3,4-diméthoxyphényl)quinazolin-2-yl]-cyclopropyl-amine,
le 4-[2-amino-6-(3,4-diméthoxyphényl)quinazolin-4-yl]-benzamide,
la 4-(3,4-diméthoxyphényl)-6-(3-fluoro-4-méthoxy-phényl)quinazolin-2-ylamine,
l'ester méthylique de l'acide 4-[2-amino-4-(3,4-diméthoxyphényl)quinazolin-6-yl]-2-méthoxybenzoïque,
la 4-(3,4-diméthoxyphényl)-6-(3,4,5-trimétboxyphényl)-quinazolin-2-ylamine,
la 6-(3-chloro-4-n-propoxyphényl)-4-(3,4-diméthoxy-phényl)quinazolin-2-ylamine,
le 4-[2-amino-4-(3,4-diméthoxyphényl)quinazolin-6-yl]phénol,
la 6-(2,3-dihydrobenzo[1,4]dioxin-6-yl)-4-(3,4-diméthoxyphényl)quinazolin-2-ylamine,
la 4-(benzo[1,3]dioxol-5-yl)-6-(3,4-diméthoxyphényl)-, quinazolin-2-ylamine,
la 6-(3,4-diméthoxyphényl)-4-(3,4,5-triméthoxyphényl)-quinazolin-2-ylamine,
la 6-(3,4-diméthoxyphényl)-4-(6-méthoxy-pyridin-3-yl)quinazolin-2-ylamine,
la 4,6-bis(3,4-diméthoxyphényl)-quinazolin-2-ylamine,
la 4,6-bis(6-méthoxy-pyridin-3-yl)quinazolin-2-ylamine,
la 4-(3,4-diméthoxyphényl)-6-(6-méthoxy-pyridin-3-yl)-quinazolin-2-ylamine,
la N-[4,6-bis-(3,4-diméthoxyphényl)quinazolin-2-yl]-N-méthyl-amine,
l'acide 4-[6-(3,4-diméthoxyphényl)quinazolin-4-yl]-2-méthoxybensoïque,
l'acide 4-[6-(3,4-diméthoxyphényl)quinazolin-4-yl]-2-méthoxybensoïque N-méthylamide,
le 4-[6-(3,4-diméthoxyphényl)quinazolin-4-yl]-2-méthoxybenzamide,
le 4-[6-(3,4-diméthoxyphényl)quinazolin-4-yl]-2-méthoxy-N,N-diméthylamide,
la {4-[6-(3,4-diméthoxyphényl)quinazolin-4-yl]-2-méthoxyphényl}-(4-méthyl-pipérazin-1-yl)méthanone,
la {4-[6-(3,4-diméthoxyphényl)quinazolin-4-yl]-2-méthoxyphényl}-morpholin-4-yl-méthanone,
la 4-(1,2,4-triazol-1-yl)-6-(3,4-diméthoxyphényl)-quinazoline,
la 4-(3,4-diméthoxyphényl)-6-[3-méthoxy-4-(2-méthoxyéthoxy)phényl]quinazoline,
la 5-[4-(3,4-diméthoxyphényl)quinazolin-6-yl]-pyridine 2-ylamine,
le 4-[6-(3,4-diméthoxyphényl) quinazolin-4-yl]-N,N-diméthylbenzènesulfonamide,
la 6-(3,4-diméthoxyphényl)-4-pyrazol-1-yl-quinazoline,
la 6-(3,4-diméthoxyphényl)-4-[1,2,4]triazol-1-yl-quinazoline, et
la 6-(3,4-diméthoxyphényl)-4-pyrrol-1-yl-quinazoline ;
ou dans chaque cas un Ni-oxyde de celui-ci, un tautomère de celui-ci et/ou un sel pharmaceutiquement acceptable de celui-ci.

8. Composé de formule I selon la revendication 1, dans laquelle
R¹ est hydrogène, amine, méthylamino, n-propylamino ou cyclopropylamino ;
R² est phényle, 4-(2-aminopyrimidin-5-ylméthyl)-phényle, 3-(2-méthoxy-6-trifluorométhyl)pyridin-3-yl-phényle, 3-méthoxyphényle, 4-méthoxyphényle, 3,4-diméthoxyphényle, 3,4,5-triméthoxylphényle, 3-chloro-4-n-propoxyphényle, 4-carboxy-3-méthoxyphényle, 4-(2-pyridin-2-yloxy-éthoxylphényle, (4-pyrimidin-4-yloxyéthoxy)phényle, 4-méthoxycarbonyl-3-méthoxyphényle, 4-carbamoylphényle, 4-N-méthylcarbamoyl-3-méthoxyphényle, 4-(N,N-diméthyl-carbamoyl)-3-méthoxyphényle, 4-4-méthylpipérazin-1-carbonyl)-3-méthoxyphényle, 4-(4-morpholin-1-carbonyl)-3-méthhoxyphényle, benzo[1,3]dioxol-5-yle, 2,3-dihydrobenzo[1,4]dioxin-6-yle, 4-[2-(4-pyrrolidino-pipéridin-1-yl)-éthoxy]-phényle, 4-(pipérazin-1-yl)-phényle, 4-[2-(4-méthyl-pipérazin-1-yl)-éthoy]-phényle, 4-[2-(4-éthyl-pipérazin-1-yl)-éthoxy]-phényle, 4-(4-méthyl-pipérazin-1-carbonylméthoxy)phényle, 4-(4-éthyl-pipérazin-1-carbonylméthoxy)phényle, 4-(4-pyrrolidino-pipéridin-1-carbonylméthoxy)phényle, 4-[N-(2-diméthylamino-éthyl)-N-méthylcarbamoyl]-phényle, 4-[(R,S ou R,S)-3-diéthylamino-pyrrolidin-1-carbonyl)-phényle, 1 4-sulfamoylphényle, 4-'N,N"diméthyl" sulfamoylphényle, 4-[N-méthyl-N,-2-(pyrrolidino-éthyl)-sulfamoyl]phényle, 4-pyrazolyl-phényle, pyrrolyle, pyrazolyle, thiophényle, 1,2, 4-triazol-1-yle, 6-méthoxy--pyridin-3-yle ou 6-pipérazino-pyridin-3-yle ;
R³ est hydrogène ;
R⁴ est 4-(2-amino-pyrimidin-5-ylméthyl)-phényle, 3-(2-méthoxy-6-trifluorométhyl)pyridin-3-yl-phényle, 3-hydroxyphényle, 4-hydroxyphényle, 4-hydroxy-3-méthoxyphényle, 3-hydroxy-4-n-propoxyphényle, 3,4-diméthoxyphényle, 3,4,5-triméthoxyphenyle, 4-éthoxy-3-méthoxyphényle, 3-(2-méthoxyéthoxy)-4-méthoxyphényle, 3-'méthoxy-4- (2-méthoxyéthoxy)-phényle, 3-fluoro-4-méthoxyphenyle, 3-chloro-4-n-propoxyphényle, 4-(2-pyridin-2-yloxyéthoxy)-phényle, 4-(2-pyrimidin-4-yloxyéthoxy)-phényle, 4-[2-(4-pyrrolidin-pipéridin-1-yl) -éthoxy]-phényle, 4-[2-(4-méthyl-pipérazin-1-yl)-éthoxy]-phényle, 4-[2-(4-éthyl-pipérazin-1-yl)-éthoxy]-phényle, 4-(4-méthyl-pipérazin-1-carbonylméthoxy)-phényle, 4-(4-éthyl-pipérazin-1-carbonylméthoxy)-phényle, 4-(4-pyrrolidino-pipéridin-1-carbonylméthoxy)-phényle, 4-[N-(2,-diméthylamino-éthyl)-N-méthyl-carbamoyl]-phényle, 4-[(R,S ou R,S)-3-diéthylamino-pyrrolidin-1-carbonyl)phényle, 4-méthoxycarbonyl-3-méthoxyphényle, 4-carbamoylphényle, N-diméthyl-aminosulfonylphényle, 4-[N-méthyl-N-2-(pyrrolidino-éthyl)-sulfamoyl]phényle, benzo[1,3]dioxol-5-yl-3, 2,3-dihydrobenzo[1,4]dioxin-6-yle, pyridine-3-yle, 6-méthoxy-pyridin-3-yle, 5-méthoxy-pyridin-3-yle, 2-amino-pyridin-4-yle, 6-amino-pyridin-3-yl, 6-(pipérazin-1-yl)-pyridin-3-yle, 6-(4-tertio-butoxycarbonyl-pipérazin-1-yl) -pyridin-3-yle, 2-(pipérazin-1-yl)-pyridin-4-yle ou 2-(4-tertio-butoxycarbonyl-pipérazin-1-yl)-pyridin-4-yle : et R⁵ est hydrogène
ou un tautomère de celui-ci ou un N-oxyde de celui-ci, ou un seul pharmaceutiquement acceptable, ou un hydrate ou solvant de celui-ci.

9. Composé de formule 1 salon à revendication 1, choisi dans le groupe constitué par les composés suivants :
la 6-(3,4-diméthoxyphényl)-4-(4-éthoxy-3-méthoxy-phényl)quinazoline ;
la 6-(3,4-diméthoxyphenyl)-4-[3-méthoxy-4-(2-methoxy-éthoxy)phényl]quinazoline ;
la 1-(3,4-diméthoxyphényl)-6-(2-méthoxypyridin-4-yl)-quinazoline ;
la 6-(3,4-dimethoxyphényl)-4-(2-méthoxypyridin-4-yl)-quinazoline ;
l'ester tertio-butylique de l'acide (3-{4-[4-(3,4-diméthoxyphenyl)quinazolin-6-yl]-2-methoxyphénoxy}-propyl)carbamique ;
l'ester tertio-butylique de l'acide (3-{4-[6-(3,4-diméthoxyphényl)quinazolin-4-yl]-2-méthoxyphénoxy}-propyl)carbamique;
l'ester tertio-butylique de l'acide (2-{4-[4-(3,4-diméthoxyphényl)quinazolin-6-yl]-2-méthoxyphénoxy}-ethyl)carbamique ;
l'ester tertio-butylique de l'acide (2-{4-[6-(3,4-diméthoxyphénl)quinazolin-4-yl]-2-méthoxyphénoxoy}-éthyl)carbamique ;
la 6-(3,4-diméthoxyphényl)-4-(3-éthoxyphényl) quinazoline ;
la 4-(3-chlorophényl)-6-(3,4-diméthoxyphényl)-quinazoline ;
la 4-(3-benzyloxy-4-méthoxyphényl)-6-(3,4-diméthoxy)-phényl)quinazoline ;
l'ester méthylique de l'acide 4-[6-(3,4-diméthoxyphényl)quinazolin-4-yl]benzoïque ;
la 4-(3,4-diméthoxyphényl)-6-(5-méthoxypyridin-3-yl)-quinasoline ;
l'ester méthylique de l'acide 4-[4-(3,4-diméthoxyphényl)quinazoli-6-yl]-2-méthoxybenzoïque ;
la 4-(3,4-diméthoxyphényl)-6-quinoléin-3-yl-quinazoline ;
le 4-[6-(5-méthoxypyridin-3-yl)quinazolin-4-yl]-benzamide ;
le 4-[6-(6-amino-5-trifluorométhyl-pyridin-3-yl)-quinazolin-4-yl]-henzamide ;
la 5-[4-(3,4-diméthoxyphényl)quinazolin-6-yl]-3-trifluorométhyl-pyridin-2-ylamine
le 3-[4-(3,4-diméthoxyphényl)quinazolin-6-yl]-benzénesulfonamide ;
la 4-benzo[1,3]dioxol-5-yl-6-(3-fluoro-4-méthoxy-phényl)quinazoline ;
la 4-(3-benzyloxy-4-methoxyphényl)-6-(3,4-diméthoxyphényl)quinazoline ;
le 3-[4-(3,4-diméthoxyphényl)quinazolin-6-yl]-benzamide ;
la 4-(4-chlorophényl)-6-(3,4-diméthoxyphényl)-quinazoline ;
la 6-(3,4-diméthoxyphényl)-4-(4-trifluorométhyl-phényl)quinazoline ;
la 6-(3-chloro-4-méthoxyphényl)-4-(3,4-diméthoxy-phenyl)quinazoline ;
la 6-(3,4-diméthoxyphényl)-4-thiophén-3-yl-quinazoline ;
la 4-(3,4-diméthoxyphényl)-6-(1H-pyrrolo[2,3-b]pyridin-5-yl)quinazoline ;
le 4-[6-(1H-pyrrolo[2,3-b]pyridin-5-yl)quinazolin-4-yl]-benzamide ;
le 4-[6-(6-amino-pyridin-3-yl)quinazolin-4-yl]-benzamide ;
la 6-(3-benzyloxy-4-méthoxyphényl)-4-(3,4-diméthoxy-phényl)quinazoline ;
la 4-(4-chlorophényl)-6-(3-fluoro-4-méthoxyphényl)-quinazoline ;
le 4-[6-(4-éthoxy-3-méthoxyphényl)quinazolin-1-yl]-benzamide ;
le 4-[6-(3,4-diéthoxyphényl)quinazolin-4-yl]benzamide ;
la 4-(3,4-diméthoxyphényl)-6-furan-3-yl-quinazoline ;
le 4-[6-(3,4-diméthoxyphényl)quinazolin-4-yl]-benzonitrile ;
le 4-[6-(6-aminopyridin-3-yl)quinazolin-4-yl]-benzonitrile ;
le 4-[4-(3,4-diméthoxyphenyl)quinazolin-6-yl]-benzaldehyde ;
la 4-biphényl-4-yl-6-(3,4-diméthoxyphényl)quinazoline ;
la 4-(3,4-diéthoxyphényl)-6-(3,4-diméthoxyphényl)-quinazoline ;
la 6-(3,4-diméthoxyphényl)-4-(4-méthoxy-3-trifluoro-méthoxyphényl)quinazoline;
la 4-(3,4-diméthoxyphényl)-6-(4-méthoxy-3-trifluoro-méthoxyphényl)quinazoline ;
le 5-[4-(3,4-diméthoxyphényl)quinazolin-6-yl]-pyridine-2-carbonitrile;
la 4-(4-bromophenyl)-6-(3,4-diméthoxyphényl) quinazoline ;
la 6-(3,4-diéthoxy-phényl)-4-(3,4-diméthoxyphényl) - quinazoline ;
la 6-(3,4-diméthoxyphényl)-4-(4-trifluorométhoxy-phényl)quinazoline ;
la 6-(3,4-diméthoxyphényl)-4-(4-fluorophényl)-quinazoline ;
la 6-(3,4-diméthoxyphényl)-4-(3-fluorophényl)-quinazoline ;
la 6-(3,4-diméthoxyphényl)-4-(4-méthanesulfonylphényl)-quinazoline ;
le N-{4-[6-(3,4-diméthoxyphényl)quinazolin-4-yl]-phényl}acétamide ;
le 5-[4-(4-diméthoxyphényl)quinazolin-6-yl]-nicotinonitrile ;
la {5-[4-(3,4-diméthoxyphényl)quinazolin-6-yl]-pyridin-2-yl}-isobutylamine ;
la 6-(3,4-diméthoxyphényl)-4-(6-morpholin-4-yl-pyridin-3-yl)quinazoline ;
la {5-[4-(3,4-diméthoxyphenyl)quinazolin-6-yl]-6-yl 2-yl}-diméthylamine ;
la {5-[4-(3,4-diméthoxyphényl)quinazolin-6-yl]-pyridin-2-yl}-méthylamine ;
le 2-{5-[4-(3,4-diméthoxyphényl)quinazolin-6-yl]-pyridin-2-ylamino}éthanol ;
l'ester tertio-butylique de l'acide 4-{5-[6-(3,4-diméthoxyphényl)quinazolin-4-yl]-pyridin-2-yl}-pipérazine-1-carboxylique ;
la 4-(2,3-dihydrobenzo[1,4]dioxin-6-yl)-6-(3,4-diméthoxyphényl)-quinazolin-2-ylamine ;
la 6-(6-amino-pyridin-3-yl)-4-(3,4-diméthoxyphényl)-quinazolin-2-ylamine ;
la 4-(3,4-diméthoxyphényl)-6-quinoléin-3-yl-quinazolin-2-ylamine ;
le 4-[6-(3,4-diméthoxyphényl)-quinazolin-4-yl]-N,N-diméthylbenzamide ;
le 4-[6-(3,4-diméthoxyphényl)quinazolin-4-yl]-N-méthyl-benzamide ;
la {4-[6-(3,4-diméthoxyphényl)quinazolin-4-yl]-phényl}-(4-méthylpipérazin-1-yl)méthanone :
la {4-[6-(3,4-diméthoxyphényl)quinazolin-4-yl)-phényl}-morpholin-4-yl-méthanone ;
la C-{4'[6-(3,4'-diméthoxyphényl)quinazolin-4-yl]-biphényl-4-yl}méthylamine ;
la 6-(3,4-diméthoxyphényl)-4-(4-méthoxybiphényl-4-yl)quinazoline ;
le {4'-[6-(3,4-diméthoxyphényl)quinazolin-4-yl]-biphényl-4-yl}méthanol ;
le 4'-[6-(3,4-diméthoxyphényl)quinazolin-4-yl]-biphényl-4-o1 ;
la 4-(3',4'-diméthoxybiphényl-4-yl)-6-(3,4-diméthoxy-phényl)quinazoline ;
la 6-(3,4-diméthoxyphényl)-4-(3,4',5'-triméthoxy-biphényl-4-yl)quinazoline;
l'acide 4'-[6-(3,4-diméthoxyphényl)quinazolin-4-yl]-biphenyl-4-carboxylique amide ;
la 6-(3,4-diméthoxyphényl)-4-(4'-méthoxyméthyl-biphényl-4-yl)quinazoline ;
la 3-{4-[4-(3,4-diméthoxyphényl)quinazolin-6-yl]-2-méthoxyphénoxy}propylamine ;
la 2-{4-[4-(3,4-diméthoxyphényl)quinazolin-6-yl]-2-méthoxyphénoxy}éthylamine ;
la 3-{5-[4'-(3,4-diméthoxyphényl)quinazolin-6-yl]-2-méthoxyphénoxy}propylamine ;
la 2-{5-[4-{3,4-diméthoxyphényl}quinazolin-6-yl]-2-méthoxyphénoxy}éthylamine ;
la 4-(3,4-diméthoxyphényl)-6-(3-éthoxy-4-méthoxyphényl)quinazoline ;
le 4-[4-(3,4-diméthoxyphényl)quinazolin-6-yl]-2-méthoxybenzamide ;
l'acide 4-[4-(3,4-diméthoxyphényl)quinazolin-6-yl]-2-méthoxybenzoïque ;
l'acide 5-[4-(3,4-diméthoxyphényl)quinazolin-6-yl]-pyridine-2-carboxylique amide ;
la C-{5-[4-(3,4-diméthoxyphényl)quinazolin-6-yl]-pyridin-2-yl}méthylamine ;
la 6-(3,4-diméthoxyphényl)-4-[6-(4-méthanesulfonyl-pipérazin-1-yl)pyridin-3-yl]quinazoline ;
la 1-(4-{5-[6-(3,4--diméthoxyphényl)quinazolin-4-yl]-pyridin-2-yl}pipérazin-1-yl)éthanone ;
la 1-{4-[6-(3,4-diméthoxyphényl)quinazolin-4-yl]-phényl}pyrrolidin-2-one ;
la 1-{4-[6-(3,4-diméthoxyphényl)quinazolin-4-yl]-phenyl}azétidin-2-one ;
la 1-{4-[6-(3,4-diméthoxyphényl)quinazolin-4-yl]-phényl}pipéridin-2-one ;
la 3-{4-[6-(3,4-diméthoxyphényl)quinazolin-4-yl]-phényl}oxazolidin-2-one;
la 1-{4-[6-(3,4-diméthoxyphényl)quinazolin-4-yl]phényl}-3-méthyl-imidazolidin-2-one ;
la 4,6-bis-(3,4-dimethoxyphényl)-5-fluoro-quinazoline ;
le 4-[6-(3,4-diméthoxyphényl)-5-fluoro-quinazoln-4-yl]-benzamide ;
la (5-[4-(3,4-diméthoxyphényl)quinazolin-6-yl]-pyridin-2-yl)-(2-méthoxyéthyl) amine ; et
la 4-(3,4-diméthoxyphényl)-6-(4,4,5,5-tétraméthyl-[1,3,2]dioxaborolan-2-yl)quinazoline
ou un tautomère de celui-ci ou un N-oxyde de celui-ci,
ou un sel pharmaceutiquement acceptable, ou un hydrate ou solvat de celui-ci,

10. Composé de formule 1, un N-oxyde de celui-ci, un tautomère de celui-ci et/ou un sel pharmaceutiquement acceptable de celui-ci, selon l'une quelconque des revendications 1 à 9, pour une utilisation dans le traitement, y compris le traitement prophylactique, d'un animal à sang chaud, notamment un être humain.

11. composé de formule I, un N-oxyde de celui-ci, un tautomère de celui-ci et/ou un sel pharmaceutiquement acceptable de celui-ci, selon la revendication 10, ou l'utilisation est contre une ou plusieurs maladies choisies dans le groupe constitué par les maladies prolifératives, inflammatoires, les maladies allergiques, les maladies obstructives des voies respiratoires, et les troubles se produisant couramment dans le cadre des greffes, notamment une ou plusieurs maladies qui répondent à l'inhibition de kinases de la famille des protéines kinases apparentées à la P13 kinase, notamment de l'activité des lipides kinases et/ou de la PI3 kinase (PI3K) et/ou de la mTOR et/ou de l'ADN protéine kinase et/ou de l'ATM et/ou de l'ATR et/ou de l'hSMG-1.

12. Préparation pharmaceutique, comprenant un composé de formule I, un N-oxyde de celui-ci, un tautomère de celui-ci et/ou in seul pharmaceutiquement acceptable de celui-ci, selon l'une quelconque des revendications 1 à 9, et au moins un véhicule pharmaceutiquement acceptable.

13. Méthode ou procédé de fabrication d'une préparation pharmaceutique, comprenant le mélange d'un composé de formule I, d'un N-oxyde de celui-ci, d'un tautomère de celui-ci et/ou d'un sel pharmaceutiquement acceptable de celui-ci, selon l'une quelconque des revendications 1 à 9, avec au moins un matériau véhicule pharmaceutiquement acceptable.

14. Procédé de fabrication d'un composé de formule T selon l'une quelconque des revendications 1 à 9, comprenant
a) pour la fabrication d'un composé de formule I dans laquelle R⁴ est lié au motif de quinazoline central dans la formule I par l'intermédiaire d'un atome de carbone, la réaction d'un composé de formule IIA, 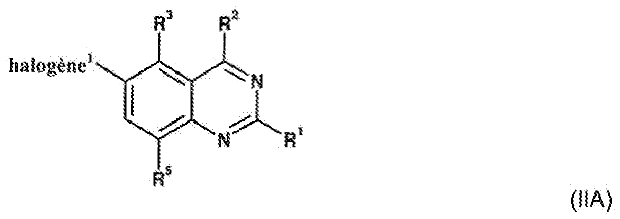 dans laquelle R¹, R², R³ est R⁵ sont tels que définis pour un composé de formule 1 et dans laquelle halogène¹ est halogène, préférablement chloro, bromo ou iodo, ou est trifluorométhanesulfonyloxy, dans des conditions de couplage croisé avec un acide boronique ou un ester de l'acide boroniqu-e de formule III,
R⁴-D (III)
dans laquelle R⁴ est tel que défini pour un composé de formule I et est Lié par l'intermédiaire d'un atome de carbone à D et D est B(OH₂) ou un groupement de formule A, 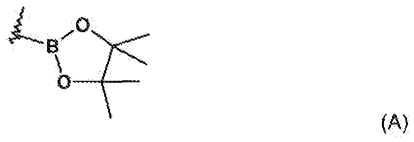 ou
b) pour la fabrication d'un composé de formule T dans laquelle R² est lié au motif de quinazoline central dans la formule 1 par l'intermédiaire d'un atome de carbone, la réaction d'un composé de formule IIB, 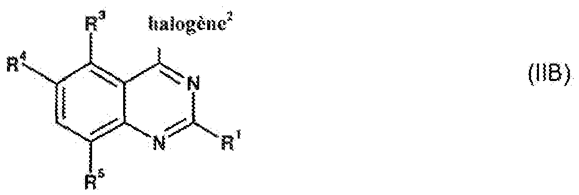 dans laquelle R¹, R³, R⁴ et R⁵ sont tels que définis pour un composé de formule I et halogène² est halogène, préférablement chloro, bromo ou iodo, ou est trifluorométhanesulfonyloxy, dans des conditions de couplage croisé avec un acide boronique ou un ester de l'acide boronique de formule IV,
R²-D (IV)
dans laquelle R² est tel que défini pour un composé de formule I et est lié par l'intermédiaire d'un atome de carbone à D et D est -B(OH₂) ou un groupement de formule A donnée ci-dessus ;
ou
c) pour la fabrication d'un composé de formule I dans laquelle R² et R⁴ sont identiques et sont liés au motif de quinazoline central dans la formule I par l'intermédiaire d'un atome de carbone, la réaction d'un composé de formule IIC, 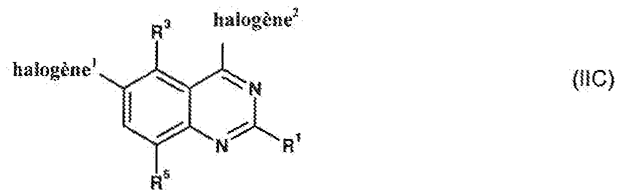 dans laquelle R¹, R³ et R⁵ sont tels que définis pour un composé de formule I et halogène¹ et halogène² sont, indépendamment l'un de l'autre, halogèno, préférablement chloro, bromo ou iodo, ou est trifluorométhanesulfonyloxy, avec un acide boronique ou un ester de l'acide boronique de formule V,
R^{2,4}-D (V)
dans laquelle R^{2,4} est un motif R² ou R⁴ lié par l'intermédiaire d'un atome de carbone à D et est par ailleurs tel que défini pour un composé de formule I et D est -B(OH₂) ou un groupement de formule A donnée ci-dessus ;
ou
d) pour la fabrication d'un composé de formule 1 dans laquelle R¹ est amino, N-mono-C₁-C₁₀-alkylamino ou N-mono-C₃-C₁₀-cycloalkylamino, la réaction d'un composé de formule IID, 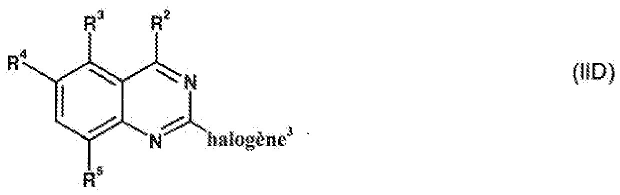 dans laquelle R², R³, R⁴ et R⁵ sont tels que définis pour un compose de formule I et dans laquelle halogène³ est halogéno, préférablement chlore, bromo ou iodo, ou est trifluorométhanesulfonyloxy, avec une aminé de formule VI
R^{1*}-H (VI)
dans laquelle R^{1*} est amino, N-mono-C₁-C₁₀-alkylamino ou N-mono-C₃-C₁₀-cycloalkylamino ;
ou
e) pour la fabrication d'un composé de formule I dans laquelle R⁴ est hétéroaryle ayant au moins un azote de cycle et est lié au motif de quinazoline central dans la formule I par l'intermédiaire d'un atome d'azote, la réaction d'un composé de formule IIA donnée ci-dessus dans a) avec un composé de formule VII,
R^{4*}-H (VII)
dans laquelle R^{4*} est un hétéroaryle azoté ayant au moins un azote de cycle et est lié à l'hydrogène dans la formule VII par l'intermédiaire d'un atome d'azote, dans des conditions de substitution ;
ou
f) pour la fabrication d'un composé de formule I dans laquelle R² est hétéroaryle ayant au moins un azote de cycle et est lié au motif de quinazoline central dans la formule I par l'intermédiaire d'un atome d'azote, la réaction d'un composé de formule IIB donnée ci-dessus dans b) avec un composé de formule VIII,
R^{2*}-H (VIII)
dans laquelle R^{6*} est un hétéroaryle azoté ayant au moins un azote de cycle et est lié à l'hydrogène dans la formule VIII par l'intermédiaire d'un atome d'azote, dans des conditions de substitution ;
ou
g) pour la fabrication d'un composé de formule I dans laquelle R² et R⁴ sont identiques et sont hétéroaryle ayant au moins un azote de cycle et chacun d'entre eux est lié au motif de quinazoline central dans la formule I par l'intermédiaire d'un atome d'azote, la réaction d'un composé de formule IX,
4^{2,4*}-H (IX)
dans laquelle R^{2,4*} est hétéroaryle ayant au moins un atome d'azote et dans laquelle R^{2,4*} est un motif R² ou R⁴ lié par l'intermédiaire d'un atome d'azote à l'hydrogène illustré dans la formule IX et est par ailleurs tel que défini pour un composé de formule I, dans des conditions de substitution avec un composé de formule IIC mentionné ci-dessus ;
ou
h) pour la fabrication d'un composé de formule 1 dans laquelle R⁴ est lié au motif de quinazoline central dans la formule I par l'intermédiaire d'un atome de carbone, la réaction d'un composé d'acide boronique ou d'ester de l'acide boronique de formule IIA*, 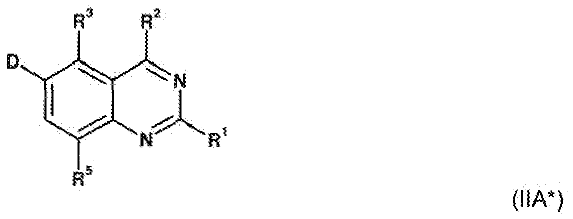 dans laquelle R¹, R², R³ et R⁵ sont tels que définis pour un composé de formule I et dans laquelle D est -B(OH₂) ou un groupement de formule A, 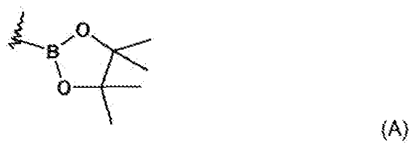 dans des conditions de couplage croisé avec un composé de formule III*,
R⁴-Hal (III*)
dans laquelle R⁴ est tel que défini pour un composé de formule I et est lié par l'intermédiaire d'un atome de carbone à Hal et Hal est halogène, préférablement chloro, bromo ou iodo, ou est trifluorométhanesulfonyloxy,
où dans l'une quelconque des réactions représentées dans a) à h), les groupements fonctionnels dans les produits de départ peuvent être présents sous forme protégée est dans les composés obtenables de formule I portant un ou plusieurs groupements protecteurs, de tels groupements protecteurs sont éliminées ;
et, si on le souhaite, un composé de formule I obtenable selon une variante de procédé choisie parmi a) à g) est converti en un composé différent de formule I, un sel obtenable d'un composé de formule I est converti en un sel différent de celui-ci, un compose libre obtenable de formule I est converti en un sel de celui-ci, et/ou un isomère obtenable d'un composé de formule 1 est séparé d'un ou plusieurs isomères obtenables différents de formule I.

15. Utilisation d'un composé de formule I, d'un N-oxyde de celui-ci, d'un tautomère de celui-ci et/ou d'un sel pharmaceutiquement acceptable de celui-ci, selon l'une quelconque des revendications 1 à 9, pour la préparation d'une préparation pharmaceutique destinée au traitement d'une maladie choisie dans le groupe constitué par les maladies prolifératives, inflammatoires, les maladies allergiques, les maladies obstructives des voies respiratoires, et les troubles se produisant couramment dans le cadre des greffes, notamment une ou plusieurs maladies qui répondent à l'inhibition de kinases de la famille des protéines kinases apparentées à la PI3 kinase, notamment de l'activité des lipides kinases et/ou de la PI3 kinase (PI3K) et/ou de la mTOR et/ou de l'ADN protéine kinase et/ou de l'ATM et/ou de l'ATR et/ou de l'hSMG-1.

16. Combinaison d'un composé de formule I selon l'une quelconque des revendications 1 à 9 avec un ou plusieurs autres agents thérapeutiques.
